# EUROPÄISCHE PATENTSCHRIFT

(11) **EP 0 960 095 B1**
(45) Veröffentlichungstag und Bekanntmachung des Hinweises auf die Patenterteilung: **01.10.2003**
(21) Anmeldenummer: 97954746.0
(22) Anmeldetag: 19.12.1997
(51) Int. Cl.: C07C 251/60, C07C 251/86, A01N 33/24, A01N 33/26

(54) **3-AMINOCARBONYL/3-AMINOTHIOCARBONYL-SUBSTITUIERTE 2-BENZOYL- CYCLOHEXAN-1,3-DIONE MIT HERBIZIDER WIRKUNG**
3-AMINOCARBONYL/3-AMINOTHIOCARBONYL-SUBSTITUTED 2-BENZOYL- CYCLOHEXAN-1,3-DIONES WITH HERBICIDAL EFFECT
2-BENZOYL-CYCLOHEXANE-1,3-DIONES 3-AMINOCARBONYL / 3-AMINOTHIOCARBONYL-SUBSTITUEES A ACTION HERBICIDE

(30) Priorität: 03.01.1997 DE 19700097
(43) Veröffentlichungstag der Anmeldung: 01.12.1999
(73) Patentinhaber: BASF AKTIENGESELLSCHAFT, 67056 Ludwigshafen (DE)
(72) Erfinder: KARDORFF, Uwe, D-68159 Mannheim (DE); HILL, Regina, Luise, D-67346 Speyer (DE); RACK, Michael, D-69123 Heidelberg (DE); VON DEYN, Wolfgang, D-67435 Neustadt (DE); ENGEL, Stefan, D-65510 Idstein (DE); OTTEN, Martina, D-67069 Ludwigshafen (DE); WITSCHEL, Matthias, D-67061 Ludwigshafen (DE); BAUMANN, Ernst, D-67373 Dudenhofen (DE); RHEINHEIMER, Joachim, D-67063 Ludwigshafen (DE); MAYER, Guido, D-67433 Neustadt (DE); MISSLITZ, Ulf, D-67433 Neustadt (DE); WESTPHALEN, Karl-Otto, D-67346 Speyer (DE); WALTER, Helmut, D-67283 Obrigheim (DE)
(86) Internationale Anmeldenummer: EP9707211
(87) Internationale Veröffentlichungsnummer: WO98029383

(56) Entgegenhaltungen:
- EP-A- 0 137 963
- WO-A-96/26200
- WO-A-97/46530
- DATABASE CROSSFIRE beilstein RN = , XP002063835 & ,

## Beschreibung

Die vorliegende Erfindung betrifft 2-Benzoyl-cyclohexan-1,3-dione der Formel I 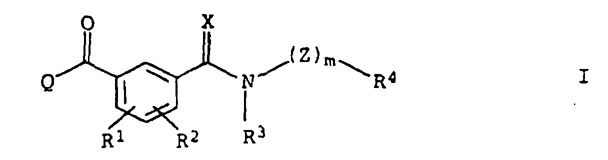 in der die Variablen folgende Bedeutung haben:
- R¹, R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵, -OCOR⁶, -OSO₂R⁶, -SH, -S(O)ₙR⁷, -SO₂OR⁵, -SO₂NR⁵R⁸, -NR⁸SO₂R⁶ oder -NR⁸COR⁶;
- R³: Wasserstoff, C₁-C₆-Alkyl, C₁-C₆-Halogenalkyl, C₃-C₆-Alkenyl oder C₃-C₆-Alkinyl;
- R⁴: Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei folgenden Gruppen tragen können:
Hydroxy, Mercapto, Amino, Cyano, R¹⁰, -OR¹⁰, -SR¹⁰, - NR⁸R¹⁰, =NOR¹⁰, -OCOR¹⁰, -SCOR¹⁰, -NR⁸COR¹⁰, -CO₂R¹⁰, - COSR¹⁰, CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Phenyl-C₁-C₄-alkyl, Hetaryl, Phenoxy, Phenyl-C₁-C₄-alkoxy und Hetaryloxy, wobei die acht letztgenannten Reste ihrerseits substituiert sein können;
- X: Sauerstoff oder Schwefel;
- Z: Sauerstoff oder NR⁸;
- m: 0 oder 1;
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁶: C₁-C₆-Alkyl oder C₁-C₆-Halogenalkyl;
- R⁷: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁸: Wasserstoff oder C₁-C₆-Alkyl;
- R⁹: C₁-C₆-Alkyl, C₃-C₆-Alkenyl, C₃-C₆-Alkinyl, Phenyl oder Benzyl;
- R¹⁰: C₁-C₆-Alkyl, C₁-C₆-Halogenalkyl, C₃-C₆-Alkenyl oder C₃-C₆-Alkinyl;
- Q: ein gegebenenfalls substituierter in 2-Stellung verknüpfter Cyclohexan-1,3-dionring;
wobei m 1 bedeutet, wenn R³ für Wasserstoff steht;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur beispielsweise aus EP-A 278 742, EP-A 298 680, EP-A 320 864 und WO 96/14285 sind 2-Benzoyl-cyclohexan-1,3-dione bekannt. Weiterhin werden in WO/26200 2-Benzoyl-cyclohexan-1,3-dion-Derivate beschrieben, die in 3-Position des Benzoylrestes einen Heterocyclus tragen.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 2-Benzoyl-cyclohexan-1,3-dione der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können ebenso je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschstenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Hervorzuheben sind die erfindungsgemäßen Verbindungen der Formel I, wobei die Variable Q einen in 2-Stellung verknüpften Cyclohexan-1,3-dionring der Formel II darstellt, 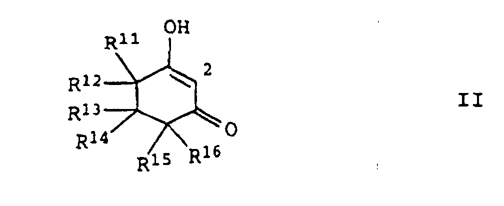 wobei II auch stellvertretend für die tautomeren Formeln II' und II'' steht, 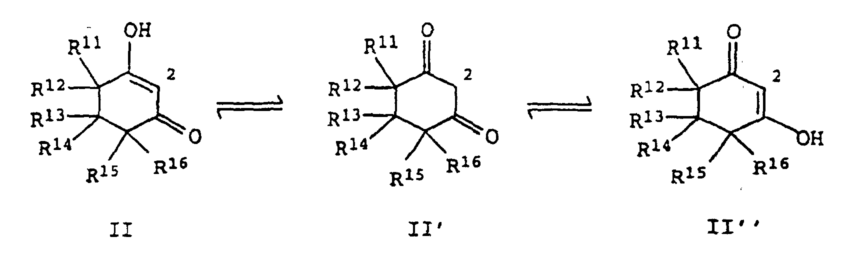 wobei
- R¹¹, R¹², R¹⁴ und R¹⁶: für Wasserstoff oder C₁-C₄-Alkyl stehen;
- R¹³: für Wasserstoff, C₁-C₄-Alkyl oder C₃-C₄-Cycloalkyl steht, wobei die. beiden letztgenannten Gruppen einen bis drei der folgenden Substituenten tragen können:
Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,4-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
- R¹⁵: für Wässerstoff, C₁-C₄-Alkyl oder C₁-C₆-Alkoxycarbonyl steht;
oder
- R¹³ und R¹⁶: gemeinsam eine π-Bindung oder einen drei- bis sechsgliedrigen carbocyclischen Ring bilden;
oder
die CR¹³R¹⁴-Einheit durch C=O ersetzt sein kann.

Ebenso hervorzuheben sind die erfindungsgemäßen Verbindungen der Formel I, wobei
- R⁴: Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, - COSR¹⁰, -CONR⁸R¹⁰, C₁-C₄-Alkyliminooxy, C₁-C₄-Alkoxyamino. C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Phenyl-C₁-C₄-alkyl, Hetaryl, Phenoxy, Phenyl-C₁-C₄-alkoxy und Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
bedeuten.

Die für die Substituenten R¹-R¹⁶ oder als Reste an Phenyl-, Hetaryl- und Heterocylylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylthio, Alkylsulfonyl-, Alkylcarbonyl-, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, Propyl, 1-Methylethyl, Butyl, 1-Methylpropyl, 2-Methylpropyl und 1,1-Dimethylethyl;
- C₁-C₄-Alkyl, sowie die Alkylteile von C₁-C₄-Alkylcarbonyl: C₂-C₄-Alkyl, wie voranstehend genannt sowie Methyl;
- C₂-C₆-Alkyl, sowie die Alkylteile von C₁-C₆-Alkoxy-C₂-C₆alkyl: C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl. 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1.1,2-Trimethylpropyl, 1-Ethyl-1-methylpropyl und 1-Ethyl-3-methylpropyl;
- C₁-C₆-Alkyl, sowie die Alkylteile von C₁-C₆-Alkoxy-C₁-C₆-alkyl: C₂-C₆-Alkyl, wie voranstehend genannt, sowie Methyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl. Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6 -Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- -C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2.2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3.3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylthio: Methylthio, Ethylthio, Propylthio, 1-Methylethylthio, Butylthio, 1-Methylpropylthio, 2-Methylpropylthio und 1,1-Dimethylethylthio;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl. 2-Methyl-but-1-en-1-yl, 3-Methyl-but-1-en-1-yl, 1-Methyl -but-2 -en-1-yl, 2-Methyl-but-2-en-1-yl, 3-Methyl-but-2-en-1-yl, 1-Methyl-but-3-en-1-yl. 2-Methyl-but-3-en-1-yl, 3-Methyl-but-3-en-1-yl, 1,1-Dimethyl-prop-2-en-1-yl, 1,2-Dimethyl-prop-1-en-1-yl. 1,2-Dimethyl-prop-2-en-1-yl, 1-Ethyl-prop-l-en-2-yl, 1-Ethyl-prop-2-en-1-yl, Hex-1-en-1-yl. Hex-2-en-1-yl. Hex-3-en-1-yl, Hex-4-en-1-yl, Hex-5-en-1-yl, 1-Methyl-pent-1-en-1 -yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, l,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₄-Cycloalkyl: Cyclopropyl und Cyclobutyl;
- C₃-C₆-Cycloalkyl: C₃-C₄-Cycloalkyl, wie voranstehend genannt, sowie Cyclopentyl und Cyclohexyl;
- C₄-C₆-Cycloalkenyl: Cyclobuten-1-yl, cyclobutan-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1.3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl. 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl. 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,9,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy:
   aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Puryl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1.3,5-Triazin-2-yl, 1,2,4-Triazin-3-yl, 1,2,4,5-Tetrazin-3-yl, sowie die entsprechenden benzokondensierten Derivate.

Alle Phenyl- und Hetarylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, - OR⁵ oder -S(O)ₙR⁷;
besonders bevorzugt Nitro, Halogen wie z.B. Fluor, Chlor oder Brom, C₁-C₆-Halogenalkyl, wie z.B. Trifluormethyl, -OR⁵ oder - SO₂R⁷ ;
- R²: Wasserstoff, Nitro, Halogen, Cyano, Rhodano, C₁-C₆-Alkyl, C₁-C₆-Halogenalkyl C₁-C₆-Alkoxy-C₁-C₆-alkyl, C₂-C₆-Alkenyl, C₂-C₆-Alkinyl, -OR⁵ oder -S(O)ₙR⁷;
besonders bevorzugt Wasserstoff, Nitro, Halogen wie z.B. Fluor, Chlor oder Brom, C₁-C₆-Alkyl, wie z,B. Methyl oder Ethyl, C₁-C₆-Halogenalkyl, wie z.B. Trifluormethyl, -OR⁵ oder - SO₂R⁷;
- R³: Wasserstoff, C₁-C₆-Alkyl, wie z.B. Methyl, Ethyl, Propyl oder Butyl, oder C₁-C₆-Halogenalkyl, wie z.B. Difluormethyl oder Trifluormethyl;
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR¹⁰, =NOR¹⁰, -OCOR¹⁰, -CO₂R¹⁰, -COSR¹⁰, -CONR⁸R¹⁰, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Phenyl-C₁-C₄-alkyl, Hetaryl, Phenoxy, Phenyl-C₁-C₄-alkoxy oder Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
- X: Sauerstoff oder Schwefel;
besonders bevorzugt Sauerstoff;
- Z: Sauerstoff, NH oder NCH₃;
- m: 0 oder 1
- n: 0 oder 2
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
besonders bevorzugt Methyl, Ethyl, Trifluormethyl, Difluormethyl, Methoxyethyl, Allyl oder Propargyl;
- R⁷: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
besonders bevorzugt Methyl, Ethyl, Trifluormethyl, Difluormethyl, Methoxyethyl, Allyl oder Propargyl;
- R⁸: Wasserstoff oder C₁-C₆-Alkyl;
- R¹⁰: C₁-C₆-Alkyl, C₁-C₆-Halogenalkyl, C₃-C₆-Alkenyl oder C₃-C₆-Alkinyl;
- R¹¹, R¹², R¹⁴, R¹⁶: Wasserstoff oder C₁-C₄-Alkyl;
besonders bevorzugt Wasserstoff, Methyl oder Ethyl;
- R¹³: Wasserstoff, C₁-C₄-Alkyl, C₃-C₄-Cycloalkyl, wobei die beiden letztgenannten Gruppen gegebenenfalls einen bis drei der folgenden Substituenten tragen können: Halogen, C₁-C₄-Alkoxy oder C₁-C₄-Alkylthio;
Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl 1,4-Dioxan-2-yl, 1,3-Oxathiolan-2-yl, 1,3-Oxathian-2-yl, 1,3-Dithian-2-yl oder 1,3-Dithiolan-2-yl, wob,ei die sechs letztgenannten Gruppen jeweils einen bis drei C₁-C₄-Alkylreste tragen können;
besonders bevorzugt Wasserstoff, Methyl, Ethyl, Cyclopropyl, Di(methoxy)methyl, Di(ethoxy)methyl, 2-Ethylthiopropyl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-y 1,3-Dioxan-2-yl, 1,4-Dioxan-2-yl,
5,5-Dimethyl-1-3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
- R¹⁵: Wasserstoff, C₁-C₄-Alkyl oder C₁-C₄-Alkoxycarbonyl;
besonders bevorzugt Wasserstoff, Methyl oder Methoxycarbonyl.

Ebenso kann vorteilhaft in Betracht kommen, daß R¹³ und R¹⁶ eine π-Bindung ausbilden, so daß ein Doppelbindungssystem entsteht.

Die CR¹³R¹⁴-Einheit kann auch vorteilhaft durch C=O ersetzt werden.

Besonders bevorzugt sind Verbindungen der Formel I mit m = 1. Ebenso besonders bevorzugt sind Verbindungen mit m = 0 und R³, R⁴ + Wasserstoff.

Insbesondere bevorzugt sind Verbindungen der Formel I mit m = 1.

Außerordentlich bevorzugt sind Verbindungen der Formel Ia (≙I wobei R¹ in Position 4 des Phenylringes und R² in Position 2 des Phenylringes gebunden sind).

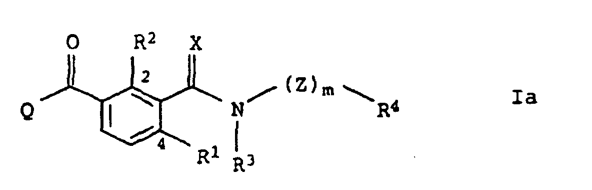

Insbesondere außerordentlich bevorzugt sind die Verbindungen der Formel Ia in der die Variablen R1 bis R³, Q, X, Z und m die oben genannte Bedeutung haben und
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR¹⁰, =NOR¹⁰, -OCOR¹⁰, -CO₂R¹⁰, - COSR¹⁰, -CONR⁸R¹⁰, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Phenyl-C₁-C₄-alkyl, Hetaryl, Phenoxy, Phenyl-C₁-C₄-alkoxy oder Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
bedeutet.

Insbesonderst außerordentlichst bevorzugt sind die Verbindungen Ia1 (≙ I mit R¹ = C1, R¹¹, R¹², R¹³, R¹⁴, R¹⁵, R¹⁶ = H, X = Sauerstoff und m = 1, wobei R¹ in 4-Position des Phenylringes und R² in 2-Position des Phenylringes gebunden sind), insbesondere die Verbindungen der Tabelle 1.

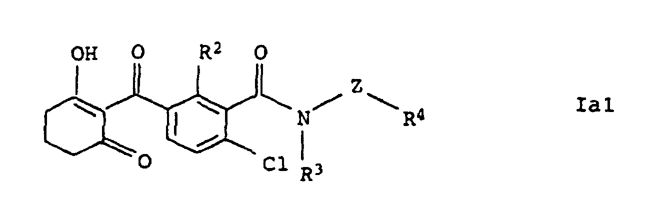

**Tabelle 1**

| Nr. | R² | R³ | R⁴ | Z |
|---|---|---|---|---|
| Ia1.1 | Cl | H | CH₃ | O |
| Ia1.2 | Cl | CH₃ | CH₃ | O |
| Ia1.3 | Cl | CH₂CH₃ | CH₃ | O |
| Ia1.4 | Cl | (CH₂)₂CH₃ | CH₃ | O |
| Ia1.5 | Cl | (CH₂)₃CH₃ | CH₃ | O |
| Ia1.6 | Cl | H | C₂H₅ | O |
| Ia1.7 | Cl | CH₃ | C₂H₅ | O |
| Ia1.8 | Cl | CH₂CH₃ | C₂H₅ | O |
| Ia1.9 | Cl | (CH₂)₂CH₃ | C₂H₅ | O |
| Ia1.10 | Cl | (CH₂)₃CH₃ | C₂H₅ | O |
| Ia1.11 | Cl | H | (CH₂)₂CH₃ | O |
| Ia1.12 | Cl | CH₃ | (CH₂)₂CH₃ | O |
| Ia1.13 | Cl | CH₂CH₃ | (CH₂)₂CH₃ | O |
| Ia1.14 | Cl | (CH₂)₂CH₃ | (CH₂)₂CH₃ | O |
| Ia1.15 | Cl | (CH₂)₃CH₃ | (CH₂)₂CH₃ | O |
| Ia1.16 | Cl | H | CH(CH₃)₂ | O |
| Ia1.17 | Cl | CH₃ | CH(CH₃)₂ | O |
| Ia1.18 | Cl | CH₂CH₃ | CH(CH₃)₂ | O |
| Ia1.19 | Cl | (CH₂)₂CH₃ | CH(CH₃)₂ | O |
| Ia1.20 | Cl | (CH₂)₃CH₃ | CH (CH₃ )₂ | O |
| Ia1.21 | Cl | H | (CH₂)₃CH₃ | O |
| Ia1.22 | Cl | CH₃ | (CH₂)₃CH₃ | O |
| Ia1.23 | Cl | CH₂CH₃ | (CH₂)₃CH₃ | O |
| Ia1.24 | Cl | (CH₂)₂CH₃ | (CH₂)₃CH₃ | O |
| Ia1.25 | Cl | (CH₂)₃CH₃ | (CH₂)₃CH₃ | O |
| Ia1.26 | Cl | H | CH₂CH(CH₃)₂ | O |
| Ia1.27 | Cl | CH₃ | CH₂CH(CH₃)₂ | O |
| Ia1.28 | Cl | CH₂CH₃ | CH₂CH(CH₃)₂ | O |
| Ia1.29 | Cl | (CH₂)₂CH₃ | CH₂CH(CH₃)₂ | O |
| Ia1.30 | Cl | (CH₂)₃CH₃ | CH₂CH(CH₃)₂ | O |
| Ia1.31 | Cl | H | CH(CH₃)CH₂CH₃ | O |
| Ia1.32 | Cl | CH₃ | CH(CH₃)CH₂CH₃ | O |
| Ia1.33 | Cl | CH₂CH₃ | CH(CH₃)CH₂CH₃ | O |
| Ia1.34 | Cl | (CH₂)₂CH₃ | CH(CH₃)CH₂CH₃ | O |
| Ia1.35 | Cl | (CH₂)₃CH₃ | CH(CH₃)CH₂CH₃ | O |
| Ia1.36 | Cl | H | (CH₂)₂-C₆H₅ | O |
| Ia1.37 | Cl | CH₃ | (CH₂)₂-C₆H₅ | O |
| Ia1.38 | Cl | CH₂CH₃ | (CH₂)₂-C₆H₅ | O |
| Ia1.39 | Cl | (CH₂)₂CH₃ | (CH₂)₂-C₆H₅ | O |
| Ia1.40 | Cl | (CH₂)₃CH₃ | (CH₂)₂-C₆H₅ | O |
| Ia1.41 | Cl | H | CH₂CH(CH₃)-C₆H₅ | O |
| Ia1.42 | Cl | CH₃ | CH₂CH(CH₃)-C₆H₅ | O |
| Ia1.43 | Cl | CH₂CH₃ | CH₂CH(CH₃)-C₆H₅ | O |
| Ia1.44 | Cl | (CH₂)₂CH₃ | CH₂CH(CH₃)-C₆H₅ | O |
| Ia1.45 | Cl | (CH₂)₃CH₃ | CH₂CH(CH₃)-C₆H₅ | O |
| Ia1.46 | Cl | H | CH(CH₃)CH₂-C₆H₅ | O |
| Ia1.47 | Cl | CH₃ | CH(CH₃)CH₂-C₆H₅ | O |
| Ia1.48 | Cl | CH₂CH₃ | CH(CH₃)CH₂-C₆H₅ | O |
| Ia1.49 | Cl | (CH₂)₂CH₃ | CH(CH₃)CH₂-C₆H₅ | O |
| Ia1.50 | Cl | (CH₂)₃CH₃ | CH(CH₃)CH₂-C₆H₅ | O |
| Ia1.51 | Cl | H | CH(CH₃)CH(CH₃)-C₆H₅ | O |
| Ia1.52 | Cl | CH₃ | CH(CH₃)CH(CH₃)-C₆H₅ | O |
| Ia1.53 | Cl | CH₂CH₃ | CH(CH₃)CH(CH₃)-C₆H₅ | O |
| Ia1.54 | Cl | (CH₂)₂CH₃ | CH(CH₃)CH(CH₃)-C₆H₅ | O |
| Ia1.55 | Cl | (CH₂)₃CH₃ | CH(CH₃)CH(CH₃)-C₆H₅ | O |
| Ia1.56 | Cl | H | (CH₂)₂-(4-Cl-C₆H₄) | O |
| Ia1.57 | Cl | CH₃ | (CH₂)₂-(4-Cl-C₆H₄) | O |
| Ia1.58 | Cl | CH₂CH₃ | (CH₂)₂-(4-Cl-C₆H₄) | O |
| la1.59 | Cl | (CH₂)₂CH₃ | (CH₂)₂-(4-Cl-C₆H₄) | O |
| Ia1.60 | Cl | (CH₂)₃CH₃ | (CH₂)₂-(4-Cl-C₆H₄) | O |
| Ia1.61 | Cl | H | CH₂CH(CH₃)-(4-Cl-C₆H₄) | O |
| Ia1.62 | Cl | CH₃ | CH₂CH(CH₃)-(4-Cl-C₆H₄) | O |
| Ia1.63 | Cl | CH₂CH₃ | CH₂CH(CH₃)-(4-Cl-C₆H₄) | O |
| Ia1.64 | Cl | (CH₂)₂CH₃ | CH₂CH(CH₃)-(4-Cl-C₆H₄) | O |
| Ia1.65 | Cl | (CH₂)₃CH₃ | CH₂CH(CH₃)-(4-Cl-C₆H₄) | O |
| Ia1.66 | Cl | H | CH(CH₃)CH₂-(4-Cl-C₆H₄) | O |
| Ia1.67 | Cl | CH₃ | CH(CH₃)CH₂-(4-Cl-C₆H₄) | O |
| Ia1.68 | Cl | CH₂CH₃ | CH(CH₃)CH₂-(4-Cl-C₆H₄) | O |
| Ia1.69 | Cl | (CH₂)₂CH₃ | CH(CH₃)CH₂(4-Cl-C₆H₄) | O |
| Ia1.70 | Cl | (CH₂)₃CH₃ | CH(CH₃)CH₂(4-Cl-C₆H₄) | O |
| Ia1.71 | Cl | H | CH(CH₃)CH(CH₃)-(4-Cl-C₆H₄) | O |
| Ia1.72 | Cl | CH₃ | CH(CH₃)CH(CH₃)-(4-Cl-C₆H₄) | O |
| Ia1.73 | Cl | CH₂CH₃ | CH(CH₃)CH(CH₃)-(4-Cl-C₆H₄) | O |
| Ia1.74 | Cl | (CH₂)₂CH₃ | CH(CH₃)CH(CH₃)-(4-Cl-C₆H₄) | O |
| Ia1.75 | Cl | (CH₂)₃CH₃ | CH(CH₃)CH(CH₃)-(4-Cl-C₆H₄) | O |
| Ia1.76 | Cl | H | (CH₂)₂- (2,4-Cl₂-C₆H₃) | O |
| Ia1.77 | Cl | CH₃ | (CH₂)₂-(2,4-Cl₂-C₆H₃) | O |
| Ia1.78 | Cl | CH₂CH₃ | (CH₂)₂-(2,4-Cl₂-C₆H₃) | O |
| Ia1.79 | Cl | (CH₂)₂CH₃ | (CH₂)₂-(2,4-Cl₂-C₆H₃) | O |
| Ia1.80 | Cl | (CH₂)₃CH₃ | (CH₂)₂-(2,4 Cl₂-C₆H₃) | O |
| Ia1.81 | Cl | H | CH₂CH(CH₃)-(2,4-Cl₂-C₆H₃) | O |
| Ia1.82 | Cl | CH₃ | CH₂CH(CH₃)-(2,4-Cl₂-C₆H₃) | O |
| Ia1.83 | Cl | CH₂CH₃ | CH₂CH(CH₃)-(2,4-Cl₂-C₆H₃) | O |
| Ia1.84 | Cl | (CH₂)₂CH₃ | CH₂CH(CH₃)-(2,4-Cl₂-C₆H₃) | O |
| Ia1.85 | Cl | (CH₂)₃CH₃ | CH₂CH(CH₃)-(2,4-Cl₂-C₆H₃) | O |
| Ia1.86 | Cl | H | CH(CH₃)CH₂-(2,4-Cl₂-C₆H₃) | O |
| Ia1.87 | Cl | CH₃ | CH(CH₃)CH₂-(2,4-Cl₂-C₆H₃) | O |
| Ia1.88 | Cl | CH₂CH₃ | CH(CH₃)CH₂-(2,4-Cl₂-C₆H₃) | O |
| Ia1.89 | Cl | (CH₂)₂CH₃ | CH(CH₃)CH₂-(2,4-Cl₂-C₆H₃) | O |
| Ia1.90 | Cl | (CH₂)₃CH₃ | CH(CH₃)CH₂-(2,4-Cl₂-C₆H₃) | O |
| Ia1.91 | Cl | H | CH(CH₃)CH(CH₃)-(2,4-Cl₂-C₆H₃) | O |
| Ia1.92 | Cl | CH₃ | CH(CH₃)CH(CH₃)-(2,4-Cl₂-C₆H₃) | O |
| Ia1.93 | Cl | CH₂CH₃ | CH(CH₃)CH(CH₃)-(2,4-Cl₂-C₆H₃) | O |
| Ia1.94 | Cl | (CH₂)₂CH₃ | CH(CH₃)CH (CH₃)-(2,4-Cl₂-C₆H₃) | O |
| Ia1.95 | Cl | (CH₂)₃CH₃ | CH(CH₃)CH(CH₃)-(2,4-Cl₂-C₆H₃) | O |
| Ia1.96 | Cl | H | (CH₂)₂-O-C₆H₅ | O |
| Ia1.97 | Cl | CH₃ | (CH₂)₂-O-C₆H₅ | O |
| Ia1.98 | Cl | CH₂CH₃ | (CH₂)₂-O-C₆H₅ | O |
| Ia1.99 | Cl | (CH₂)₂CH₃ | (CH₂)₂-O-C₆H₅ | O |
| Ia1.100 | Cl | (CH₂)₃CH₃ | (CH₂)₂-O-C₆H₅ | O |
| Ia1.101 | Cl | H | CH₂CH(CH₃)-O-C₆H₅ | O |
| Ia1.102 | Cl | CH₃ | CH₂CH(CH₃)-O-C₆H₅ | O |
| Ia1.103 | Cl | CH₂CH₃ | CH₂CH(CH₃)-O-C₆H₅ | O |
| Ia1.104 | Cl | (CH₂)₂CH₃ | CH₂CH(CH₃)-O-C₆H₅ | O |
| Ia1.105 | Cl | (CH₂)₃CH₃ | CH₂CH(CH₃)-O-C₆H₅ | O |
| Ia1.106 | Cl | H | CH(CH₃)CH₂-O-C₆H₅ | O |
| Ia1.107 | Cl | CH₃ | CH(CH₃)CH₂-O-C₆H₅ | O |
| Ia1.108 | Cl | CH₂CH₃ | CH(CH₃)CH₂-O-C₆H₅ | O |
| Ia1.109 | Cl | (CH₂)₂CH₃ | CH(CH₃)CH₂-O-C₆H₅ | O |
| Ia1.110 | Cl | (CH₂)₃CH₃ | CH(CH₃)CH₂-O-C₆H₅ | O |
| Ia1.111 | Cl | H | CH(CH₃)CH(CH₃)-O-C₆H₅ | O |
| Ia1.112 | Cl | CH₃ | CH(CH₃)CH(CH₃)-O-C₆H₅ | O |
| Ia1.113 | Cl | CH₂CH₃ | CH(CH₃)CH(CH₃)-O-C₆H₅ | O |
| Ia1.114 | Cl | (CH₂)₂CH₃ | CH(CH₃)CH(CH₃)-O-C₆H₅ | O |
| Ia1.115 | Cl | (CH₂)₃CH₃ | CH(CH₃)CH(CH₃)-O-C₆H₅ | O |
| Ia1.116 | Cl | H | (CH₂)₂-O-(4-Cl-C₆H₄) | O |
| Ia1.117 | Cl | CH₃ | (CH₂)₂-O-(4-Cl-C₆H₄) | O |
| Ia1.118 | Cl | CH₂CH₃ | (CH₂)₂-O-(4-Cl-C₆H₄) | O |
| Ia1.119 | Cl | (CH₂)₂CH₃ | (CH₂)₂-O-(4-Cl-C₆H₄) | O |
| Ia1.120 | Cl | (CH₂)₃CH₃ | (CH₂)₂-O-(4-Cl-C₆H₄) | O |
| Ia1.121 | Cl | H | CH₂CH(CH₃)-O-(4-Cl-C₆H₄) | O |
| Ia1.122 | Cl | CH₃ | CH₂CH(CH₃)-O-(4-Cl-C₆H₄) | O |
| Ia1.123 | Cl | CH₂CH₃ | CH₂CH(CH₃)-O-(4-Cl-C₆H₄) | O |
| Ia1.124 | Cl | (CH₂)₂CH₃ | CH₂CH(CH₃)-O-(4-Cl-C₆H₄) | O |
| Ia1.125 | Cl | (CH₂)₃CH₃ | CH₂CH(CH₃)-O-(4-Cl-C₆H₄) | O |
| Ia1.126 | Cl | H | CH(CH₃)CH₂-O-(4-Cl-C₆H₄) | O |
| Ia1.127 | Cl | CH₃ | CH(CH₃)CH₂-O-(4-Cl-C₆H₄) | O |
| Ia1.128 | Cl | CH₂CH₃ | CH(CH₃)CH₂-O-(4-Cl-C₆H₄) | O |
| Ia1.129 | Cl | (CH₂)₂CH₃ | CH(CH₃)CH₂-O-(4-Cl-C₆H₄) | O |
| Ia1.130 | Cl | (CH₂)₃CH₃ | CH(CH₃)CH₂-O-(4-Cl-C₆H₄) | O |
| Ia1.131 | Cl | H | CH(CH₃)CH(CH₃)-O-(4-Cl-C₆H₄) | O |
| Ia1.132 | Cl | CH₃ | CH(CH₃)CH(CH₃)-O-(4-Cl-C₆H₄) | O |
| Ia1.133 | Cl | CH₂CH₃ | CH(CH₃)CH(CH₃)-O-(4-Cl-C₆H₄) | O |
| Ia1.134 | Cl | (CH₂)₂CH₃ | CH(CH₃)CH(CH₃)-O-(4-Cl-C₆H₄) | O |
| Ia1.135 | Cl | (CH₂)₃CH₃ | CH(CH₃)CH(CH₃)-O-(4-Cl-C₆H₄) | O |
| Ia1.136 | C1 | H | (CH₂)₂-O-(2,4-Cl₂-C₆H₃) | O |
| Ia1.137 | C1 | CH₃ | (CH₂)₂-O-(2,4-Cl₂-C₆H₃) | O |
| Ia1.138 | C1 | CH₂CH₃ | (CH₂)₂-O-(2,4-Cl₂-C₆H₃) | O |
| Ia1.139 | Cl | (CH₂)₂CH₃ | (CH₂)₂-O-(2,4-Cl₂-C₆H₃) | O |
| Ia1.140 | Cl | (CH₂)₃CH₃ | (CH₂)₂-O-(2,4-Cl₂-C₆H₃) | O |
| Ia1.141 | Cl | H | CH₂CH(CH₃)-O-(2,4-Cl₂-C₆H₃) | O |
| Ia1.142 | Cl | CH₃ | CH₂CH(CH₃)-O-(2,4-Cl₂-C₆H₃) | O |
| Ia1.143 | Cl | CH₂CH₃ | CH₂CH(CH₃)-O-(2,4-Cl₂-C₆H₃) | O |
| Ia1.144 | Cl | (CH₂)₂CH₃ | CH₂CH(CH₃)-O-(2,4-Cl₂-C₆H₃) | O |
| Ia1.145 | Cl | (CH₂)₃CH₃ | CH₂CH(CH₃)-O-(2,4-Cl₂-C₆H₃) | O |
| Ia1.146 | Cl | H | CH(CH₃)CH₂-O-(2,4-Cl₂-C₆H₃) | O |
| Ia1.147 | Cl | CH₃ | CH(CH₃)CH₂-O-(2,4-Cl₂-C₆H₃) | O |
| Ia1.148 | Cl | CH₂CH₃ | CH(CH₃)CH₂-O-(2,4-Cl₂-C₆H₃) | O |
| Ia1.149 | Cl | (CH₂)₂CH₃ | CH (CH₃)CH₂-O-(2,4-Cl₂-C₆H₃) | O |
| Ia1.150 | Cl | (CH₂)₃CH₃ | CH(CH₃)CH₂-O-(2,4-Cl₂-C₆H₃) | O |
| Ia1.151 | Cl | H | CH(CH₃)CH(CH₃)-O-(2,4-Cl₂-C₆H₃) | O |
| Ia1.152 | Cl | CH₃ | CH(CH₃)CH(CH₃)-O-(2,4-Cl₂-C₆H₃) | O |
| Ia1.153 | Cl | CH₂CH₃ | CH(CH₃)CH(CH₃) -O-(2,4-Cl₂-C₆H₃) | O |
| Ia1.154 | Cl | (CH₂)₂CH₃ | CH(CH₃)CH(CH₃)-O-(2,4-Cl₂-C₆H₃) | O |
| Ia1.155 | Cl | (CH₂)₃CH₃ | CH(CH₃)CH(CH₃)-O-(2,4-Cl₂-C₆H₃) | O |
| Ia1.156 | Cl | H | CH₃ | NH |
| Ia1.157 | Cl | CH₃ | CH₃ | NH |
| Ia1.158 | Cl | CH₂CH₃ | CH₃ | NH |
| Ia1.159 | Cl | (CH₂)₂CH₃ | CH₃ | NH |
| Ia1.160 | Cl | (CH₂)₃CH₃ | CH₃ | NH |
| Ia1.161 | Cl | H | C₂H₅ | NH |
| Ia1.162 | Cl | CH₃ | C₂H₅ | NH |
| Ia1.163 | Cl | CH₂CH₃ | C₂H₅ | NH |
| Ia1.164 | Cl | (CH₂)₂CH₃ | C₂H₅ | NH |
| Ia1.165 | Cl | (CH₂)₃CH₃ | C₂H₅ | NH |
| Ia1.166 | Cl | H | (CH₂)₂CH₃ | NH |
| Ia1.167 | Cl | CH₃ | (CH₂)₂CH₃ | NH |
| Ia1.168 | Cl | CH₂CH₃ | (CH₂)₂CH₃ | NH |
| Ia1.169 | Cl | (CH₂)₂CH₃ | (CH₂)₂CH₃ | NH |
| Ia1.170 | Cl | (CH₂)₃CH₃ | (CH₂)₂CH₃ | NH |
| Ia1.171 | Cl | H | CH(CH₃)₂ | NH |
| Ia1.172 | Cl | CH₃ | CH(CH₃)₂ | NH |
| Ia1.173 | Cl | CH₂CH₃ | CH(CH₃)₂ | NH |
| Ia1.174 | Cl | (CH₂)₂CH₃ | CH(CH₃)₂ | NH |
| Ia1.175 | Cl | (CH₂)₃CH₃ | CH(CH₃)₂ | NH |
| Ia1.176 | Cl | H | (CH₂)₃CH₃ | NH |
| Ia1.177 | Cl | CH₃ | (CH₂)₃CH₃ | NH |
| Ia1.178 | Cl | CH₂CH₃ | (CH₂)₃CH₃ | NH |
| Ia1.179 | Cl | (CH₂)₂CH₃ | (CH₂)₃CH₃ | NH |
| Ia1.180 | Cl | (CH₂)₃CH₃ | (CH₂)₃CH₃ | NH |
| Ia1.181 | Cl | H | CH₂CH(CH₃)₂ | NH |
| Ia1.182 | Cl | CH₃ | CH₂CH(CH₃)₂ | NH |
| Ia1.183 | Cl | CH₂CH₃ | CH₂CH(CH₃)₂ | NH |
| Ia1.184 | Cl | (CH₂)₂CH₃ | CH₂CH(CH₃)₂ | NH |
| Ia1.185 | Cl | (CH₂)₃CH₃ | CH₂CH(CH₃)₂ | NH |
| Ia1.186 | Cl | H | CH(CH₃)CH₂CH₃ | NH |
| Ia1.187 | Cl | CH₃ | CH(CH₃)CH₂CH₃ | NH |
| Ia1.188 | Cl | CH₂CH₃ | CH(CH₃)CH₂CH₃ | NH |
| Ia1.189 | Cl | (CH₂)₂CH₃ | CH(CH₃)CH₂CH₃ | NH |
| Ia1.190 | Cl | (CH₂)₃CH₃ | CH(CH₃)CH₂CH₃ | NH |
| Ia1.191 | Cl | H | (CH₂)₂-C₆H₅ | NH |
| Ia1.192 | Cl | CH₃ | (CH₂)₂-C₆H₅ | NH |
| Ia1.193 | Cl | CH₂CH₃ | (CH₂)₂-C₆H₅ | NH |
| Ia1.194 | Cl | (CH₂)₂CH₃ | (CH₂)₂-C₆H₅ | NH |
| Ia1.195 | Cl | (CH₂)₃CH₃ | (CH₂)₂-C₆H₅ | NH |
| Ia1.196 | Cl | H | CH₂CH(CH₃)-C₆H₅ | NH |
| Ia1.197 | Cl | CH₃ | CH₂CH(CH₃)-C₆H₅ | NH |
| Ia1.198 | Cl | CH₂CH₃ | CH₂CH(CH₃)-C₆H₅ | NH |
| Ia1.199 | Cl | (CH₂)₂CH₃ | CH₂CH(CH₃)-C₆H₅ | NH |
| Ia1.200 | Cl | (CH₂)₃CH₃ | CH₂CH(CH₃)-C₆H₅ | NH |
| Ia1.201 | Cl | H | CH(CH₃)CH₂-C₆H₅ | NH |
| Ia1.202 | Cl | CH₃ | CH(CH₃)CH₂-C₆H₅ | NH |
| Ia1.203 | Cl | CH₂CH₃ | CH(CH₃)CH₂-C₆H₅ | NH |
| Ia1.204 | Cl | (CH₂)₂CH₃ | CH(CH₃)CH₂-C₆H₅ | NH |
| Ia1.205 | Cl | (CH₂)₃CH₃ | CH(CH₃)CH₂-C₆H₅ | NH |
| Ia1.206 | Cl | H | CH(CH₃)CH(CH₃)-C₆H₅ | NH |
| Ia1.207 | Cl | CH₃ | CH(CH₃)CH(CH₃)-C₆H₅ | NH |
| Ia1.208 | Cl | CH₂CH₃ | CH(CH₃)CH(CH₃)-C₆H₅ | NH |
| Ia1.209 | Cl | (CH₂)₂CH₃ | CH(CH₃)CH(CH₃)-C₆H₅ | NH |
| Ia1.210 | Cl | (CH₂)₃CH₃ | CH(CH₃)CH(CH₃)-C₆H₅ | NH |
| Ia1.211 | Cl | H | (CH₂)₂- (4-Cl-C₆H₄) | NH |
| Ia1.212 | Cl | CH₃ | (CH₂)₂-(4-Cl-C₆H₄) | NH |
| Ia1.213 | Cl | CH₂CH₃ | (CH₂)₂-(4-Cl-C₆H₄) | NH |
| Ia1.214 | Cl | (CH₂)₂CH₃ | (CH₂)₂- (4-Cl-C₆H₄) | NH |
| Ia1.215 | Cl | (CH₂)₃CH₃ | (CH₂)₂-(4-Cl-C₆H₄) | NH |
| Ia1.216 | Cl | H | CH₂CH(CH₃)-(4-Cl-C₆H₄) | NH |
| Ia1.217 | Cl | CH₃ | CH₂CH(CH₃)-(4-Cl-C₆H₄) | NH |
| Ia1.218 | Cl | CH₂CH₃ | CH₂CH(CH₃)-(4-Cl-C₆H₄) | NH |
| Ia1.219 | Cl | (CH₂)₂CH₃ | CH₂CH(CH₃)-(4-Cl-C₆H₄) | NH |
| Ia1.220 | Cl | (CH₂)₃CH₃ | CH₂CH(CH₃)-(4-Cl-C₆H₄) | NH |
| Ia1.221 | Cl | H | CH(CH₃)CH₂-(4-Cl-C₆H₄) | NH |
| Ia1.222 | Cl | CH₃ | CH(CH₃)CH₂-(4-Cl-C₆H₄) | NH |
| Ia1.223 | Cl | CH₂CH₃ | CH(CH₃)CH₂-(4-Cl-C₆H₄) | NH |
| Ia1.224 | Cl | (CH₂)₂CH₃ | CH(CH₃)CH₂-(4-Cl-C₆H₄) | NH |
| Ia1.225 | Cl | (CH₂)₃CH₃ | CH(CH₃)CH₂-(4-Cl-C₆H₄) | NH |
| Ia1.226 | Cl | H | CH(CH₃)CH(CH₃)-(4-Cl-C₆H₄) | NH |
| Ia1.227 | Cl | CH₃ | CH(CH₃)CH(CH₃)-(4-Cl-C₆H₄) | NH |
| Ia1.228 | Cl | CH₂CH₃ | CH(CH₃)CH(CH₃)-(4-Cl-C₆H₄) | NH |
| Ia1.229 | Cl | (CH₂)₂CH₃ | CH(CH₃)CH(CH₃)-(4-Cl-C₆H₄) | NH |
| Ia1.230 | Cl | (CH₂)₃CH₃ | CH(CH₃)CH(CH₃)-(4-Cl-C₆H₄) | NH |
| Ia1.231 | Cl | H | (CH₂)₂-(2,4-Cl₂-C₆H₃) | NH |
| Ia1.232 | Cl | CH₃ | (CH₂)₂-(2,4-Cl₂-C₆H₃) | NH |
| Ia1.233 | Cl | CH₂CH₃ | (CH₂)₂-(2,4-Cl₂-C₆H₃) | NH |
| Ia1.234 | Cl | (CH₂)₂CH₃ | (CH₂)₂-(2,4-Cl₂-C₆H₃) | NH |
| Ia1.235 | Cl | (CH₂)₃CH₃ | (CH₂)₂-(2,4-Cl₂-C₆H₃) | NH |
| Ia1.236 | Cl | H | CH₂CH(CH₃)-(2,4-Cl₂-C₆H₃) | NH |
| Ia1.237 | Cl | CH₃ | CH₂CH(CH₃)-(2,4-Cl₂-C₆H₃) | NH |
| Ia1.238 | Cl | CH₂CH₃ | CH₂CH(CH₃)-(2,4-Cl₂-C₆H₃) | NH |
| Ia1.239 | Cl | (CH₂)₂CH₃ | CH₂CH(CH₃)-(2,4-Cl₂-C₆H₃) | NH |
| Ia1.240 | Cl | (CH₂)₃CH₃ | CH₂CH(CH₃)-(2,4-Cl₂-C₆H₃) | NH |
| Ia1.241 | Cl | H | CH(CH₃)CH₂-(2,4-Cl₂-C₆H₃) | NH |
| Ia1.242 | Cl | CH₃ | CH(CH₃)CH₂-(2,4-Cl₂-C₆H₃) | NH |
| Ia1.243 | Cl | CH₂CH₃ | CH(CH₃)CH₂-(2,4-Cl₂-C₆H₃) | NH |
| Ia1.244 | Cl | (CH₂)₂CH₃ | CH(CH₃)CH₂-(2,4-Cl₂-C₆H₃) | NH |
| Ia1.245 | Cl | (CH₂)₃CH₃ | CH(CH₃)CH₂-(2,4-Cl₂-C₆H₃) | NH |
| Ia1.246 | Cl | H | CH(CH₃)CH(CH₃)-(2,4-Cl₂-C₆H₃) | NH |
| Ia1.247 | Cl | CH₃ | CH(CH₃)CH(CH₃)-(2,4-Cl₂-C₆H₃) | NH |
| Ia1.248 | Cl | CH₂CH₃ | CH(CH₃)CH(CH₃)-(2,4-Cl₂-C₆H₃) | NH |
| Ia1.249 | Cl | (CH₂)₂CH₃ | CH(CH₃)CH(CH₃)-(2,4-Cl₂-C₆H₃) | NH |
| Ia1.250 | Cl | (CH₂)₃CH₃ | CH(CH₃)CH(CH₃)-(2,4-Cl₂-C₆H₃) | NH |
| Ia1.251 | Cl | H | (CH₂)₂-O-C₆H₅ | NH |
| Ia1.252 | Cl | CH₃ | (CH₂)₂-O-C₆H₅ | NH |
| Ia1.253 | Cl | CH₂CH₃ | (CH₂)₂-O-C₆H₅ | NH |
| Ia1.254 | Cl | (CH₂)₂CH₃ | (CH₂)₂-O-C₆H₅ | NH |
| Ia1.255 | Cl | (CH₂)₃CH₃ | (CH₂) ₂-O-C₆H₅ | NH |
| Ia1.256 | Cl | H | CH₂CH(CH₃-O-C₆H₅ | NH |
| Ia1.257 | Cl | CH₃ | CH₂CH(CH₃)-O-C₆H₅ | NH |
| Ia1.258 | Cl | CH₂CH₃ | CH₂CH(CH₃)-O-C₆H₅ | NH |
| Ia1.259 | Cl | (CH₂)₂CH₃ | CH₂CH(CH₃)-O-C₆H₅ | NH |
| Ia1.260 | Cl | (CH₂)₃CH₃ | CH₂CH(CH₃)-O-C₆H₅ | NH |
| Ia1.261 | Cl | H | CH(CH₃)CH₂-O-C₆H₅ | NH |
| Ia1.262 | Cl | CH₃ | CH(CH₃)CH₂-O-C₆H₅ | NH |
| Ia1.263 | Cl | CH₂CH₃ | CH(CH₃)CH₂-O-C₆H₅ | NH |
| Ia1.264 | Cl | (CH₂)₂CH₃ | CH(CH₃)CH₂-O-C₆H₅ | NH |
| Ia1.265 | Cl | (CH₂)₃CH₃ | CH(CH₃)CH₂-O-C₆H₅ | NH |
| Ia1.266 | Cl | H | CH(CH₃)CH(CH₃)-O-C₆H₅ | NH |
| Ia1.267 | Cl | CH₃ | CH(CH₃)CH(CH₃)-O-C₆H₅ | NH |
| Ia1.268 | Cl | CH₂CH₃ | CH(CH₃)CH(CH₃)-O-C₆H₅ | NH |
| Ia1.269 | Cl | (CH₂)₂CH₃ | CH(CH₃)CH(CH₃)-O-C₆H₅ | NH |
| Ia1.270 | Cl | (CH₂)₃CH₃ | CH(CH₃)CH(CH₃)-O-C₆H₅ | NH |
| Ia1.271 | Cl | H | (CH₂)₂-O-(4-Cl-C₆H₄) | NH |
| Ia1.272 | Cl | CH₃ | (CH₂)₂-O-(4-Cl-C₆H₄) | NH |
| Ia1.273 | Cl | CH₂CH₃ | (CH₂)₂-O-(4-Cl-C₆H₄) | NH |
| Ia1.274 | Cl | (CH₂)₂CH₃ | (CH₂)₂-O-(4-Cl-C₆H₄) | NH |
| Ia1.275 | Cl | (CH₂)₃CH₃ | (CH₂)₂-O-(4-Cl -C₆H₄) | NH |
| Ia1.276 | Cl | H | CH₂CH(CH₃)-O-(4-Cl-C₆H₄) | NH |
| Ia1.277 | Cl | CH₃ | CH₂CH(CH₃)-O-(4-Cl-C₆H₄) | NH |
| Ia1.278 | Cl | CH₂CH₃ | CH₂CH(CH₃)-O-(4-Cl-C₆H₄) | NH |
| Ia1.279 | Cl | (CH₂)₂CH₃ | CH₂CH(CH₃)-O-(4-Cl-C₆H₄) | NH |
| Ia1.280 | Cl | (CH₂)₃CH₃ | CH₂CH(CH₃)-O-(4-Cl-C₆H₄) | NH |
| Ia1.281 | Cl | H | CH(CH₃)CH₂-O-(4-Cl-C₆H₄) | NH |
| Ia1.282 | Cl | CH₃ | CH(CH₃)CH₂-O-(4-Cl-C₆H₄) | NH |
| Ia1.283 | Cl | CH₂CH₃ | CH(CH₃)CH₂-O-(4-Cl-C₆H₄) | NH |
| Ia1.284 | Cl | (CH₂)₂CH₃ | CH(CH₃)CH₂-O-(4-Cl-C₆H₄) | NH |
| Ia1.285 | Cl | (CH₂)₃CH₃ | CH(CH₃)CH₂-O-(4-Cl-C₆H₄) | NH |
| Ia1.286 | Cl | H | CH(CH₃)CH(CH₃)-O-(4-Cl-C₆H₄) | NH |
| Ia1.287 | Cl | CH₃ | CH(CH₃)CH(CH₃)-O-(4-Cl-C₆H₄) | NH |
| Ia1.288 | Cl | CH₂CH₃ | CH(CH₃)CH(CH₃)-O-(4-Cl-C₆H₄) | NH |
| Ia1.289 | Cl | (CH₂)₂CH₃ | CH(CH₃)CH(CH₃)-O-(4-Cl-C₆H₄) | NH |
| Ia1.290 | Cl | (CH₂)₃CH₃ | CH(CH₃)CH(CH₃)-O-(4-Cl-C₆H₄) | NH |
| Ia1.291 | Cl | H | (CH₂)₂-O-(2,4-Cl₂-C₆H₃) | NH |
| Ia1.292 | Cl | CH₃ | (CH₂)₂-O-(2,4-Cl₂-C₆H₃) | NH |
| Ia1.293 | Cl | CH₂CH₃ | (CH₂)₂-O-(2.4-Cl₂-C₆H₃) | NH |
| Ia1.294 | Cl | (CH₂)₂CH₃ | (CH₂)₂-O-(2,4-Cl₂-C₆H₃) | NH |
| Ia1.295 | Cl | (CH₂)₃CH₃ | (CH₂)₂-O-(2,4-Cl₂-C₆H₃) | NH |
| Ia1.296 | Cl | H | CH₂CH(CH₃)-O-(2,4-Cl₂-C₆H₃) | NH |
| Ia1.297 | Cl | CH₃ | CH₂CH(CH₃)-O-(2,4-Cl₂-C₆H₃) | NH |
| Ia1.298 | Cl | CH₂CH₃ | CH₂CH(CH₃)-O-(2,4-Cl₂-C₆H₃) | NH |
| Ia1.299 | Cl | (CH₂)₂CH₃ | CH₂CH(CH₃)-O-(2,4-Cl₂-C₆H₃) | NH |
| Ia1.300 | Cl | (CH₂)₃CH₃ | CH₂CH(CH₃)-O-(2,4-Cl₂-C₆H₃) | NH |
| Ia1.301 | Cl | H | CH(CH₃)CH₂-O-(2,4-Cl₂-C₆H₃) | NH |
| Ia1.302 | Cl | CH₃ | CH(CH₃)CH₂-O-(2,4-Cl₂-C₆H₃) | NH |
| Ia1.303 | Cl | CH₂CH₃ | CH(CH₃)CH₂-O-(2,4-Cl₂-C₆H₃) | NH |
| Ia1.304 | Cl | (CH₂)₂CH₃ | CH(CH3)CH₂-O-(2,4-Cl₂-C₆H₃) | NH |
| Ia1.305 | Cl | (CH₂)₃CH₃ | CH(CH₃)CH₂-O-(2,4-Cl₂-C₆H₃) | NH |
| Ia1.306 | Cl | H | CH(CH₃)CH(CH₃)-O-(2,4-Cl₂-C₆H₃) | NH |
| Ia1.307 | Cl | CH₃ | CH(CH₃)CH(CH₃)-O-(2,4-Cl₂-C₆H₃) | NH |
| Ia1.308 | Cl | CH₂CH₃ | CH(CH₃)CH(CH₃)-O-(2,4-Cl₂-C₆H₃) | NH |
| Ia1.309 | Cl | (CH₂)₂CH₃ | CH(CH₃)CH(CH₃)-O-(2,4-Cl₂-C₆H₃) | NH |
| Ia1.310 | Cl | (CH₂)₃CH₃ | CH(CH₃)CH(CH₃)-O-(2,4-Cl₂-C₆H₃) | NH |
| Ia1.311 | Cl | H | CH₃ | NCH₃ |
| Ia1.312 | Cl | CH₃ | CH₃ | NCH₃ |
| Ia1.313 | Cl | CH₂CH₃ | CH₃ | NCH₃ |
| Ia1.314 | Cl | (CH₂)₂CH₃ | CH₃ | NCH₃ |
| Ia1.315 | Cl | (CH₂)₃CH₃ | CH₃ | NCH₃ |
| Ia1.316 | Cl | H | C₂H₅ | NCH₃ |
| Ia1.317 | Cl | CH₃ | C₂H₅ | NCH₃ |
| Ia1.318 | Cl | CH₂CH₃ | C₂H₅ | NCH₃ |
| Ia1.319 | Cl | (CH₂)₂CH₃ | C₂H₅ | NCH₃ |
| Ia1.320 | Cl | (CH₂)₃CH₃ | C₂H₅ | NCH₃ |
| Ia1.321 | Cl | H | (CH₂)₂CH₃ | NCH₃ |
| Ia1.322 | Cl | CH₃ | (CH₂)₂CH₃ | NCH₃ |
| Ia1.323 | Cl | CH₂CH₃ | (CH₂)₂CH₃ | NCH₃ |
| Ia1.324 | Cl | (CH₂)₂CH₃ | (CH₂)₂CH₃ | NCH₃ |
| Ia1.325 | Cl | (CH₂)₃CH₃ | (CH₂)₂CH₃ | NCH₃ |
| Ia1.326 | Cl | H | CH(CH₃)₂ | NCH₃ |
| Ia1.327 | Cl | CH₃ | CH(CH₃)₂ | NCH₃ |
| Ia1.328 | Cl | CH₂CH₃ | CH(CH₃)₂ | NCH₃ |
| Ia1.329 | Cl | (CH₂)₂CH₃ | CH(CH₃)₂ | NCH₃ |
| Ia1.330 | Cl | (CH₂)₃CH₃ | CH(CH₃)₂ | NCH₃ |
| Ia1.331 | Cl | H | (CH₂)₃CH₃ | NCH₃ |
| Ia1.332 | Cl | CH₃ | (CH₂)₃CH₃ | NCH₃ |
| Ia1.333 | Cl | CH₂CH₃ | (CH₂)₃CH₃ | NCH₃ |
| Ia1.334 | Cl | (CH₂)₂CH₃ | (CH₂)₃CH₃ | NCH₃ |
| Ia1.335 | Cl | (CH₂)₃CH₃ | (CH₂)₃CH₃ | NCH₃ |
| Ia1.336 | Cl | H | CH₂CH(CH₃)₂ | NCH₃ |
| Ia1.337 | Cl | CH₃ | CH₂CH(CH₃)₂ | NCH₃ |
| Ia1.338 | Cl | CH₂CH₃ | CH₂CH(CH3)₂ | NCH₃ |
| Ia1.339 | Cl | (CH₂)₂CH₃ | CH₂CH(CH₃)₂ | NCH₃ |
| Ia1.340 | Cl | (CH₂)₃CH₃ | CH₂CH(CH₃)₂ | NCH₃ |
| Ia1.341 | Cl | H | CH(CH₃)CH₂CH₃ | NCH₃ |
| Ia1.342 | Cl | CH₃ | CH(CH₃)CH₂CH₃ | NCH₃ |
| Ia1.343 | Cl | CH₂CH₃ | CH(CH₃)CH₂CH₃ | NCH₃ |
| Ia1.344 | Cl | (CH₂)₂CH₃ | CH(CH₃)CH₂CH₃ | NCH₃ |
| Ia1.345 | Cl | (CH₂)₃CH₃ | CH(CH₃)CH₂CH₃ | NCH₃ |
| Ia1.346 | Cl | H | (CH₂)₂-C₆H₅ | NCH₃ |
| Ia1.347 | Cl | CH₃ | (CH₂)₂-C₆H₅ | NCH₃ |
| la1.348 | Cl | CH₂CH₃ | (CH₂)₂-C₆H₅ | NCH₃ |
| Ia1.349 | Cl | (CH₂)₂CH₃ | (CH₂)₂-C₆H₅ | NCH₃ |
| Ia1.350 | Cl | (CH₂)₃CH₃ | (CH₂)₂-C₆H₅ | NCH₃ |
| Ia1.351 | Cl | H | CH₂CH(CH₃)-C₆H₅ | NCH₃ |
| Ia1.352 | Cl | CH₃ | CH₂CH(CH₃)-C₆H₅ | NCH₃ |
| Ia1.353 | Cl | CH₂CH₃ | CH₂CH(CH₃)-C₆H₅ | NCH₃ |
| Ia1.354 | Cl | (CH₂)₂CH₃ | CH₂CH(CH₃)-C₆H₅ | NCH₃ |
| Ia1.355 | Cl | (CH₂)₃CH₃ | CH₂CH(CH₃)-C₆H₅ | NCH₃ |
| Ia1.356 | Cl | H | CH(CH₃)CH₂-C₆H₅ | NCH₃ |
| Ia1.357 | Cl | CH₃ | CH(CH₃)CH₂-C₆H₅ | NCH₃ |
| Ia1.358 | Cl | CH₂CH₃ | CH(CH₃)CH₂-C₆H₅ | NCH₃ |
| Ia1.359 | Cl | (CH₂)₂CH₃ | CH(CH₃)CH₂-C₆H₅ | NCH₃ |
| Ia1.360 | Cl | (CH₂)₃CH₃ | CH(CH₃)CH₂-C₆H₅ | NCH₃ |
| Ia1.361 | Cl | H | CH(CH₃)CH(CH₃)-C₆H₅ | NCH₃ |
| Ia1.362 | Cl | CH₃ | CH(CH₃)CH(CH₃)-C₆H₅ | NCH₃ |
| Ia1.363 | Cl | CH₂CH₃ | CH(CH₃)CH(CH₃)-C₆H₅ | NCH₃ |
| Ia1.364 | Cl | (CH₂)₂CH₃ | CH(CH₃)CH(CH₃)-C₆H₅ | NCH₃ |
| Ia1.365 | Cl | (CH₂)₃CH₃ | CH(CH₃)CH(CH₃)-C₆H₅ | NCH₃ |
| Ia1.366 | Cl | H | (CH₂)₂-(4-Cl-C₆H₄) | NCH₃ |
| Ia1.367 | Cl | CH₃ | (CH₂)₂-(4-Cl-C₆H₄) | NCH₃ |
| Ia1.368 | Cl | CH₂CH₃ | (CH₂)₂-(4-Cl-C₆H₄) | NCH₃ |
| Ia1.369 | Cl | (CH₂)₂CH₃ | (CH₂)₂-(4-Cl-C₆H₄) | NCH₃ |
| Ia1.370 | Cl | (CH₂)₃CH₃ | (CH₂)₂-(4-Cl-C₆H₄) | NCH₃ |
| Ia1.371 | Cl | H | CH₂CH(CH₃)-(4-Cl-C₆H₄) | NCH₃ |
| Ia1.372 | Cl | CH₃ | CH₂CH(CH₃)-(4-Cl-C₆H₄) | NCH₃ |
| Ia1.373 | Cl | CH₂CH₃ | CH₂CH(CH₃)-(4-Cl-C₆H₄) | NCH₃ |
| Ia1.374 | Cl | (CH₂)₂CH₃ | CH₂CH(CH₃)-(4-Cl-C₆H₄) | NCH₃ |
| Ia1.375 | Cl | (CH₂)₃CH₃ | CH₂CH(CH₃)-(4-Cl-C₆H₄) | NCH₃ |
| Ia1.376 | Cl | H | CH(CH₃)CH₂-(4-Cl-C₆H₄) | NCH₃ |
| Ia1.377 | Cl | CH₃ | CH(CH₃)CH₂-(4-Cl-C₆H₄) | NCH₃ |
| Ia1.378 | Cl | CH₂CH₃ | CH(CH₃)CH₂-(4-Cl-C₆H₄) | NCH₃ |
| Ia1.379 | Cl | (CH₂)₂CH₃ | CH(CH₃)CH₂-(4-Cl-C₆H₄) | NCH₃ |
| Ia1.380 | Cl | (CH₂)₃CH₃ | CH(CH₃)CH₂-(4-Cl-C₆H₄) | NCH₃ |
| Ia1.381 | Cl | H | CH(CH₃)CH(CH₃)-(4-Cl-C₆H₄) | NCH₃ |
| Ia1.382 | Cl | CH₃ | CH(CH₃)CH(CH₃)-(4-Cl-C₆H₄) | NCH₃ |
| Ia1.383 | Cl | CH₂CH₃ | CH(CH₃)CH(CH₃)-(4-Cl-C₆H₄) | NCH₃ |
| Ia1.384 | Cl | (CH₂)₂CH₃ | CH(CH₃)CH(CH₃)-(4-Cl-C₆H₄) | NCH₃ |
| Ia1.385 | Cl | (CH₂)₃CH₃ | CH(CH₃)CH(CH₃)-(4-Cl-C₆H₄) | NCH₃ |
| Ia1.386 | Cl | H | (CH₂)₂-(2,4-Cl₂-C₆H₃) | NCH₃ |
| Ia1.387 | Cl | CH₃ | (CH2)2-(2,4-Cl₂-C₆H₃) | NCH₃ |
| Ia1.388 | Cl | CH₂CH₃ | (CH₂)₂-(2,4-Cl₂-C₆H₃) | NCH₃ |
| Ia1.389 | Cl | (CH₂)₂CH₃ | (CH₂)₂-(2,4-Cl₂-C₆H₃) | NCH₃ |
| Ia1.390 | Cl | (CH₂)₃CH₃ | (CH₂)₂-(2,4-Cl₂-C₆H₃) | NCH₃ |
| Ia1.391 | Cl | H | CH₂CH(CH₃)-(2,4-Cl₂-C₆H₃) | NCH₃ |
| Ia1.392 | Cl | CH₃ | CH₂CH(CH₃)-(2,4-Cl₂-C₆H₃) | NCH₃ |
| Ia1.393 | Cl | CH₂CH₃ | CH₂CH(CH₃)-(2,4-Cl₂-C₆H₃) | NCH₃ |
| Ia1.394 | Cl | (CH₂)₂CH₃ | CH₂CH(CH₃)-(2,4-Cl₂-C₆H₃) | NCH₃ |
| Ia1.395 | Cl | (CH₂)₃CH₃ | CH₂CH(CH₃)-(2,4-Cl₂-C₆H₃) | NCH₃ |
| Ia1.396 | Cl | H | CH(CH₃)CH₂-(2,4-Cl₂-C₆H₃) | NCH₃ |
| Ia1.397 | Cl | CH₃ | CH(CH₃)CH₂-(2,4-Cl₂-C₆H₃) | NCH₃ |
| Ia1.398 | Cl | CH₂CH₃ | CH(CH₃)CH₂-(2,4-Cl₂-C₆H₃) | NCH₃ |
| Ia1.399 | Cl | (CH₂)₂CH₃ | CH(CH₃)CH₂-(2,4-Cl₂-C₆H₃) | NCH₃ |
| Ia1.400 | Cl | (CH₂)₃CH₃ | CH(CH₃)CH₂-(2,4-Cl₂-C₆H₃) | NCH₃ |
| Ia1.401 | Cl | H | CH(CH₃)CH(CH₃)-(2,4-Cl₂-C₆H₃) | NCH₃ |
| Ia1.402 | Cl | CH₃ | CH(CH₃)CH(CH₃)-(2,4-Cl₂-C₆H₃) | NCH₃ |
| Ia1.403 | Cl | CH₂CH₃ | CH(CH₃)CH(CH₃)-(2,4-Cl₂-C₆H₃) | NCH₃ |
| Ia1.404 | Cl | (CH₂)₂CH₃ | CH(CH₃)CH(CH₃)-(2,4-Cl₂-C₆H₃) | NCH₃ |
| Ia1.405 | Cl | (CH₂)₃CH₃ | CH(CH₃)CH(CH₃)-(2, 4-Cl₂-C₆H₃) | NCH₃ |
| Ia1.406 | Cl | H | (CH₂)₂-O-C₆H₅ | NCH₃ |
| Ia1.407 | Cl | CH₃ | (CH₂)₂-O-C₆H₅ | NCH₃ |
| Ia1.408 | Cl | CH₂CH₃ | (CH₂)₂-O-C₆H₅ | NCH₃ |
| Ia1.409 | Cl | (CH₂)₂CH₃ | (CH₂)₂-O-C₆H₅ | NCH₃ |
| Ia1.410 | Cl | (CH₂)₃CH₃ | (CH₂)₂-O-C₆H₅ | NCH₃ |
| Ia1.411 | Cl | H | CH₂CH(CH₃)-O-C₆H₅ | NCH₃ |
| Ia1.412 | Cl | CH₃ | CH₂CH(CH₃)-O-C₆H₅ | NCH₃ |
| Ia1.413 | Cl | CH₂CH₃ | CH₂CH(CH₃)-O-C₆H₅ | NCH₃ |
| Ia1.414 | Cl | (CH₂)₂CH₃ | CH₂CH(CH₃)-O-C₆H₅ | NCH₃ |
| Ia1.415 | Cl | (CH₂)₃CH₃ | CH₂CH(CH₃)-O-C₆H₅ | NCH₃ |
| Ia1.416 | Cl | H | CH(CH₃)CH₂-O-C₆H₅ | NCH₃ |
| Ia1.417 | Cl | CH₃ | CH(CH₃)CH₂-O-C₆H₅ | NCH₃ |
| Ia1.418 | Cl | CH₂CH₃ | CH(CH₃)CH₂-O-C₆H₅ | NCH₃ |
| Ia1.419 | Cl | (CH₂)₂CH₃ | CH(CH₃)CH₂-O-C₆H₅ | NCH₃ |
| Ia1.420 | Cl | (CH₂)₃CH₃ | CH(CH₃)CH₂-O-C₆H₅ | NCH₃ |
| Ia1.421 | Cl | H | CH(CH₃)CH(CH₃)-O-C₆H₅ | NCH₃ |
| Ia1.422 | Cl | CH₃ | CH(CH₃)CH(CH₃)-O-C₆H₅ | NCH₃ |
| Ia1.423 | Cl | CH₂CH₃ | CH(CH₃)CH(CH₃)-O-C₆H₅ | NCH₃ |
| Ia1.424 | Cl | (CH₂)₂CH₃ | CH(CH₃)CH(CH₃)-O-C₆H₅ | NCH₃ |
| Ia1.425 | Cl | (CH₂)₃CH₃ | CH(CH₃)CH(CH₃)-O-C₆H₅ | NCH₃ |
| Ia1.426 | Cl | H | (CH₂)₂-O-(4-Cl-C₆H₄) | NCH₃ |
| Ia1.427 | Cl | CH₃ | (CH₂)₂-O-(4-Cl-C₆H₄) | NCH₃ |
| Ia1.428 | Cl | CH₂CH₃ | (CH₂)₂-O-(4-Cl-C₆H₄) | NCH₃ |
| Ia1.429 | Cl | (CH₂)₂CH₃ | (CH₂)₂-O-(4-Cl-C₆H₄) | NCH₃ |
| Ia1.430 | Cl | (CH₂)₃CH₃ | (CH₂)₂-O-(4-Cl-C₆H₄) | NCH₃ |
| Ia1.431 | Cl | H | CH₂CH(CH₃)-O-(4-Cl-C₆H₄) | NCH₃ |
| Ia1.432 | Cl | CH₃ | CH₂CH(CH₃)-O-(4-Cl-C₆H₄) | NCH₃ |
| Ia1.433 | Cl | CH₂CH₃ | CH₂CH(CH₃)-O-(4-Cl-C₆H₄) | NCH₃ |
| Ia1.434 | Cl | (CH₂)₂CH₃ | CH₂CH(CH₃)-O-(4-Cl-C₆H₄) | NCH₃ |
| Ia1.435 | Cl | (CH₂)₃CH₃ | CH₂CH(CH₃)-O-(4-Cl-C₆H₄) | NCH₃ |
| Ia1.436 | Cl | H | CH(CH₃)CH₂-O-(4-Cl-C₆H₄) | NCH₃ |
| Ia1.437 | Cl | CH₃ | CH(CH₃)CH₂-O-(4-Cl-C₆H₄) | NCH₃ |
| Ia1.438 | Cl | CH₂CH₃ | CH(CH₃)CH₂-O-(4-Cl-C₆H₄) | NCH₃ |
| Ia1.439 | Cl | (CH₂)₂CH₃ | CH(CH₃)CH₂-O-(4-Cl-C₆H₄) | NCH₃ |
| Ia1.440 | Cl | (CH₂)₃CH₃ | CH(CH₃)CH₂-O-(4-Cl-C₆H₄) | NCH₃ |
| Ia1.441 | Cl | H | CH(CH₃)CH(CH₃)-O-(4-Cl-C₆H₄) | NCH₃ |
| Ia1.442 | Cl | CH₃ | CH(CH₃)CH(CH₃)-O-(4-Cl-C₆H₄) | NCH₃ |
| Ia1.443 | Cl | CH₂CH₃ | CH(CH₃)CH(CH₃)-O-(4-Cl-C₆H₄) | NCH₃ |
| Ia1.444 | Cl | (CH₂)₂CH₃ | CH(CH₃)CH(CH₃)-O-(4-Cl-C₆H₄) | NCH₃ |
| Ia1.445 | Cl | (CH₂)₃CH₃ | CH(CH₃)CH(CH₃)-O-(4-Cl-C₆H₄) | NCH₃ |
| Ia1.446 | Cl | H | (CH₂)₂-O-(2,4-Cl₂-C₆H₃) | NCH₃ |
| Ia1.447 | Cl | CH₃ | (CH₂)₂-O-(2,4-Cl₂-C₆H₃) | NCH₃ |
| Ia1.448 | Cl | CH₂CH₃ | (CH₂)₂-O-(2,4-Cl₂-C₆H₃) | NCH₃ |
| Ia1.449 | Cl | (CH₂)₂CH₃ | (CH₂)₂-O-(2,4-Cl₂-C₆H₃) | NCH₃ |
| Ia1.450 | Cl | (CH₂)₃CH₃ | (CH₂)₂-O-(2,4-Cl₂-C₆H₃) | NCH₃ |
| Ia1.451 | Cl | H | CH₂CH(CH₃)-O-(2,4-Cl₂-C₆H₃) | NCH₃ |
| Ia1.452 | Cl | CH₃ | CH₂CH(CH₃)-O- (2,4-Cl₂-C₆H₃) | NCH₃ |
| Ia1.453 | Cl | CH₂CH₃ | CH₂CH(CH₃)-O-(2,4-Cl₂-C₆H₃) | NCH₃ |
| Ia1.454 | Cl | (CH₂)₂CH₃ | CH₂CH(CH₃)-O-(2,4-Cl₂-C₆H₃) | NCH₃ |
| Ia1.455 | Cl | (CH₂)₃CH₃ | CH₂CH(CH₃)-O-(2,4-Cl₂-C₆H₃) | NCH₃ |
| Ia1.456 | Cl | H | CH(CH₃)CH₂-O- (2,4-Cl₂-C₆H₃) | NCH₃ |
| Ia1.457 | Cl | CH₃ | CH(CH₃)CH₂-O-(2,4-Cl₂-C₆H₃) | NCH₃ |
| Ia1.458 | Cl | CH₂CH₃ | CH(CH₃)CH₂-O-(2,4-Cl₂-C₆H₃) | NCH₃ |
| Ia1.459 | Cl | (CH₂)₂CH₃ | CH(CH₃)CH₂-O-(2,4-Cl₂-C₆H₃) | NCH₃ |
| Ia1.460 | Cl | (CH₂)₃CH₃ | CH(CH₃)CH₂-O-(2,4-Cl₂-C₆H₃) | NCH₃ |
| Ia1.461 | Cl | H | CH(CH₃)CH(CH₃)-O-(2,4-Cl₂-C₆H₃) | NCH₃ |
| Ia1.462 | Cl | CH₃ | CH(CH₃)CH(CH₃)-O-(2,4-Cl₂-C₆H₃) | NCH₃ |
| Ia1.463 | Cl | CH₂CH₃ | CH(CH₃)CH(CH₃)-O-(2,4-Cl₂-C₆H₃) | NCH₃ |
| Ia1.462 | Cl | (CH₂)₂CH₃ | CH(CH₃)CH(CH₃)-O-(2,4-Cl₂-C₆H₃) | NCH₃ |
| Ia1.465 | Cl | (CH₂)₃CH₃ | CH(CH₃)CH(CH₃)-O-(2,4-Cl₂-C₆H₃) | NCH₃ |
| Ia1.466 | CH₃ | H | CH₃ | O |
| Ia1.467 | CH₃ | CH₃ | CH₃ | O |
| Ia1.468 | CH₃ | CH₂CH₃ | CH₃ | O |
| Ia1.469 | CH₃ | (CH₂)₂CH₃ | CH₃ | O |
| Ia1.470 | CH₃ | (CH₂)₃CH₃ | CH₃ | O |
| Ia1.471 | CH₃ | H | C₂H₅ | O |
| Ia1.472 | CH₃ | CH₃ | C₂H₅ | O |
| Ia1.473 | CH₃ | CH₂CH₃ | C₂H₅ | O |
| Ia1.474 | CH₃ | (CH₂)₂CH₃ | C₂H₅ | O |
| Ia1.475 | CH₃ | (CH₂)₃CH₃ | C₂H₅ | O |
| Ia1.476 | CH₃ | H | (CH₂)₂CH₃ | O |
| Ia1.477 | CH₃ | CH₃ | (CH₂)₂CH₃ | O |
| Ia1.478 | CH₃ | CH₂CH₃ | (CH₂)₂CH₃ | O |
| Ia1.479 | CH₃ | (CH₂)₂CH₃ | (CH₂)₂CH₃ | O |
| Ia1.480 | CH₃ | (CH₂)₃CH₃ | (CH₂)₂CH₃ | O |
| Ia1.481 | CH₃ | H | CH(CH₃)₂ | O |
| Ia1.482 | CH₃ | CH₃ | CH(CH₃)₂ | O |
| Ia1.483 | CH₃ | CH₂CH₃ | CH(CH₃)₂ | O |
| Ia1.484 | CH₃ | (CH₂)₂CH₃ | CH(CH₃)₂ | O |
| Ia1.485 | CH₃ | (CH₂)₃CH₃ | CH(CH₃)₂ | O |
| Ia1.486 | CH₃ | H | (CH₂)₃CH₃ | O |
| Ia1.487 | CH₃ | CH₃ | (CH₂)₃CH₃ | O |
| Ia1.488 | CH₃ | CH₂CH₃ | (CH₂)₃CH₃ | O |
| Ia1.489 | CH₃ | (CH₂)₂CH₃ | (CH₂)₃CH₃ | O |
| Ia1.490 | CH₃ | (CH₂)₃CH₃ | (CH₂)₃CH₃ | O |
| Ia1.491 | CH₃ | H | CH₂CH(CH₃)₂ | O |
| Ia1.492 | CH₃ | CH₃ | CH₂CH(CH₃)₂ | O |
| Ia1.493 | CH₃ | CH₂CH₃ | CH₂CH(CH₃)₂ | O |
| Ia1.494 | CH₃ | (CH₂)₂CH₃ | CH₂CH(CH₃)₂ | O |
| Ia1.495 | CH₃ | (CH₂)₃CH₃ | CH₂CH(CH₃)₂ | O |
| Ia1.496 | CH₃ | H | CH(CH₃)CH₂CH₃ | O |
| Ia1.497 | CH₃ | CH₃ | CH(CH₃)CH₂CH₃ | O |
| Ia1.498 | CH₃ | CH₂CH₃ | CH(CH₃)CH₂CH₃ | O |
| Ia1.499 | CH₃ | (CH₂)₂CH₃ | CH(CH₃)CH₂CH₃ | O |
| Ia1.500 | CH₃ | (CH₂)₃CH₃ | CH(CH₃)CH₂CH₃ | O |
| Ia1.501 | CH₃ | H | (CH₂)₂-C₆H₅ | O |
| Ia1.502 | CH₃ | CH₃ | (CH₂)₂-C₆H₅ | O |
| Ia1.503 | CH₃ | CH₂CH₃ | (CH₂)₂-C₆H₅ | O |
| Ia1.504 | CH₃ | (CH₂)₂CH₃ | (CH₂)₂-C₆H₅ | O |
| Ia1.505 | CH₃ | (CH₂)₃CH₃ | (CH₂)₂-C₆H₅ | O |
| Ia1.506 | CH₃ | H | CH₂CH(CH₃)-C₆H₅ | O |
| Ia1.507 | CH₃ | CH₃ | CH₂CH(CH₃)-C₆H₅ | O |
| Ia1.508 | CH₃ | CH₂CH₃ | CH₂CH(CH₃)-C₆H₅ | O |
| Ia1.509 | CH₃ | (CH₂)₂CH₃ | CH₂CH(CH₃)-C₆H₅ | O |
| Ia1.510 | CH₃ | (CH₂)₃CH₃ | CH₂CH(CH₃)-C₆H₅ | O |
| Ia1.511 | CH₃ | H | CH(CH₃)CH₂-C₆H₅ | O |
| Ia1.512 | CH₃ | CH₃ | CH(CH₃)CH₂-C₆Hₛ | O |
| Ia1.513 | CH₃ | CH₂CH₃ | CH(CH₃)CH₂-CₑH₅ | O |
| Ia1.514 | CH₃ | (CH₂)₂CH₃ | CH(CH₃)CH₂-C₆H₅ | O |
| Ia1.515 | CH₃ | (CH₂)₃CH₃ | CH(CH₃)CH₂-C₆H₅ | O |
| Ia1.516 | CH₃ | H | CH(CH₃)CH(CH₃)-C₆H₅ | O |
| Ia1.517 | CH₃ | CH₃ | CH(CH₃)CH(CH₃)-C₆H₅ | O |
| Ia1.518 | CH₃ | CH₂CH₃ | CH(CH₃)CH(CH₃)-C₆H₅ | O |
| Ia1.519 | CH₃ | (CH₂)₂CH₃ | CH(CH₃)CH(CH₃)-C₆H₅ | O |
| Ia1.520 | CH₃ | (CH₂)₃CH₃ | CH(CH₃)CH(CH₃)-C₆H₅ | O |
| Ia1.521 | CH₃ | H | (CH₂)₂-(4-Cl-C₆H₄) | O |
| Ia1.522 | CH₃ | CH₃ | (CH₂)₂-(4-Cl-C₆H₄) | O |
| Ia1.523 | CH₃ | CH₂CH₃ | (CH₂)₂-(4-Cl-C₆H₄) | O |
| Ia1.524 | CH₃ | (CH₂)₂CH₃ | (CH₂)₂-(4-Cl-C₆H₄) | O |
| Ia1.525 | CH₃ | (CH₂)₃CH₃ | (CH₂)₂-(4-Cl-C₆H₄) | O |
| Ia1.526 | CH₃ | H | CH₂CH(CH₃)-(4-Cl-C₆H₄) | O |
| Ia1.527 | CH₃ | CH₃ | CH₂CH(CH₃)-(4-Cl-C₆H₄) | O |
| Ia1.528 | CH₃ | CH₂CH₃ | CH₂CH(CH₃-(4-Cl-C₆H₄) | O |
| Ia1.529 | CH₃ | (CH₂)₂CH₃ | CH₂CH(CH₃)-(4-Cl-C₆H₄) | O |
| Ia1.530 | CH₃ | (CH₂)₃CH₃ | CH₂CH(CH₃)-(4-Cl-C₆H₄) | O |
| Ia1.531 | CH₃ | H | CH(CH₃)CH₂-(4-Cl-C₆H₄) | O |
| Ia1.532 | CH₃ | CH₃ | CH(CH₃)CH₂-(4-Cl-C₆H₄) | O |
| Ia1.533 | CH₃ | CH₂CH₃ | CH(CH₃)CH₂-(4-Cl-C₆H₄) | O |
| Ia1.534 | CH₃ | (CH₂)₂CH₃ | CH(CH₃)CH₂-(4-Cl-C₆H₄) | O |
| Ia1.535 | CH₃ | (CH₂)₃CH₃ | CH(CH₃)CH₂-(4-Cl-C₆H₄) | O |
| Ia1.536 | CH₃ | H | CH(CH₃)CH(CH₃)-(4-Cl-C₆H₄) | O |
| Ia1.537 | CH₃ | CH₃ | CH(CH₃)CH(CH₃)-(4-Cl-C₆H₄) | O |
| Ia1.538 | CH₃ | CH₂CH₃ | CH(CH₃)CH(CH₃)-(4-Cl-C₆H₄) | O |
| Ia1.539 | CH₃ | (CH₂)₂CH₃ | CH(CH₃)CH(CH₃)-(4-Cl-C₆H₄) | O |
| Ia1.540 | CH₃ | (CH₂)₃CH₃ | CH(CH₃)CH(CH₃)-(4-Cl-C₆H₄) | O |
| Ia1.541 | CH₃ | H | (CH₂)₂-(2,4-Cl₂-C₆H₃) | O |
| Ia1.542 | CH₃ | CH₃ | (CH₂)₂-(2,4-Cl₂-C₆H₃) | O |
| Ia1.543 | CH₃ | CH₂CH₃ | (CH₂)₂-(2,4-Cl₂-C₆H₃) | O |
| Ia1.544 | CH₃ | (CH₂)₂CH₃ | (CH₂)₂-(2,4-Cl₂-C₆H₃) | O |
| Ia1.545 | CH₃ | (CH₂)₃CH₃ | (CH₂)₂-(2,4-Cl₂-C₆H₃) | O |
| Ia1.546 | CH₃ | H | CH₂CH(CH₃)-(2,4-Cl₂-C₆H₃) | O |
| Ia1.547 | CH₃ | CH₃ | CH₂CH(CH₃)-(2,4-Cl₂-C₆H₃) | O |
| Ia1.548 | CH₃ | CH₂CH₃ | CH₂CH(CH₃)-(2,4-Cl₂-C₆H₃) | O |
| Ia1.549 | CH₃ | (CH₂)₂CH₃ | CH₂CH(CH₃)-(2,4-Cl₂-C₆H₃) | O |
| Ia1.550 | CH₃ | (CH₂)₃CH₃ | CH₂CH(CH₃)-(2,4-Cl₂-C₆H₃) | O |
| Ia1.551 | CH₃ | H | CH(CH₃)CH₂-(2,4-Cl₂-C₆H₃) | O |
| Ia1.552 | CH₃ | CH₃ | CH(CH₃)CH₂-(2,4-Cl₂-C₆H₃) | O |
| Ia1.553 | CH₃ | CH₂CH₃ | CH(CH₃)CH₂-(2,4-Cl₂-C₆H₃) | O |
| Ia1.554 | CH₃ | (CH₂)₂CH₃ | CH(CH₃)CH₂-(2,4-Cl₂-C₆H₃) | O |
| Ia1.555 | CH₃ | (CH₂)₃CH₃ | CH(CH₃)CH₂-(2,4-Cl₂-C₆H₃) | O |
| Ia1.556 | CH₃ | H | CH(CH₃)CH(CH₃)-(2,4-Cl₂-C₆H₃) | O |
| Ia1.557 | CH₃ | CH₃ | CH(CH₃)CH(CH₃)-(2,4-Cl₂-C₆H₃) | O |
| Ia1.558 | CH₃ | CH₂CH₃ | CH(CH₃)CH(CH₃)-(2,4-Cl₂-C₆H₃) | O |
| Ia1.559 | CH₃ | (CH₂)₂CH₃ | CH(CH₃)CH(CH₃)-(2,4-Cl₂-C₆H₃) | O |
| Ia1.560 | CH₃ | (CH₂)₃CH₃ | CH(CH₃)CH(CH₃)-(2,4-Cl₂-C₆H₃) | O |
| Ia1.561 | CH₃ | H | (CH₂)₂-O-C₆H₅ | O |
| Ia1.562 | CH₃ | CH₃ | (CH₂)₂-O-C₆H₅ | O |
| Ia1.563 | CH₃ | CH₂CH₃ | (CH₂)₂-O-C₆H₅ | O |
| Ia1.564 | CH₃ | (CH₂)₂CH₃ | (CH₂)₂-O-C₆H₅ | O |
| Ia1.565 | CH₃ | (CH₂)₃CH₃ | (CH₂)₂-O-C₆H₅ | O |
| la1.566 | CH₃ | H | CH₂CH(CH₃)-O-C₆H₅ | O |
| Ia1.567 | CH₃ | CH₃ | CH₂CH(CH₃)-O-C₆H₅ | O |
| Ia1.568 | CH₃ | CH₂CH₃ | CH₂CH(CH₃)-O-C₆H₅ | O |
| Ia1.569 | CH₃ | (CH₂)₂CH₃ | CH₂CH(CH₃)-O-C₆H₅ | O |
| Ia1.570 | CH₃ | (CH₂)₃CH₃ | CH₂CH(CH₃)-O-C₆H₅ | O |
| Ia1.571 | CH₃ | H | CH(CH₃)CH₂-O-C₆H₅ | O |
| Ia1.572 | CH₃ | CH₃ | CH(CH₃)CH₂-O-C₆H₅ | O |
| Ia1.573 | CH₃ | CH₂CH₃ | CH(CH₃)CH₂-O-C₆H₅ | O |
| Ia1.574 | CH₃ | (CH₂)₂CH₃ | CH(CH₃)CH₂-O-C₆H₅ | O |
| Ia1.575 | CH₃ | (CH₂)₃CH₃ | CH(CH₃)CH₂-O-C₆H₅ | O |
| Ia1.576 | CH₃ | H | CH(CH₃)CH(CH₃)-O-C₆H₅ | O |
| Ia1.577 | CH₃ | CH₃ | CH(CH₃)CH(CH₃)-O-C₆H₅ | O |
| Ia1.578 | CH₃ | CH₂CH₃ | CH(CH₃)CH(CH₃)-O-C₆H₅ | O |
| Ia1.579 | CH₃ | (CH₂)₂CH₃ | CH(CH₃)CH(CH₃)-O-C₆H₅ | O |
| Ia1.580 | CH₃ | (CH₂)₃CH₃ | CH(CH₃)CH(CH₃)-O-C₆H₅ | O |
| Ia1.581 | CH₃ | H | (CH₂)₂-O-(4-Cl-C₆H₄) | O |
| Ia1.582 | CH₃ | CH₃ | (CH₂)₂-O-(4-Cl-C₆H₄) | O |
| Ia1.583 | CH₃ | CH₂CH₃ | (CH₂)₂-O-(4-Cl-C₆H₄) | O |
| Ia1.584 | CH₃ | (CH₂)₂CH₃ | (CH₂)₂-O-(4-Cl-C₆H₄) | O |
| Ia1.585 | CH₃ | (CH₂)₃CH₃ | (CH₂)₂-O-(4-Cl-C₆H₄) | O |
| Ia1.586 | CH₃ | H | CH₂CH(CH₃)-O-(4-Cl-C₆H₄) | O |
| Ia1.587 | CH₃ | CH₃ | CH₂CH(CH₃)-O-(4-Cl-C₆H₄) | O |
| Ia1.588 | CH₃ | CH₂CH₃ | CH₂CH(CH₃)-O-(4-Cl-C₆H₄) | O |
| Ia1.589 | CH₃ | (CH₂)₂CH₃ | CH₂CH(CH₃)-O-(4-Cl-C₆H₄) | O |
| Ia1.590 | CH₃ | (CH₂)₃CH₃ | CH₂CH(CH₃)-O-(4-Cl-C₆H₄) | O |
| Ia1.591 | CH₃ | H | CH(CH₃)CH₂-O-(4-Cl-C₆H₄) | O |
| Ia1.592 | CH₃ | CH₃ | CH(CH₃)CH₂-O-(4-Cl-C₆H₄) | O |
| Ia1.593 | CH₃ | CH₂CH₃ | CH(CH₃)CH₂-O-(4-Cl-C₆H₄) | O |
| Ia1.594 | CH₃ | (CH₂)₂CH₃ | CH(CH₃)CH₂-O-(4-Cl-C₆H₄) | O |
| Ia1.595 | CH₃ | (CH₂)₃CH₃ | CH(CH₃)CH₂-O-(4-Cl-C₆H₄) | O |
| Ia1.596 | CH₃ | H | CH(CH₃)CH(CH₃)-O-(4-Cl-C₆H₄) | O |
| Ia1.597 | CH₃ | CH₃ | CH(CH₃)CH(CH₃)-O-(4-Cl-C₆H₄) | O |
| Ia1.598 | CH₃ | CH₂CH₃ | CH(CH₃)CH(CH₃)-O-(4-Cl-C₆H₄) | O |
| Ia1.599 | CH₃ | (CH₂)₂CH₃ | CH(CH₃)CH(CH₃)-O-(4-Cl-C₆H₄) | O |
| Ia1.600 | CH₃ | (CH₂)₃CH₃ | CH(CH₃)CH(CH₃)-O-(4-Cl-C₆H₄) | O |
| Ia1.601 | CH₃ | H | (CH₂)₂-O-(2,4-Cl₂-C₆H₃) | O |
| Ia1.602 | CH₃ | CH₃ | (CH₂)₂-O-(2,4-Cl₂-C₆H₃) | O |
| Ia1.603 | CH₃ | CH₂CH₃ | (CH₂)₂-O-(2,4-Cl₂-C₆H₃) | O |
| Ia1.604 | CH₃ | (CH₂)₂CH₃ | (CH₂)₂-O-(2,4-Cl₂-C₆H₃) | O |
| Ia1.605 | CH₃ | (CH₂)₃CH₃ | (CH₂)₂-O-(2,4-Cl₂-C₆H₃) | O |
| Ia1.606 | CH₃ | H | CH₂CH(CH₃)-O-(2,4-Cl₂-C₆H₃) | O |
| Ia1.607 | CH₃ | CH₃ | CH₂CH(CH₃)-O-(2,4-Cl₂-C₆H₃) | O |
| Ia1.608 | CH₃ | CH₂CH₃ | CH₂CH(CH₃)-O-(2,4-Cl₂-C₆H₃) | O |
| Ia1.609 | CH₃ | (CH₂)₂CH₃ | CH₂CH(CH₃)-O-(2,4-Cl₂-C₆H₃) | O |
| Ia1.610 | CH₃ | (CH₂)₃CH₃ | CH₂CH(CH₃)-O-(2,4-Cl₂-C₆H₃) | O |
| Ia1.611 | CH₃ | H | CH(CH₃)CH₂-O-(2,4-Cl₂-C₆H₃) | O |
| Ia1.612 | CH₃ | CH₃ | CH(CH₃)CH₂-O-(2,4-Cl₂-C₆H₃) | O |
| Ia1.613 | CH₃ | CH₂CH₃ | CH(CH₃)CH₂-O-(2,4-Cl₂-C₆H₃) | O |
| Ia1.614 | CH₃ | (CH₂)₂CH₃ | CH(CH₃)CH₂-O-(2,4-Cl₂-C₆H₃) | O |
| Ia1.615 | CH₃ | (CH₂)₃CH₃ | CH(CH₃)CH₂-O-(2,4-Cl₂-C₆H₃) | O |
| Ia1.616 | CH₃ | H | CH(CH₃)CH(CH₃)-O-(2,4-Cl₂-C₆H₃) | O |
| Ia1.617 | CH₃ | CH₃ | CH(CH₃)CH(CH₃)-O-(2,4-Cl₂-C₆H₃) | O |
| Ia1.618 | CH₃ | CH₂CH₃ | CH(CH₃)CH(CH₃)-O-(2,4-Cl₂-C₆H₃) | O |
| Ia1.619 | CH₃ | (CH₂)₂CH₃ | CH(CH₃)CH(CH₃)-O-(2,4-Cl₂-C₆H₃) | O |
| Ia1.620 | CH₃ | (CH₂)₃CH₃ | CH(CH₃)CH(CH₃)-O-(2,4-Cl₂-C₆H₃) | O |
| Ia1.621 | CH₃ | H | CH₃ | NH |
| Ia1.622 | CH₃ | CH₃ | CH₃ | NH |
| Ia1.623 | CH₃ | CH₂CH₃ | CH₃ | NH |
| Ia1.624 | CH₃ | (CH₂)₂CH₃ | CH₃ | NH |
| Ia1.625 | CH₃ | (CH₂)₃CH₃ | CH₃ | NH |
| Ia1.626 | CH₃ | H | C₂H₅ | NH |
| Ia1.627 | CH₃ | CH₃ | C₂H₅ | NH |
| Ia1.628 | CH₃ | CH₂CH₃ | C₂H₅ | NH |
| Ia1.629 | CH₃ | (CH₂)₂CH₃ | C₂H₅ | NH |
| Ia1.630 | CH₃ | (CH₂)₃CH₃ | C₂H₅ | NH |
| Ia1.631 | CH₃ | H | (CH₂)₂CH₃ | NH |
| Ia1.632 | CH₃ | CH₃ | (CH₂)₂CH₃ | NH |
| Ia1.633 | CH₃ | CH₂CH₃ | (CH₂)₂CH₃ | NH |
| Ia1.634 | CH₃ | (CH₂)₂CH₃ | (CH₂)₂CH₃ | NH |
| Ia1.635 | CH₃ | (CH₂)₃CH₃ | (CH₂)₂CH₃ | NH |
| Ia1.636 | CH₃ | H | CH(CH₃)₂ | NH |
| Ia1.637 | CH₃ | CH₃ | CH(CH₃)₂ | NH |
| Ia1.638 | CH₃ | CH₂CH₃ | CH(CH₃)₂ | NH |
| Ia1.639 | CH₃ | (CH₂)₂CH₃ | CH(CH₃)₂ | NH |
| Ia1.640 | CH₃ | (CH₂)₃CH₃ | CH(CH₃)₂ | NH |
| Ia1.641 | CH₃ | H | (CH₂)₃CH₃ | NH |
| Ia1.642 | CH₃ | CH₃ | (CH₂)₃CH₃ | NH |
| Ia1.643 | CH₃ | CH₂CH₃ | (CH₂)₃CH₃ | NH |
| Ia1.644 | CH₃ | (CH₂)₂CH₃ | (CH₂)₃CH₃ | NH |
| Ia1.645 | CH₃ | (CH₂)₃CH₃ | (CH₂)₃CH₃ | NH |
| Ia1.646 | CH₃ | H | CH₂CH(CH₃)₂ | NH |
| Ia1.647 | CH₃ | CH₃ | CH₂CH(CH₃)₂ | NH |
| Ia1.648 | CH₃ | CH₂CH₃ | CH₂CH(CH₃)₂ | NH |
| Ia1.649 | CH₃ | (CH₂)₂CH₃ | CH₂CH(CH₃)₂ | NH |
| Ia1.650 | CH₃ | (CH₂)₃CH₃ | CH₂CH(CH₃)₂ | NH |
| Ia1.651 | CH₃ | H | CH(CH₃)CH₂CH₃ | NH |
| Ia1.652 | CH₃ | CH₃ | CH(CH₃)CH₂CH₃ | NH |
| Ia1.653 | CH₃ | CH₂CH₃ | CH(CH₃)CH₂CH₃ | NH |
| Ia1.654 | CH₃ | (CH₂)₂CH₃ | CH(CH₃)CH₂CH₃ | NH |
| Ia1.655 | CH₃ | (CH₂)₃CH₃ | CH(CH₃)CH₂CH₃ | NH |
| Ia1.656 | CH₃ | H | (CH₂)₂-C₆H₅ | NH |
| Ia1.657 | CH₃ | CH₃ | (CH₂)₂-C₆H₅ | NH |
| Ia1.658 | CH₃ | CH₂CH₃ | (CH₂)₂-C₆H₅ | NH |
| Ia1.659 | CH₃ | (CH₂)₂CH₃ | (CH₂)₂-C₆H₅ | NH |
| Ia1.660 | CH₃ | (CH₂)₃CH₃ | (CH₂)₂-C₆R₅ | NH |
| Ia1.661 | CH₃ | H | CH₂CH(CH₃)-C₆H₅ | NH |
| Ia1.662 | CH₃ | CH₃ | CH₂CH(CH₃)-C₆H₅ | NH |
| Ia1.663 | CH₃ | CH₂CH₃ | CH₂CH(CH₃)-C₆H₅ | NH |
| Ia1.664 | CH₃ | (CH₂)₂CH₃ | CH₂CH(CH₃)-C₆H₅ | NH |
| Ia1.665 | CH₃ | (CH₂)₃CH₃ | CH₂CH(CH₃)-C₆H₅ | NH |
| Ia1.666 | CH₃ | H | CH(CH₃)CH₂-C₆H₅ | NH |
| Ia1.667 | CH₃ | CH₃ | CH(CH₃)CH₂-C₆H₅ | NH |
| Ia1.668 | CH₃ | CH₂CH₃ | CH(CH₃)CH₂-C₆H₅ | NH |
| Ia1.669 | CH₃ | (CH₂)₂CH₃ | CH(CH₃)CH₂-C₆H₅ | NH |
| Ia1.670 | CH₃ | (CH₂)₃CH₃ | CH(CH₃)CH₂-C₆H₅ | NH |
| Ia1.671 | CH₃ | H | CH(CH₃)CH(CH₃)-C₆H₅ | NH |
| Ia1.672 | CH₃ | CH₃ | CH(CH₃)CH(CH₃)-C₆H5 | NH |
| Ia1.673 | CH₃ | CH₂CH₃ | CH(CH₃)CH(CH₃)-C₆H₅ | NH |
| Ia1.674 | CH₃ | (CH₂)₂CH₃ | CH(CH₃)CH(CH₃)-C₆H₅ | NH |
| Ia1.675 | CH₃ | (CH₂)₃CH₃ | CH(CH₃)CH(CH₃)-C₆H₅ | NH |
| Ia1.676 | CH₃ | H | (CH₂)₂-(4-Cl-C₆H₄) | NH |
| Ia1.677 | CH₃ | CH₃ | (CH₂)₂-(4-Cl-C₆H₄) | NH |
| Ia1.678 | CH₃ | CH₂CH₃ | (CH₂)₂-(4-Cl-C₆H₄) | NH |
| Ia1.679 | CH₃ | (CH₂)₂CH₃ | (CH₂)₂- (4-Cl-C₆H₄) | NH |
| Ia1.680 | CH₃ | (CH₂)₃CH₃ | (CH₂)₂-(4-Cl-C₆H₄) | NH |
| Ia1.681 | CH₃ | H | CH₂CH(CH₃)-(4-Cl-C₆H₄) | NH |
| Ia1.682 | CH₃ | CH₃ | CH₂CH(CH₃)-(4-Cl-C₆H₄) | NH |
| Ia1.683 | CH₃ | CH₂CH₃ | CH₂CH(CH₃)-(4-Cl-C₆H₄) | NH |
| Ia1.684 | CH₃ | (CH₂)₂CH₃ | CH₂CH(CH₃)-(4-Cl-C₆H₄) | NH |
| Ia1.685 | CH₃ | (CH₂)₃CH₃ | CH₂CH(CH₃)-(4-Cl-C₆H₄) | NH |
| Ia1.686 | CH₃ | H | CH(CH₃)CH₂-(4-Cl-C₆H₄) | NH |
| Ia1.687 | CH₃ | CH₃ | CH(CH₃)CH₂-(4-Cl-C₆H₄) | NH |
| Ia1.688 | CH₃ | CH₂CH₃ | CH(CH₃)CH₂-(4-Cl-C₆H₄) | NH |
| Ia1.689 | CH₃ | (CH₂)₂CH₃ | CH(CH₃)CH₂-(4-Cl-C₆H₄) | NH |
| Ia1.690 | CH₃ | (CH₂)₃CH₃ | CH(CH₃)CH₂-(4-Cl-C₆H₄) | NH |
| Ia1.691 | CH₃ | H | CH(CH₃)CH(CH₃)-(4-Cl-C₆H₄) | NH |
| Ia1.692 | CH₃ | CH₃ | CH(CH₃)CH(CH₃)-(4-Cl-C₆H₄) | NH |
| Ia1.693 | CH₃ | CH₂CH₃ | CH(CH₃)CH(CH₃)-(4-Cl-C₆H₄) | NH |
| Ia1.694 | CH₃ | (CH₂)₂CH₃ | CH(CH₃)CH(CH₃)-(4-Cl-C₆H₄) | NH |
| Ia1.695 | CH₃ | (CH₂)₃CH₃ | CH(CH₃)CH(CH₃)-(4-Cl-C₆H₄) | NH |
| Ia1.696 | CH₃ | H | (CH₂)₂-(2,4-Cl₂-C₆H₃) | NH |
| Ia1.697 | CH₃ | CH₃ | (CH₂)₂-(2,4-Cl₂-C₆H₃) | NH |
| Ia1.698 | CH₃ | CH₂CH₃ | (CH₂)₂-(2,4-Cl₂-C₆H₃) | NH |
| Ia1.699 | CH₃ | (CH₂)₂CH₃ | CH₂-(2,4-Cl₂-C₆H₃) | NH |
| Ia1.700 | CH₃ | (CH₂)₃CH₃ | CH₂-(2,4-Cl₂-C₆H₃) | NH |
| Ia1.701 | CH₃ | H | CH₂CH(CH₃)-(2,4-Cl₂-C₆H₃) | NH |
| Ia1.702 | CH₃ | CH₃ | CH₂CH(CH₃)-(2,4-Cl₂-C₆H₃) | NH |
| Ia1.703 | CH₃ | CH₂CH₃ | CH₂CH(CH₃)-(2,4-Cl₂-C₆H₃) | NH |
| Ia1.704 | CH₃ | (CH₂)₂CH₃ | CH₂CH(CH₃)-(2,4-Cl₂-C₆H₃) | NH |
| Ia1.705 | CH₃ | (CH₂)₃CH₃ | CH₂CH(CH₃)-(2,4-Cl₂-C₆H₃) | NH |
| Ia1.706 | CH₃ | H | CH(CH₃CH₂)-(2,4-Cl₂-C₆H₃) | NH |
| Ia1.707 | CH₃ | CH₃ | CH(CH₃CH₂)-(2,4-Cl₂-C₆H₃) | NH |
| Ia1.708 | CH₃ | CH₂CH₃ | CH(CH₃CH₂)-(2,4-Cl₂-C₆H₃) | NH |
| Ia1.709 | CH₃ | (CH₂)₂CH₃ | CH(CH₃CH₂)-(2,4-Cl₂-C₆H₃) | NH |
| Ia1.710 | CH₃ | (CH₂)₃CH₃ | CH(CH₃CH₂)-(2,4-Cl₂-C₆H₃) | NH |
| Ia1.711 | CH₃ | H | CH(CH₃)-(2,4-Cl₂-C₆H₃) | NH |
| Ia1.712 | CH₃ | CH₃ | CH(CH₃)-(2,4-Cl₂-C₆H₃) | NH |
| Ia1.713 | CH₃ | CH₂CH₃ | CH(CH₃)-(2,4-Cl₂-C₆H₃) | NH |
| Ia1.714 | CH₃ | (CH₂)₂CH₃ | CH(CH₃)-(2,4-Cl₂-C₆H₃) | NH |
| Ia1.715 | CH₃ | (CH₂)₃CH₃ | CH(CH₃)-(2,4-Cl₂-C₆H₃) | NH |
| Ia1.716 | CH₃ | H | (CH₂)₂-O-C₆H₅ | NH |
| Ia1.717 | CH₃ | CH₃ | (CH₂)₂-O-C₆H₅ | NH |
| Ia1.718 | CH₃ | CH₂CH₃ | (CH₂)₂-O-C₆H₅ | NH |
| Ia1.719 | CH₃ | (CH₂)₂CH₃ | (CH₂)₂-O-C₆H₅ | NH |
| Ia1.720 | CH₃ | (CH₂)₃CH₃ | (CH₂)₂-O-C₆H₅ | NH |
| Ia1.721 | CH₃ | H | CH₂CH(CH₃)-O-C₆H₅ | NH |
| Ia1.722 | CH₃ | CH₃ | CH₂CH(CH₃)-O-C₆H₅ | NH |
| Ia1.723 | CH₃ | CH₂CH₃ | CH₂CH(CH₃)-O-C₆H₅ | NH |
| Ia1.724 | CH₃ | (CH₂)₂CH₃ | CH₂CH(CH₃)-O-C₆H₅ | NH |
| Ia1.725 | CH₃ | (CH₂)₃CH₃ | CH₂CH(CH₃)-O-C₆H₅ | NH |
| Ia1.726 | CH₃ | H | CH(CH₃)CH₂-O-C₆H₅ | NH |
| Ia1.727 | CH₃ | CH₃ | CH(CH₃)CH₂-O-C₆H₅ | NH |
| Ia1.728 | CH₃ | CH₂CH₃ | CH(CH₃)CH₂-O-C₆H₅ | NH |
| Ia1.729 | CH₃ | (CH₂)₂CH₃ | CH(CH₃)CH₂-O-C₆H₅ | NH |
| Ia1.730 | CH₃ | (CH₂)₃CH₃ | CH(CH₃)CH₂-O-C₆H₅ | NH |
| Ia1.731 | CH₃ | H | CH(CH₃)CH(CH₃)-O-C₆H₅ | NH |
| Ia1.732 | CH₃ | CH₃ | CH(CH₃)CH(CH₃)-O-C₆H₅ | NH |
| Ia1.733 | CH₃ | CH₂CH₃ | CH(CH₃)CH(CH₃)-O-C₆H₅ | NH |
| Ia1.734 | CH₃ | (CH₂)₂CH₃ | CH(CH₃)CH(CH₃)-O-C₆H₅ | NH |
| Ia1.735 | CH₃ | (CH₂)₃CH₃ | CH(CH₃)CH(CH₃)-O-C₆H₅ | NH |
| Ia1.736 | CH₃ | H | (CH₂)₂-O-(4-Cl-C₆H₄) | NH |
| Ia1.737 | CH₃ | CH₃ | (CH₂)₂-O-(4-Cl-C₆H₄) | NH |
| Ia1.738 | CH₃ | CH₂CH₃ | (CH₂)₂-O-(4-Cl-C₆H₄) | NH |
| Ia1.739 | CH₃ | (CH₂)₂CH₃ | (CH₂)₂-O-(4-Cl-C₆H₄) | NH |
| Ia1.740 | CH₃ | (CH₂)₃CH₃ | (CH₂)₂-O-(4-Cl-C₆H₄) | NH |
| Ia1.741 | CH₃ | H | CH₂CH(CH₃)-O-(4-Cl-C₆H₄) | NH |
| Ia1.742 | CH₃ | CH₃ | CH₂CH(CH₃)-O-(4-Cl-C₆H₄) | NH |
| Ial.743 | CH₃ | CH₂CH₃ | CH₂CH(CH₃)-O-(4-Cl-C₆H₄) | NH |
| Ia1.744 | CH₃ | (CH₂)₂CH₃ | CH₂CH(CH₃)-O-(4-Cl-C₆H₄) | NH |
| Ia1.745 | CH₃ | (CH₂)₃CH₃ | CH₂CH(CH₃)-O-(4-Cl-C₆H₄) | NH |
| Ia1.746 | CH₃ | H | CH(CH₃)CH₂-O-(4-Cl-C₆H₄) | NH |
| Ia1.747 | CH₃ | CH₃ | CH(CH₃)CH₂-O-(4-Cl-C₆H₄) | NH |
| Ia1.748 | CH₃ | CH₂CH₃ | CH(CH₃)CH₂-O-(4-Cl-C₆H₄) | NH |
| Ia1.749 | CH₃ | (CH₂)₂CH₃ | CH(CH₃)CH₂-O-(4-Cl-C₆H₄) | NH |
| Ia1.750 | CH₃ | (CH₂)₃CH₃ | CH(CH₃)CH₂-O-(4-Cl-C₆H₄) | NH |
| Ia1.751 | CH₃ | H | CH(CH₃)CH(CH₃)-O-(4-Cl-C₆H₄) | NH |
| Ia1.752 | CH₃ | CH₃ | CH(CH₃)CH(CH₃)-O-(4-Cl-C₆H₄) | NH |
| Ia1.753 | CH₃ | CH₂CH₃ | CH(CH₃)CH(CH₃)-O-(4-Cl-C₆H₄) | NH |
| Ia1.754 | CH₃ | (CH₂)₂CH₃ | CH(CH₃)CH(CH₃)-O-(4-Cl-C₆H₄) | NH |
| Ia1.755 | CH₃ | (CH₂)₃CH₃ | CH(CH₃)CH(CH₃)-O-(4-Cl-C₆H₄) | NH |
| Ia1.756 | CH₃ | H | (CH₂)₂-O-(2,4-Cl₂-C₆H₃) | NH |
| Ia1.757 | CH₃ | CH₃ | (CH₂)₂-O-(2,4-Cl₂-C₆H₃) | NH |
| Ia1.758 | CH₃ | CH₂CH₃ | (CH₂)₂-O-(2,4-Cl₂-C₆H₃) | NH |
| Ia1.759 | CH₃ | (CH₂)₂CH₃ | (CH₂)₂-O-(2,4-Cl₂-C₆H₃) | NH |
| Ia1.760 | CH₃ | (CH₂)₃CH₃ | (CH₂)₂-O-(2,4-Cl₂-C₆H₃) | NH |
| Ia1.761 | CH₃ | H | CH₂CH(CH₃)-O-(2,4-Cl₂-C₆H₃) | NH |
| Ia1.762 | CH₃ | CH₃ | CH₂CH(CH₃)-O-(2,4-Cl₂-C₆H₃) | NH |
| Ia1.763 | CH₃ | CH₂CH₃ | CH₂CH(CH₃)-O-(2,4-Cl₂-C₆H₃) | NH |
| Ia1.764 | CH₃ | (CH₂)₂CH₃ | CH₂CH(CH₃)-O-(2,4-Cl₂-C₆H₃) | NH |
| Ia1.765 | CH₃ | (CH₂)₃CH₃ | CH₂CH(CH₃)-O-(2,4-Cl₂-C₆H₃) | NH |
| Ia1.766 | CH₃ | H | CH(CH₃)CH₂-O-(2,4-Cl₂-C₆H₃) | NH |
| Ia1.767 | CH₃ | CH₃ | CH(CH₃)CH₂-O-(2,4-Cl₂-C₆H₃) | NH |
| Ia1.768 | CH₃ | CH₂CH₃ | CH(CH₃)CH₂-O-(2,4-Cl₂-C₆H₃) | NH |
| Ia1.769 | CH₃ | (CH₂)₂CH₃ | CH(CH₃)CH₂-O-(2,4-Cl₂-C₆H₃) | NH |
| Ia1.770 | CH₃ | (CH₂)₃CH₃ | CH(CH₃)CH₂-O-(2,4-Cl₂-C₆H₃) | NH |
| Ia1.771 | CH₃ | H | CH(CH₃)CH(CH₃)-O-(2,4-Cl₂-C₆H₃) | NH |
| Ia1.772 | CH₃ | CH₃ | CH(CH₃)CH(CH₃)-O-(2,4-Cl₂-C₆H₃) | NH |
| Ia1.773 | CH₃ | CH₂CH₃ | CH(CH₃)CH(CH₃)-O-(2,4-Cl₂-C₆H₃) | NH |
| Ia1.774 | CH₃ | (CH₂)₂CH₃ | CH(CH₃)CH(CH₃)-O-(2,4-Cl₂-C₆H₃) | NH |
| Ia1.775 | CH₃ | (CH₂)₃CH₃ | CH(CH₃)CH(CH₃)-O-(2,4-Cl₂-C₆H₃) | NH |
| Ia1.776 | CH₃ | H | CH₃ | NCH₃ |
| Ia1.777 | CH₃ | CH₃ | CH₃ | NCH₃ |
| Ia1.778 | CH₃ | CH₂CH₃ | CH₃ | NCH₃ |
| Ia1.779 | CH₃ | (CH₂)₂CH₃ | CH₃ | NCH₃ |
| Ia1.780 | CH₃ | (CH₂)₃CH₃ | CH₃ | NCH₃ |
| Ia1.781 | CH₃ | H | C₂H₅ | NCH₃ |
| Ia1.782 | CH₃ | CH₃ | C₂H₅ | NCH₃ |
| Ia1.783 | CH₃ | CH₂CH₃ | C₂H₅ | NCH₃ |
| Ia1.784 | CH₃ | (CH₂)₂CH₃ | C₂H₅ | NCH₃ |
| Ia1.785 | CH₃ | (CH₂)₃CH₃ | C₂H₅ | NCH₃ |
| Ia1.786 | CH₃ | H | (CH₂)₂CH₃ | NCH₃ |
| Ia1.787 | CH₃ | CH₃ | (CH₂)₂CH₃ | NCH₃ |
| Ia1.788 | CH₃ | CH₂CH₃ | (CH₂)₂CH₃ | NCH₃ |
| Ia1.789 | CH₃ | (CH₂)₂CH₃ | (CH₂)₂CH₃ | NCH₃ |
| Ia1.790 | CH₃ | (CH₂)₃CH₃ | (CH₂)₂CH₃ | NCH₃ |
| Ia1.791 | CH₃ | H | CH(CH₃)₂ | NCH₃ |
| Ia1.792 | CH₃ | CH₃ | CH(CH₃)₂ | NCH₃ |
| Ia1.793 | CH₃ | CH₂CH₃ | CH(CH₃)₂ | NCH₃ |
| Ia1.794 | CH₃ | (CH₂)₂CH₃ | CH(CH₃)₂ | NCH₃ |
| Ia1.795 | CH₃ | (CH₂)₃CH₃ | CH(CH₃)₂ | NCH₃ |
| Ia1.796 | CH₃ | H | (CH₂)₃CH₃ | NCH₃ |
| Ia1.797 | CH₃ | CH₃ | (CH₂)₃CH₃ | NCH₃ |
| Ia1.798 | CH₃ | CH₂CH₃ | (CH₂)₃CH₃ | NCH₃ |
| Ia1.799 | CH₃ | (CH₂)₂CH₃ | (CH₂)₃CH₃ | NCH₃ |
| Ia1.800 | CH₃ | (CH₂)₃CH₃ | (CH₂)₃CH₃ | NCH₃ |
| Ia1.801 | CH₃ | H | CH₂CH(CH₃)₂ | NCH₃ |
| Ia1.802 | CH₃ | CH₃ | CH₂CH(CH₃)₂ | NCH₃ |
| Ia1.803 | CH₃ | CH₂CH₃ | CH₂CH(CH₃)₂ | NCH₃ |
| Ia1.804 | CH₃ | (CH₂)₂CH₃ | CH₂CH(CH₃)₂ | NCH₃ |
| Ia1.805 | CH₃ | (CH₂)₃CH₃ | CH₂CH(CH₃)₂ | NCH₃ |
| Ia1.806 | CH₃ | H | CH(CH₃)CH₂CH₃ | NCH₃ |
| Ia1.807 | CH₃ | CH₃ | CH(CH₃)CH₂CH₃ | NCH₃ |
| Ia1.808 | CH₃ | CH₂CH₃ | CH(CH₃)CH₂CH₃ | NCH₃ |
| Ia1.809 | CH₃ | (CH₂)₂CH₃ | CH(CH₃)CH₂CH₃ | NCH₃ |
| Ia1.810 | CH₃ | (CH₂)₃CH₃ | CH(CH₃)CH₂CH₃ | NCH₃ |
| Ia1.811 | CH₃ | H | (CH₂)₂-C₆H₅ | NCH₃ |
| Ia1.812 | CH₃ | CH₃ | (CH₂)₂-C₆H₅ | NCH₃ |
| Ia1.813 | CH₃ | CH₂CH₃ | (CH₂)₂-C₆H₅ | NCH₃ |
| Ia1.814 | CH₃ | (CH₂)₂CH₃ | (CH₂)₂-C₆H₅ | NCH₃ |
| Ia1.815 | CH₃ | (CH₂)₃CH₃ | (CH₂)₂-C₆H₅ | NCH₃ |
| Ia1.816 | CH₃ | H | CH₂CH(CH₃)-C₆H₅ | NCH₃ |
| Ia1.817 | CH₃ | CH₃ | CH₂CH(CH₃)-C₆H₅ | NCH₃ |
| Ia1.818 | CH₃ | CH₂CH₃ | CH₂CH(CH₃)-C₆H₅ | NCH₃ |
| Ia1.819 | CH₃ | (CH₂)₂CH₃ | CH₂CH(CH₃)-C₆H₃ | NCH₃ |
| Ia1.820 | CH₃ | (CH₂)₃CH₃ | CH₂CH(CH₃)-C₆H₅ | NCH₃ |
| Ia1.821 | CH₃ | H | CH(CH₃)CH₂-C₆H₅ | NCH₃ |
| Ia1.822 | CH₃ | CH₃ | CH(CH₃)CH₂-C₆H₅ | NCH₃ |
| Ia1.823 | CH₃ | CH₂CH₃ | CH(CH₃)CH₂-C₆H₅ | NCH₃ |
| Ia1.824 | CH₃ | (CH₂)₂CH₃ | CH(CH₃)CH₂-C₆H₅ | NCH₃ |
| Ia1.825 | CH₃ | (CH₂)₃CH₃ | CH(CH₃)CH₂-C₆H₅ | NCH₃ |
| Ia1.826 | CH₃ | H | CH(CH₃)CH(CH₃)-C₆H₅ | NCH₃ |
| Ia1.827 | CH₃ | CH₃ | CH(CH₃)CH(CH₃)-C₆H₅ | NCH₃ |
| Ia1.828 | CH₃ | CH₂CH₃ | CH(CH₃)CH(CH₃)-C₆H₅ | NCH₃ |
| Ia1.829 | CH₃ | (CH₂)₂CH₃ | CH(CH₃)CH(CH₃)-C₆H₅ | NCH₃ |
| Ia1.830 | CH₃ | (CH₂)₃CH₃ | CH(CH₃)CH(CH₃)-C₆H₅ | NCH₃ |
| Ia1.831 | CH₃ | H | (CH₂)₂-(4-Cl-C₆H₄) | NCH₃ |
| Ia1.832 | CH₃ | CH₃ | (CH₂)₂-(4-Cl-C₆H₄) | NCH₃ |
| Ia1.833 | CH₃ | CH₂CH₃ | (CH₂)₂-(4-Cl-C₆H₄) | NCH₃ |
| Ia1.834 | CH₃ | (CH₂)₂CH₃ | (CH₂)₂-(4-Cl-C₆H₄) | NCH₃ |
| Ia1.835 | CH₃ | (CH₂)₃CH₃ | (CH₂)₂-(4-Cl-C₆H₄) | NCH₃ |
| Ia1.836 | CH₃ | H | CH₂CH(CH₃)-(4-Cl-C₆H₄) | NCH₃ |
| Ia1.837 | CH₃ | CH₃ | CH₂CH(CH₃)-(4-Cl-C₆H₄) | NCH₃ |
| Ia1.838 | CH₃ | CH₂CH₃ | CH₂CH(CH₃)-(4-Cl-C₆H₄) | NCH₃ |
| Ia1.839 | CH₃ | (CH₂)₂CH₃ | CH₂CH(CH₃)-(4-Cl-C₆H₄) | NCH₃ |
| Ia1.840 | CH₃ | (CH₂)₃CH₃ | CH₂CH(CH₃)-(4 -Cl-C₆H₄) | NCH₃ |
| Ia1.841 | CH₃ | H | CH(CH₃)CH₂-(4-Cₗ-C₆H₄) | NCH₃ |
| Ia1.842 | CH₃ | CH₃ | CH(CH₃)CH₂-(4-Cl-C₆H₄) | NCH₃ |
| Ia1.843 | CH₃ | CH₂CH₃ | CH(CH₃)CH₂-(4-Cl-C₆H₄) | NCH₃ |
| Ia1.844 | CH₃ | (CH₂)₂CH₃ | CH(CH₃)CH₂-(4-Cl-C₆H₄) | NCH₃ |
| Ia1.845 | CH₃ | (CH₂)₃CH₃ | CH(CH₃)CH₂-(4-Cl-C₆H₄) | NCH₃ |
| Ia1.846 | CH₃ | H | CH(CH₃)CH(CH₃)-(4-Cl-C₆H₄) | NCH₃ |
| Ia1.847 | CH₃ | CH₃ | CH(CH₃)CH(CH₃)-(4-Cl-C₆H₄) | NCH₃ |
| Ia1.848 | CH₃ | CH₂CH₃ | CH(CH₃)CH(CH₃)-(4-Cl-C₆H₄) | NCH₃ |
| Ia1.849 | CH₃ | (CH₂)₂CH₃ | CH(CH₃)CH(CH₃)-(4-Cl-C₆H₄) | NCH₃ |
| Ia1.850 | CH₃ | (CH₂)₃CH₃ | CH(CH₃)CH(CH₃)-(4-Cl-C₆H₄) | NCH₃ |
| Ia1.851 | CH₃ | H | (CH₂)₂-(2,4-Cl₂-C₆H₃) | NCH₃ |
| Ia1.852 | CH₃ | CH₃ | (CH₂)₂-(2,4-Cl₂-C₆H₃) | NCH₃ |
| Ia1.853 | CH₃ | CH₂CH₃ | (CH₂)₂-(2,4-Cl₂-C₆H₃) | NCH₃ |
| Ia1.854 | CH₃ | (CH₂)₂CH₃ | (CH₂)₂-(2,4-Cl₂-C₆H₃) | NCH₃ |
| Ia1.855 | CH₃ | (CH₂)₃CH₃ | (CH₂)₂-(2,4-Cl₂-C₆H₃) | NCH₃ |
| Ia1.856 | CH₃ | H | CH₂CH(CH₃)-(2,4-Cl₂-C₆H₃) | NCH₃ |
| Ia1.857 | CH₃ | CH₃ | CH₂CH(CH₃)-(2,4-Cl₂-C₆H₃) | NCH₃ |
| Ia1.858 | CH₃ | CH₂CH₃ | CH₂CH(CH₃)-(2,4-Cl₂-C₆H₃) | NCH₃ |
| Ia1.859 | CH₃ | (CH₂)₂CH₃ | CH₂CH(CH₃)-(2,4-Cl₂-C₆H₃) | NCH₃ |
| Ia1.860 | CH₃ | (CH₂)₃CH₃ | CH₂CH(CH₃)-(2,4-Cl₂-C₆H₃) | NCH₃ |
| Ia1.861 | CH₃ | H | CH(CH₃)CH₂-(2,4-Cl₂-C₆H₃) | NCH₃ |
| Ia1.862 | CH₃ | CH₃ | CH(CH₃)CH₂-(2,4-Cl₂-C₆H₃) | NCH₃ |
| Ia1.863 | CH₃ | CH₂CH₃ | CH(CH₃)CH₂-(2,4-Cl₂-C₆H₃) | NCH₃ |
| Ia1.864 | CH₃ | (CH₂)₂CH₃ | CH(CH₃)CH₂-(2,4-Cl₂-C₆H₃) | NCH₃ |
| Ia1.865 | CH₃ | (CH₂)₃CH₃ | CH(CH₃)CH₂-(2,4-Cl₂-C₆H₃) | NCH₃ |
| Ia1.866 | CH₃ | H | CH(CH₃)CH(CH₃)-(2,4-Cl₂-C₆H₃) | NCH₃ |
| Ia1.867 | CH₃ | CH₃ | CH(CH₃)CH(CH₃)-(2,4-Cl₂-C₆H₃) | NCH₃ |
| Ia1.868 | CH₃ | CH₂CH₃ | CH(CH₃)CH(CH₃)-(2,4-Cl₂-C₆H₃) | NCH₃ |
| Ia1.869 | CH₃ | (CH₂)₂CH₃ | CH(CH₃)CH(CH₃)-(2,4-Cl₂-C₆H₃) | NCH₃ |
| Ia1.870 | CH₃ | (CH₂)₃CH₃ | CH(CH₃)CH(CH₃)-(2,4-Cl₂-C₆H₃) | NCH₃ |
| Ia1.871 | CH₃ | H | (CH₂)₂-O-C₆H₅ | NCH₃ |
| Ia1.872 | CH₃ | CH₃ | (CH₂)₂-O-C₆H₅ | NCH₃ |
| Ia1.873 | CH₃ | CH₂CH₃ | (CH₂) ₂-O-C₆H₅ | NCH₃ |
| Ia1.874 | CH₃ | (CH₂)₂CH₃ | (CH₂)₂-O-C₆H₅ | NCH₃ |
| Ia1.875 | CH₃ | (CH₂)₃CH₃ | (CH₂)₂-O-C₆H₅ | NCH₃ |
| Ia1.876 | CH₃ | H | CH₂CH(CH₃)-O-C₆H₅ | NCH₃ |
| Ia1.877 | CH₃ | CH₃ | CH₂CH(CH₃)-O-C₆H₅ | NCH₃ |
| Ia1.878 | CH₃ | CH₂CH₃ | CH₂CH(CH₃)-O-C₆H₅ | NCH₃ |
| Ia1.879 | CH₃ | (CH₂)₂CH₃ | CH₂CH(CH₃)-O-C₆H₅ | NCH₃ |
| Ia1.880 | CH₃ | (CH₂)₃CH₃ | CH₂CH(CH₃)-O-C₆H₅ | NCH₃ |
| Ia1.881 | CH₃ | H | CH(CH₃)CH₂-O-C₆H₅ | NCH₃ |
| Ia1.882 | CH₃ | CH₃ | CH(CH₃)CH₂-O-C₆H₅ | NCH₃ |
| Ia1.883 | CH₃ | CH₂CH₃ | CH(CH₃) CH₂-O-C₆H₅ | NCH₃ |
| Ia1.884 | CH₃ | (CH₂)₂CH₃ | CH(CH₃)CH₂-O-C₆H₅ | NCH₃ |
| Ia1.885 | CH₃ | (CH₂)₃CH₃ | CH(CH₃)CH₂-O-C₆H₅ | NCH₃ |
| Ia1.886 | CH₃ | H | CH(CH₃)CH(CH₃)-O-C₆H₅ | NCH₃ |
| Ia1.887 | CH₃ | CH₃ | CH(CH₃)CH(CH₃)-O-C₆H₅ | NCH₃ |
| Ia1.888 | CH₃ | CH₂CH₃ | CH(CH₃)CH(CH₃)-O-C₆H₅ | NCH₃ |
| Ia1.889 | CH₃ | (CH₂)₂CH₃ | CH(CH₃)CH(CH₃)-O-C₆H₅ | NCH₃ |
| Ia1.890 | CH₃ | (CH₂)₃CH₃ | CH(CH₃)CH(CH₃)-O-C₆H₅ | NCH₃ |
| Ia1.891 | CH₃ | H | (CH₂)₂-O-(4-Cl-C₆H₄) | NCH₃ |
| Ia1.892 | CH₃ | CH₃ | (CH₂)₂-O-(4-Cl-C₆H₄) | NCH₃ |
| Ia1.893 | CH₃ | CH₂CH₃ | (CH₂)₂-O-(4-Cl-C₆H₄) | NCH₃ |
| Ia1.894 | CH₃ | (CH₂)₂CH₃ | (CH₂)₂-O-(4-Cl-C₆H₄) | NCH₃ |
| Ia1.895 | CH₃ | (CH₂)₃CH₃ | (CH₂)₂-O-(4-Cl-C₆H₄) | NCH₃ |
| Ia1.896 | CH₃ | H | CH₂CH(CH₃)-O-(4-Cl-C₆H₄) | NCH₃ |
| Ia1.897 | CH₃ | CH₃ | CH₂CH(CH₃)-O-(4-Cl-C₆H₄) | NCH₃ |
| Ia1.898 | CH₃ | CH₂CH₃ | CH₂CH(CH₃)-O-(4-Cl-C₆H₄) | NCH₃ |
| Ia1.899 | CH₃ | (CH₂)₂CH₃ | CH₂CH(CH₃)-O-(4-Cl-C₆H₄) | NCH₃ |
| Ia1.900 | CH₃ | (CH₂)₃CH₃ | CH₂CH(CH₃)-O-(4-Cl-C₆H₄) | NCH₃ |
| Ia1.901 | CH₃ | H | CH(CH₃)CH₂-O-(4-Cl-C₆H₄) | NCH₃ |
| Ia1.902 | CH₃ | CH₃ | CH(CH₃)CH₂-O-(4-Cl-C₆H₄) | NCH₃ |
| Ia1.903 | CH₃ | CH₂CH₃ | CH(CH₃)CH₂-O-(4-Cl-C₆H₄) | NCH₃ |
| Ia1.904 | CH₃ | (CH₂)₂CH₃ | CH(CH₃)CH₂-O-(4-Cl-C₆H₄) | NCH₃ |
| Ia1.905 | CH₃ | (CH₂)₃CH₃ | CH(CH₃)CH₂-O-(4-Cl-C₆H₄) | NCH₃ |
| Ia1.906 | CH₃ | H | CH(CH₃)CH(CH₃)-O-(4-Cl-C₆H₄) | NCH₃ |
| Ia1.907 | CH₃ | CH₃ | CH(CH₃)CH(CH₃)-O-(4-Cl-C₆H₄) | NCH₃ |
| Ia1.908 | CH₃ | CH₂CH₃ | CH(CH₃)CH(CH₃)-O-(4-Cl-C₆H₄) | NCH₃ |
| Ia1.909 | CH₃ | (CH₂)₂CH₃ | CH(CH₃)CH(CH₃)-O-(4-Cl-C₆H₄) | NCH₃ |
| Ia1.910 | CH₃ | (CH₂)₃CH₃ | CH(CH₃)CH(CH₃)-O-(4-Cl-C₆H₄) | NCH₃ |
| Ia1.911 | CH₃ | H | (CH₂)₂-O-(2,4-Cl₂-C₆H₃) | NCH₃ |
| Ia1.912 | CH₃ | CH₃ | (CH₂)₂-O-(2,4-Cl₂-C₆H₃) | NCH₃ |
| Ia1.913 | CH₃ | CH₂CH₃ | (CH₂)₂-O-(2,4-Cl₂-C₆H₃) | NCH₃ |
| Ia1.914 | CH₃ | (CH₂)₂CH₃ | (CH2)2-O-(2,4-Cl₂-C₆H₃) | NCH₃ |
| Ia1.915 | CH₃ | (CH₂)₃CH₃ | (CH₂)₂-O-(2,4-Cl₂-C₆H₃) | NCH₃ |
| Ia1.916 | CH₃ | H | CH₂CH(CH₃)-O-(2,4-Cl₂-C₆H₃) | NCH₃ |
| Ia1.917 | CH₃ | CH₃ | CH₂CH(CH₃)-O-(2,4-Cl₂-C₆H₃) | NCH₃ |
| Ia1.918 | CH₃ | CH₂CH₃ | CH₂CH(CH₃)-O-(2,4-Cl₂-C₆H₃) | NCH₃ |
| Ia1.919 | CH₃ | (CH₂)₂CH₃ | CH₂CH(CH₃)-O-(2,4-Cl₂-C₆H₃) | NCH₃ |
| Ia1.920 | CH₃ | (CH₂)₃CH₃ | CH₂CH(CH₃)-O-(2,4-Cl₂-C₆H₃) | NCH₃ |
| Ia1.921 | CH₃ | H | CH(CH₃)CH₂-O-(2,4-Cl₂-C₆H₃) | NCH₃ |
| Ia1.922 | CH₃ | CH₃ | CH(CH₃)CH₂-O-(2,4-Cl₂-C₆H₃) | NCH₃ |
| Ia1.923 | CH₃ | CH₂CH₃ | CH(CH₃)CH₂-O-(2,4-Cl₂-C₆H₃) | NCH₃ |
| Ia1.924 | CH₃ | (CH₂)₂CH₃ | CH(CH₃)CH₂-O-(2,4-Cl₂-C₆H₃) | NCH₃ |
| Ia1.925 | CH₃ | (CH₂)₃CH₃ | CH(CH₃)CH₂-O-(2,4-Cl₂-C₆H₃) | NCH₃ |
| Ia1.926 | CH₃ | H | CH(CH₃)CH(CH₃)-O-(2,4-Cl₂-C₆H₃) | NCH₃ |
| Ia1.927 | CH₃ | CH₃ | CH(CH₃)CH(CH₃)-O-(2,4-Cl₂-C₆H₃) | NCH₃ |
| Ia1.928 | CH₃ | CH₂CH₃ | CH(CH₃)CH(CH₃)-O-(2,4-Cl₂-C₆H₃) | NCH₃ |
| Ia1.929 | CH₃ | (CH₂)₂CH₃ | CH(CH₃)CH(CH₃)-O-(2,4-Cl₂-C₆H₃) | NCH₃ |
| Ia1.930 | CH₃ | (CH₂)₃CH₃ | CH(CH₃)CH(CH₃)-O-(2,4-Cl₂-C₆H₃) | NCH₃ |
| Ia1.931 | OCH₃ | H | CH₃ | O |
| Ia1.932 | OCH₃ | CH₃ | CH₃ | O |
| Ia1.933 | OCH₃ | CH₂CH₃ | CH₃ | O |
| Ia1.934 | OCH₃ | (CH₂)₂CH₃ | CH₃ | O |
| Ia1.935 | OCH₃ | (CH₂)₃CH₃ | CH₃ | O |
| Ia1.936 | OCH₃ | H | C₂H₅ | O |
| Ia1.937 | OCH₃ | CH₃ | C₂H₅ | O |
| Ia1.938 | OCH₃ | CH₂CH₃ | C₂H₅ | O |
| Ia1.939 | OCH₃ | (CH₂)₂CH₃ | C₂H₅ | O |
| Ia1.940 | OCH₃ | (CH₂)₃CH₃ | C₂H₅ | O |
| Ia1.941 | OCH₃ | H | (CH₂)₂CH₃ | O |
| Ia1.942 | OCH₃ | CH₃ | (CH₂)₂CH₃ | O |
| Ia1.943 | OCH₃ | CH₂CH₃ | (CH₂)₂CH₃ | O |
| Ia1.944 | OCH₃ | (CH₂)₂CH₃ | (CH₂)₂CH₃ | O |
| Ia1.945 | OCH₃ | (CH₂)₃CH₃ | (CH₂)₂CH₃ | O |
| Ia1.946 | OCH₃ | H | CH(CH₃)₂ | O |
| Ia1.947 | OCH₃ | CH₃ | CH(CH₃)₂ | O |
| Ia1.948 | OCH₃ | CH₂CH₃ | CH(CH₃)₂ | O |
| Ia1.949 | OCH₃ | (CH₂)₂CH₃ | CH(CH₃)₂ | O |
| Ia1.950 | OCH₃ | (CH₂)₃CH₃ | CH(CH₃)₂ | O |
| Ia1.951 | OCH₃ | H | (CH₂)₃CH₃ | O |
| Ia1.952 | OCH₃ | CH₃ | (CH₂)₃CH₃ | O |
| Ia1.953 | OCH₃ | CH₂CH₃ | (CH₂)₃CH₃ | O |
| Ia1.954 | OCH₃ | (CH₂)₂CH₃ | (CH₂)₃CH₃ | O |
| Ia1.955 | OCH₃ | (CH₂)₃CH₃ | (CH₂)₃CH₃ | O |
| Ia1.956 | OCH₃ | H | CH₂CH(CH₃)₂ | O |
| Ia1.957 | OCH₃ | CH₃ | CH₂CH(CH₃)₂ | O |
| Ia1.958 | OCH₃ | CH₂CH₃ | CH₂CH(CH₃)₂ | O |
| Ia1.959 | OCH₃ | (CH₂)₂CH₃ | CH₂CH(CH₃)₂ | O |
| Ia1.960 | OCH₃ | (CH₂)₃CH₃ | CH₂CH(CH₃)₂ | O |
| Ia1.961 | OCH₃ | H | CH(CH₃)CH₂CH₃ | O |
| Ia1.962 | OCH₃ | CH₃ | CH(CH₃)CH₂CH₃ | O |
| Ia1.963 | OCH₃ | CH₂CH₃ | CH(CH₃)CH₂CH₃ | O |
| Ia1.964 | OCH₃ | (CH₂)₂CH₃ | CH(CH₃)CH₂CH₃ | O |
| Ia1.965 | OCH₃ | (CH₂)₃CH₃ | CH(CH₃)CH₂CH₃ | O |
| Ia1.966 | OCH₃ | H | (CH₂)₂-C₆H₅ | O |
| Ia1.967 | OCH₃ | CH₃ | (CH₂)₂-C₆H₅ | O |
| Ia1.968 | OCH₃ | CH₂CH₃ | (CH₂)₂-C₆H₅ | O |
| Ia1.969 | OCH₃ | (CH₂)₂CH₃ | (CH₂)₂-C₆H₅ | O |
| Ia1.970 | OCH₃ | (CH₂)₃CH₃ | (CH₂)₂-C₆H₅ | O |
| Ia1.971 | OCH₃ | H | CH₂CH(CH₃)-C₆H₅ | O |
| Ia1.972 | OCH₃ | CH₃ | CH₂CH(CH₃)-C₆H₅ | O |
| Ia1.973 | OCH₃ | CH₂CH₃ | CH₂CH(CH₃)-C₆H₅ | O |
| Ia1.974 | OCH₃ | (CH₂)₂CH₃ | CH₂CH(CH₃)-C₆H₅ | O |
| Ia1.975 | OCH₃ | (CH₂)₃CH₃ | CH₂CH(CH₃)-C₆H₅ | O |
| Ia1.976 | OCH₃ | H | CH(CH₃)CH₂-C₆H₅ | O |
| Ia1.977 | OCH₃ | CH₃ | CH(CH₃)CH₂-C₆H₅ | O |
| Ia1.978 | OCH₃ | CH₂CH₃ | CH(CH₃)CH₂-C₆H₅ | O |
| Ia1.979 | OCH₃ | (CH₂)₂CH₃ | CH(CH₃)CH₂-C₆H₅ | O |
| Ia1.980 | OCH₃ | (CH₂)₃CH₃ | CH(CH₃)CH₂-C₆H₅ | O |
| Ia1.981 | OCH₃ | H | CH(CH₃)CH(CH₃)-C₆H₅ | O |
| Ia1.982 | OCH₃ | CH₃ | CH(CH₃)CH(CH₃)-C₆H₅ | O |
| Ia1.983 | OCH₃ | CH₂CH₃ | CH(CH₃)CH(CH₃)-C₆H₅ | O |
| Ia1.984 | OCH₃ | (CH₂)₂CH₃ | CH(CH₃)CH(CH₃)-C₆H₅ | O |
| Ia1.985 | OCH₃ | (CH₂)₃CH₃ | CH(CH₃)CH(CH₃)-C₆H₅ | O |
| Ia1.986 | OCH₃ | H | (CH₂)₂-(4-Cl-C₆H₄) | O |
| Ia1.987 | OCH₃ | CH₃ | (CH₂)₂-(4-Cl-C₆H₄) | O |
| Ia1.988 | OCH₃ | CH₂CH₃ | (CH₂)₂-(4-Cl-C₆H₄) | O |
| Ia1.989 | OCH₃ | (CH₂)₂CH₃ | (CH₂)₂-(4-Cl-C₆H₄) | O |
| Ia1.990 | OCH₃ | (CH₂)₃CH₃ | (CH₂)₂-(4-Cl-C₆H₄) | O |
| Ia1.991 | OCH₃ | H | CH₂CH(CH₃)-(4-Cl-C₆H₄) | O |
| Ia1.992 | OCH₃ | CH₃ | CH₂CH(CH₃)-(4-Cl-C₆H₄) | O |
| Ia1.993 | OCH₃ | CH₂CH₃ | CH₂CH(CH₃)-(4-Cl-C₆H₄) | O |
| Ia1.994 | OCH₃ | (CH₂)₂CH₃ | CH₂CH(CH₃)-(4-Cl-C₆H₄) | O |
| Ia1.995 | OCH₃ | (CH₂)₃CH₃ | CH₂CH(CH₃)-(4-Cl-C₆H₄) | O |
| Ia1.996 | OCH₃ | H | CH(CH₃)CH₂-(4-Cl-C₆H₄) | O |
| Ia1.997 | OCH₃ | CH₃ | CH(CH₃)CH₂-(4-Cl-C₆H₄) | O |
| Ia1.998 | OCH₃ | CH₂CH₃ | CH(CH₃)CH₂-(4-Cl-C₆H₄) | O |
| Ia1.999 | OCH₃ | (CH₂)₂CH₃ | CH(CH₃)CH₂-(4-Cl-C₆H₄) | O |
| Ia1.1000 | OCH₃ | (CH₂)₃CH₃ | CH(CH₃)CH₂-(4-Cl-C₆H₄) | O |
| Ia1.1001 | OCH₃ | H | CH(CH₃)CH(CH₃)-(4-Cl-C₆H₄) | O |
| Ia1.1002 | OCH₃ | CH₃ | CH(CH₃)CH(CH₃)-(4-Cl-C₆H₄) | O |
| Ia1.1003 | OCH₃ | CH₂CH₃ | CH(CH₃)CH(CH₃)-(4-Cl-C₆H₄) | O |
| Ia1.1004 | OCH₃ | (CH₂)₂CH₃ | CH(CH₃)CH(CH₃)-(4-Cl-C₆H₄) | O |
| Ia1.1005 | OCH₃ | (CH₂)₃CH₃ | CH(CH₃)CH(CH₃)-(4-Cl-C₆H₄) | O |
| Ia1.1006 | OCH₃ | H | (CH₂)₂-(2,4-Cl₂-C₆H₃) | O |
| Ia1.1007 | OCH₃ | CH₃ | (CH₂)₂-(2,4-Cl₂-C₆H₃) | O |
| Ia1.1008 | OCH₃ | CH₂CH₃ | (CH₂)₂-(2,4-Cl₂-C₆H₃) | O |
| Ia1.1009 | OCH₃ | (CH₂)₂CH₃ | (CH₂)₂-(2,4-Cl₂-C₆H₃) | O |
| la1.1010 | OCH₃ | (CH₂)₃CH₃ | (CH₂)₂-(2,4-Cl₂-C₆H₃) | O |
| Ia1.1011 | OCH₃ | H | (CH₂)CH(CH₃)-(2,4-Cl₂-C₆H₃) | O |
| Ia1.1012 | OCH₃ | CH₃ | CH₂CH(CH₃)-(2,4-Cl₂-C₆H₃) | O |
| Ia1.1013 | OCH₃ | CH₂CH₃ | CH₂CH(CH₃)-(2,4-Cl₂-C₆H₃) | O |
| Ia1.1014 | OCH₃ | (CH₂)₂CH₃ | CH₂CH(CH₃)-(2,4-Cl₂-C₆H₃) | O |
| Ia1.1015 | OCH₃ | (CH₂)₃CH₃ | CH₂CH(CH₃)-(2,4-Cl₂-C₆H₃) | O |
| Ia1.1016 | OCH₃ | H | CH(CH₃)CH₂-(2,4-Cl₂-C₆H₃) | O |
| Ia1.1017 | OCH₃ | CH₃ | CH(CH₃)CH₂-(2,4-Cl₂-C₆H₃) | O |
| Ia1.1018 | OCH₃ | CH₂CH₃ | CH(CH₃)CH₂-(2,4-Cl₂-C₆H₃) | O |
| Ia1.1019 | OCH₃ | (CH₂)₂CH₃ | CH(CH₃)CH₂-(2,4-Cl₂-C₆H₃) | O |
| Ia1.1020 | OCH₃ | (CH₂)₃CH₃ | CH(CH₃)CH₂-(2,4-Cl₂-C₆H₃) | O |
| Ia1.1021 | OCH₃ | H | CH(CH₃)CH(CH₃)-(2,4-Cl₂-C₆H₃) | O |
| Ia1.1022 | OCH₃ | CH₃ | CH(CH₃)CH(CH₃)-(2,4-Cl₂-C₆H₃) | O |
| Ia1.1023 | OCH₃ | CH₂CH₃ | CH(CH₃)CH(CH₃-(2,4-Cl₂-C₆H₃) | O |
| Ia1.1024 | OCH₃ | (CH₂)₂CH₃ | CH(CH₃)CH(CH₃)-(2,4-Cl₂-C₆H₃) | O |
| Ia1.1025 | OCH₃ | (CH₂)₃CH₃ | CH(CH₃)CH(CH₃)-(2,4-Cl₂-C₆H₃) | O |
| Ia1.1026 | OCH₃ | H | (CH₂)₂-O-C₆H₅ | O |
| Ia1.1027 | OCH₃ | CH₃ | (CH₂)₂-O-C₆H₅ | O |
| Ia1.1028 | OCH₃ | CH₂CH₃ | (CH₂)₂-O-C₆H₅ | O |
| Ia1.1029 | OCH₃ | (CH₂)₂CH₃ | (CH₂)₂-O-C₆H₅ | O |
| Ia1.1030 | OCH₃ | (CH₂)₃CH₃ | (CH₂)₂-O-C₆H₅ | O |
| Ia1.1031 | OCH₃ | H | CH₂CH(CH₃)-O-C₆H₅ | O |
| Ia1.1032 | OCH₃ | CH₃ | CH₂CH(CH₃)-O-C₆H₅ | O |
| Ia1.1033 | OCH₃ | CH₂CH₃ | CH₂CH(CH₃)-O-C₆H₅ | O |
| Ia1.1034 | OCH₃ | (CH₂)₂CH₃ | CH₂CH(CH₃)-O-C₆H₅ | O |
| Ia1.1035 | OCH₃ | (CH₂)₃CH₃ | CH₂CH(CH₃)-O-C₆H₅ | O |
| Ia1.1036 | OCH₃ | H | CH(CH₃)CH₂-O-C₆H₅ | O |
| Ia1.1037 | OCH₃ | CH₃ | CH(CH₃)CH₂-O-C₆H₅ | O |
| Ia1.1038 | OCH₃ | CH₂CH₃ | CH(CH₃)CH₂-O-C₆H₅ | O |
| Ia1.1039 | OCH₃ | (CH₂)₂CH₃ | CH(CH₃)CH₂-O-C₆H₅ | O |
| Ia1.1040 | OCH₃ | (CH₂)₃CH₃ | CH(CH₃)CH₂-O-C₆H₅ | O |
| Ia1.1041 | OCH₃ | H | CH(CH₃)CH(CH₃)-O-C₆H₅ | O |
| Ia1.1042 | OCH₃ | CH₃ | CH(CH₃)CH(CH₃)-O-C₆H₅ | O |
| Ia1.1043 | OCH₃ | CH₂CH₃ | CH(CH₃)CH(CH₃)-O-C₆H₅ | O |
| Ia1.1044 | OCH₃ | (CH₂)₂CH₃ | CH(CH₃)CH(CH₃)-O-C₆H₅ | O |
| Ia1.1045 | OCH₃ | (CH₂)₃CH₃ | CH(CH₃)CH(CH₃)-O-C₆H₅ | O |
| Ia1.1046 | OCH₃ | H | (CH₂)₂-O-(4-Cl-C₆H₄) | O |
| Ia1.1047 | OCH₃ | CH₃ | (CH₂)₂-O-(4-Cl-C₆H₄) | O |
| Ia1.1048 | OCH₃ | CH₂CH₃ | (CH₂)₂-O-(4-Cl-C₆H₄) | O |
| Ia1.1049 | OCH₃ | (CH₂)₂CH₃ | (CH₂)₂-O-(4-Cl-C₆H₄) | O |
| Ia1.1050 | OCH₃ | (CH₂)₃CH₃ | (CH₂)₂-O-(4-Cl-C₆H₄) | O |
| Ia1.1051 | OCH₃ | H | CH₂CH(CH₃)-O-(4-Cl-C₆H₄) | O |
| Ia1.1052 | OCH₃ | CH₃ | CH₂CH(CH₃)-O-(4-Cl-C₆H₄) | O |
| Ia1.1053 | OCH₃ | CH₂CH₃ | CH₂CH(CH₃)-O-(4-Cl-C₆H₄) | O |
| Ia1.1054 | OCH₃ | (CH₂)₂CH₃ | CH₂CH(CH₃)-O-(4-Cl-C₆H₄) | O |
| Ia1.1055 | OCH₃ | (CH₂)₃CH₃ | CH₂CH(CH₃)-O-(4-Cl-C₆H₄) | O |
| Ia1.1056 | OCH₃ | H | CH(CH₃)CH₂-O-(4-Cl-C₆H₄) | O |
| Ia1.1057 | OCH₃ | CH₃ | CH(CH₃)CH₂-O-(4-Cl-C₆H₄) | O |
| Ia1.1058 | OCH₃ | CH₂CH₃ | CH(CH₃)CH₂-O-(4-Cl-C₆H₄) | O |
| Ia1.1059 | OCH₃ | (CH₂)₂CH₃ | CH(CH₃)CH₂-O-(4-Cl-C₆H₄) | O |
| Ia1.1060 | OCH₃ | (CH₂)₃CH₃ | CH(CH₃)CH₂-O-(4-Cl-C₆H₄) | O |
| Ia1.1061 | OCH₃ | H | CH(CH₃)CH(CH₃)-O-(4-Cl-C₆H₄) | O |
| Ia1.1062 | OCH₃ | CH₃ | CH(CH₃)CH(CH₃)-O-(4-Cl-C₆H₄) | O |
| Ia1.1063 | OCH₃ | CH₂CH₃ | CH(CH₃)CH(CH₃)-O-(4-Cl-C₆H₄) | O |
| Ia1.1064 | OCH₃ | (CH₂)₂CH₃ | CH(CH₃)CH(CH₃)-O-(4-Cl-C₆H₄) | O |
| Ia1.1065 | OCH₃ | (CH₂)₃CH₃ | CH(CH₃)CH(CH₃)-O-(4-Cl-C₆H₄) | O |
| Ia1.1066 | OCH₃ | H | (CH₂)₂-O-(2,4-Cl₂-C₆H₃) | O |
| Ia1.1067 | OCH₃ | CH₃ | (CH₂)₂-O-(2,4-Cl₂-C₆H₃) | O |
| Ia1.1068 | OCH₃ | CH₂CH₃ | (CH₂)₂-O-(2,4-Cl₂-C₆H₃) | O |
| Ia1.1069 | OCH₃ | (CH₂)₂CH₃ | (CH₂)₂-O-(2,4-Cl₂-C₆H₃) | O |
| Ia1.1070 | OCH₃ | (CH₂)₃CH₃ | (CH₂)₂-O-(2,4-Cl₂-C₆H₃) | O |
| Ia1.1071 | OCH₃ | H | CH₂CH(CH₃)-O-(2,4-Cl₂-C₆H₃) | O |
| Ia1.1072 | OCH₃ | CH₃ | CH₂CH(CH₃)-O-(2,4-Cl₂-C₆H₃) | O |
| Ial.1073 | OCH₃ | CH₂CH₃ | CH₂CH(CH₃)-O-(2,4-Cl₂-C₆H₃) | O |
| Ia1.1074 | OCH₃ | (CH₂)₂CH₃ | CH₂CH(CH₃)-O-(2,4-Cl₂-C₆H₃) | O |
| Ia1.1075 | OCH₃ | (CH₂)₃CH₃ | CH₂CH(CH₃)-O-(2,4-Cl₂-C₆H₃) | O |
| Ia1.1076 | OCH₃ | H | CH(CH₃)CH₂-O-(2,4-Cl₂-C₆H₃) | O |
| Ia1.1077 | OCH₃ | CH₃ | CH(CH₃)CH₂-O-(2,4-Cl₂-C₆H₃) | O |
| Ia1.1078 | OCH₃ | CH₂CH₃ | CH(CH₃)CH₂-O-(2,4-Cl₂-C₆H₃) | O |
| Ia1.1079 | OCH₃ | (CH₂)₂CH₃ | CH(CH₃)CH₂-O-(2,4-Cl₂-C₆H₃) | O |
| Ia1.1080 | OCH₃ | (CH₂)₃CH₃ | CH(CH₃)CH₂-O-(2,4-Cl₂-C₆H₃) | O |
| Ia1.1081 | OCH₃ | H | CH(CH₃)CH(CH₃)-O-(2,4-Cl₂-C₆H₃) | O |
| Ia1.1082 | OCH₃ | CH₃ | CH(CH₃)CH(CH₃)-O-(2,4-Cl₂-C₆H₃) | O |
| Ia1.1083 | OCH₃ | CH₂CH₃ | CH(CH₃)CH(CH₃)-O-(2,4-Cl₂-C₆H₃) | O |
| Ia1.1084 | OCH₃ | (CH₂)₂CH₃ | CH(CH₃)CH(CH₃)-O-(2,4-Cl₂-C₆H₃) | O |
| Ia1.1085 | OCH₃ | (CH₂)₃CH₃ | CH(CH₃)CH(CH₃)-O-(2,4-Cl₂-C₆H₃) | O |
| Ia1.1086 | OCH₃ | H | CH₃ | NH |
| Ia1.1087 | OCH₃ | CH₃ | CH₃ | NH |
| Ia1.1088 | OCH₃ | CH₂CH₃ | CH₃ | NH |
| Ia1.1089 | OCH₃ | (CH₂)₂CH₃ | CH₃ | NH |
| Ia1.1090 | OCH₃ | (CH₂)₃CH₃ | CH₃ | NH |
| la1.1091 | OCH₃ | H | C₂H₅ | NH |
| Ia1.1092 | OCH₃ | CH₃ | C₂H₅ | NH |
| Ia1.1093 | OCH₃ | CH₂CH₃ | C₂H₅ | NH |
| Ia1.1094 | OCH₃ | (CH₂)₂CH₃ | C₂H₅ | NH |
| Ia1.1095 | OCH₃ | (CH₂)₃CH₃ | C₂H₅ | NH |
| Ia1.1096 | OCH₃ | H | (CH₂)₂CH₃ | NH |
| Ia1.1097 | OCH₃ | CH₃ | (CH₂)₂CH₃ | NH |
| Ia1.1098 | OCH₃ | CH₂CH₃ | (CH₂)₂CH₃ | NH |
| Ia1.1099 | OCH₃ | (CH₂)₂CH₃ | (CH₂)₂CH₃ | NH |
| Ia1.1100 | OCH₃ | (CH₂)₃CH₃ | (CH₂)₂CH₃ | NH |
| Ia1.1101 | OCH₃ | H | CH(CH₃)₂ | NH |
| Ia1.1102 | OCH₃ | CH₃ | CH(CH₃)₂ | NH |
| Ia1.1103 | OCH₃ | CH₂CH₃ | CH(CH₃)₂ | NH |
| Ia1.1104 | OCH₃ | (CH₂)₂CH₃ | CH(CH₃)₂ | NH |
| Ia1.1105 | OCH₃ | (CH₂)₃CH₃ | CH(CH₃)₂ | NH |
| Ia1.1106 | OCH₃ | H | (CH₂)₃CH₃ | NH |
| Ia1.1107 | OCH₃ | CH₃ | (CH₂)₃CH₃ | NH |
| Ia1.1108 | OCH₃ | CH₂CH₃ | (CH₂)₃CH₃ | NH |
| Ia1.1109 | OCH₃ | (CH₂)₂CH₃ | (CH₂)₃CH₃ | NH |
| Ia1.1110 | OCH₃ | (CH₂)₃CH₃ | (CH₂)₃CH₃ | NH |
| Ia1.1111 | OCH₃ | H | CH₂CH(CH₃)₂ | NH |
| Ia1.1112 | OCH₃ | CH₃ | CH₂CH(CH₃)₂ | NH |
| Ia1.1113 | OCH₃ | CH₂CH₃ | CH₂CH(CH₃)₂ | NH |
| Ia1.1114 | OCH₃ | (CH₂)₂CH₃ | CH₂CH(CH₃)₂ | NH |
| Ia1.1115 | OCH₃ | (CH₂)₃CH₃ | CH₂CH(CH₃)₂ | NH |
| Ia1.1116 | OCH₃ | H | CH₂CH(CH₃)₂ | NH |
| Ia1.1117 | OCH₃ | CH₃ | CH(CH₃)CH₂CH₃ | NH |
| Ia1.1118 | OCH₃ | CH₂CH₃ | CH(CH₃)CH₂CH₃ | NH |
| Ia1.1119 | OCH₃ | (CH₂)₂CH₃ | CH(CH₃)CH₂CH₃ | NH |
| Ia1.1120 | OCH₃ | (CH₂)₃CH₃ | CH(CH₃)CH₂CH₃ | NH |
| Ia1.1121 | OCH₃ | H | (CH₂)₂-C₆H₅ | NH |
| Ia1.1122 | OCH₃ | CH₃ | (CH₂)₂-C₆H₅ | NH |
| Ia1.1123 | OCH₃ | CH₂CH₃ | (CH₂)₂-C₆H₅ | NH |
| Ia1.1124 | OCH₃ | (CH₂)₂CH₃ | (CH₂)₂-C₆H₅ | NH |
| Ia1.1125 | OCH₃ | (CH₂)₃C_{H3} | (CH₂)₂-C₆H₅ | NH |
| Ia1.1126 | OCH₃ | H | CH₂CH(CH₃)-C₆H₅ | NH |
| Ia1.1127 | OCH₃ | CH₃ | CH₂CH(CH₃)-C₆H₅ | NH |
| Ia1.1128 | OCH₃ | CH₂CH₃ | CH₂CH(CH₃)-C₆H₅ | NH |
| Ia1.1129 | OCH₃ | (CH₂)₂CH₃ | CH₂CH(CH₃)-C₆H₅ | NH |
| Ia1.1130 | OCH₃ | (CH₂)₃CH₃ | CH₂CH(CH₃)-C₆H₅ | NH |
| Ia1.1131 | OCH₃ | H | CH(CH₃)CH₂-C₆H₅ | NH |
| Ia1.1132 | OCH₃ | CH₃ | CH(CH₃)CH₂-C₆H₅ | NH |
| Ia1.1133 | OCH₃ | CH₂CH₃ | CH(CH₃)CH₂-C₆H₅ | NH |
| Ia1.1134 | OCH₃ | (CH₂)₂CH₃ | CH(CH₃)CH₂-C₆H₅ | NH |
| Ia1.1135 | OCH₃ | (CH₂)₃CH₃ | CH(CH₃)CH₂-C₆H₅ | NH |
| Ia1.1136 | OCH₃ | H | CH(CH₃)CH(CH₃)-C₆H₅ | NH |
| Ia1.1137 | OCH₃ | CH₃ | CH(CH₃)CH(CH₃)-C₆H₅ | NH |
| Ia1.1138 | OCH₃ | CH₂CH₃ | CH(CH₃)CH(CH₃)-C₆H₅ | NH |
| Ia1.1139 | OCH₃ | (CH₂)₂CH₃ | CH(CH₃)CH(CH₃)-C₆H₅ | NH |
| Ia1.1140 | OCH₃ | (CH₂)₃CH₃ | CH(CH₃)CH(CH₃)-C₆H₅ | NH |
| Ia1.1141 | OCH₃ | H | (CH₂)₂-(4-Cl-C₆H₄) | NH |
| Ia1.1142 | OCH₃ | CH₃ | (CH₂)₂-(4-Cl-C₆H₄) | NH |
| Ia1.1143 | OCH₃ | CH₂CH₃ | (CH₂)₂-(4-Cl-C₆H₄) | NH |
| Ia1.1144 | OCH₃ | (CH₂)₂CH₃ | (CH₂)₂-(4-Cl-C₆H₄) | NH |
| Ia1.1145 | OCH₃ | (CH₂)₃CH₃ | (CH₂)₂-(4-Cl-C₆H₄) | NH |
| Ia1.1146 | OCH₃ | H | CH₂CH(CH₃)-(4-Cl-C₆H₄) | NH |
| Ia1.1147 | OCH₃ | CH₃ | CH₂CH(CH₃)-(4-Cl-C₆H₄) | NH |
| Ia1.1148 | OCH₃ | CH₂CH₃ | CH₂CH(CH₃)-(4-Cl-C₆H₄) | NH |
| Ia1.1149 | OCH₃ | (CH₂)₂CH₃ | CH₂CH(CH₃)-(4-Cl-C₆H₄) | NH |
| Ia1.1150 | OCH₃ | (CH₂)₃CH₃ | CH₂CH(CH₃)-(4-Cl-C₆H₄) | NH |
| Ia1.1151 | OCH₃ | H | CH(CH₃)CH₂-(4-Cl-C₆H₄) | NH |
| Ia1.1152 | OCH₃ | CH₃ | CH(CH₃)CH₂-(4-Cl-C₆H₄) | NH |
| Ia1.1153 | OCH₃ | CH₂CH₃ | CH(CH₃)CH₂-(4-Cl-C₆H₄) | NH |
| Ia1.1154 | OCH₃ | (CH₂)₂CH₃ | CH(CH₃)CH₂-(4-Cl-C₆H₄) | NH |
| Ia1.1155 | OCH₃ | (CH₂)₃CH₃ | CH(CH₃)CH₂-(4-Cl-C₆H₄) | NH |
| Ia1.1156 | OCH₃ | H | CH(CH₃)CH(CH₃)-(4-Cl-C₆H₄) | NH |
| Ia1.1157 | OCH₃ | CH₃ | CH(CH₃)CH(CH₃)-(4-Cl-C₆H₄) | NH |
| Ia1.1158 | OCH₃ | CH₂CH₃ | CH(CH₃)CH(CH₃)-(4-Cl-C₆H₄) | NH |
| Ia1.1159 | OCH₃ | (CH₂)₂CH₃ | CH(CH₃)CH(CH₃)-(4-Cl-C₆H₄) | NH |
| Ia1.1160 | OCH₃ | (CH₂)₃CH₃ | CH(CH₃)CH(CH₃)-(4-Cl-C₆H₄) | NH |
| Ia1.1161 | OCH₃ | H | (CH₂)₂-(2,4-Cl₂-C₆H₃) | NH |
| Ia1.1162 | OCH₃ | CH₃ | (CH₂)₂-(2,4-Cl₂-C₆H₃) | NH |
| Ia1.1163 | OCH₃ | CH₂CH₃ | (CH₂)₂-(2,4-Cl₂-C₆H₃) | NH |
| Ia1.1164 | OCH₃ | (CH₂)₂CH₃ | (CH₂)₂-(2,4-Cl₂-C₆H₃) | NH |
| Ia1.1165 | OCH₃ | (CH₂)₃CH₃ | (CH₂)₂-(2,4-Cl₂-C₆H₃) | NH |
| Ia1.1166 | OCH₃ | H | CH₂CH(CH₃)-(2,4-Cl₂-C₆H₃) | NH |
| Ia1.1167 | OCH₃ | CH₃ | CH₂CH(CH₃)-(2,4-Cl₂-C₆H₃) | NH |
| Ia1.1168 | OCH₃ | CH₂CH₃ | CH₂CH(CH₃)-(2,4-Cl₂-C₆H₃) | NH |
| Ia1.1169 | OCH₃ | (CH₂)₂CH₃ | CH₂CH(CH₃)-(2,4-Cl₂-C₆H₃) | NH |
| Ia1.1170 | OCH₃ | (CH₂)₃CH₃ | CH₂CH(CH₃)-(2,4-Cl₂-C₆H₃) | NH |
| Ia1.1171 | OCH₃ | H | CH(CH₃)CH₂-(2,4-Cl₂-C₆H₃) | NH |
| Ia1.1172 | OCH₃ | CH₃ | CH(CH₃)CH₂-(2,4-Cl₂-C₆H₃) | NH |
| Ia1.1173 | OCH₃ | CH₂CH₃ | CH(CH₃)CH₂-(2, 4-Cl₂-C₆H₃) | NH |
| Ia1.1174 | OCH₃ | (CH₂)₂CH₃ | CH(CH₃)CH₂-(2,4-Cl₂-C₆H₃) | NH |
| Ia1.1175 | OCH₃ | (CH₂)₃CH₃ | CH(CH₃)CH₂-(2,4-Cl₂-C₆H₃) | NH |
| Ia1.1176 | OCH₃ | H | CH(CH₃)CH(CH₃)-(2,4-Cl₂-C₆H₃) | NH |
| Ia1.1177 | OCH₃ | CH₃ | CH(CH₃)CH(CH₃)-(2,4-Cl₂-C₆H₃) | NH |
| Ia1.1178 | OCH₃ | CH₂CH₃ | CH(CH₃)CH(CH₃)-(2,4-Cl₂-C₆H₃) | NH |
| Ia1.1179 | OCH₃ | (CH₂)₂CH₃ | CH(CH₃)CH(CH₃)-(2,4-Cl₂-C₆H₃) | NH |
| Ia1.1180 | OCH₃ | (CH₂)₃CH₃ | CH(CH₃)CH(CH₃)-(2,4-Cl₂-C₆H₃) | NH |
| Ia1.1181 | OCH₃ | H | (CH₂)₂-O-C₆H₅ | NH |
| Ia1.1182 | OCH₃ | CH₃ | (CH₂)₂-O-C₆H₅ | NH |
| Ia1.1183 | OCH₃ | CH₂CH₃ | (CH₂)₂-O-C₆H₅ | NH |
| Ia1.1184 | OCH₃ | (CH₂)₂CH₃ | (CH₂) ₂-O-C₆H₅ | NH |
| Ia1.1185 | OCH₃ | (CH₂)₃CH₃ | (CH₂)₂-O-C₆H₅ | NH |
| Ia1.1186 | OCH₃ | H | CH₂CH(CH₃)-O-C₆H₅ | NH |
| Ia1.1187 | OCH₃ | CH₃ | CH₂CH(CH₃)-O-C₆H₅ | NH |
| Ia1.1188 | OCH₃ | CH₂CH₃ | CH₂CH(CH₃)-O-C₆H₅ | NH |
| Ia1.1189 | OCH₃ | (CH₂)₂CH₃ | CH₂CH(CH₃)-O-C₆H₅ | NH |
| Ia1.1190 | OCH₃ | (CH₂)₃CH₃ | CH₂CH(CH₃)-O-C₆H₅ | NH |
| Ia1.1191 | OCH₃ | H | CH(CH₃)CH₂-O-C₆H₅ | NH |
| Ia1.1192 | OCH₃ | CH₃ | CH(CH₃)CH₂-O-C₆H₅ | NH |
| Ia1.1193 | OCH₃ | CH₂CH₃ | CH(CH₃)CH₂-O-C₆H₅ | NH |
| Ia1.1194 | OCH₃ | (CH₂)₂CH₃ | CH(CH₃)CH₂-O-C₆H₅ | NH |
| Ia1.1195 | OCH₃ | (CH₂)₃CH₃ | CH(CH₃)CH₂-O-C₆H₅ | NH |
| Ia1.1196 | OCH₃ | H | CH(CH₃)CH(CH₃)-O-C₆H₅ | NH |
| Ia1.1197 | OCH₃ | CH₃ | CH(CH₃)CH(CH₃)-O-C₆H₅ | NH |
| Ia1.1198 | OCH₃ | CH₂CH₃ | CH(CH₃)CH(CH₃)-O-C₆H₅ | NH |
| Ia1.1199 | OCH₃ | (CH₂)₂CH₃ | CH(CH₃)CH(CH₃)-O-C₆H₅ | NH |
| Ia1.1200 | OCH₃ | (CH₂)₃CH₃ | CH(CH₃)CH(CH₃)-O-C₆H₅ | NH |
| Ia1.1201 | OCH₃ | H | (CH₂)₂-O-(4-Cl-C₆H₄) | NH |
| Ia1.1202 | OCH₃ | CH₃ | (CH₂)₂-O-(4-Cl-C₆H₄) | NH |
| Ia1.1203 | OCH₃ | CH₂CH₃ | (CH₂)₂-O-(4-Cl-C₆H₄) | NH |
| Ia1.1204 | OCH₃ | (CH₂)₂CH₃ | (CH₂)₂-O-(4-Cl-C₆H₄) | NH |
| Ia1.1205 | OCH₃ | (CH₂)₃CH₃ | (CH₂)₂-O-(4-Cl-C₆H₄) | NH |
| Ia1.1206 | OCH₃ | H | CH₂CH(CH₃)-O-(4-Cl-C₆H₄) | NH |
| Ia1.1207 | OCH₃ | CH₃ | CH₂CH(CH₃)-O-(4-Cl-C₆H₄) | NH |
| Ia1.1208 | OCH₃ | CH₂CH₃ | CH₂CH(CH₃)-O-(4-Cl-C₆H₄) | NH |
| Ia1.1209 | OCH₃ | (CH₂)₂CH₃ | CH₂CH(CH₃)-O-(4-Cl-C₆H₄) | NH |
| Ia1.1210 | OCH₃ | (CH₂)₃CH₃ | CH₂CH(CH₃)-O-(4-Cl-C₆H₄) | NH |
| Ia1.1211 | OCH₃ | H | CH(CH₃)CH₂-O-(4-Cl-C₆H₄) | NH |
| Ia1.1212 | OCH₃ | CH₃ | CH(CH₃)CH₂-O-(4-Cl-C₆H₄) | NH |
| Ia1.1213 | OCH₃ | CH₂CH₃ | CH(CH₃)CH₂-O-(4-Cl-C₆H₄) | NH |
| Ia1.1214 | OCH₃ | (CH₂)₂CH₃ | CH(CH₃)CH₂-O-(4-Cl-C₆H₄) | NH |
| Ia1.1215 | OCH₃ | (CH₂)₃CH₃ | CH(CH₃)CH₂-O-(4-Cl-C₆H₄) | NH |
| Ia1.1216 | OCH₃ | H | CH(CH₃)CH(CH₃)-O-(4-Cl-C₆H₄) | NH |
| Ia1.1217 | OCH₃ | CH₃ | CH(CH₃)CH(CH₃)-O-(4-Cl-C₆H₄) | NH |
| Ia1.1218 | OCH₃ | CH₂CH₃ | CH(CH₃)CH(CH₃)-O-(4-Cl-C₆H₄) | NH |
| Ia1.1219 | OCH₃ | (CH₂)₂CH₃ | CH(CH₃)CH(CH₃)-O-(4-Cl-C₆H₄) | NH |
| Ia1.2220 | OCH₃ | (CH₂)₃CH₃ | CH(CH₃)CH(CH₃)-O-(4-Cl-C₆H₄) | NH |
| Ia1.1221 | OCH₃ | H | (CH₂)₂-O-(2,4-Cl₂-C₆H₃) | NH |
| Ia1.1222 | OCH₃ | CH₃ | (CH₂)₂-O-(2,4-Cl₂-C₆H₃) | NH |
| Ia1.1223 | OCH₃ | CH₂CH₃ | (CH₂)₂-O-(2,4-Cl₂-C₆H₃) | NH |
| Ia1.1224 | OCH₃ | (CH₂)₂CH₃ | (CH₂)₂-O-(2,4-Cl₂-C₆H₃) | NH |
| Ia1.1225 | OCH₃ | (CH₂)₃CH₃ | (CH₂)₂-O-(2,4-Cl₂-C₆H₃) | NH |
| Ia1.1226 | OCH₃ | H | CH₂CH(CH₃)-O-(2,4-Cl₂-C₆H₃) | NH |
| Ia1.1227 | OCH₃ | CH₃ | CH₂CH(CH₃)-O-(2,4-Cl₂-C₆H₃) | NH |
| Ia1.1228 | OCH₃ | CH₂CH₃ | CH₂CH(CH₃)-O-(2,4-Cl₂-C₆H₃) | NH |
| Ia1.1229 | OCH₃ | (CH₂)₂CH₃ | CH₂CH(CH₃)-O-(2,4-Cl₂-C₆H₃) | NH |
| Ia1.1230 | OCH₃ | (CH₂)₃CH₃ | CH₂CH(CH₃)-O-(2,4-Cl₂-C₆H₃) | NH |
| Ia1.1231 | OCH₃ | H | CH(CH₃) CH₂-O-(2,4-Cl₂-C₆H₃) | NH |
| Ia1.1232 | OCH₃ | CH₃ | CH(CH₃)CH₂-O-(2,4-Cl₂-C₆H₃) | NH |
| Ia1.1233 | OCH₃ | CH₂CH₃ | CH(CH₃)CH₂-O-(2,4-Cl₂-C₆H₃) | NH |
| Ia1.1234 | OCH₃ | (CH₂)₂CH₃ | CH(CH₃)CH₂-O-(2,4-Cl₂-C₆H₃) | NH |
| Ia1.1235 | OCH₃ | (CH₂)₃CH₃ | CH(CH₃)CH₂-O-(2,4-Cl₂-C₆H₃) | NH |
| Ia1.1236 | OCH₃ | H | CH(CH₃)CH(CH₃)-O- (2,4-Cl₂-C₆H₃) | NH |
| Ia1.1237 | OCH₃ | CH₃ | CH(CH₃)CH(CH₃)-O-(2,4-Cl₂-C₆H₃) | NH |
| Ia1.1238 | OCH₃ | CH₂CH₃ | CH(CH₃)CH(CH₃)-O-(2,4-Cl₂-C₆H₃) | NH |
| Ia1.1239 | OCH₃ | (CH₂)₂CH₃ | CH(CH₃)CH(CH₃)-O-(2,4-Cl₂-C₆H₃) | NH |
| Ia1.1240 | OCH₃ | (CH₂)₃CH₃ | CH(CH₃)CH(CH₃)-O-(2,4-Cl₂-C₆H₃) | NH |
| Ia1.1241 | OCH₃ | H | CH₃ | NCH₃ |
| Ia1.1242 | OCH₃ | CH₃ | CH₃ | NCH₃ |
| Ia1.1243 | OCH₃ | CH₂CH₃ | CH₃ | NCH₃ |
| Ia1.1244 | OCH₃ | (CH₂)₂CH₃ | CH₃ | NCH₃ |
| Ia1.1245 | OCH₃ | (CH₂)₃CH₃ | CH₃ | NCH₃ |
| Ia1.1246 | OCH₃ | H | C₂H₅ | NCH₃ |
| Ia1.1247 | OCH₃ | CH₃ | C₂H₅ | NCH₃ |
| Ia1.1248 | OCH₃ | CH₂CH₃ | C₂H₅ | NCH₃ |
| Ia1.1249 | OCH₃ | (CH₂)₂CH₃ | C₂H₅ | NCH₃ |
| Ia1.1250 | OCH₃ | (CH₂)₃CH₃ | C₂H₅ | NCH₃ |
| Ia1.1251 | OCH₃ | H | (CH₂)₂CH₃ | NCH₃ |
| Ia1.1252 | OCH₃ | CH₃ | (CH₂)₂CH₃ | NCH₃ |
| Ia1.1253 | OCH₃ | CH₂CH₃ | (CH₂)₂CH₃ | NCH₃ |
| Ia1.1254 | OCH₃ | (CH₂)₂CH₃ | (CH₂)₂CH₃ | NCH₃ |
| Ia1.1255 | OCH₃ | (CH₂)₃CH₃ | (CH₂)₂CH₃ | NCH₃ |
| Ia1.1256 | OCH₃ | H | CH(CH₃)₂ | NCH₃ |
| Ia1.1257 | OCH₃ | CH₃ | CH(CH₃)₂ | NCH₃ |
| Ia1.1258 | OCH₃ | CH₂CH₃ | CH(CH₃)₂ | NCH₃ |
| Ia1.1259 | OCH₃ | (CH₂)₂CH₃ | CH(CH₃)₂ | NCH₃ |
| Ia1.1260 | OCH₃ | (CH₂)₃CH₃ | CH(CH₃)₂ | NCH₃ |
| Ia1.1261 | OCH₃ | H | (CH₂)₃CH₃ | NCH₃ |
| Ia1.1262 | OCH₃ | CH₃ | (CH₂)₃CH₃ | NCH₃ |
| Ia1.1263 | OCH₃ | CH₂CH₃ | (CH₂)₃CH₃ | NCH₃ |
| Ia1.1264 | OCH₃ | (CH₂)₂CH₃ | (CH₂)₃CH₃ | NCH₃ |
| Ia1.1265 | OCH₃ | (CH₂)₃CH₃ | (CH₂)₃CH₃ | NCH₃ |
| Ia1.1266 | OCH₃ | H | CH₂CH(CH₃)₂ | NCH₃ |
| Ia1.1267 | OCH₃ | CH₃ | CH₂CH(CH₃)₂ | NCH₃ |
| Ia1.1268 | OCH₃ | CH₂CH₃ | CH₂CH(CH₃)₂ | NCH₃ |
| Ia1.1269 | OCH₃ | (CH₂)₂CH₃ | CH₂CH(CH₃)₂ | NCH₃ |
| Ia1.1270 | OCH₃ | (CH₂)₃CH₃ | CH₂CH(CH₃)₂ | NCH₃ |
| Ia1.1271 | OCH₃ | H | CH(CH₃)CH₂CH₃ | NCH₃ |
| Ia1.1272 | OCH₃ | CH₃ | CH(CH₃)CH₂CH₃ | NCH₃ |
| Ia1.1273 | OCH₃ | CH₂CH₃ | CH(CH₃)CH₂CH₃ | NCH₃ |
| Ia1.1274 | OCH₃ | (CH₂)₂CH₃ | CH(CH₃)CH₂CH₃ | NCH₃ |
| Ia1.1275 | OCH₃ | (CH₂)₃CH₃ | CH(CH₃)CH₂CH₃ | NCH₃ |
| Ia1.1276 | OCH₃ | H | (CH₂)₂-C₆H₅ | NCH₃ |
| Ia1.1277 | OCH₃ | CH₃ | (CH₂)₂-C₆H₅ | NCH₃ |
| Ia1.1278 | OCH₃ | CH₂CH₃ | (CH₂)₂-C6Hs | NCH₃ |
| Ia1.1279 | OCH₃ | (CH₂)₂CH₃ | (CH₂)₂-C₆H₅ | NCH₃ |
| Ia1.1280 | OCH₃ | (CH₂)₃CH₃ | (CH₂)₂-C₆H₅ | NCH₃ |
| Ia1.1281 | OCH₃ | H | CH₂CH(CH₃)-C₆H₅ | NCH₃ |
| Ia1.1282 | OCH₃ | CH₃ | CH₂CH(CH₃)-C₆H₅ | NCH₃ |
| Ia1.1283 | OCH₃ | CH₂CH₃ | CH₂CH(CH₃)-C₆H₅ | NCH₃ |
| Ia1.1284 | OCH₃ | (CH₂)₂CH₃ | CH₂CH(CH₃)-C₆H₅ | NCH₃ |
| Ia1.1285 | OCH₃ | (CH₂)₃CH₃ | CH₂CH(CH₃)-C₆H₅ | NCH₃ |
| Ia1.1286 | OCH₃ | H | CH(CH₃)CH₂-C₆H₅ | NCH₃ |
| Ia1.1287 | OCH₃ | CH₃ | CH(CH₃)CH₂-C₆H₅ | NCH₃ |
| Ia1.1288 | OCH₃ | CH₂CH₃ | CH(CH₃)CH₂-C₆H₅ | NCH₃ |
| Ia1.1289 | OCH₃ | (CH₂)₂CH₃ | CH(CH₃)CH₂-C₆H₅ | NCH₃ |
| Ia1.1290 | OCH₃ | (CH₂)₃CH₃ | CH(CH₃)CH₂-C₆H₅ | NCH₃ |
| Ia1.1291 | OCH₃ | H | CH(CH₃)CH(CH₃)-C₆H₅ | NCH₃ |
| Ia1.1292 | OCH₃ | CH₃ | CH(CH₃)CH(CH₃)-C₆H₅ | NCH₃ |
| Ia1.1293 | OCH₃ | CH₂CH₃ | CH(CH₃)CH(CH₃)-C₆H₅ | NCH₃ |
| Ia1.1294 | OCH₃ | (CH₂)₂CH₃ | CH(CH₃)CH(CH₃)-C₆H₅ | NCH₃ |
| Ia1.1295 | OCH₃ | (CH₂)₃CH₃ | CH(CH₃)CH(CH₃)-C₆H₅ | NCH₃ |
| Ia1.1296 | OCH₃ | H | (CH₂)₂-(4-Cl-C₆H₄) | NCH₃ |
| Ia1.1297 | OCH₃ | CH₃ | (CH₂)₂-(4-Cl-C₆H₄) | NCH₃ |
| Ia1.1298 | OCH₃ | CH₂CH₃ | (CH₂)₂-(4-Cl-C₆H₄) | NCH₃ |
| Ia1.1299 | OCH₃ | (CH₂)₂CH₃ | (CH₂)₂-(4-Cl-C₆H₄) | NCH₃ |
| Ia1.1300 | OCH₃ | (CH₂)₃CH₃ | (CH₂)₂-(4-Cl-C₆H₄) | NCH₃ |
| Ia1.1301 | OCH₃ | H | CH₂CH(CH₃)-(4-Cl-C₆H₄) | NCH₃ |
| Ia1.1302 | OCH₃ | CH₃ | CH₂CH(CH₃)-(4-Cl-C₆H₄) | NCH₃ |
| Ia1.1303 | OCH₃ | CH₂CH₃ | CH₂CH(CH₃)-(4-Cl-C₆H₄) | NCH₃ |
| Ia1.1304 | OCH₃ | (CH₂)₂CH₃ | CH₂CH(CH₃)-(4-Cl-C₆H₄) | NCH₃ |
| Ia1.1305 | OCH₃ | (CH₂)₃CH₃ | CH₂CH(CH₃)-(4-Cl-C₆H₄) | NCH₃ |
| Ia1.1306 | OCH₃ | H | CH(CH₃)CH₂-(4-Cl-C₆H₄) | NCH₃ |
| Ia1.1307 | OCH₃ | CH₃ | CH(CH₃)CH₂-(4-Cl-C₆H₄) | NCH₃ |
| Ia1.1308 | OCH₃ | CH₂CH₃ | CH(CH₃)CH₂-(4-Cl-C₆H₄) | NCH₃ |
| Ia1.1309 | OCH₃ | (CH₂)₂CH₃ | CH(CH₃)CH₂-(4-Cl-C₆H₄) | NCH₃ |
| Ia1.1310 | OCH₃ | (CH₂)₃CH₃ | CH(CH₃)CH₂-(4-Cl-C₆H₄) | NCH₃ |
| Ia1.1311 | OCH₃ | H | CH(CH₃)CH(CH₃)-(4-Cl-C₆H₄) | NCH₃ |
| Ia1.1312 | OCH₃ | CH₃ | CH(CH₃)CH(CH₃)-(4-Cl-C₆H₄) | NCH₃ |
| Ia1.1313 | OCH₃ | CH₂CH₃ | CH(CH₃)CH(CH₃)-(4-Cl-C₆H₄) | NCH₃ |
| Ia1.1314 | OCH₃ | (CH₂)₂CH₃ | CH(CH₃)CH(CH₃)-(4-Cl-C₆H₄) | NCH₃ |
| Ia1.1315 | OCH₃ | (CH₂)₃CH₃ | CH(CH₃)CH(CH₃)-(4-Cl-C₆H₄) | NCH₃ |
| Ia1.1316 | OCH₃ | H | (CH₂)₂-(2,4-Cl₂-C₆H₃) | NCH₃ |
| Ia1.1317 | OCH₃ | CH₃ | (CH₂)₂-(2,4-Cl₂-C₆H₃) | NCH₃ |
| Ia1.1318 | OCH₃ | CH₂CH₃ | (CH₂)₂-(2,4-Cl₂-C₆H₃) | NCH₃ |
| Ia1.1319 | OCH₃ | (CH₂)₂CH₃ | (CH₂)₂-(2,4-Cl₂-C₆H₃) | NCH₃ |
| Ia1.1320 | OCH₃ | (CH₂)₃CH₃ | (CH₂)₂-(2,4-Cl₂-C₆H₃) | NCH₃ |
| Ia1.1321 | OCH₃ | H | CH₂CH(CH₃)-(2,4-Cl₂-C₆H₃) | NCH₃ |
| Ia1.1322 | OCH₃ | CH₃ | CH₂CH(CH₃)-(2,4-Cl₂-C₆H₃) | NCH₃ |
| Ia1.1323 | OCH₃ | CH₂CH₃ | CH₂CH(CH₃)-(2,4-Cl₂-C₆H₃) | NCH₃ |
| Ia1.1324 | OCH₃ | (CH₂)₂CH₃ | CH₂CH(CH₃)-(2,4-Cl₂-C₆H₃) | NCH₃ |
| Ia1.1325 | OCH₃ | (CH₂)₃CH₃ | CH₂CH(CH₃)-(2,4-Cl₂-C₆H₃) | NCH₃ |
| Ia1.1326 | OCH₃ | H | CH(CH₃)CH₂-(2,4-Cl₂-C₆H₃) | NCH₃ |
| Ia1.1327 | OCH₃ | CH₃ | CH(CH₃)CH₂-(2,4-Cl₂-C₆H₃) | NCH₃ |
| Ia1.1328 | OCH₃ | CH₂CH₃ | CH(CH₃)CH₂-(2,4-Cl₂-C₆H₃) | NCH₃ |
| Ia1.1329 | OCH₃ | (CH₂)₂CH₃ | CH(CH₃)CH₂-(2,4-Cl₂-C₆H₃) | NCH₃ |
| Ia1.1330 | OCH₃ | (CH₂)₃CH₃ | CH(CH₃)CH₂-(2,4-Cl₂-C₆H₃) | NCH₃ |
| Ia1.1331 | OCH₃ | H | CH(CH₃)CH(CH₃)-(2.4-Cl₂-C₆H₃) | NCH₃ |
| Ia1.1332 | OCH₃ | CH₃ | CH(CH₃)CH(CH₃)-(2,4-Cl₂-C₆H₃) | NCH₃ |
| Ia1.1333 | OCH₃ | CH₂CH₃ | CH(CH₃)CH(CH₃)-(2,4-Cl₂-C₆H₃) | NCH₃ |
| Ia1.1334 | OCH₃ | (CH₂)₂CH₃ | CH(CH₃)CH(CH₃)-(2,4-Cl₂-C₆H₃) | NCH₃ |
| Ia1.1335 | OCH₃ | (CH₂)₃CH₃ | CH(CH₃)CH(CH₃)-(2,4-Cl₂-C₆H₃) | NCH₃ |
| Ia1.1336 | OCH₃ | H | (CH₂)₂-O-C₆H₅ | NCH₃ |
| Ia1.1337 | OCH₃ | CH₃ | (CH₂)₂-O-C₆H₅ | NCH₃ |
| Ia1.1338 | OCH₃ | CH₂CH₃ | (CH₂)₂-O-C₆H₅ | NCH₃ |
| Ia1.1339 | OCH₃ | (CH₂)₂CH₃ | (CH₂)₂-O-C₆H₅ | NCH₃ |
| Ia1.1340 | OCH₃ | (CH₂)₃CH₃ | (CH₂)₂-O-C₆H₅ | NCH₃ |
| Ia1.1341 | OCH₃ | H | CH₂CH(CH₃)-O-C₆H₅ | NCH₃ |
| Ia1.1342 | OCH₃ | CH₃ | CH₂CH(CH₃)-O-C₆H₅ | NCH₃ |
| Ia1.1343 | OCH₃ | CH₂CH₃ | CH₂CH(CH₃)-O-C₆H₅ | NCH₃ |
| Ia1.1344 | OCH₃ | (CH₂)₂CH₃ | CH₂CH(CH₃)-O-C₆H₅ | NCH₃ |
| Ia1.1345 | OCH₃ | (CH₂)₃CH₃ | CH₂CH(CH₃)-O-C₆H₅ | NCH₃ |
| Ia1.1346 | OCH₃ | H | CH(CH₃)CH₂-O-C₆H₅ | NCH₃ |
| Ia1.1347 | OCH₃ | CH₃ | CH(CH₃)CH₂-O-C₆H₅ | NCH₃ |
| Ia1.1348 | OCH₃ | CH₂CH₃ | CH(CH₃)CH₂-O-C₆H₅ | NCH₃ |
| Ia1.1349 | OCH₃ | (CH₂)₂CH₃ | CH(CH₃)CH₂-O-C₆H₅ | NCH₃ |
| Ia1.1350 | OCH₃ | (CH₂)₃CH₃ | CH(CH₃)CH₂-O-C₆H₅ | NCH₃ |
| Ia1.1351 | OCH₃ | H | CH(CH₃)CH(CH₃)-O-C₆H₅ | NCH₃ |
| Ia1.1352 | OCH₃ | CH₃ | CH(CH₃)CH(CH₃)-O-C₆H₅ | NCH₃ |
| Ia1.1353 | OCH₃ | CH₂CH₃ | CH(CH₃)CH(CH₃)-O-C₆H₅ | NCH₃ |
| Ia1.1354 | OCH₃ | (CH₂)₂CH₃ | CH(CH₃)CH(CH₃)-O-C₆H₅ | NCH₃ |
| Ia1.1355 | OCH₃ | (CH₂)₃CH₃ | CH(CH₃)CH(CH₃)-O-C₆H₅ | NCH₃ |
| Ia1.1356 | OCH₃ | H | (CH₂)₂-O-(4-Cl-C₆H₄) | NCH₃ |
| Ia1.1357 | OCH₃ | CH₃ | (CH₂)₂-O-(4-Cl-C₆H₄) | NCH₃ |
| Ia1.1358 | OCH₃ | CH₂CH₃ | (CH₂)₂-O-(4-Cl-C₆H₄) | NCH₃ |
| Ia1.1359 | OCH₃ | (CH₂)₂CH₃ | (CH₂)₂-O-(4-Cl-C₆H₄) | NCH₃ |
| Ia1.1360 | OCH₃ | (CH₂)₃CH₃ | (CH₂)₂-O-(4-Cl-C₆H₄) | NCH₃ |
| Ia1.1361 | OCH₃ | H | CH₂CH(CH₃)-O-(4-Cl-C₆H₄) | NCH₃ |
| Ia1.1362 | OCH₃ | CH₃ | CH₂CH(CH₃)-O-(4-Cl-C₆H₄) | NCH₃ |
| Ia1.1363 | OCH₃ | CH₂CH₃ | CH₂CH(CH₃)-O-(4-Cl-C₆H₄) | NCH₃ |
| Ia1.1364 | OCH₃ | (CH₂)₂CH₃ | CH₂CH(CH₃)-O-(4-Cl-C₆H₄) | NCH₃ |
| Ia1.1365 | OCH₃ | (CH₂)₃CH₃ | CH₂CH(CH₃)-O-(4-Cl-C₆H₄) | NCH₃ |
| Ia1.1366 | OCH₃ | H | CH(CH₃)CH₂-O-(4-Cl-C₆H₄) | NCH₃ |
| Ia1.1367 | OCH₃ | CH₃ | CH(CH₃)CH₂-O-(4-Cl-C₆H₄) | NCH₃ |
| Ia1.1368 | OCH₃ | CH₂CH₃ | CH(CH₃)CH₂-O-(4-Cl-C₆H₄) | NCH₃ |
| Ia1.1369 | OCH₃ | (CH₂)₂CH₃ | CH(CH₃)CH₂-O-(4-Cl-C₆H₄) | NCH₃ |
| la1.1370 | OCH₃ | (CH₂)₃CH₃ | CH(CH₃)CH₂-O-(4-Cl-C₆H₄) | NCH₃ |
| Ia1.1371 | OCH₃ | H | CH(CH₃)CH(CH₃)-O-(4-Cl-C₆H₄) | NCH₃ |
| Ia1.1372 | OCH₃ | CH₃ | CH(CH₃)CH(CH₃)-O-(4-Cl-C₆H₄) | NCH₃ |
| Ia1.1373 | OCH₃ | CH₂CH₃ | CH(CH₃)CH(CH₃)-O-(4-Cl-C₆H₄) | NCH₃ |
| Ia1.1374 | OCH₃ | (CH₂)₂CH₃ | CH(CH₃)CH(CH₃)-O-(4-Cl-C₆H₄) | NCH₃ |
| Ia1.1375 | OCH₃ | (CH₂)₃CH₃ | CH(CH₃)CH(CH₃)-O-(4-Cl-C₆H₄) | NCH₃ |
| Ia1.1376 | OCH₃ | H | (CH₂)₂-O-(2,4-Cl₂-C₆H₃) | NCH₃ |
| Ia1.1377 | OCH₃ | CH₃ | (CH₂)₂-O-(2,4-Cl₂-C₆H₃) | NCH₃ |
| Ia1.1378 | OCH₃ | CH₂CH₃ | (CH₂)₂-O-(2,4-Cl₂-C₆H₃) | NCH₃ |
| Ia1.1379 | OCH₃ | (CH₂)₂CH₃ | (CH₂)₂-O-(2,4-Cl₂-C₆H₃) | NCH₃ |
| Ia1.1380 | OCH₃ | (CH₂)₃CH₃ | (CH₂)₂-O-(2,4-Cl₂-C₆H₃) | NCH₃ |
| Ia1.1381 | OCH₃ | H | CH₂CH(CH₃)-O-(2,4-Cl₂-C₆H₃) | NCH₃ |
| Ia1.1382 | OCH₃ | CH₃ | CH₂CH(CH₃)-O-(2,4-Cl₂-C₆H₃) | NCH₃ |
| Ia1.1383 | OCH₃ | CH₂CH₃ | CH₂CH(CH₃)-O-(2,4-Cl₂-C₆H₃) | NCH₃ |
| Ia1.1384 | OCH₃ | (CH₂)₂CH₃ | CH₂CH(CH₃)-O-(2,4-Cl₂-C₆H₃) | NCH₃ |
| Ia1.1385 | OCH₃ | (CH₂)₃CH₃ | CH₂CH(CH₃)-O-(2,4-Cl₂-C₆H₃) | NCH₃ |
| Ia1.1386 | OCH₃ | H | CH(CH₃)CH₂-O-(2,4-Cl₂-C₆H₃) | NCH₃ |
| Ia1.1387 | OCH₃ | CH₃ | CH(CH₃)CH₂-O-(2,4-Cl₂-C₆H₃) | NCH₃ |
| Ia1.1388 | OCH₃ | CH₂CH₃ | CH(CH₃)CH₂-O-(2,4-Cl₂-C₆H₃) | NCH₃ |
| Ia1.1389 | OCH₃ | (CH₂)₂CH₃ | CH(CH₃)CH₂-O-(2,4-Cl₂-C₆H₃) | NCH₃ |
| Ia1.1390 | OCH₃ | (CH₂)₃CH₃ | CH(CH₃)CH₂-O-(2,4-Cl₂-C₆H₃) | NCH₃ |
| Ia1.1391 | OCH₃ | H | CH(CH₃)CH(CH₃)-O-(2,4-Cl₂-C₆H₃) | NCH₃ |
| Ia1.1392 | OCH₃ | CH₃ | CH(CH₃)CH(CH₃)-O-(2,4-Cl₂-C₆H₃) | NCH₃ |
| Ia1.1393 | OCH₃ | CH₂CH₃ | CH(CH₃)CH(CH₃)-O-(2,4-Cl₂-C₆H₃) | NCH₃ |
| Ia1.1394 | OCH₃ | (CH₂)₂CH₃ | CH(CH₃)CH(CH₃)-O-(2,4-Cl₂-C₆H₃) | NCH₃ |
| Ia1.1395 | OCH₃ | (CH₂)₃CH₃ | CH(CH₃)CH(CH₃)-O-(2,4-Cl₂-C₆H₃) | NCH₃ |
| Ia1.1396 | CF₃ | H | CH₃ | O |
| Ia1.1397 | CF₃ | CH₃ | CH₃ | O |
| Ia1.1398 | CF₃ | CH₂CH₃ | CH₃ | O |
| Ia1.1399 | CF₃ | (CH₂)₂CH₃ | CH₃ | O |
| Ia1.1400 | CF₃ | (CH₂)₃CH₃ | CH₃ | O |
| Ia1.1401 | CF₃ | H | C₂H₅ | O |
| Ia1.1402 | CF₃ | CH₃ | C₂H₅ | O |
| Ia1.1403 | CF₃ | CH₂CH₃ | C₂H₅ | O |
| Ia1.1404 | CF₃ | (CH₂)₂CH₃ | C₂H₅ | O |
| Ia1.1405 | CF₃ | (CH₂)₃CH₃ | C₂H₅ | O |
| Ia1.1406 | CF₃ | H | (CH₂)₂CH₃ | O |
| Ia1.1407 | CF₃ | CH₃ | (CH₂)₂CH₃ | O |
| Ia1.1408 | CF₃ | CH₂CH₃ | (CH₂)₂CH₃ | O |
| Ia1.1409 | CF₃ | (CH₂)₂CH₃ | (CH₂)₂CH₃ | O |
| Ia1.1410 | CF₃ | (CH₂)₃CH₃ | (CH₂)₂CH₃ | O |
| Ia1.1411 | CF₃ | H | CH(CH₃)₂ | O |
| Ia1.1412 | CF₃ | CH₃ | CH(CH₃)₂ | O |
| Ia1.1413 | CF₃ | CH₂CH₃ | CH(CH₃)₂ | O |
| Ia1.1414 | CF₃ | (CH₂)₂CH₃ | CH(CH₃)₂ | O |
| Ia1.1415 | CF₃ | (CH₂)₃CH₃ | CH(CH₃)₂ | O |
| Ia1.1416 | CF₃ | H | (CH₂)₃CH₃ | O |
| Ia1.1417 | CF₃ | CH₃ | (CH₂)₃CH₃ | O |
| Ia1.1418 | CF₃ | CH₂CH₃ | (CH₂)₃CH₃ | O |
| Ia1.1419 | CF₃ | (CH₂)₂CH₃ | (CH₂)₃CH₃ | O |
| Ia1.1420 | CF₃ | (CH₂)₃CH₃ | (CH₂)₃CH₃ | O |
| Ia1.1421 | CF₃ | H | CH₂CH(CH₃)₂ | O |
| Ia1.1422 | CF₃ | CH₃ | CH₂CH(CH₃)₂ | O |
| Ia1.1423 | CF₃ | CH₂CH₃ | CH₂CH(CH₃)₂ | O |
| Ia1.1424 | CF₃ | (CH₂)₂CH₃ | CH₂CH(CH₃)₂ | O |
| Ia1.1425 | CF₃ | (CH₂)₃CH₃ | CH₂CH(CH₃)₂ | O |
| Ia1.1426 | CF₃ | H | CH(CH₃)CH₂CH₃ | O |
| Ia1.1427 | CF₃ | CH₃ | CH(CH₃)CH₂CH₃ | O |
| Ia1.1428 | CF₃ | CH₂CH₃ | CH(CH₃)CH₂CH₃ | O |
| Ia1.1429 | CF₃ | (CH₂)₂CH₃ | CH(CH₃)CH₂CH₃ | O |
| Ia1.1430 | CF₃ | (CH₂)₃CH₃ | CH(CH₃)CH₂CH₃ | O |
| Ia1.1431 | CF₃ | H | (CH₂)₂-C₆H₅ | O |
| Ia1.1432 | CF₃ | CH₃ | (CH₂)₂-C₆H₅ | O |
| Ia1.1433 | CF₃ | CH₂CH₃ | (CH₂)₂-C₆H₅ | O |
| Ia1.1434 | CF₃ | (CH₂)₂CH₃ | (CH₂)₂-C₆H₅ | O |
| Ia1.1435 | CF₃ | (CH₂)₃CH₃ | (CH₂)₂-C₆H₅ | O |
| Ia1.1436 | CF₃ | H | CH₂CH(CH₃)-C₆H₅ | O |
| Ia1.1437 | CF₃ | CH₃ | CH₂CH(CH₃)-C₆H₅ | O |
| Ia1.1438 | CF₃ | CH₂CH₃ | CH₂CH(CH₃)-C₆H₅ | O |
| Ia1.1439 | CF₃ | (CH₂)₂CH₃ | CH₂CH(CH₃)-C₆H₅ | O |
| Ia1.1440 | CF₃ | (CH₂)₃CH₃ | CH₂CH(CH₃)-C₆H₅ | O |
| Ia1.1441 | CF₃ | H | CH(CH₃)CH₂-C₆H₅ | O |
| Ia1.1442 | CF₃ | CH₃ | CH(CH₃)CH₂-C₆H₅ | O |
| Ia1.1443 | CF₃ | CH₂CH₃ | CH(CH₃)CH₂-C₆H₅ | O |
| Ia1.1444 | CF₃ | (CH₂)₂CH₃ | CH(CH₃)CH₂-C₆H₅ | O |
| Ia1.1445 | CF₃ | (CH₂)₃CH₃ | CH(CH₃)CH₂-C₆H₅ | O |
| Ia1.1446 | CF₃ | H | CH(CH₃)CH(CH₃)-C₆H₅ | O |
| Ia1.1447 | CF₃ | CH₃ | CH(CH₃)CH(CH₃)-C₆H₅ | O |
| Ia1.1448 | CF₃ | CH₂CH₃ | CH(CH₃)CH(CH₃)-C₆H₅ | O |
| Ia1.1449 | CF₃ | (CH₂)₂CH₃ | CH(CH₃)CH(CH₃)-C₆H₅ | O |
| Ia1.1450 | CF₃ | (CH₂)₃CH₃ | CH(CH₃)CH(CH₃)-C₆H₅ | O |
| Ia1.1451 | CF₃ | H | (CH₂)₂-(4-Cl-C₆H₄) | O |
| Ia1.1452 | CF₃ | CH₃ | (CH₂)₂-(4-Cl-C₆H₄) | O |
| Ia1.1453 | CF₃ | CH₂CH₃ | (CH₂)₂-(4-Cl-C₆H₄) | O |
| Ia1.1454 | CF₃ | (CH₂)₂CH₃ | (CH₂)₂-(4-Cl-C₆H₄) | O |
| Ia1.1455 | CF₃ | (CH₂)₃CH₃ | (CH₂)₂-(4-Cl-C₆H₄) | O |
| Ia1.1456 | CF₃ | H | CH₂CH (CH₃)-(4-Cl-C₆H₄) | O |
| Ia1.1457 | CF₃ | CH₃ | CH₂CH(CH₃)-(4-Cl-C₆H₄) | O |
| Ia1.1458 | CF₃ | CH₂CH₃ | CH₂CH(CH₃)-(4-Cl-C₆H₄) | O |
| Ia1.1459 | CF₃ | (CH₂)₂CH₃ | CH₂CH(CH₃)-(4-Cl-C₆H₄) | O |
| Ia1.1460 | CF₃ | (CH₂)₃CH₃ | CH₂CH(CH₃)-(4-Cl-C₆H₄) | O |
| Ia1.1461 | CF₃ | H | CH(CH₃)CH₂-(4-Cl-C₆H₄) | O |
| Ia1.1462 | CF₃ | CH₃ | CH(CH₃)CH₂-(4-Cl-C₆H₄) | O |
| Ia1.1463 | CF₃ | CH₂CH₃ | CH(CH₃)CH₂-(4-Cl-C₆H₄) | O |
| Ia1.1464 | CF₃ | (CH₂)₂CH₃ | CH(CH₃)CH₂-(4-Cl-C₆H₄) | O |
| Ia1.1465 | CF₃ | (CH₂)₃CH₃ | CH(CH₃)CH₂-(4-Cl-C₆H₄) | O |
| Ia1.1466 | CF₃ | H | CH(CH₃)CH(CH₃)-(4-Cl-C₆H₄) | O |
| Ia1.1467 | CF₃ | CH₃ | CH(CH₃)CH(CH₃)-(4-Cl-C₆H₄) | O |
| Ia1.1468 | CF₃ | CH₂CH₃ | CH(CH₃)CH(CH₃)-(4-Cl-C₆H₄) | O |
| Ia1.1469 | CF₃ | (CH₂)₂CH₃ | CH(CH₃)CH(CH₃)-(4-Cl-C₆H₄) | O |
| Ia1.1470 | CF₃ | (CH₂)₃CH₃ | CH(CH₃)CH(CH₃)-(4-Cl-C₆H₄) | O |
| Ia1.1471 | CF₃ | H | (CH₂)₂-(2,4-Cl₂-C₆H₃) | O |
| Ia1.1472 | CF₃ | CH₃ | (CH₂)₂-(2,4-Cl₂-C₆H₃) | O |
| Ia1.1473 | CF₃ | CH₂CH₃ | (CH₂)₂-(2,4-Cl₂-C₆H₃) | O |
| Ia1.1474 | CF₃ | (CH₂)₂CH₃ | (CH₂)₂-(2,4-Cl₂-C₆H₃) | O |
| Ia1.1475 | CF₃ | (CH₂)₃CH₃ | (CH₂)₂-(2,4-Cl₂-C₆H₃) | O |
| Ia1.1476 | CF₃ | H | CH₂CH(CH₃)-(2,4-Cl₂-C₆H₃) | O |
| Ia1.1477 | CF₃ | CH₃ | CH₂CH(CH₃)-(2,4-Cl₂-C₆H₃) | O |
| Ia1.1478 | CF₃ | CH₂CH₃ | CH₂CH(CH₃)-(2,4-Cl₂-C₆H₃) | O |
| Ia1.1479 | CF₃ | (CH₂)₂CH₃ | CH₂CH(CH₃)-(2,4-Cl₂-C₆H₃) | O |
| Ia1.1480 | CF₃ | (CH₂)₃CH₃ | CH₂CH(CH₃)-(2,4-Cl₂-C₆H₃) | O |
| Ia1.1481 | CF₃ | H | CH(CH₃)CH₂-(2,4-Cl₂-C₆H₃) | O |
| Ia1.1482 | CF₃ | CH₃ | CH(CH₃)CH₂-(2,4-Cl₂-C₆H₃) | O |
| Ia1.1483 | CF₃ | CH₂CH₃ | CH(CH₃)CH₂-(2,4-Cl₂-C₆H₃) | O |
| Ia1.1484 | CF₃ | (CH₂)₂CH₃ | CH(CH₃)CH₂-(2,4-Cl₂-C₆H₃) | O |
| Ia1.1485 | CF₃ | (CH₂)₃CH₃ | CH(CH₃)CH₂-(2,4-Cl₂-C₆H₃) | O |
| Ia1.1486 | CF₃ | H | CH(CH₃)CH(CH₃)-(2,4-Cl₂-C₆H₃) | O |
| Ia1.1487 | CF₃ | CH₃ | CH(CH₃)CH(CH₃)-(2,4-Cl₂-C₆H₃) | O |
| Ia1.1488 | CF₃ | CH₂CH₃ | CH(CH₃)CH(CH₃)-(2,4-Cl₂-C₆H₃) | O |
| Ia1.1489 | CF₃ | (CH₂)₂CH₃ | CH(CH₃)CH(CH₃)-(2,4-Cl₂-C₆H₃) | O |
| Ia1.1490 | CF₃ | (CH₂)₃CH₃ | CH(CH₃)CH(CH₃)-(2,4-Cl₂-C₆H₃) | O |
| Ia1.1491 | CF₃ | H | (CH₂)₂-O-C₆H₅ | O |
| Ia1.1492 | CF₃ | CH₃ | (CH₂)₂-O-C₆H₅ | O |
| Ia1.1493 | CF₃ | CH₂CH₃ | (CH₂)₂-O-C₆H₅ | O |
| Ia1.1494 | CF₃ | (CH₂)₂CH₃ | (CH₂)₂-O-C₆H₅ | O |
| Ia1.1495 | CF₃ | (CH₂)₃CH₃ | (CH₂)₂-O-C₆H₅ | O |
| Ia1.1496 | CF₃ | H | CH₂CH(CH₃)-O-C₆H₅ | O |
| Ia1.1497 | CF₃ | CH₃ | CH₂CH(CH₃)-O-C₆H₅ | O |
| Ia1.1498 | CF₃ | CH₂CH₃ | CH₂CH(CH₃)-O-C₆H₅ | O |
| Ia1.1499 | CF₃ | (CH₂)₂CH₃ | CH₂CH(CH₃)-O-C₆H₅ | O |
| Ia1.1500 | CF₃ | (CH₂)₃CH₃ | CH₂CH(CH₃)-O-C₆H₅ | O |
| Ia1.1501 | CF₃ | H | CH(CH₃)CH₂-O-C₆H₅ | O |
| Ia1.1502 | CF₃ | CH₃ | CH(CH₃)CH₂-O-C₆H₅ | O |
| Ia1.1503 | CF₃ | CH₂CH₃ | CH(CH₃)CH₂-O-C₆H₅ | O |
| Ia1.1504 | CF₃ | (CH₂)₂CH₃ | CH(CH₃)CH₂-O-C₆H₅ | O |
| Ia1.1505 | CF₃ | (CH₂)₃CH₃ | CH(CH₃)CH₂-O-C₆H₅ | O |
| Ia1.1506 | CF₃ | H | CH(CH₃)CH(CH₃)-O-C₆H₅ | O |
| Ia1.1507 | CF₃ | CH₃ | CH(CH₃)CH(CH₃)-O-C₆H₅ | O |
| Ia1.1508 | CF₃ | CH₂CH₃ | CH(CH₃)CH(CH₃)-O-C₆H₅ | O |
| Ia1.1509 | CF₃ | (CH₂)₂CH₃ | CH(CH₃)CH(CH₃)-O-C₆H₅ | O |
| Ia1.1510 | CF₃ | (CH₂)₃CH₃ | CH(CH₃)CH(CH₃)-O-C₆H₅ | O |
| Ia1.1511 | CF₃ | H | (CH₂)₂-O-(4-Cl-C₆H₄) | O |
| Ia1.1512 | CF₃ | CH₃ | (CH₂)₂-O-(4-Cl-C₆H₄) | O |
| Ia1.1513 | CF₃ | CH₂CH₃ | (CH₂)₂-O-(4-Cl-C₆H₄) | O |
| Ia1.1514 | CF₃ | (CH₂)₂CH₃ | (CH₂)₂-O-(4-Cl-C₆H₄) | O |
| Ia1.1515 | CF₃ | (CH₂)₃CH₃ | (CH₂)₂-O-(4-Cl-C₆H₄) | O |
| Ia1.1516 | CF₃ | H | CH₂CH(CH₃)-O-(4-Cl-C₆H₄) | O |
| Ia1.1517 | CF₃ | CH₃ | CH₂CH(CH₃)-O-(4-Cl-C₆H₄) | O |
| Ia1.1518 | CF₃ | CH₂CH₃ | CH₂CH(CH₃)-O-(4-Cl-C₆H₄) | O |
| Ia1.1519 | CF₃ | (CH₂)₂CH₃ | CH₂CH(CH₃)-O-(4-Cl-C₆H₄) | O |
| Ia1.1520 | CF₃ | (CH₂)₃CH₃ | CH₂CH(CH₃)-O-(4-Cl-C₆H₄) | O |
| Ia1.1521 | CF₃ | H | CH(CH₃)CH₂-O-(4-Cl-C₆H₄) | O |
| Ia1.1522 | CF₃ | CH₃ | CH(CH₃)CH₂-O-(4-Cl-C₆H₄) | O |
| Ia1.1523 | CF₃ | CH₂CH₃ | CH(CH₃)CH₂-O-(4-Cl-C₆H₄) | O |
| Ia1.1524 | CF₃ | (CH₂)₂CH₃ | CH(CH₃)CH₂-O-(4-Cl-C₆H₄) | O |
| Ia1.1525 | CF₃ | (CH₂)₃CH₃ | CH(CH₃)CH₂-O-(4-Cl-C₆H₄) | O |
| Ia1.1526 | CF₃ | H | CH(CH₃)CH(CH₃)-O-(4-Cl-C₆H₄) | O |
| Ia1.1527 | CF₃ | CH₃ | CH(CH₃)CH(CH₃)-O-(4-Cl-C₆H₄) | O |
| Ia1.1528 | CF₃ | CH₂CH₃ | CH(CH₃)CH(CH₃)-O-(4-Cl-C₆H₄) | O |
| Ia1.1529 | CF₃ | (CH₂)₂CH₃ | CH(CH₃)CH(CH₃)-O-(4-Cl-C₆H₄) | O |
| Ia1.1530 | CF₃ | (CH₂)₃CH₃ | CH(CH₃)CH(CH₃)-O-(4-Cl-C₆H₄) | O |
| Ia1.1531 | CF₃ | H | (CH₂)₂-O-(2,4-Cl₂-C₆H₃) | O |
| Ia1.1532 | CF₃ | CH₃ | (CH₂)₂-O-(2,4-Cl₂-C₆H₃) | O |
| Ia1.1533 | CF₃ | CH₂CH₃ | (CH₂)₂-O-(2,4-Cl₂-C₆H₃) | O |
| Ia1.1534 | CF₃ | (CH₂)₂CH₃ | (CH₂)₂-O-(2,4-Cl₂-C₆H₃) | O |
| Ia1.1535 | CF₃ | (CH₂)₃CH₃ | (CH₂)₂-O-(2,4-Cl₂-C₆H₃) | O |
| Ia1.1536 | CF₃ | H | CH₂CH(CH₃)-O-(2,4-Cl₂-C₆H₃) | O |
| Ia1.1537 | CF₃ | CH₃ | CH₂CH(CH₃)-O-(2,4-Cl₂-C₆H₃) | O |
| Ia1.1538 | CF₃ | CH₂CH₃ | CH₂CH(CH₃)-O-(2,4-Cl₂-C₆H₃) | O |
| Ia1.1539 | CF₃ | (CH₂)₂CH₃ | CH₂CH(CH₃)-O-(2,4-Cl₂-C₆H₃) | O |
| Ia1.1540 | CF₃ | (CH₂)₃CH₃ | CH₂CH(CH₃)-O-(2,4-Cl₂-C₆H₃) | O |
| Ia1.1541 | CF₃ | H | CH(CH₃)CH₂-O-(2,4-Cl₂-C₆H₃) | O |
| Ia1.1542 | CF₃ | CH₃ | CH(CH₃)CH₂-O-(2,4-Cl₂-C₆H₃) | O |
| Ia1.1543 | CF₃ | CH₂CH₃ | CH(CH₃)CH₂-O-(2,4-Cl₂-C₆H₃) | O |
| Ia1.1544 | CF₃ | (CH₂)₂CH₃ | CH(CH₃)CH₂-O-(2,4-Cl₂-C₆H₃) | O |
| Ia1.1545 | CF₃ | (CH₂)₃CH₃ | CH(CH₃)CH₂-O-(2,4-Cl₂-C₆H₃) | O |
| Ia1.1546 | CF₃ | H | CH(CH₃)CH(CH₃)-O-(2,4-Cl₂-C₆H₃) | O |
| Ia1.1547 | CF₃ | CH₃ | CH(CH₃)CH(CH₃)-O-(2,4-Cl₂-C₆H₃) | O |
| Ia1.1548 | CF₃ | CH₂CH₃ | CH(CH₃)CH(CH₃)-O-(2,4-Cl₂-C₆H₃) | O |
| Ia1.1549 | CF₃ | (CH₂)₂CH₃ | CH(CH₃)CH(CH₃)-O-(2,4-Cl₂-C₆H₃) | O |
| Ia1.1550 | CF₃ | (CH₂)₃CH₃ | CH(CH₃)CH(CH₃)-O-(2,4-Cl₂-C₆H₃) | O |
| Ia1.1551 | CF₃ | H | CH₃ | NH |
| Ia1.1552 | CF₃ | CH₃ | CH₃ | NH |
| Ia1.1553 | CF₃ | CH₂CH₃ | CH₃ | NH |
| Ia1.1554 | CF₃ | (CH₂)₂CH₃ | CH₃ | NH |
| Ia1.1555 | CF₃ | (CH₂)₃CH₃ | CH₃ | NH |
| Ia1.1556 | CF₃ | H | C₂H₅ | NH |
| Ia1.1557 | CF₃ | CH₃ | C₂H₅ | NH |
| Ia1.1558 | CF₃ | CH₂CH₃ | C₂H₅ | NH |
| Ia1.1559 | CF₃ | (CH₂)₂CH₃ | C₂H₅ | NH |
| Ia1.1560 | CF₃ | (CH₂)₃CH₃ | C₂H₅ | NH |
| Ia1.1561 | CF₃ | H | (CH₂)₂CH₃ | NH |
| Ia1.1562 | CF₃ | CH₃ | (CH₂)₂CH₃ | NH |
| Ia1.1563 | CF₃ | CH₂CH₃ | (CH₂)₂CH₃ | NH |
| Ia1.1564 | CF₃ | (CH₂)₂CH₃ | (CH₂)₂CH₃ | NH |
| Ia1.1565 | CF3 | (CH₂)₃CH₃ | (CH₂)₂CH₃ | NH |
| Ia1.1566 | CF₃ | H | CH(CH₃)₂ | NH |
| Ia1.1567 | CF₃ | CH₃ | CH(CH₃)₂ | NH |
| Ia1.1568 | CF₃ | CH₂CH₃ | CH(CH₃)₂ | NH |
| Ia1.1569 | CF₃ | (CH₂)₂CH₃ | CH(CH₃)₂ | NH |
| Ia1.1570 | CF₃ | (CH₂)₃CH₃ | CH(CH₃)₂ | NH |
| Ia1.1571 | CF₃ | H | (CH₂)₃CH₃ | NH |
| Ia1.1572 | CF₃ | CH₃ | (CH₂)₃CH₃ | NH |
| Ia1.1573 | CF₃ | CH₂CH₃ | (CH₂)₃CH₃ | NH |
| Ia1.1574 | CF₃ | (CH₂)₂CH₃ | (CH₂)₃CH₃ | NH |
| Ia1.1575 | CF₃ | (CH₂)₃CH₃ | (CH₂)₃CH₃ | NH |
| Ia1.1576 | CF₃ | H | CH₂CH(CH₃)₂ | NH |
| Ia1.1577 | CF₃ | CH₃ | CH₂CH(CH₃)₂ | NH |
| Ia1.1578 | CF₃ | CH₂CH₃ | CH₂CH(CH₃)₂ | NH |
| Ia1.1579 | CF₃ | (CH₂)₂CH₃ | CH₂CH(CH₃)₂ | NH |
| Ia1.1580 | CF₃ | (CH₂)₃CH₃ | CH₂CH(CH₃)₂ | NH |
| Ia1.1581 | CF₃ | H | CH(CH₃)CH₂CH₃ | NH |
| Ia1.1582 | CF₃ | CH₃ | CH(CH₃)CH₂CH₃ | NH |
| Ia1.1583 | CF₃ | CH₂CH₃ | CH(CH₃)CH₂CH₃ | NH |
| Ia1.1584 | CF₃ | (CH₂)₂CH₃ | CH(CH₃)CH₂CH₃ | NH |
| Ia1.1585 | CF₃ | (CH₂)₃CH₃ | CH(CH₃)CH₂CH₃ | NH |
| Ia1.1586 | CF₃ | H | (CH₂)₂-C₆H₅ | NH |
| Ia1.1587 | CF₃ | CH₃ | (CH₂)₂-C₆H₅ | NH |
| Ia1.1588 | CF₃ | CH₂CH₃ | (CH₂)₂-C₆H₅ | NH |
| Ia1.1589 | CF₃ | (CH₂)₂CH₃ | (CH₂)₂-C₆H₅ | NH |
| Ia1.1590 | CF₃ | (CH₂)₃CH₃ | (CH₂)₂-C₆H₅ | NH |
| Ia1.1591 | CF₃ | H | CH₂CH(CH₃)-C₆H₅ | NH |
| Ia1.1592 | CF₃ | CH₃ | CH₂CH(CH₃)-C₆H₅ | NH |
| Ia1.1593 | CF₃ | CH₂CH₃ | CH₂CH(CH₃)-C₆H₅ | NH |
| Ia1.1594 | CF₃ | (CH₂)₂CH₃ | CH₂CH(CH₃)-C₆H₅ | NH |
| Ia1.1595 | CF₃ | (CH₂)₃CH₃ | CH₂CH(CH₃)-C₆H₅ | NH |
| Ia1.1596 | CF₃ | H | CH(CH₃)CH₂-C₆H₅ | NH |
| Ia1.1597 | CF₃ | CH₃ | CH(CH₃)CH₂-C₆H₅ | NH |
| Ia1.1598 | CF₃ | CH₂CH₃ | CH(CH₃)CH₂-C₆H₅ | NH |
| Ia1.1599 | CF₃ | (CH₂)₂CH₃ | CH(CH₃)CH₂-C₆H₅ | NH |
| Ia1.1600 | CF₃ | (CH₂)₃CH₃ | CH(CH₃)CH₂-C₆H₅ | NH |
| Ia1.1601 | CF₃ | H | CH(CH₃)CH(CH₃)-C₆H₅ | NH |
| Ia1.1602 | CF₃ | CH₃ | CH(CH₃)CH(CH₃)-C₆H₅ | NH |
| Ia1.1603 | CF₃ | CH₂CH₃ | CH(CH₃)CH(CH₃)-C₆H₅ | NH |
| Ia1.1604 | CF₃ | (CH₂)₂CH₃ | CH(CH₃)CH(CH₃)-C₆H₅ | NH |
| Ia1.1605 | CF₃ | (CH₂)₃CH₃ | CH(CH₃)CH(CH₃)-C₆H₅ | NH |
| Ia1.1606 | CF₃ | H | (CH₂)₂-(4-Cl-C₆H₄) | NH |
| Ia1.1607 | CF₃ | CH₃ | (CH₂)₂ (4-Cl-C₆H₄) | NH |
| Ia1.1608 | CF₃ | CH₂CH₃ | (CH₂)₂-(4-Cl-C₆H₄) | NH |
| Ia1.1609 | CF₃ | (CH₂)₂CH₃ | (CH₂)₂-(4-Cl-C₆H₄) | NH |
| Ia1.1610 | CF₃ | (CH₂)₃CH₃ | (CH₂)₂-(4-Cl-C₆H₄ ) | NH |
| Ia1.1611 | CF₃ | H | CH₂CH(CH₃)-(4-Cl-C₆H₄) | NH |
| Ia1.1612 | CF₃ | CH₃ | CH₂CH(CH₃)-(4-Cl-C₆H₄) | NH |
| Ia1.1613 | CF₃ | CH₂CH₃ | CH₂CH(CH₃)-(4-Cl-C₆H₄) | NH |
| Ia1.1614 | CF₃ | (CH₂)₂CH₃ | CH₂CH(CH₃)-(4-Cl-C₆H₄) | NH |
| Ia1.1615 | CF₃ | (CH₂)₃CH₃ | CH₂CH(CH₃)-(4-Cl-C₆H₄ ) | NH |
| Ia1.1616 | CF₃ | H | CH(CH₃)CH₂-(4-Cl-C₆H₄) | NH |
| Ia1.1617 | CF₃ | CH₃ | CH(CH₃)CH₂-(4-Cl-C₆H₄) | NH |
| Ia1.1618 | CF₃ | CH₂CH₃ | CH(CH₃)CH₂-(4-Cl-C₆H₄) | NH |
| Ia1.1619 | CF₃ | (CH₂)₂CH₃ | CH(CH₃)CH₂-(4-Cl-C₆H₄) | NH |
| Ia1.1620 | CF₃ | (CH₂)₃CH₃ | CH(CH₃)CH₂-(4-Cl-C₆H₄) | NH |
| Ia1.1621 | CF₃ | H | CH(CH₃)CH(CH₃)-(4-Cl-C₆H₄) | NH |
| Ia1.1622 | CF₃ | CH₃ | CH(CH₃)CH(CH₃)-(4-Cl-C₆H₄) | NH |
| Ia1.1623 | CF₃ | CH₂CH₃ | CH(CH₃)CH(CH₃)-(4-Cl-C₆H₄) | NH |
| Ia1.1624 | CF₃ | (CH₂)₂CH₃ | CH(CH₃)CH(CH₃)-(4-Cl-C₆H₄) | NH |
| Ia1.1625 | CF₃ | (CH₂)₃CH₃ | CH(CH₃)CH(CH₃)-(4-Cl-C₆H₄) | NH |
| Ia1.1626 | CF₃ | H | (CH₂)₂-(2,4-Cl₂-C₆H₃) | NH |
| Ia1.1627 | CF₃ | CH₃ | (CH₂)₂-(2,4-Cl₂-C₆H₃) | NH |
| Ia1.1628 | CF₃ | CH₂CH₃ | (CH₂)₂-(2,4-Cl₂-C₆H₃) | NH |
| Ia1.1629 | CF₃ | (CH₂)₂CH₃ | (CH₂)₂-(2,4-Cl₂-C₆H₃) | NH |
| Ia1.1630 | CF₃ | (CH₂)₃CH₃ | (CH₂)₂-(2,4-Cl₂-C₆H₃) | NH |
| Ia1.1631 | CF₃ | H | CH₂CH(CH₃)-(2,4-Cl₂-C₆H₃) | NH |
| Ia1.1632 | CF₃ | CH₃ | CH₂CH(CH₃)-(2,4-Cl₂-C₆H₃) | NH |
| Ia1.1633 | CF₃ | CH₂CH₃ | CH₂CH(CH₃)-(2,4-Cl₂-C₆H₃) | NH |
| Ia1.1634 | CF₃ | (CH₂)₂CH₃ | CH₂CH(CH₃)-(2,4-Cl₂-C₆H₃) | NH |
| Ia1.1635 | CF₃ | (CH₂)₃CH₃ | CH₂CH(CH₃)-(2,4-Cl₂-C₆H₃) | NH |
| Ia1.1636 | CF₃ | H | CH(CH₃)CH₂-(2,4-Cl₂-C₆H₃) | NH |
| Ia1.1637 | CF₃ | CH₃ | CH(CH₃)CH₂-(2,4-Cl₂-C₆H₃) | NH |
| Ia1.1638 | CF₃ | CH₂CH₃ | CH(CH₃)CH₂-(2,4-Cl₂-C₆H₃) | NH |
| Ia1.1639 | CF₃ | (CH₂)₂CH₃ | CH(CH₃)CH₂-(2,4-Cl₂-C₆H₃) | NH |
| Ia1.1640 | CF₃ | (CH₂)₃CH₃ | CH(CH₃)CH₂-(2,4-Cl₂-C₆H₃) | NH |
| Ia1.1641 | CF₃ | H | CH(CH₃)CH(CH₃)-(2,4-Cl₂-C₆H₃) | NH |
| Ia1.1642 | CF₃ | CH₃ | CH(CH₃)CH(CH₃)-(2,4-Cl₂-C₆H₃) | NH |
| Ia1.1643 | CF₃ | CH₂CH₃ | CH(CH₃)CH(CH₃)-(2,4-Cl₂-C₆H₃) | NH |
| Ia1.1644 | CF₃ | (CH₂)₂CH₃ | CH(CH₃)CH(CH₃)- (2,4-Cl₂-C₆H₃) | NH |
| Ia1.1645 | CF₃ | (CH₂)₃CH₃ | CH(CH₃)CH(CH₃)-(2,4-Cl₂-C₆H₃) | NH |
| Ia1.1646 | CF₃ | H | (CH₂)₂-O-C₆H₅ | NH |
| Ia1.1647 | CF₃ | CH₃ | (CH₂)₂-O-C₆H₅ | NH |
| Ia1.1648 | CF₃ | CH₂CH₃ | (CH₂)₂-O-C₆H₅ | NH |
| Ia1.1649 | CF₃ | (CH₂)₂CH₃ | (CH₂)₂-O-C₆H₅ | NH |
| Ia1.1650 | CF₃ | (CH₂)₃CH₃ | (CH₂)₂-O-C₆H₅ | NH |
| Ia1.1651 | CF₃ | H | CH₂CH(CH₃)-O-C₆H₅ | NH |
| Ia1.1652 | CF₃ | CH₃ | CH₂CH(CH₃)-O-C₆H₅ | NH |
| Ia1.1653 | CF₃ | CH₂CH₃ | CH₂CH(CH₃)-O-C₆H₅ | NH |
| Ia1.1654 | CF₃ | (CH₂)₂CH₃ | CH₂CH(CH₃)-O-C₆H₅ | NH |
| Ia1.1655 | CF₃ | (CH₂)₃CH₃ | CH₂CH(CH₃)-O-C₆H₅ | NH |
| Ia1.1656 | CF₃ | H | CH(CH₃)CH₂-O-C₆H₅ | NH |
| Ia1.1657 | CF₃ | CH₃ | CH(CH₃)CH₂-O-C₆H₅ | NH |
| Ia1.1658 | CF₃ | CH₂CH₃ | CH(CH₃)CH₂-O-C₆H₅ | NH |
| Ia1.1659 | CF₃ | (CH₂)₂CH₃ | CH(CH₃)CH₂-O-C₆H₅ | NH |
| Ia1.1660 | CF₃ | (CH₂)₃CH₃ | CH(CH₃)CH₂-O-C₆H₅ | NH |
| Ia1.1661 | CF₃ | H | CH(CH₃)CH(CH₃)-O-C₆H₅ | NH |
| Ia1.1662 | CF₃ | CH₃ | CH(CH₃)CH(CH₃)-O-C₆H₅ | NH |
| Ia1.1663 | CF₃ | CH₂CH₃ | CH(CH₃)CH(CH₃)-O-C₆H₅ | NH |
| Ia1.1664 | CF₃ | (CH₂)₂CH₃ | CH(CH₃)CH(CH₃)-O-C₆H₅ | NH |
| Ia1.1665 | CF₃ | (CH₂)₃CH₃ | CH(CH₃)CH(CH₃)-O-C₆H₅ | NH |
| Ia1.1666 | CF₃ | H | (CH₂)₂-O-(4-Cl-C₆H₄) | NH |
| Ia1.1667 | CF₃ | CH₃ | (CH₂)₂-O-(4-Cl-C₆H₄) | NH |
| Ia1.1668 | CF₃ | CH₂CH₃ | (CH₂)₂-O-(4-Cl-C₆H₄) | NH |
| Ia1.1669 | CF₃ | (CH₂)₂CH₃ | (CH₂)₂-O-(4-Cl-C₆H₄) | NH |
| Ia1.1670 | CF₃ | (CH₂)₃CH₃ | (CH₂)₂-O-(4-Cl-C₆H₄) | NH |
| Ia1.1671 | CF₃ | H | CH₂CH(CH₃)-O-(4-Cl-C₆H₄) | NH |
| Ia1.1672 | CF₃ | CH₃ | CH₂CH(CH₃)-O-(4-C1-C₆H₄) | NH |
| Ia1.1673 | CF₃ | CH₂CH₃ | CH₂CH(CH₃)-O-(4-Cl-C₆H₄) | NH |
| Ia1.1674 | CF₃ | (CH₂)₂CH₃ | CH₂CH(CH₃-O-(4-Cl-C₆H₄) | NH |
| Ia1.1675 | CF₃ | (CH₂)₃CH₃ | CH₂CH(CH₃)-O-(4-Cl-C₆H₄) | NH |
| Ia1.1676 | CF₃ | H | CH(CH₃)CH₂-O-(4-Cl-C₆H₄) | NH |
| Ia1.1677 | CF₃ | CH₃ | CH(CH₃)CH₂-O-(4-Cl-C₆H₄) | NH |
| Ia1.1678 | CF₃ | CH₂CH₃ | CH(CH₃)CH₂-O-(4-Cl-C₆H₄) | NH |
| Ia1.1679 | CF₃ | (CH₂)₂CH₃ | CH(CH₃)CH₂-O-(4-Cl-C₆H₄) | NH |
| Ia1.1680 | CF₃ | (CH₂)₃CH₃ | CH(CH₃)CH₂-O-(4-Cl-C₆H₄) | NH |
| Ia1.1681 | CF₃ | H | CH(CH₃)CH(CH₃)-O-(4-Cl-C₆H₄) | NH |
| Ia1.1682 | CF₃ | CH₃ | CH(CH₃)CH(CH₃)-O-(4-Cl-C₆H₄) | NH |
| Ia1.1683 | CF₃ | CH₂CH₃ | CH(CH₃)CH(CH₃)-O-(4-Cl-C₆H₄) | NH |
| Ia1.1684 | CF₃ | (CH₂)₂CH₃ | CH(CH₃)CH(CH₃)-O-(4-Cl-C₆H₄) | NH |
| Ia1.1685 | CF₃ | (CH₂)₃CH₃ | CH(CH₃)CH(CH₃)-O-(4-Cl-C₆H₄) | NH |
| Ia1.1686 | CF₃ | H | (CH₂)₂-O-(2,4-Cl₂-C₆H₃) | NH |
| Ia1.1687 | CF₃ | CH₃ | (CH₂)₂-O-(2,4-Cl₂-C₆H₃) | NH |
| Ia1.1688 | CF₃ | CH₂CH₃ | (CH₂)₂-O-(2,4-Cl₂-C₆H₃) | NH |
| Ia1.1689 | CF₃ | (CH₂)₂CH₃ | (CH₂)₂-O-(2,4-Cl₂-C₆H₃) | NH |
| Ia1.1690 | CF₃ | (CH₂)₃CH₃ | (CH₂)₂-O-(2,4-Cl₂-C₆H₃) | NH |
| Ia1.1691 | CF₃ | H | CH₂CH(CH₃)-O-(2,4-Cl₂-C₆H₃) | NH |
| Ia1.1692 | CF₃ | CH₃ | CH₂CH(CH₃)-O-(2,4-Cl₂-C₆H₃) | NH |
| Ia1.1693 | CF₃ | CH₂CH₃ | CH₂CH(CH₃)-O-(2,4-Cl₂-C₆H₃) | NH |
| Ia1.1694 | CF₃ | (CH₂)₂CH₃ | CH₂CH(CH₃)-O-(2,4-Cl₂-C₆H₃) | NH |
| Ia1.1695 | CF₃ | (CH₂)₃CH₃ | CH₂CH(CH₃)-O-(2,4-Cl₂-C₆H₃) | NH |
| Ia1.1696 | CF₃ | H | CH(CH₃)CH₂-O-(2,4-Cl₂-C₆H₃) | NH |
| Ia1.1697 | CF₃ | CH₃ | CH(CH₃)CH₂-O-(2,4-Cl₂-C₆H₃) | NH |
| Ia1.1698 | CF₃ | CH₂CH₃ | CH(CH₃)CH₂-O-(2,4-Cl₂-C₆H₃) | NH |
| Ia1.1699 | CF₃ | (CH₂)₂CH₃ | CH(CH₃)CH₂-O-(2,4-Cl₂-C₆H₃) | NH |
| Ia1.1700 | CF₃ | (CH₂)₃CH₃ | CH(CH₃)CH₂-O-(2,4-Cl₂-C₆H₃) | NH |
| Ia1.1701 | CF₃ | H | CH(CH₃)CH(CH₃)-O-(2,4-Cl₂-C₆H₃) | NH |
| Ia1.1702 | CF₃ | CH₃ | CH(CH₃)CH(CH₃)-O-(2,4-Cl₂-C₆H₃) | NH |
| Ia1.1703 | CF₃ | CH₂CH₃ | CH(CH₃)CH(CH₃)-O-(2,4-Cl₂-C₆H₃) | NH |
| Ia1.1704 | CF₃ | (CH₂)₂CH₃ | CH(CH₃)CH(CH₃)-O-(2,4-Cl₂-C₆H₃) | NH |
| Ia1.1705 | CF₃ | (CH₂)₃CH₃ | CH(CH₃)CH(CH₃)-O-(2,4-Cl₂-C₆H₃) | NH |
| Ia1.1706 | CF₃ | H | CH₃ | NCH₃ |
| Ia1.1707 | CF₃ | CH₃ | CH₃ | NCH₃ |
| Ia1.1708 | CF₃ | CH₂CH₃ | CH₃ | NCH₃ |
| Ia1.1709 | CF₃ | (CH₂)₂CH₃ | CH₃ | NCH₃ |
| Ia1.1710 | CF₃ | (CH₂)₃CH₃ | CH₃ | NCH₃ |
| Ia1.1711 | CF₃ | H | C₂H₅ | NCH₃ |
| Ia1.1712 | CF₃ | CH₃ | C₂H₅ | NCH₃ |
| Ia1.1713 | CF₃ | CH₂CH₃ | C₂H₅ | NCH₃ |
| Ia1.1714 | CF₃ | (CH₂)₂CH₃ | C₂H₅ | NCH₃ |
| Ia1.1715 | CF₃ | (CH₂)₃CH₃ | C₂H₅ | NCH₃ |
| Ia1.1716 | CF₃ | H | (CH₂)₂CH₃ | NCH₃ |
| Ia1.1717 | CF₃ | CH₃ | (CH₂)₂CH₃ | NCH₃ |
| Ia1.1718 | CF₃ | CH₂CH₃ | (CH₂)₂CH₃ | NCH₃ |
| Ia1.1719 | CF₃ | (CH₂)₂CH₃ | (CH₂)₂CH₃ | NCH₃ |
| Ia1.1720 | CF₃ | (CH₂)₃CH₃ | (CH₂)₂CH₃ | NCH₃ |
| Ia1.1721 | CF₃ | H | CH(CH₃)₂ | NCH₃ |
| Ia1.1722 | CF₃ | CH₃ | CH(CH₃)₂ | NCH₃ |
| Ia1.1723 | CF₃ | CH₂CH₃ | CH(CH₃)₂ | NCH₃ |
| Ia1.1724 | CF₃ | (CH₂)₂CH₃ | CH(CH₃)₂ | NCH₃ |
| Ia1.1725 | CF₃ | (CH₂)₃CH₃ | CH(CH₃)₂ | NCH₃ |
| Ia1.1726 | CF₃ | H | (CH₂)₃CH₃ | NCH₃ |
| Ia1.1727 | CF₃ | CH₃ | (CH₂)₃CH₃ | NCH₃ |
| Ia1.1728 | CF₃ | CH₂CH₃ | (CH₂)₃CH₃ | NCH₃ |
| Ia1.1729 | CF₃ | (CH₂)₂CH₃ | (CH₂)₃CH₃ | NCH₃ |
| Ia1.1730 | CF₃ | (CH₂)₃CH₃ | (CH₂)₃CH₃ | NCH₃ |
| Ia1.1731 | CF₃ | H | CH₂CH(CH₃)₂ | NCH₃ |
| Ia1.1732 | CF₃ | CH₃ | CH₂CH(CH₃)₂ | NCH₃ |
| Ia1.1733 | CF₃ | CH₂CH₃ | CH₂CH(CH₃)₂ | NCH₃ |
| Ia1.1734 | CF₃ | (CH₂)₂CH₃ | CH₂CH(CH₃)₂ | NCH₃ |
| Ia1.1735 | CF₃ | (CH₂)₃CH₃ | CH₂CH(CH₃)₂ | NCH₃ |
| Ia1.1736 | CF₃ | H | CH(CH₃)CH₂CH₃ | NCH₃ |
| Ia1.1737 | CF₃ | CH₃ | CH(CH₃)CH₂CH₃ | NCH₃ |
| Ia1.1738 | CF₃ | CH₃CH₃ | CH(CH₃)CH₂CH₃ | NCH₃ |
| Ia1.1739 | CF₃ | (CH₂)₂CH₃ | CH(CH₃)CH₂CH₃ | NCH₃ |
| Ia1.1740 | CF₃ | (CH₂)₃CH₃ | CH(CH₃)CH₂CH₃ | NCH₃ |
| Ia1.1741 | CF₃ | H | (CH₂)₂-C₆H₅ | NCH₃ |
| Ia1.1742 | CF₃ | CH₃ | (CH₂)₂-C₆H₅ | NCH₃ |
| Ia1.1743 | CF₃ | CH₂CH₃ | (CH₂)₂-C₆H₅ | NCH₃ |
| Ia1.1744 | CF₃ | (CH₂)₂CH₃ | (CH₂)₂-C₆H₅ | NCH₃ |
| Ia1.1745 | CF₃ | (CH₂)₃CH₃ | (CH₂)₂-C₆H₅ | NCH₃ |
| Ia1.1746 | CF₃ | H | CH₂CH(CH₃)-C₆H₅ | NCH₃ |
| Ia1.1747 | CF₃ | CH₃ | CH₂CH(CH₃)-C₆H₅ | NCH₃ |
| Ia1.1748 | CF₃ | CH₂CH₃ | CH2_{C}H(CH₃)-C₆H₅ | NCH₃ |
| Ia1.1749 | CF₃ | (CH₂)₂CH₃ | CH₂CH(CH₃)-C₆H₅ | NCH₃ |
| Ia1.1750 | CF₃ | (CH₂)₃CH₃ | CH₂CH(CH₃)-C₆H₅ | NCH₃ |
| Ia1.1751 | CF₃ | H | CH(CH₃)CH₂-C₆H₅ | NCH₃ |
| Ia1.1752 | CF₃ | CH₃ | CH(CH₃)CH₂-C₆H₅ | NCH₃ |
| Ia1.1753 | CF₃ | CH₂CH₃ | CH(CH₃)CH₂-C₆H₅ | NCH₃ |
| Ia1.1754 | CF₃ | (CH₂)₂CH₃ | CH(CH₃)CH₂-C₆H₅ | NCH₃ |
| Ia1.1755 | CF₃ | (CH₂)₃CH₃ | CH(CH₃)CH₂-C₆H₅ | NCH₃ |
| Ia1.1756 | CF₃ | H | CH(CH₃)CH(CH₃)-C₆H₅ | NCH₃ |
| Ia1.1757 | CF₃ | CH₃ | CH(CH₃)CH(CH₃)-C₆H₅ | NCH₃ |
| Ia1.1758 | CF₃ | CH₂CH₃ | CH(CH₃)CH(CH₃)-C₆H₅ | NCH₃ |
| Ia1.1759 | CF₃ | (CH₂)₂CH₃ | CH(CH₃)CH(CH₃)-C₆H₅ | NCH₃ |
| Ia1.1760 | CF₃ | (CH₂)₃CH₃ | CH(CH₃)CH(CH₃)-C₆H₅ | NCH₃ |
| Ia1.1761 | CF₃ | H | (CH₂)₂-(4-Cl-C₆H₄) | NCH₃ |
| Ia1.1762 | CF₃ | CH₃ | (CH₂)₂-(4-Cl-C₆H₄) | NCH₃ |
| Ia1.1763 | CF₃ | CH₂CH₃ | (CH₂)₂-(4-Cl-C₆H₄) | NCH₃ |
| Ia1.1764 | CF₃ | (CH₂)₂CH₃ | (CH₂)₂-(4-Cl-C₆H₄) | NCH₃ |
| Ia1.1765 | CF₃ | (CH₂)₃CH₃ | (CH₂)₂-(4-Cl-C₆H₄) | NCH₃ |
| Ia1.1766 | CF₃ | H | CH₂CH(CH₃)-(4-Cl-C₆H₄) | NCH₃ |
| Ia1.1767 | CF₃ | CH₃ | CH₂CH(CH₃)-(4-Cl-C₆H₄) | NCH₃ |
| Ia1.1768 | CF₃ | CH₂CH₃ | CH₂CH(CH₃)-(4-Cl-C₆H₄) | NCH₃ |
| Ia1.1769 | CF₃ | (CH₂)₂CH₃ | CH₂CH(CH₃)-(4-Cl-C₆H₄) | NCH₃ |
| Ia1.1770 | CF₃ | (CH₂)₃CH₃ | CH₂CH(CH₃)-(4-Cl-C₆H₄) | NCH₃ |
| Ia1.1771 | CF₃ | H | CH(CH₃)CH₂-(4-Cl-C₆H₄) | NCH₃ |
| Ia1.1772 | CF₃ | CH₃ | CH(CH₃)CH₂-(4-Cl-C₆H₄) | NCH₃ |
| Ia1.1773 | CF₃ | CH₂CH₃ | CH(CH₃)CH₂-(4-Cl-C₆H₄) | NCH₃ |
| la1.1774 | CF₃ | (CH₂)₂CH₃ | CH(CH₃)CH₂-(4-Cl-C₆H₄) | NCH₃ |
| Ia1.1775 | CF₃ | (CH₂)₃CH₃ | CH(CH₃)CH₂-(4-Cl-C₆H₄) | NCH₃ |
| Ia1.1776 | CF₃ | H | CH(CH₃)CH(CH₃)-(4-Cl-C₆H₄) | NCH₃ |
| Ia1.1777 | CF₃ | CH₃ | CH(CH₃)CH(CH₃)-(4-Cl-C₆H₄) | NCH₃ |
| Ia1.1778 | CF₃ | CH₂CH₃ | CH(CH₃)CH(CH₃)-(4-Cl-C₆H₄) | NCH₃ |
| Ia1.1779 | CF₃ | (CH₂)₂CH₃ | CH(CH₃)CH(CH₃)-(4-Cl-C₆H₄) | NCH₃ |
| Ia1.1780 | CF₃ | (CH₂)₃CH₃ | CH(CH₃)CH(CH₃)-(4-Cl-C₆H₄) | NCH₃ |
| Ia1.1781 | CF3 | H | (CH₂)₂-(2,4-Cl₂-C₆H₃) | NCH₃ |
| Ia1.1782 | CF₃ | CH₃ | (CH₂)₂-(2,4-Cl₂-C₆H₃) | NCH₃ |
| Ia1.1783 | CF₃ | CH₂CH₃ | (CH₂)₂-(2,4-Cl₂-C₆H₃) | NCH₃ |
| Ia1.1784 | CF₃ | (CH₂)₂CH₃ | (CH₂)₂-(2,4-Cl₂-C₆H₃) | NCH₃ |
| Ia1.1785 | CF₃ | (CH₂)₃CH₃ | (CH₂)₂-(2,4-Cl₂-C₆H₃) | NCH₃ |
| Ia1.1786 | CF₃ | H | CH₂CH(CH₃)-(2,4-Cl₂-C₆H₃) | NCH₃ |
| Ia1.1787 | CF₃ | CH₃ | CH₂CH(CH₃)-(2,4-Cl₂-C₆H₃) | NCH₃ |
| Ia1.1788 | CF₃ | CH₂CH₃ | CH₂CH(CH₃)-(2,4-Cl₂-C₆H₃) | NCH₃ |
| Ia1.1789 | CF₃ | (CH₂)₂CH₃ | CH₂CH(CH₃)-(2,4-Cl₂-C₆H₃) | NCH₃ |
| Ia1.1790 | CF₃ | (CH₂)₃CH₃ | CH₂CH(CH₃)-(2,4-Cl₂-C₆H₃) | NCH₃ |
| Ia1.1791 | CF₃ | H | CH(CH₃)CH₂-(2,4-Cl₂-C₆H₃) | NCH₃ |
| Ia1.1792 | CF₃ | CH₃ | CH(CH₃)CH₂-(2,4-Cl₂-C₆H₃) | NCH₃ |
| Ia1.1793 | CF₃ | CH₂CH₃ | CH(CH₃)CH₂-(2,4-Cl₂-C₆H₃) | NCH₃ |
| Ia1.1794 | CF₃ | (CH₂)₂CH₃ | CH(CH₃)CH₂-(2,4-Cl₂-C₆H₃) | NCH₃ |
| Ia1.1795 | CF₃ | (CH₂)₃CH₃ | CH(CH₃)CH₂-(2,4-Cl₂-C₆H₃) | NCH₃ |
| Ia1.1796 | CF₃ | H | CH(CH₃)CH(CH₃)-(2,4-Cl₂-C₆H₃) | NCH₃ |
| Ia1.1797 | CF₃ | CH₃ | CH(CH₃)CH(CH₃)-(2,4-Cl₂-C₆H₃) | NCH₃ |
| Ia1.1798 | CF₃ | CH₂CH₃ | CH(CH₃)CH(CH₃)-(2,4-Cl₂-C₆H₃) | NCH₃ |
| Ia1.1799 | CF₃ | (CH₂)₂CH₃ | CH(CH₃)CH(CH₃)-(2,4-Cl₂-C₆H₃) | NCH₃ |
| Ia1.1800 | CF₃ | (CH₂)₃CH₃ | CH(CH₃)CH(CH₃)-(2,4-Cl₂-C₆H₃) | NCH₃ |
| Ia1.1801 | CF₃ | H | (CH₂)₂-O-C₆H₅ | NCH₃ |
| Ia1.1802 | CF₃ | CH₃ | (CH₂)₂-O-C₆H₅ | NCH₃ |
| Ia1.1803 | CF₃ | CH₂CH₃ | (CH₂)₂-O-C₆H₅ | NCH₃ |
| Ia1.1804 | CF₃ | (CH₂)₂CH₃ | (CH₂)₂-O-C₆H₅ | NCH₃ |
| Ia1.1805 | CF₃ | (CH₂)₃CH₃ | (CH₂)₂-O-C₆H₅ | NCH₃ |
| Ia1.1806 | CF₃ | H | CH₂CH(CH₃)-O-C₆H₅ | NCH₃ |
| Ia1.1807 | CF₃ | CH₃ | CH₂CH(CH₃)-O-C₆H₅ | NCH₃ |
| Ia1.1808 | CF₃ | CH₂CH₃ | CH₂CH(CH₃)-O-C₆H₅ | NCH₃ |
| Ia1.1809 | CF₃ | (CH₂)₂CH₃ | CH₂CH(CH₃)-O-C₆H₅ | NCH₃ |
| Ia1.1810 | CF₃ | (CH₂)₃CH₃ | CH₂CH(CH₃)-O-C₆H₅ | NCH₃ |
| Ia1.1811 | CF₃ | H | CH(CH₃)CH₂-O-C₆H₅ | NCH₃ |
| Ia1.1812 | CF₃ | CH₃ | CH(CH₃)CH₂-O-C₆H₅ | NCH₃ |
| Ia1.1813 | CF₃ | CH₂CH₃ | CH(CH₃)CH₂-O-C₆H₅ | NCH₃ |
| Ia1.1814 | CF₃ | (CH₂)₂CH₃ | CH(CH₃)CH₂-O-C₆H₅ | NCH₃ |
| Ia1.1815 | CF₃ | (CH₂)₃CH₃ | CH(CH₃)CH₂-O-C₆H₅ | NCH₃ |
| Ia1.1816 | CF₃ | H | CH(CH₃)CH(CH₃)-O-C₆H₅ | NCH₃ |
| Ia1.1817 | CF₃ | CH₃ | CH(CH₃)CH(CH₃)-O-C₆H₅ | NCH₃ |
| Ia1.1818 | CF₃ | CH₂CH₃ | CH(CH₃)CH(CH₃)-O-C₆H₅ | NCH₃ |
| Ia1.1819 | CF₃ | (CH₂)₂CH₃ | CH(CH₃)CH(CH₃)-O-C₆H₅ | NCH₃ |
| Ia1.1820 | CF₃ | (CH₂)₃CH₃ | CH(CH₃)CH(CH₃)-O-C₆H₅ | NCH3 |
| Ia1.1821 | CF₃ | H | (CH₂)₂-O-(4-Cl-C₆H₄) | NCH₃ |
| Ia1.1822 | CF₃ | CH₃ | (CH₂)₂-O-(4-Cl-C₆H₄) | NCH₃ |
| Ia1.1823 | CF3 | CH₂CH₃ | (CH₂)₂-O-(4-Cl-C₆H₄) | NCH₃ |
| Ia1.1824 | CF₃ | (CH₂)₂CH₃ | (CH₂)₂-O-(4-Cl-C₆H₄) | NCH₃ |
| Ia1.1825 | CF₃ | (CH₂)₃CH₃ | (CH₂)₂-O-(4-Cl-C₆H₄) | NCH₃ |
| Ia1.1826 | CF₃ | H | CH₂CH(CH₃)-O-(4-Cl-C₆H₄) | NCH₃ |
| Ia1.1827 | CF₃ | CH₃ | CH₂CH(CH₃)-O-(4-Cl-C₆H₄) | NCH₃ |
| Ia1.1828 | CF₃ | CH₂CH₃ | CH₂CH(CH₃)-O-(4-Cl-C₆H₄) | NCH₃ |
| Ia1.1829 | CF₃ | (CH₂)₂CH₃ | CH₂CH(CH₃)-O-(4-Cl-C₆H₄) | NCH₃ |
| Ia1.1830 | CF₃ | (CH2)₃CH₃ | CH₂CH₂(CH₃)-O-(4-Cl-C₆H₄) | NCH₃ |
| Ia1.1831 | CF₃ | H | CH(CH₃)CH₂-O-(4-Cl-C₆H₄) | NCH₃ |
| Ia1.1832 | CF₃ | CH₃ | CH(CH₃)CH₂-O-(4-Cl-C₆H₄) | NCH₃ |
| Ia1.1833 | CF₃ | CH₂CH₃ | CH(CH₃)CH₂-O-(4-Cl-C₆H₄) | NCH₃ |
| Ia1.1834 | CF₃ | (CH₂)₂CH₃ | CH(CH₃)CH₂-O-(4-Cl-C₆H₄) | NCH₃ |
| Ia1.1835 | CF₃ | (CH₂)₃CH₃ | CH(CH₃)CH₂-O-(4-Cl-C₆H₄) | NCH₃ |
| Ia1.1836 | CF₃ | H | CH(CH₃)CH(CH₃)-O-(4-Cl-C₆H₄) | NCH₃ |
| Ia1.1837 | CF₃ | CH₃ | CH(CH₃)CH(CH₃)-O-(4-Cl-C₆H₄) | NCH₃ |
| Ia1.1838 | CF₃ | CH₂CH₃ | CH(CH₃)CH(CH₃)-O-(4-Cl-C₆H₄) | NCH₃ |
| Ia1.1839 | CF₃ | (CH₂)₂CH₃ | CH(CH₃)CH(CH₃)-O-(4-Cl-C₆H₄) | NCH₃ |
| Ia1.1840 | CF₃ | (CH₂)₃CH₃ | CH(CH₃)CH(CH₃)-O-(4-Cl-C₆H₄) | NCH₃ |
| Ia1.1841 | CF₃ | H | (CH₂)₂-O-(2,4-Cl₂-C₆H₃) | NCH₃ |
| Ia1.1842 | CF₃ | CH₃ | (CH₂)₂-O-(2,4-Cl₂-C₆H₃) | NCH₃ |
| Ia1.1843 | CF₃ | CH₂CH₃ | (CH₂)₂-O-(2,4-Cl₂-C₆H₃) | NCH₃ |
| Ia1.1844 | CF₃ | (CH₂)₂CH₃ | (CH₂)₂-O-(2,4-Cl₂-C₆H₃) | NCH₃ |
| Ia1.1845 | CF₃ | (CH₂)₃CH₃ | (CH₂)₂-O-(2,4-Cl₂-C₆H₃) | NCH₃ |
| Ia1.1846 | CF₃ | H | CH₂CH(CH₃)-O-(2,4-Cl₂-C₆H₃) | NCH₃ |
| Ia1.1847 | CF₃ | CH₃ | CH₂CH(CH₃)-O-(2,4-Cl₂-C₆H₃) | NCH₃ |
| Ia1.1848 | CF₃ | CH₂CH₃ | CH₂CH(CH₃)-O-(2,4-Cl₂-C₆H₃) | NCH₃ |
| Ia1.1849 | CF₃ | (CH₂)₂CH₃ | CH₂CH(CH₃)-O-(2,4-Cl₂-C₆H₃) | NCH₃ |
| Ia1.1850 | CF₃ | (CH₂)₃CH₃ | CH₂CH(CH₃)-O-(2,4-Cl₂-C₆H₃) | NCH₃ |
| Ia1.1851 | CF₃ | H | CH(CH₃)CH₂-O-(2,4-Cl₂-C₆H₃) | NCH₃ |
| Ia1.1852 | CF₃ | CH₃ | CH(CH₃)CH₂-O-(2,4-Cl₂-C₆H₃) | NCH₃ |
| Ia1.1853 | CF₃ | CH₂CH₃ | CH(CH₃)CH₂-O-(2,4-Cl₂-C₆H₃) | NCH₃ |
| Ia1.1854 | CF₃ | (CH₂)₂CH₃ | CH(CH₃)CH₂O-(2,4-Cl₂-C₆H₃) | NCH₃ |
| Ia1.1855 | CF₃ | (CH₂)₃CH₃ | CH(CH₃)CH₂-O-(2,4-Cl₂-C₆H₃) | NCH₃ |
| Ia1.1856 | CF₃ | H | CH(CH₃)CH₃-O-(2,4-Cl₂-C₆H₃) | NCH₃ |
| Ia1.1857 | CF₃ | CH₃ | CH(CH₃)CH₃-O-(2,4-Cl₂-C₆H₃) | NCH₃ |
| Ia1.1858 | CF₃ | CH₂CH₃ | CH(CH₃)CH₃-O-(2,4-Cl₂-C₆H₃) | NCH₃ |
| Ia1.1859 | CF₃ | (CH₂)₂CH₃ | CH(CH₃)CH₃-O-(2,4-Cl₂-C₆H₃) | NCH₃ |
| Ia1.1860 | CF₃ | (CH₂)₃CH₃ | CH(CH₃)CH₃-O-(2,4-Cl₂-C₆H₃) | NCH₃ |
| Ia1.1861 | SO₂CH₃ | H | CH₃ | O |
| Ia1.1862 | SO₂CH₃ | CH₃ | CH₃ | O |
| Ia1.1863 | SO₂CH₃ | CH₂CH₃ | CH₃ | O |
| Ia1.1864 | SO₂CH₃ | (CH₂)₂CH₃ | CH₃ | O |
| Ia1.1865 | SO₂CH₃ | (CH₂)₃CH₃ | CH₃ | O |
| Ia1.1866 | SO₂CH₃ | H | C₂H₅ | O |
| Ia1.1867 | SO₂CH₃ | CH₃ | C₂H₅ | O |
| Ia1.1868 | SO₂CH₃ | CH₂CH₃ | C₂H₅ | O |
| Ia1.1869 | SO₂CH₃ | (CH₂)₂CH₃ | C₂H₅ | O |
| Ia1.1870 | SO₂CH₃ | (CH₂)₃CH₃ | C₂H₅ | O |
| Ia1.1871 | SO₂CH₃ | H | (CH₂)₂CH₃ | O |
| Ia1.1872 | SO₂CH₃ | CH₃ | (CH₂)₂CH₃ | O |
| Ia1.1873 | SO₂CH₃ | CH₂CH₃ | (CH₂)₂CH₃ | O |
| Ia1.1874 | SO₂CH₃ | (CH₂)₂CH₃ | (CH₂)₂CH₃ | O |
| Ia1.1875 | SO₂CH₃ | (CH₂)₃CH₃ | (CH₂)₂CH₃ | O |
| Ia1.1876 | SO₂CH₃ | H | CH(CH₃)₂ | O |
| Ia1.1877 | SO₂CH₃ | CH₃ | CH(CH₃)₂ | O |
| Ia1.1878 | SO₂CH₃ | CH₂CH₃ | CH(CH₃)₂ | O |
| Ia1.1879 | SO₂CH₃ | (CH₂)₂CH₃ | CH(CH₃)₂ | O |
| Ia1.1880 | SO₂CH₃ | (CH₂)₃CH₃ | CH(CH₃)₂ | O |
| Ia1.1881 | SO₂CH₃ | H | (CH₂)₃CH₃ | O |
| Ia1.1882 | SO₂CH₃ | CH₃ | (CH₂)₃CH₃ | O |
| Ia1.1883 | SO₂CH₃ | CH₂CH₃ | (CH₂)₃CH₃ | O |
| Ia1.1884 | SO₂CH₃ | (CH₂)₂CH₃ | (CH₂)₃CH₃ | O |
| Ia1.1885 | SO₂CH₃ | (CH₂)₃CH₃ | (CH₂)₃CH₃ | O |
| Ia1.1886 | SO₂CH₃ | H | CH₂CH(CH₃)₂ | O |
| Ia1.1887 | SO₂CH₃ | CH₃ | CH₂CH(CH₃)₂ | O |
| Ia1.1888 | SO₂CH₃ | CH₂CH₃ | CH₂CH(CH₃)₂ | O |
| Ia1.1889 | SO₂CH₃ | (CH₂)₂CH₃ | CH₂CH(CH₃)₂ | O |
| Ia1.1890 | SO₂CH₃ | (CH₂)₃CH₃ | CH₂CH(CH₃)₂ | O |
| Ia1.1891 | SO₂CH₃ | H | CH(CH₃)CH₂CH₃ | O |
| Ia1.1892 | SO₂CH₃ | CH₃ | CH(CH₃)CH₂CH₃ | O |
| Ia1.1893 | SO₂CH₃ | CH₂CH₃ | CH(CH₃)CH₂CH₃ | O |
| Ia1.1894 | SO₂CH₃ | (CH₂)₂CH₃ | CH(CH₃)CH₂CH₃ | O |
| Ia1.1895 | SO₂CH₃ | (CH₂)₃CH₃ | CH(CH₃)CH₂CH₃ | O |
| Ia1.1896 | SO₂CH₃ | H | (CH₂)₂-C₆H₅ | O |
| Ia1.1897 | SO₂CH₃ | CH₃ | (CH₂)₂-C₆H₅ | O |
| Ia1.1898 | SO₂CH₃ | CH₂CH₃ | (CH₂)₂-C₆H₅ | O |
| Ia1.1899 | SO₂CH₃ | (CH₂)₂CH₃ | (CH₂)₂-C₆H₅ | O |
| Ia1.1900 | SO₂CH₃ | (CH₂)₃CH₃ | (CH₂)₂-C₆H₅ | O |
| Ia1.1901 | SO₂CH₃ | H | CH₂CH(CH₃)-C₆H₅ | O |
| Ia1.1902 | SO₂CH₃ | CH₃ | CH₂CH(CH₃)-C₆H₅ | O |
| Ia1.1903 | SO₂CH₃ | CH₂CH₃ | CH₂CH(CH₃)-C₆H₅ | O |
| Ia1.1904 | SO₂CH₃ | (CH₂)₂CH₃ | CH₂CH(CH₃)-C₆H₅ | O |
| Ia1.1905 | SO₂CH₃ | (CH₂)₃CH₃ | CH₂CH(CH₃)-C₆H₅ | O |
| Ia1.1906 | SO₂CH₃ | H | CH(CH₃)CH₂-C₆H₅ | O |
| Ia1.1907 | SO₂CH₃ | CH₃ | CH(CH₃)CH₂-C₆H₅ | O |
| Ia1.1908 | SO₂CH₃ | CH₂CH₃ | CH(CH₃)CH₂-C₆H₅ | O |
| Ia1.1909 | SO₂CH₃ | (CH₂)₂CH₃ | CH(CH₃)CH₂-C₆H₅ | O |
| Ia1.1910 | SO₂CH₃ | (CH₂)₃CH₃ | CH(CH₃)CH₂-C₆H₅ | O |
| Ia1.1911 | SO₂CH₃ | H | CH(CH₃)CH(CH₃)-C₆H₅ | O |
| Ia1.1912 | SO₂CH₃ | CH₃ | CH(CH₃)CH(CH₃)-C₆H₅ | O |
| Ia1.1913 | SO₂CH₃ | CH₂CH₃ | CH(CH₃)CH(CH₃)-C₆H₅ | O |
| Ia1.1914 | SO₂CH₃ | (CH₂)₂CH₃ | CH(CH₃)CH(CH₃)-C₆H₅ | O |
| Ia1.1915 | SO₂CH₃ | (CH₂)₃CH₃ | CH(CH₃)CH(CH₃)-C₆H₅ | O |
| Ia1.1916 | SO₂CH₃ | H | (CH₂)₂-(4-Cl-C₆H₄) | O |
| Ia1.1917 | SO₂CH₃ | CH₃ | (CH₂)₂-(4-Cl-C₆H₄) | O |
| Ia1.1918 | SO₂CH₃ | CH₂CH₃ | (CH₂)₂-(4-Cl-C₆H₄) | O |
| Ia1.1919 | SO₂CH₃ | (CH₂)₂CH₃ | (CH₂)₂-(4-Cl-C₆H₄) | O |
| Ia1.1920 | SO₂CH₃ | (CH₂)₃CH₃ | (CH₂)₂-(4-Cl-C₆H₄) | O |
| Ia1.1921 | SO₂CH₃ | H | CH₂CH(CH₃)-(4-Cl-C₆H₄) | O |
| Ia1.1922 | SO₂CH₃ | CH₃ | CH₂CH(CH₃)-(4-Cl-C₆H₄) | O |
| Ia1.1923 | SO₂CH₃ | CH₂CH₃ | CH₂CH(CH₃)-(4-Cl-C₆H₄) | O |
| Ia1.1924 | SO₂CH₃ | (CH₂)₂CH₃ | CH₂CH(CH₃)-(4-Cl-C₆H₄) | O |
| Ia1.1925 | SO₂CH₃ | (CH₂)₃CH₃ | CH₂CH(CH₃)-(4-Cl-C₆H₄) | O |
| Ia1.1926 | SO₂CH₃ | H | CH(CH₃)CH₂-(4-Cl-C₆H₄) | O |
| Ia1.1927 | SO₂CH₃ | CH₃ | CH(CH₃)CH₂-(4-Cl-C₆H₄) | O |
| Ia1.1928 | SO₂CH₃ | CH₂CH₃ | CH(CH₃)CH₂-(4-Cl-C₆H₄) | O |
| Ia1.1929 | SO₂CH₃ | (CH₂)₂CH₃ | CH(CH₃)CH₂-(4-Cl-C₆H₄) | O |
| Ia1.1930 | SO₂CH₃ | (CH₂)₃CH₃ | CH(CH₃)CH₂-(4-Cl-C₆H₄) | O |
| Ia1.1931 | SO₂CH₃ | H | CH(CH₃)CH(CH₃)-(4-Cl-C₆H₄) | O |
| Ia1.1932 | SO₂CH₃ | CH₃ | CH(CH₃)CH(CH₃)-(4-Cl-C₆H₄) | O |
| Ia1.1933 | SO₂CH₃ | CH₂CH₃ | CH(CH₃)CH(CH₃)-(4-Cl-C₆H₄) | O |
| Ia1.1934 | SO₂CH₃ | (CH₂)₂CH₃ | CH(CH₃)CH(CH₃)-(4-Cl-C₆H₄) | O |
| Ia1.1935 | SO₂CH₃ | (CH₂)₃CH₃ | CH(CH₃)CH(CH₃)-(4-Cl-C₆H₄) | O |
| Ia1.1936 | SO₂CH₃ | H | (CH₂)₂-(2,4-Cl₂C₆H₃) | O |
| Ia1.1937 | SO₂CH₃ | CH₃ | (CH₂)₂-(2,4-Cl₂C₆H₃) | O |
| Ia1.1938 | SO₂CH₃ | CH₂CH₃ | (CH₂)₂-(2,4-Cl₂C₆H₃) | O |
| Ia1.1939 | SO₂CH₃ | (CH₂)₂CH₃ | (CH₂)₂-(2,4-Cl₂-C₆H₃) | O |
| Ia1.1940 | SO₂CH₃ | (CH₂)₃CH₃ | (CH₂)₂-(2,4-Cl₂-C₆H₃) | O |
| Ia1.1941 | SO₂CH₃ | H | CH₂CH(CH₃)-(2,4-Cl₂-C₆H₃) | O |
| Ia1.1942 | SO₂CH₃ | CH₃ | CH₂CH(CH₃)-(2,4-Cl₂-C₆H₃) | O |
| Ia1.1943 | SO₂CH₃ | CH₂CH₃ | CH₂CH(CH₃)-(2,4-Cl₂-C₆H₃) | O |
| Ia1.1944 | SO₂CH₃ | (CH₂)₂CH₃ | CH₂CH(CH₃)-(2,4-Cl₂-C₆H₃) | O |
| Ia1.1945 | SO₂CH₃ | (CH₂)₃CH₃ | CH₂CH(CH₃)-(2,4-Cl₂-C₆H₃) | O |
| Ia1.1946 | SO₂CH₃ | H | CH(CH₃)CH₂-(2,4-Cl₂-C₆H₃) | O |
| Ia1.1947 | SO₂CH₃ | CH₃ | CH(CH₃)CH₂-(2,4-Cl₂-C₆H₃) | O |
| Ia1.1948 | SO₂CH₃ | CH₂CH₃ | CH(CH₃)CH₂-(2,4-Cl₂-C₆H₃) | O |
| Ia1.1949 | SO₂CH₃ | (CH₂)₂CH₃ | CH(CH₃)CH₂-(2,4-Cl₂-C₆H₃) | O |
| Ia1.1950 | SO₂CH₃ | (CH₂)₃CH₃ | CH(CH₃)CH₂-(2,4-Cl₂-C₆H₃) | O |
| Ia1.1951 | SO₂CH₃ | H | CH(CH₃)CH(CH₃)-(2,4-Cl₂-C₆H₃) | O |
| Ia1.1952 | SO₂CH₃ | CH₃ | CH(CH₃)CH(CH₃)-(2,4-Cl₂-C₆H₃) | O |
| Ia1.1953 | SO₂CH₃ | CH₂CH₃ | CH(CH₃)CH(CH₃)-(2,4-Cl₂-C₆H₃) | O |
| Ia1.1954 | SO₂CH₃ | (CH₂)₂CH₃ | CH(CH₃)CH(CH₃)-(2,4-Cl₂-C₆H₃) | O |
| Ia1.1955 | SO₂CH₃ | (CH₂)₃CH₃ | CH(CH₃)CH(CH₃)-(2,4-Cl₂-C₆H₃) | O |
| Ia1.1956 | SO₂CH₃ | H | (CH₂)₂-O-C₆H₅ | O |
| Ia1.1957 | SO₂CH₃ | CH₃ | (CH₂)₂-O-C₆H₅ | O |
| Ia1.1958 | SO₂CH₃ | CH₂CH₃ | (CH₂)₂-O-C₆H₅ | O |
| Ia1.1959 | SO₂CH₃ | (CH₂)₂CH₃ | (CH₂)₂-O-C₆H₅ | O |
| Ia1.1960 | SO₂CH₃ | (CH₂)₃CH₃ | (CH₂)₂-O-C₆H₅ | O |
| Ia1.1961 | SO₂CH₃ | H | CH₂CH(CH₃)-O-C₆H₅ | O |
| Ia1.1962 | SO₂CH₃ | CH₃ | CH₂CH(CH₃)-O-C₆H₅ | O |
| Ia1.1963 | SO₂CH₃ | CH₂CH₃ | CH₂CH(CH₃)-O-C₆H₅ | O |
| Ia1.1964 | SO₂CH₃ | (CH₂)₂CH₃ | CH₂CH(CH₃)-O-C₆H₅ | O |
| Ia1.1965 | SO₂CH₃ | (CH₂)₃CH₃ | CH₂CH(CH₃)-O-C₆H₅ | O |
| Ia1.1966 | SO₂CH₃ | H | CH(CH₃)CH₂-O-C₆H₅ | O |
| Ia1.1967 | SO₂CH₃ | CH₃ | CH(CH₃)CH₂-O-C₆H₅ | O |
| Ia1.1968 | SO₂CH₃ | CH₂CH₃ | CH(CH₃)CH₂-O-C₆H₅ | O |
| Ia1.1969 | SO₂CH₃ | (CH₂)₂CH₃ | CH(CH₃)CH₂-O-C₆H₅ | O |
| Ia1.1970 | SO₂CH₃ | (CH₂)₃CH₃ | CH(CH₃)CH₂-O-C₆H₅ | O |
| Ia1.1971 | SO₂CH₃ | H | CH(CH₃)CH(CH₃)-O-C₆H₅ | O |
| Ia1.1972 | SO₂CH₃ | CH₃ | CH(CH₃)CH(CH₃)-O-C₆H₅ | O |
| Ia1.1973 | SO₂CH₃ | CH₂CH₃ | CH(CH₃)CH(CH₃)-O-C₆H₅ | O |
| Ia1.1974 | SO₂CH₃ | (CH₂)₂CH₃ | CH(CH₃)CH(CH₃)-O-C₆H₅ | O |
| Ia1.1975 | SO₂CH₃ | (CH₂)₃CH₃ | CH(CH₃)CH(CH₃)-O-C₆H₅ | O |
| Ia1.1976 | SO₂CH₃ | H | (CH₂)₂-O-(4-Cl-C₆H₄) | O |
| Ia1.1977 | SO₂CH₃ | CH₃ | (CH₂)₂-O-(4-Cl-C₆H₄) | O |
| Ia1.1978 | SO₂CH₃ | CH₂CH₃ | (CH₂)₂-O-(4-Cl-C₆H₄) | O |
| Ia1.1979 | SO₂CH₃ | (CH₂)₂CH₃ | (CH₂)₂-O-(4-Cl-C₆H₄) | O |
| Ia1.1980 | SO₂CH₃ | (CH₂)₃CH₃ | (CH₂)₂-O-(4-Cl-C₆H₄) | O |
| Ia1.1981 | SO₂CH₃ | H | CH₂CH(CH₃)-O-(4-Cl-C₆H₄) | O |
| Ia1.1982 | SO₂CH₃ | CH₃ | CH₂CH(CH₃)-O-(4-Cl-C₆H₄) | O |
| Ia1.1983 | SO₂CH₃ | CH₂CH₃ | CH₂CH(CH₃)-O-(4-Cl-C₆H₄) | O |
| Ia1.1984 | SO₂CH₃ | (CH₂)₂CH₃ | CH₂CH(CH₃)-O-(4-Cl-C₆H₄) | O |
| Ia1.1985 | SO₂CH₃ | (CH₂)₃CH₃ | CH₂CH(CH₃)-O-(4-Cl-C₆H₄) | O |
| Ia1.1986 | SO₂CH₃ | H | CH(CH₃)CH₂-O-(4-Cl-C₆H₄) | O |
| Ia1.1987 | SO₂CH₃ | CH₃ | CH(CH₃)CH₂-O-(4-Cl-C₆H₄) | 0 |
| Ia1.1988 | SO₂CH₃ | CH₂CH₃ | CH(CH₃)CH₂-O-(4-Cl-C₆H₄) | O |
| Ia1.1989 | SO₂CH₃ | (CH₂)₂CH₃ | CH(CH₃)CH₂-O-(4-Cl-C₆H₄) | O |
| Ia1.1990 | SO₂CH₃ | (CH₂)₃CH₃ | CH (CH₃)CH₂-O-(4-Cl-C₆H₄) | O |
| Ia1.1991 | SO₂CH₃ | H | CH(CH₃)CH(CH₃)-O-(4-Cl-C₆H₄) | O |
| Ia1.1992 | SO₂CH₃ | CH₃ | CH(CH₃)CH(CH₃)-O-(4-Cl-C₆H₄) | O |
| Ia1.1993 | SO₂CH₃ | CH₂CH₃ | CH(CH₃)CH(CH₃)-O-(4-Cl-C₆H₄) | O |
| Ia1.1994 | SO₂CH₃ | (CH₂)₂CH₃ | CH(CH₃)CH(CH₃)-O-(4-Cl-C₆H₄) | O |
| Ia1.1995 | SO₂CH₃ | (CH₂)₃CH₃ | CH(CH₃)CH(CH₃)-O-(4-Cl-C₆H₄) | O |
| Ia1.1996 | SO₂CH₃ | H | (CH₂)₂-O-(2,4-Cl₂-C₆H₃) | O |
| Ia1.1997 | SO₂CH₃ | CH₃ | (CH₂)₂-O-(2,4-Cl₂-C₆H₃) | O |
| Ia1.1998 | SO₂CH₃ | CH₂CH₃ | (CH₂)₂-O-(2,4-Cl₂-C₆H₃) | O |
| Ia1.1999 | SO₂CH₃ | (CH₂)₂CH₃ | (CH₂)₂-O-(2,4-Cl₂-C₆H₃) | O |
| Ia1.2000 | SO₂CH₃ | (CH₂)₃CH₃ | (CH₂)₂-O-(2,4-Cl₂-C₆H₃) | O |
| Ia1.2001 | SO₂CH₃ | H | CH₂CH(CH₃)-O-(2,4-Cl₂-C₆H₃) | O |
| Ia1.2002 | SO₂CH₃ | CH₃ | CH₂CH(CH₃)-O-(2,4-Cl₂-C₆H₃) | O |
| Ia1.2003 | SO₂CH₃ | CH₂CH₃ | CH₂CH(CH₃)-O-(2,4-Cl₂-C₆H₃) | O |
| Ia1.2004 | SO₂CH₃ | (CH₂)₂CH₃ | CH₂CH(CH₃)-O-(2,4-Cl₂-C₆H₃) | o |
| Ia1.2005 | SO₂CH₃ | (CH₂)₃CH₃ | CH₂CH(CH₃)-O-(2,4-Cl₂-C₆H₃) | O |
| Ia1.2006 | SO₂CH₃ | H | CH(CH₃)CH₂-O-(2,4-Cl₂-C₆H₃) | O |
| Ia1.2007 | SO₂CH₃ | CH₃ | CH(CH₃)CH₂-O-(2,4-Cl₂-C₆H₃) | O |
| Ia1.2008 | SO₂CH₃ | CH₂CH₃ | CH(CH₃)CH₂-O-(2,4-Cl₂-C₆H₃) | O |
| Ia1.2009 | SO₂CH₃ | (CH₂)₂CH₃ | CH(CH₃)CH₂-O-(2,4-Cl₂-C₆H₃) | O |
| Ia1.2010 | SO₂CH₃ | (CH₂)₃CH₃ | CH(CH₃)CH₂-O-(2,4-Cl₂-C₆H₃) | O |
| Ia1.2011 | SO₂CH₃ | H | CH(CH₃)CH(CH₃)-O-(2,4-Cl₂-C₆H₃) | O |
| Ia1.2012 | SO₂CH₃ | CH₃ | CH(CH₃)CH(CH₃)-O-(2,4-Cl₂-C₆H₃) | O |
| Ia1.2013 | SO₂CH₃ | CH₂CH₃ | CH(CH₃)CH(CH₃)-O-(2,4-Cl₂-C₆H₃) | O |
| Ia1.2014 | SO₂CH₃ | (CH₂)₂CH₃ | CH(CH₃)CH(CH₃)-O-(2,4-Cl₂-C₆H₃) | O |
| Ia1.2015 | SO₂CH₃ | (CH₂)₃CH₃ | CH(CH₃)CH(CH₃)-O-(2,4-Cl₂-C₆H₃) | O |
| Ia1.2016 | SO₂CH₃ | H | CH₃ | NH |
| Ia1.2017 | SO₂CH₃ | CH₃ | CH₃ | NH |
| Ia1.2018 | SO₂CH₃ | CH₂CH₃ | CH₃ | NH |
| Ia1.2019 | SO₂CH₃ | (CH₂)₂CH₃ | CH₃ | NH |
| Ia1.2020 | SO₂CH₃ | (CH₂)₃CH₃ | CH₃ | NH |
| Ia1.2021 | SO₂CH₃ | H | C₂H₅ | NH |
| Ia1.2022 | SO₂CH₃ | CH₃ | C₂H₅ | NH |
| Ia1.2023 | SO₂CH₃ | CH₂CH₃ | C₂H₅ | NH |
| Ia1.2024 | SO₂CH₃ | (CH₂)₂CH₃ | C₂H₅ | NH |
| Ia1.2025 | SO₂CH₃ | (CH₂)₃CH₃ | C₂H₅ | NH |
| Ia1.2026 | SO₂CH₃ | H | (CH₂)₂CH₃ | NH |
| Ia1.2027 | SO₂CH₃ | CH₃ | (CH₂)₂CH₃ | NH |
| Ia1.2028 | SO₂CH₃ | CH₂CH₃ | (CH₂)₂CH₃ | NH |
| Ia1.2029 | SO₂CH₃ | (CH₂)₂CH₃ | (CH₂)₂CH₃ | NH |
| Ia1.2030 | SO₂CH₃ | (CH₂)₃CH₃ | (CH₂)₂CH₃ | NH |
| Ia1.2031 | SO₂CH₃ | H | CH(CH₃)₂ | NH |
| Ia1.2032 | SO₂CH₃ | CH₃ | CH(CH₃)₂ | NH |
| Ia1.2033 | SO₂CH₃ | CH₂CH₃ | CH(CH₃)₂ | NH |
| Ia1.2034 | SO₂CH₃ | (CH₂)₂CH₃ | CH(CH₃)₂ | NH |
| Ia1.2035 | SO₂CH₃ | (CH₂)₃CH₃ | CH(CH₃)₂ | NH |
| Ia1.2036 | SO₂CH₃ | H | (CH₂)₃CH₃ | NH |
| Ia1.2037 | SO₂CH₃ | CH₃ | (CH₂)₃CH₃ | NH |
| Ia1.2038 | SO₂CH₃ | CH₂CH₃ | (CH₂)₃CH₃ | NH |
| Ia1.2039 | SO₂CH₃ | (CH₂)₂CH₃ | (CH₂)₃CH₃ | NH |
| Ia1.2040 | SO₂CH₃ | (CH₂)₃CH₃ | (CH₂)₃CH₃ | NH |
| Ia1.2041 | SO₂CH₃ | H | CH₂CH(CH₃)₂ | NH |
| Ia1.2042 | SO₂CH₃ | CH₃ | CH₂CH(CH₃)₂ | NH |
| Ia1.2043 | SO₂CH₃ | CH₂CH₃ | CH₂CH(CH₃)₂ | NH |
| Ia1.2044 | SO₂CH₃ | (CH₂)₂CH₃ | CH₂CH(CH₃)₂ | NH |
| Ia1.2045 | SO₂CH₃ | (CH₂)₃CH₃ | CH₂CH(CH₃)₂ | NH |
| Ia1.2046 | SO₂CH₃ | H | CH(CH₃)CH₂CH₃ | NH |
| Ia1.2047 | SO₂CH₃ | CH₃ | CH(CH₃)CH₂CH₃ | NH |
| Ia1.2048 | SO₂CH₃ | CH₂CH₃ | CH(CH₃)CH₂CH₃ | NH |
| Ia1.2049 | SO₂CH₃ | (CH₂)₂CH₃ | CH(CH₃)CH₂CH₃ | NH |
| Ia1.2050 | SO₂CH₃ | (CH₂)₃CH₃ | CH(CH₃)CH₂CH₃ | NH |
| Ia1.2051 | SO₂CH₃ | H | (CH₂)₂-C₆H₅ | NH |
| Ia1.2052 | SO₂CH₃ | CH₃ | (CH₂)₂-C₆H₅ | NH |
| Ia1.2053 | SO₂CH₃ | CH₂CH₃ | (CH₂)₂-C₆H₅ | NH |
| Ia1.2054 | SO₂CH₃ | (CH₂)₂CH₃ | (CH₂)₂-C₆H₅ | NH |
| Ia1.2055 | SO₂CH₃ | (CH₂)₃CH₃ | (CH₂)₂-C₆H₅ | NH |
| Ia1.2056 | SO₂CH₃ | H | CH₂CH(CH₃)-C₆H₅ | NH |
| Ia1.2057 | SO₂CH₃ | CH₃ | CH₂CH(CH₃)-C₆H₅ | NH |
| Ia1.2058 | SO₂CH₃ | CH₂CH₃ | CH₂CH(CH₃)-C₆H₅ | NH |
| Ia1.2059 | SO₂CH₃ | (CH₂)₂CH₃ | CH₂CH(CH₃)-C₆H₅ | NH |
| Ia1.2060 | SO₂CH₃ | (CH₂)₃CH₃ | CH₂CH(CH₃)-C₆H₅ | NH |
| Ia1.2061 | SO₂CH₃ | H | CH(CH₃)CH₂-C₆H₅ | NH |
| Ia1.2062 | SO₂CH₃ | CH₃ | CH(CH₃)CH₂-C₆H₅ | NH |
| Ia1.2063 | SO₂CH₃ | CH₂CH₃ | CH(CH₃)CH₂-C₆H₅ | NH |
| Ia1.2064 | SO₂CH₃ | (CH₂)₂CH₃ | CH(CH₃)CH₂-C₆H₅ | NH |
| Ia1.2065 | SO₂CH₃ | (CH₂)₃CH₃ | CH(CH₃)CH₂-C₆H₅ | NH |
| Ia1.2066 | SO₂CH₃ | H | CH(CH₃)CH(CH₃)-C₆H₅ | NH |
| Ia1.2067 | SO₂CH₃ | CH₃ | CH(CH₃)CH(CH₃)-C₆H₅ | NH |
| Ia1.2068 | SO₂CH₃ | CH₂CH₃ | CH(CH₃)CH(CH₃)-C₆H₅ | NH |
| Ia1.2069 | SO₂CH₃ | (CH₂)₂CH₃ | CH(CH₃)CH(CH₃)-C₆H₅ | NH |
| Ia1.2070 | SO₂CH₃ | (CH₂)₃CH₃ | CH(CH₃)CH(CH₃)-C₆H₅ | NH |
| Ia1.2071 | SO₂CH₃ | H | (CH₂)₂-(4-Cl-C₆H₄) | NH |
| Ia1.2072 | SO₂CH₃ | CH₃ | (CH₂)₂-(4-Cl-C₆H₄) | NH |
| Ia1.2073 | SO₂CH₃ | CH₂CH₃ | (CH₂)₂-(4-Cl-C₆H₄) | NH |
| Ia1.2074 | SO₂CH₃ | (CH₂)₂CH₃ | (CH₂)₂-(4-Cl-C₆H₄) | NH |
| Ia1.2075 | SO₂CH₃ | (CH₂)₃CH₃ | (CH₂)₂-(4-Cl-C₆H₄) | NH |
| Ia1.2076 | SO₂CH₃ | H | CH₂CH(CH₃)-(4-Cl-C₆H₄) | NH |
| Ia1.2077 | SO₂CH₃ | CH₃ | CH₂CH(CH₃)-(4-Cl-C₆H₄) | NH |
| Ia1.2078 | SO₂CH₃ | CH₂CH₃ | CH₂CH(CH₃)-(4-Cl-C₆H₄) | NH |
| Ia1.2079 | SO₂CH₃ | (CH₂)₂CH₃ | CH₂CH(CH₃)-(4-Cl-C₆H₄) | NH |
| Ia1.2080 | SO₂CH₃ | (CH₂)₃CH₃ | CH₂CH(CH₃)-(4-Cl-C₆H₄) | NH |
| Ia1.2081 | SO₂CH₃ | H | CH(CH₃)CH₂-(4-Cl-C₆H₄) | NH |
| Ia1.2082 | SO₂CH₃ | CH₃ | CH(CH₃)CH₂-(4-Cl-C₆H₄) | NH |
| Ia1.2083 | SO₂CH₃ | CH₂CH₃ | CH(CH₃)CH₂-(4-Cl-C₆H₄) | NH |
| Ia1.2084 | SO₂CH₃ | (CH₂)₂CH₃ | CH(CH₃)CH₂-(4-Cl-C₆H₄) | NH |
| Ia1.2085 | SO₂CH₃ | (CH₂)₃CH₃ | CH(CH₃)CH₂-(4-Cl-C₆H₄) | NH |
| Ia1.2086 | SO₂CH₃ | H | CH(CH₃)CH(CH₃)-(4-Cl-C₆H₄) | NH |
| Ia1.2087 | SO₂CH₃ | CH₃ | CH(CH₃)CH(CH₃)-(4-Cl-C₆H₄) | NH |
| Ia1.2088 | SO₂CH₃ | CH₂CH₃ | CH(CH₃)CH(CH₃)-(4-Cl-C₆H₄) | NH |
| Ia1.2089 | SO₂CH₃ | (CH₂)₂CH₃ | CH(CH₃)CH(CH₃)-(4-Cl-C₆H₄) | NH |
| Ia1.2090 | SO₂CH₃ | (CH₂)₃CH₃ | CH(CH₃)CH(CH₃)-(4-Cl-C₆H₄) | NH |
| Ia1.2091 | SO₂CH₃ | H | (CH₂)₂-(2,4-Cl₂-C₆H₃) | NH |
| Ia1.2092 | SO₂CH₃ | CH₃ | (CH₂)₂-(2,4-Cl₂-C₆H₃) | NH |
| Ia1.2093 | SO₂CH₃ | CH₂CH₃ | (CH₂)₂-(2,4-Cl₂-C₆H₃) | NH |
| Ia1.2094 | SO₂CH₃ | (CH₂)₂CH₃ | (CH₂)₂-(2,4-Cl₂-C₆H₃) | NH |
| Ia1.2095 | SO₂CH₃ | (CH₂)₃CH₃ | (CH₂)₂-(2,4 Cl₂-C₆H₃) | NH |
| Ia1.2096 | SO₂CH₃ | H | CH₂CH(CH₃)-(2,4 Cl₂-C₆H₃) | NH |
| Ia1.2097 | SO₂CH₃ | CH₃ | CH₂CH(CH₃)-(2,4 Cl₂-C₆H₃) | NH |
| Ia1.2098 | SO₂CH₃ | CH₂CH₃ | CH₂CH(CH₃)-(2,4 Cl₂-C₆H₃) | NH |
| Ia1.2099 | SO₂CH₃ | (CH₂)₂CH₃ | CH₂CH(CH₃)-(2,4-Cl₂-C₆H₃) | NH |
| Ia1.2100 | SO₂CH₃ | (CH₂)₃CH₃ | CH₂CH(CH₃)-(2,4-Cl₂-C₆H₃) | NH |
| Ia1.2101 | SO₂CH₃ | H | CH(CH₃)CH₂-(2,4-Cl₂-C₆H₃) | NH |
| Ia1.2102 | SO₂CH₃ | CH₃ | CH(CH₃)CH₂-(2,4-Cl₂-C₆H₃) | NH |
| Ia1.2103 | SO₂CH₃ | CH₂CH₃ | CH(CH₃)CH₂-(2,4-Cl₂-C₆H₃) | NH |
| Ia1.2104 | SO₂CH₃ | (CH₂)₂CH₃ | CH(CH₃)CH₂-(2,4-Cl₂-C₆H₃) | NH |
| Ia1.2105 | SO₂CH₃ | (CH₂)₃CH₃ | CH(CH₃)CH₂-(2,4-Cl₂-C₆H₃) | NH |
| Ia1.2106 | SO₂CH₃ | H | CH(CH₃)CH(CH₃)-(2,4-Cl₂-C₆H₃) | NH |
| Ia1.2107 | SO₂CH₃ | CH₃ | CH(CH₃)CH(CH₃)-(2,4-Cl₂-C₆H₃) | NH |
| Ia1.2108 | SO₂CH₃ | CH₂CH₃ | CH(CH₃)CH(CH₃)-(2,4-Cl₂-C₆H₃) | NH |
| Ia1.2109 | SO₂CH₃ | (CH₂)₂CH₃ | CH(CH₃)CH(CH₃)-(2,4-Cl₂-C₆H₃) | NH |
| Ia1.2110 | SO₂CH₃ | (CH₂)₃CH₃ | CH(CH₃)CH(CH₃)-(2,4-Cl₂-C₆H₃) | NH |
| Ia1.2111 | SO₂CH₃ | H | (CH₂)₂-O-C₆H₅ | NH |
| Ia1.2112 | SO₂CH₃ | CH₃ | (CH₂)₂-O-C₆H₅ | NH |
| Ia1.2113 | SO₂CH₃ | CH₂CH₃ | (CH₂)₂-O-C₆H₅ | NH |
| Ia1.2114 | SO₂CH₃ | (CH₂)₂CH₃ | (CH₂)₂-O-C₆H₅ | NH |
| Ia1.2115 | SO₂CH₃ | (CH₂)₃CH₃ | (CH₂)₂-O-C₆H₅ | NH |
| Ia1.2116 | SO₂CH₃ | H | CH₂CH(CH₃)-O-C₆H₅ | NH |
| Ia1.2117 | SO₂CH₃ | CH₃ | CH₂CH(CH₃)-O-C₆H₅ | NH |
| Ia1.2118 | SO₂CH₃ | CH₂CH₃ | CH₂CH(CH₃)-O-C₆H₅ | NH |
| Ia1.2119 | SO₂CH₃ | (CH₂)₂CH₃ | CH₂CH(CH₃)-O-C₆H₅ | NH |
| Ia1.2120 | SO₂CH₃ | (CH₂)₃CH₃ | CH₂CH(CH₃)-O-C₆H₅ | NH |
| Ia1.2121 | SO₂CH₃ | H | CH(CH₃)CH₂-O-C₆H₅ | NH |
| Ia1.2122 | SO₂CH₃ | CH₃ | CH (CH₃)CH₂-O-C₆H₅ | NH |
| Ia1.2123 | SO₂CH₃ | CH₂CH₃ | CH(CH₃)CH₂-O-C₆H₅ | NH |
| Ia1.2124 | SO₂CH₃ | (CH₂)₂CH₃ | CH(CH₃)CH₂-O-C₆H₅ | NH |
| Ia1.2125 | SO₂CH₃ | (CH₂)₃CH₃ | CH(CH₃)CH₂-O-C₆H₅ | NH |
| Ia1.2126 | SO₂CH₃ | H | CH(CH₃)CH(CH₃)-O-C₆H₅ | NH |
| Ia1.2127 | SO₂CH₃ | CH₃ | CH(CH₃)CH(CH₃)-O C₆H₅ | NH |
| Ia1.2128 | SO₂CH₃ | CH₂CH₃ | CH(CH₃)CH(CH₃)-O-C₆H₅ | NH |
| Ia1.2129 | SO₂CH₃ | (CH₂)₂CH₃ | CH(CH₃)CH(CH₃)-O-C₆H₅ | NH |
| Ia1.2130 | SO₂CH₃ | (CH₂)₃CH₃ | CH(CH₃)CH(CH₃)-O-C₆H₅ | NH |
| Ia1.2131 | SO₂CH₃ | H | (CH₂)₂-O-(4-Cl-C₆H₄) | NH |
| Ia1.2132 | SO₂CH₃ | CH₃ | (CH₂)₂-O-(4-Cl-C₆H₄) | NH |
| Ia1.2133 | SO₂CH₃ | CH₂CH₃ | (CH₂)₂-O-(4-Cl-C₆H₄) | NH |
| Ia1.2134 | SO₂CH₃ | (CH₂)₂CH₃ | (CH₂)₂-O-(4-Cl-C₆H₄) | NH |
| Ia1.2135 | SO₂CH₃ | (CH₂)₃CH₃ | (CH₂)₂-O-(4-Cl-C₆H₄) | NH |
| Ia1.2136 | SO₂CH₃ | H | CH₂CH(CH₃)-O(4-Cl-C₆H₄) | NH |
| Ia1.2137 | SO₂CH₃ | CH₃ | CH₂CH(CH₃)-O-(4-Cl-C₆H₄) | NH |
| Ia1.2138 | SO₂CH₃ | CH₂CH₃ | CH₂CH(CH₃)-O(4-Cl-C₆H₄) | NH |
| Ia1.2139 | SO₂CH₃ | (CH₂)₂CH₃ | CH₂CH(CH₃)-O-(4-Cl-C₆H₄) | NH |
| Ia1.2140 | SO₂CH₃ | (CH₂)₃CH₃ | CH₂CH(CH₃)-O-(4-Cl-C₆H₄) | NH |
| Ia1.2141 | SO₂CH₃ | H | CH(CH₃)CH₂-O-(4-Cl-C₆H₄) | NH |
| Ia1.2142 | SO₂CH₃ | CH₃ | CH(CH₃)CH₂-O-(4-Cl-C₆H₄) | NH |
| Ia1.2143 | SO₂CH₃ | CH₂CH₃ | CH(CH₃)CH₂-O-(4-Cl-C₆H₄) | NH |
| Ia1.2144 | SO₂CH₃ | (CH₂)₂CH₃ | CH(CH₃)CH₂-O-(4-Cl-C₆H₄) | NH |
| Ia1.2145 | SO₂CH₃ | (CH₂)₃CH₃ | CH(CH₃)CH₂-O-(4-Cl-C₆H₄) | NH |
| Ia1.2146 | SO₂CH₃ | H | CH(CH₃)CH(CH₃)-O-(4-Cl-C₆H₄) | NH |
| Ia1.2147 | SO₂CH₃ | CH₃ | CH(CH₃)CH(CH₃)-O-(4-Cl-C₆H₄) | NH |
| Ia1.2148 | SO₂CH₃ | CH₂CH₃ | CH(CH₃)CH(CH₃)-O-(4-Cl-C₆H₄) | NH |
| Ia1.2149 | SO₂CH₃ | (CH₂)₂CH₃ | CH(CH₃)CH(CH₃)-O-(4-Cl-C₆H₄) | NH |
| Ia1.2150 | SO₂CH₃ | (CH₂)₃CH₃ | CH(CH₃)CH(CH₃)-O-(4-Cl-C₆H₄) | NH |
| Ia1.2151 | SO₂CH₃ | H | (CH₂)₂-O-(2,4-Cl₂-C₆H₃) | NH |
| Ia1.2152 | SO₂CH₃ | CH₃ | (CH₂)₂-O-(2,4-Cl₂-C₆H₃) | NH |
| Ia1.2153 | SO₂CH₃ | CH₂CH₃ | (CH₂)₂-O-(2,4-Cl₂-C₆H₃) | NH |
| Ia1.2154 | SO₂CH₃ | (CH₂)₂CH₃ | (CH₂)₂-O-(2,4-Cl₂-C₆H₃) | NH |
| Ia1.2155 | SO₂CH₃ | (CH₂)₃CH₃ | (CH₂)₂-O-(2,4-Cl₂-C₆H₃) | NH |
| Ia1.2156 | SO₂CH₃ | H | CH₂CH(CH₃)-O-(2,4-Cl₂-C₆H₃) | NH |
| Ia1.2157 | SO₂CH₃ | CH₃ | CH₂CH(CH₃)-O-(2,4-Cl₂-C₆H₃) | NH |
| Ia1.2158 | SO₂CH₃ | CH₂CH₃ | CH₂CH(CH₃)-O-(2,4-Cl₂-C₆H₃) | NH |
| Ia1.2159 | SO₂CH₃ | (CH₂)₂CH₃ | CH₂CH(CH₃)-O-(2,4-Cl₂-C₆H₃) | NH |
| Ia1.2160 | SO₂CH₃ | (CH₂)₃CH₃ | CH₂CH(CH₃)-O-(2,4-Cl₂-C₆H₃) | NH |
| Ia1.2161 | SO₂CH₃ | H | CH(CH₃)CH₂-O-(2,4-Cl₂-C₆H₃) | NH |
| Ia1.2162 | SO₂CH₃ | CH₃ | CH(CH₃)CH₂-O-(2,4-Cl₂-C₆H₃) | NH |
| Ia1.2163 | SO₂CH₃ | CH₂CH₃ | CH(CH₃)CH₂-O-(2,4-Cl₂-C₆H₃) | NH |
| Ia1-2164 | SO₂CH₃ | (CH₂)₂CH₃ | CH(CH₃)CH₂-O-(2,4-Cl₂-C₆H₃) | NH |
| Ia1.2165 | SO₂CH₃ | (CH₂)₃CH₃ | CH(CH₃)CH₂-O-(2,4-Cl₂-C₆H₃) | NH |
| Ia1.2166 | SO₂CH₃ | H | CH(CH₃)CH(CH₃)-O-(2,4-Cl₂-C₆H₃) | NH |
| Ia1.2167 | SO₂CH₃ | CH₃ | CH(CH₃)CH(CH₃)-O-(2,4-Cl₂-C₆H₃) | NH |
| Ia1.2168 | SO₂CH₃ | CH₂CH₃ | CH(CH₃)CH(CH₃)-O-(2,4-Cl₂-C₆H₃) | NH |
| Ia1.2169 | SO₂CH₃ | (CH₂)₂CH₃ | CH(CH₃)CH(CH₃)-O-(2,4-Cl₂-C₆H₃) | NH |
| Ia1.2170 | SO₂CH₃ | (CH₂)₃CH₃ | CH(CH₃)CH(CH₃)-O-(2,4-Cl₂-C₆H₃) | NH |
| Ia1.2171 | SO₂CH₃ | H | CH₃ | NCH₃ |
| Ia1.2172 | SO₂CH₃ | CH₃ | CH₃ | NCH₃ |
| Ia1.2173 | SO₂CH₃ | CH₂CH₃ | CH₃ | NCH₃ |
| Ia1.2174 | SO₂CH₃ | (CH₂)₂CH₃ | CH₃ | NCH₃ |
| Ia1.2175 | SO₂CH₃ | (CH₂)₃CH₃ | CH₃ | NCH₃ |
| Ia1.2176 | SO₂CH₃ | H | C₂H₅ | NCH₃ |
| Ia1.2177 | SO₂CH₃ | CH₃ | C₂H₅ | NCH₃ |
| Ia1.2178 | SO₂CH₃ | CH₂CH₃ | C₂H₅ | NCH₃ |
| Ia1.2179 | SO₂CH₃ | (CH₂)₂CH₃ | C₂H₅ | NCH₃ |
| Ia1.2180 | SO₂CH₃ | (CH₂)₃CH₃ | C₂H₅ | NCH₃ |
| Ia1.2181 | SO₂CH₃ | H | (CH₂)₂CH₃ | NCH₃ |
| Ia1.2182 | SO₂CH₃ | CH₃ | (CH₂)₂CH₃ | NCH₃ |
| Ia1.2183 | SO₂CH₃ | CH₂CH₃ | (CH₂)₂CH₃ | NCH₃ |
| Ia1.2184 | SO₂CH₃ | (CH₂)₂CH₃ | (CH₂)₂CH₃ | NCH₃ |
| Ia1.2185 | SO₂CH₃ | (CH₂)₃CH₃ | (CH₂)₂CH₃ | NCH₃ |
| Ia1.2186 | SO₂CH₃ | H | CH(CH₃)₂ | NCH₃ |
| Ia1.2187 | SO₂CH₃ | CH₃ | CH(CH₃)₂ | NCH₃ |
| Ia1.2188 | SO₂CH₃ | CH₂CH₃ | CH(CH₃)₂ | NCH₃ |
| Ia1.2189 | SO₂CH₃ | (CH₂)₂CH₃ | CH(CH₃)₂ | NCH₃ |
| Ia1.2190 | SO₂CH₃ | (CH₂)₃CH₃ | CH(CH₃)₂ | NCH₃ |
| Ia1.2191 | SO₂CH₃ | H | (CH₂)₃CH₃ | NCH₃ |
| Ia1.2192 | SO₂CH₃ | CH₃ | (CH₂)₃CH₃ | NCH₃ |
| Ia1.2193 | SO₂CH₃ | CH₂CH₃ | (CH₂)₃CH₃ | NCH₃ |
| Ia1.2194 | SO₂CH₃ | (CH₂)₂CH₃ | (CH₂)₃CH₃ | NCH₃ |
| Ia1.2195 | SO₂CH₃ | (CH₂)₃CH₃ | (CH₂)₃CH₃ | NCH₃ |
| Ia1.2196 | SO₂CH₃ | H | CH₂CH(CH₃)₂ | NCH₃ |
| Ia1.2197 | SO₂CH₃ | CH₃ | CH₂CH(CH₃)₂ | NCH₃ |
| Ia1.2198 | SO₂CH₃ | CH₂CH₃ | CH₂CH(CH₃)₂ | NCH₃ |
| Ia1.2199 | SO₂CH₃ | (CH₂)₂CH₃ | CH₂CH(CH₃)₂ | NCH₃ |
| Ia1.2200 | SO₂CH₃ | (CH₂)₃CH₃ | CH₂CH(CH₃)₂ | NCH₃ |
| Ia1.2201 | SO₂CH₃ | H | CH(CH₃)CH₂CH₃ | NCH₃ |
| Ia1.2202 | SO₂CH₃ | CH₃ | CH(CH₃)CH₂CH₃ | NCH₃ |
| Ia1.2203 | SO₂CH₃ | CH₂CH₃ | CH(CH₃)CH₂CH₃ | NCH₃ |
| Ia1.2204 | SO₂CH₃ | (CH₂)₂CH₃ | CH(CH₃)CH₂CH₃ | NCH₃ |
| Ia1.2205 | SO₂CH₃ | (CH₂)₃CH₃ | CH(CH₃)CH₂CH₃ | NCH₃ |
| Ia1.2206 | SO₂CH₃ | H | (CH₂)₂-C₆H₅ | NCH₃ |
| Ia1.2207 | SO₂CH₃ | CH₃ | (CH₂)₂-C₆H₅ | NCH₃ |
| Ia1.2208 | SO₂CH₃ | CH₂CH₃ | (CH₂)₂-C₆H₅ | NCH₃ |
| Ia1.2209 | SO₂CH₃ | (CH₂)₂CH₃ | (CH₂)₂-C₆H₅ | NCH₃ |
| Ia1.2210 | SO₂CH₃ | (CH₂)₃CH₃ | (CH₂)₂-C₆H₅ | NCH₃ |
| Ia1.2211 | SO₂CH₃ | H | CH₂CH(CH₃)-C₆H₅ | NCH₃ |
| Ia1.2212 | SO₂CH₃ | CH₃ | CH₂CH(CH₃)-C₆H₅ | NCH₃ |
| Ia1.2213 | SO₂CH₃ | CH₂CH₃ | CH₂CH(CH₃)-C₆H₅ | NCH₃ |
| Ia1.2214 | SO₂CH₃ | (CH₂)₂CH₃ | CH₂CH(CH₃)-C₆H₅ | NCH₃ |
| Ia1.2215 | SO₂CH₃ | (CH₂)₃CH₃ | CH₂CH(CH₃)-C₆H₅ | NCH₃ |
| Ia1.2216 | SO₂CH₃ | H | CH(CH₃)CH₂-C₆H₅ | NCH₃ |
| Ia1.2217 | SO₂CH₃ | CH₃ | CH(CH₃)CH₂-C₆H₅ | NCH₃ |
| Ia1.2218 | SO₂CH₃ | CH₂CH₃ | CH(CH₃)CH₂-C₆H₅ | NCH₃ |
| Ia1.2219 | SO₂CH₃ | (CH₂)₂CH₃ | CH(CH₃)CH₂-C₆H₅ | NCH₃ |
| Ia1.2220 | SO₂CH₃ | (CH₂)₃CH₃ | CH(CH₃)CH₂-C₆H₅ | NCH₃ |
| Ia1.2221 | SO₂CH₃ | H | CH(CH₃)CH(CH₃)-C₆H₅ | NCH₃ |
| Ia1.2222 | SO₂CH₃ | CH₃ | CH(CH₃)CH(CH₃)-C₆H₅ | NCH₃ |
| Ia1.2223 | SO₂CH₃ | CH₂CH₃ | CH(CH₃)CH(CH₃)-C₆H₅ | NCH₃ |
| Ia1.2224 | SO₂CH₃ | (CH₂)₂CH₃ | CH(CH₃)CH(CH₃)-C₆H₅ | NCH₃ |
| Ia1.2225 | SO₂CH₃ | (CH₂)₃CH₃ | CH(CH₃)CH(CH₃)-C₆H₅ | NCH₃ |
| Ia1.2226 | SO₂CH₃ | H | (CH₂)₂(4-Cl-C₆H₄) | NCH₃ |
| Ia1.2227 | SO₂CH₃ | CH₃ | (CH₂)₂-(4-Cl-C₆H₄) | NCH₃ |
| Ia1.2228 | SO₂CH₃ | CH₂CH₃ | (CH₂)₂-(4-Cl-C₆H₄) | NCH₃ |
| Ia1.2229 | SO₂CH₃ | (CH₂)₂CH₃ | (CH₂)₂-(4-Cl-C₆H₄) | NCH₃ |
| Ia1.2230 | SO₂CH₃ | (CH₂)₃CH₃ | (CH₂)₂-(4-Cl-C₆H₄) | NCH₃ |
| Ia1.2231 | SO₂CH₃ | H | CH₂CH(CH₃)-(4-Cl-C₆H₄) | NCH₃ |
| Ia1.2232 | SO₂CH₃ | CH₃ | CH₂CH(CH₃)-(4-Cl-C₆H₄) | NCH₃ |
| Ia1.2233 | SO₂CH₃ | CH₂CH₃ | CH₂CH(CH₃)-(4-Cl-C₆H₄) | NCH₃ |
| Ia1.2234 | SO₂CH₃ | (CH₂)₂CH₃ | CH₂CH(CH₃)-(4-Cl-C₆H₄) | NCH₃ |
| Ia1.2235 | SO₂CH₃ | (CH₂)₃CH₃ | CH₂CH(CH₃)-(4-Cl-C₆H₄) | NCH₃ |
| Ia1.2236 | SO₂CH₃ | H | CH(CH₃)CH₂-(4-Cl-C₆H₄) | NCH₃ |
| Ia1.2237 | SO₂CH₃ | CH₃ | CH(CH₃)CH₂-(4-Cl-C₆H₄) | NCH₃ |
| Ia1.2238 | SO₂CH₃ | CH₂CH₃ | CH(CH₃)CH₂-(4-Cl-C₆H₄) | NCH₃ |
| Ia1.2239 | SO₂CH₃ | (CH₂)₂CH₃ | CH(CH₃)CH₂-(4-Cl-C₆H₄) | NCH₃ |
| Ia1.2240 | SO₂CH₃ | (CH₂)₃CH₃ | CH(CH₃)CH₂-(4-Cl-C₆H₄) | NCH₃ |
| Ia1.2241 | SO₂CH₃ | H | CH(CH₃)CH(CH₃)-(4-Cl-C₆H₄) | NCH₃ |
| Ia1.2242 | SO₂CH₃ | CH₃ | CH(CH₃)CH(CH₃)-(4-Cl-C₆H₄) | NCH₃ |
| Ia1.2243 | SO₂CH₃ | CH₂CH₃ | CH(CH₃)CH(CH₃)-(4-Cl-C₆H₄) | NCH₃ |
| Ia1.2244 | SO₂CH₃ | (CH₂)₂CH₃ | CH(CH₃)CH(CH₃)-(4-Cl-C₆H₄) | NCH₃ |
| Ia1.2245 | SO₂CH₃ | (CH₂)₃CH₃ | CH(CH₃)CH(CH₃)-(4-Cl-C₆H₄) | NCH₃ |
| Ia1.2246 | SO₂CH₃ | H | (CH₂)₂-(2,4-Cl₂-C₆H₃) | NCH₃ |
| Ia1.2247 | SO₂CH₃ | CH₃ | (CH₂)₂-(2,4-Cl₂-C₆H₃) | NCH₃ |
| Ia1.2248 | SO₂CH₃ | CH₂CH₃ | (CH₂)₂-(2,4-Cl₂-C₆H₃) | NCH₃ |
| Ia1.2249 | SO₂CH₃ | (CH₂)₂CH₃ | (CH₂)₂-(2,4-Cl₂-C₆H₃) | NCH₃ |
| Ia1.2250 | SO₂CH₃ | (CH₂)₃CH₃ | (CH₂)₂-(2,4-Cl₂-C₆H₃) | NCH₃ |
| Ia1.2251 | SO₂CH₃ | H | CH₂CH(CH₃)-(2,4-Cl₂-C₆H₃) | NCH₃ |
| Ia1.2252 | SO₂CH₃ | CH₃ | CH₂CH(CH₃)-(2,4-Cl₂-C₆H₃) | NCH₃ |
| Ia1.2253 | SO₂CH₃ | CH₂CH₃ | CH₂CH(CH₃)-(2,4-Cl₂-C₆H₃) | NCH₃ |
| Ia1.2254 | SO₂CH₃ | (CH₂)₂CH₃ | CH₂CH(CH₃)-(2,4-Cl₂-C₆H₃) | NCH₃ |
| Ia1.2255 | SO₂CH₃ | (CH₂)₃CH₃ | CH₂CH(CH₃)-(2,4-Cl₂-C₆H₃) | NCH₃ |
| Ia1.2256 | SO₂CH₃ | H | CH(CH₃)CH₂-(2,4-Cl₂-C₆H₃) | NCH₃ |
| Ia1.2257 | SO₂CH₃ | CH₃ | CH(CH₃)CH₂-(2,4-Cl₂-C₆H₃) | NCH₃ |
| Ia1.2258 | SO₂CH₃ | CH₂CH₃ | CH(CH₃)CH₂-(2,4-Cl₂-C₆H₃) | NCH₃ |
| Ia1.2259 | SO₂CH₃ | (CH₂)₂CH₃ | CH(CH₃)CH₂-(2,4-Cl₂-C₆H₃) | NCH₃ |
| Ia1.2260 | SO₂CH₃ | (CH₂)₃CH₃ | CH(CH₃)CH₂-(2,4-Cl₂-C₆H₃) | NCH₃ |
| Ia1.2261 | SO₂CH₃ | H | CH(CH₃)CH₂-(2,4-Cl₂-C₆H₃) | NCH₃ |
| Ia1.2262 | SO₂CH₃ | CH₃ | CH(CH₃)CH(CH₃)-(2,4-Cl₂-C₆H₃) | NCH₃ |
| Ia1.2263 | SO₂CH₃ | CH₂CH₃ | CH(CH₃)CH(CH₃)-(2,4-Cl₂-C₆H₃) | NCH₃ |
| Ia1.2264 | SO₂CH₃ | (CH₂)₂CH₃ | CH(CH₃)CH(CH₃)-(2,4-Cl₂-C₆H₃) | NCH₃ |
| Ia1.2265 | SO₂CH₃ | (CH₂)₃CH₃ | CH(CH₃)CH(CH₃)-(2,4-Cl₂-C₆H₃) | NCH₃ |
| Ia1.2266 | SO₂CH₃ | H | (CH₂)-₂-O-C₆H₅ | NCH₃ |
| Ia1.2267 | SO₂CH₃ | CH₃ | (CH₂)-₂-O-C₆H₅ | NCH₃ |
| Ia1.2268 | SO₂CH₃ | CH₂CH₃ | (CH₂)-₂-O-C₆H₅ | NCH₃ |
| Ia1.2269 | SO₂CH₃ | (CH₂)₂CH₃ | (CH₂)-₂-O-C₆H₅ | NCH₃ |
| Ia1.2270 | SO₂CH₃ | (CH₂)₃CH₃ | (CH₂)-₂-O-C₆H₅ | NCH₃ |
| Ia1.2271 | SO₂CH₃ | H | CH₂CH(CH₃)-O-C₆H₅ | NCH₃ |
| Ia1.2272 | SO₂CH₃ | CH₃ | CH₂CH(CH₃)-O-C₆H₅ | NCH₃ |
| Ia1.2273 | SO₂CH₃ | CH₂CH₃ | CH₂CH(CH₃)-O-C₆H₅ | NCH₃ |
| Ia1.2274 | SO₂CH₃ | (CH₂)₂CH₃ | CH₂CH(CH₃)-O-C₆H₅ | NCH₃ |
| Ia1.2275 | SO₂CH₃ | (CH₂)₃CH₃ | CH₂CH(CH₃)-O-C₆H₅ | NCH₃ |
| Ia1.2276 | SO₂CH₃ | H | CH(CH₃)CH₂-O-C₆H₅ | NCH₃ |
| Ia1.2277 | SO₂CH₃ | CH₃ | CH(CH₃)CH₂-O-C₆H₅ | NCH₃ |
| la1.2278 | SO₂CH₃ | CH₂CH₃ | CH(CH₃)CH₂-O-C₆H₅ | NCH₃ |
| Ia1.2279 | SO₂CH₃ | (CH₂)₂CH₃ | CH(CH₃)CH₂-O-C₆H₅ | NCH₃ |
| Ia1.2280 | SO₂CH₃ | (CH₂)₃CH₃ | CH(CH₃)CH₂-O-C₆H₅ | NCH₃ |
| Ia1.2281 | SO₂CH₃ | H | CH(CH₃)CH-O-C₆H₅ | NCH₃ |
| Ia1.2282 | SO₂CH₃ | CH₃ | CH(CH₃)CH-O-C₆H₅ | NCH₃ |
| Ia1.2283 | SO₂CH₃ | CH₂CH₃ | CH(CH₃)CH-O-C₆H₅ | NCH₃ |
| Ia1.2284 | SO₂CH₃ | (CH₂)₂CH₃ | CH(CH₃)CH-O-C₆H₅ | NCH₃ |
| Ia1.2285 | SO₂CH₃ | (CH₂)₃CH₃ | CH(CH₃)CH-O-C₆H₅ | NCH₃ |
| Ia1.2286 | SO₂CH₃ | H | (CH₂)₂-O-(4-Cl-C₆H₄) | NCH₃ |
| Ia1.2287 | SO₂CH₃ | CH₃ | (CH₂)₂-O-(4-Cl-C₆H₄) | NCH₃ |
| Ia1.2288 | SO₂CH₃ | CH₂CH₃ | (CH₂)₂-O-(4-Cl-C₆H₄) | NCH₃ |
| Ia1.2289 | SO₂CH₃ | (CH₂)₂CH₃ | (CH₂)₂-O-(4-Cl-C₆H₄) | NCH₃ |
| Ia1.2290 | SO₂CH₃ | (CH₂)₃CH₃ | (CH₂)₂-O-(4-Cl-C₆H₄) | NCH₃ |
| Ia1.2291 | SO₂CH₃ | H | CH₂CH(CH₃)-O-(4-Cl-C₆H₄) | NCH₃ |
| Ia1.2292 | SO₂CH₃ | CH₃ | CH₂CH(CH₃)-O-(4-Cl-C₆H₄) | NCH₃ |
| Ia1.2293 | SO₂CH₃ | CH₂CH₃ | CH₂CH(CH₃)-O-(4-Cl-C₆H₄) | NCH₃ |
| Ia1.2294 | SO₂CH₃ | (CH₂)₂CH₃ | CH₂CH(CH₃)-O-(4-Cl-C₆H₄) | NCH₃ |
| Ia1.2295 | SO₂CH₃ | (CH₂)₃CH₃ | CH₂CH(CH₃)-O-(4-Cl-C₆H₄) | NCH₃ |
| Ia1.2296 | SO₂CH₃ | H | CH(CH₃)CH₂-O-(4-Cl-C₆H₄) | NCH₃ |
| Ia1.2297 | SO₂CH₃ | CH₃ | CH(CH₃)CH₂-O-(4-Cl-C₆H₄) | NCH₃ |
| Ia1.2298 | SO₂CH₃ | CH₂CH₃ | CH(CH₃)CH₂-O-(4-Cl-C₆H₄) | NCH₃ |
| Ia1.2299 | SO₂CH₃ | (CH₂)₂CH₃ | CH(CH₃)CH₂-O-(4-Cl-C₆H₄) | NCH₃ |
| Ia1.2300 | SO₂CH₃ | (CH₂)₃CH₃ | CH(CH₃)CH₂-O-(4-Cl-C₆H₄) | NCH₃ |
| Ia1.2301 | SO₂CH₃ | H | CH(CH₃)CH(CH₃)-O-(4-Cl-C₆H₄) | NCH₃ |
| Ia1.2302 | SO₂CH₃ | CH₃ | CH(CH₃)CH(CH₃)-O-(4-Cl**-**C₆H₄) | NCH₃ |
| Ia1.2303 | SO₂CH₃ | CH₂CH₃ | CH(CH₃)CH(CH₃)-O-(4-Cl-C₆H₄) | NCH₃ |
| Ia1.2304 | SO₂CH₃ | (CH₂)₂CH₃ | CH(CH₃)CH(CH₃)-O-(4-Cl-C₆H₄) | NCH₃ |
| Ia1.2305 | SO₂CH₃ | (CH₂)₃CH₃ | CH(CH₃)CH(CH₃)-O-(4-Cl-C₆H₄) | NCH₃ |
| Ia1.2306 | SO₂CH₃ | H | (CH₂)₂-O-(2,4-Cl₂-C₆H₃) | NCH₃ |
| Ia1.2307 | SO₂CH₃ | CH₃ | (CH₂)₂-O-(2,4-Cl₂-C₆H₃) | NCH₃ |
| Ia1.2308 | SO₂CH₃ | CH₂CH₃ | (CH₂)₂-O-(2,4-Cl₂-C₆H₃) | NCH₃ |
| Ia1.2309 | SO₂CH₃ | (CH₂)₂CH₃ | (CH₂)₂-O-(2,4-Cl₂-C₆H₃) | NCH₃ |
| Ia1.2310 | SO₂CH₃ | (CH₂)₃CH₃ | (CH₂)₂-O-(2,4-Cl₂-C₆H₃) | NCH₃ |
| Ia1.2311 | SO₂CH₃ | H | CH₂CH(CH₃)-O-(2,4-Cl₂-C₆H₃) | NCH₃ |
| Ia1.2312 | SO₂CH₃ | CH₃ | CH₂CH(CH₃)-O-(2,4-Cl₂-C₆H₃) | NCH₃ |
| Ia1.2313 | SO₂CH₃ | CH₂CH₃ | CH₂CH(CH₃)-O-(2,4-Cl₂-C₆H₃) | NCH₃ |
| Ia1.2314 | SO₂CH₃ | (CH₂)₂CH₃ | CH₂CH(CH₃)-O-(2,4-Cl₂-C₆H₃) | NCH₃ |
| Ia1.2315 | SO₂CH₃ | (CH₂)₃CH₃ | CH₂CH(CH₃)-O-(2,4-Cl₂-C₆H₃) | NCH₃ |
| Ia1.2316 | SO₂CH₃ | H | CH(CH₃)CH₂-O-(2,4-Cl₂-C₆H₃) | NCH₃ |
| Ia1.2317 | SO₂CH₃ | CH₃ | CH(CH₃)CH₂-O-(2,4-Cl₂-C₆H₃) | NCH₃ |
| Ia1.2318 | SO₂CH₃ | CH₂CH₃ | CH(CH₃)CH₂-O-(2,4-Cl₂-C₆H₃) | NCH₃ |
| Ia1.2319 | SO₂CH₃ | (CH₂)₂CH₃ | CH(CH₃)CH₂-O-(2,4-Cl₂-C₆H₃) | NCH₃ |
| Ia1.2320 | SO₂CH₃ | (CH₂)₃CH₃ | CH(CH₃)CH₂-O-(2,4-Cl₂-C₆H₃) | NCH₃ |
| Ia1.2321 | SO₂CH₃ | H | CH(CH₃)CH(CH₃)-O-(2,4-Cl₂-C₆H₃) | NCH₃ |
| Ia1.2322 | SO₂CH₃ | CH₃ | CH(CH₃)CH(CH₃)-O-(2,4-Cl₂-C₆H₃) | NCH₃ |
| Ia1.2323 | SO₂CH₃ | CH₂CH₃ | CH(CH₃)CH(CH₃)-O-(2,4-Cl₂-C₆H₃) | NCH₃ |
| Ia1.2324 | SO₂CH₃ | (CH₂)₂CH₃ | CH(CH₃)CH(CH₃)-O-(2,4-Cl₂-C₆H₃) | NCH₃ |
| Ia1.2325 | SO₂CH₃ | (CH₂)₃CH₃ | CH(CH₃)CH(CH₃)-O-(2,4-Cl₂-C₆H₃) | NCH₃ |
| Ia1.2326 | NO₂ | H | CH₃ | O |
| Ia1.2327 | NO₂ | CH₃ | CH₃ | O |
| Ia1.2328 | NO₂ | CH₂CH₃ | CH₃ | O |
| Ia1.2329 | NO₂ | (CH₂)₂CH₃ | CH₃ | O |
| Ia1.2330 | NO₂ | (CH₂)₃CH₃ | CH₃ | O |
| Ia1.2331 | NO₂ | H | C₂H₅ | O |
| Ia1.2332 | NO₂ | CH₃ | C₂H₅ | O |
| Ia1.2333 | NO₂ | CH₂CH₃ | C₂H₅ | O |
| Ia1.2334 | NO₂ | (CH₂)₂CH₃ | C₂H₅ | O |
| Ia1.2335 | NO₂ | (CH₂)₃CH₃ | C₂H₅ | O |
| Ia1.2336 | NO₂ | H | (CH₂)₂CH₃ | O |
| Ia1.2337 | NO₂ | CH₃ | (CH₂)₂CH₃ | O |
| Ia1.2338 | NO₂ | CH₂CH₃ | (CH₂)₂CH₃ | O |
| Ia1.2339 | NO₂ | (CH₂)₂CH₃ | (CH₂)₂CH₃ | O |
| Ia1.2340 | NO₂ | (CH₂)₃CH₃ | (CH₂)₂CH₃ | O |
| Ia1.2341 | NO₂ | H | CH(CH₃)₂ | O |
| Ia1.2342 | NO₂ | CH₃ | CH(CH₃)₂ | O |
| Ia1.2343 | NO₂ | CH₂CH₃ | CH(CH₃)₂ | O |
| Ia1.2344 | NO₂ | (CH₂)₂CH₃ | CH(CH₃)₂ | O |
| Ia1.2345 | NO₂ | (CH₂)₃CH₃ | CH(CH₃)₂ | O |
| Ia1.2346 | NO₂ | H | (CH₂)₃CH₃ | O |
| Ia1.2347 | NO₂ | CH₃ | (CH₂)₃CH₃ | O |
| Ia1.2348 | NO₂ | CH₂CH₃ | (CH₂)₃CH₃ | O |
| Ia1.2349 | NO₂ | (CH₂)₂CH₃ | (CH₂)₃CH₃ | O |
| Ia1.2350 | NO₂ | (CH₂)₃CH₃ | (CH₂)₃CH₃ | O |
| Ia1.2351 | NO₂ | H | CH₂CH(CH₃)₂ | O |
| Ia1.2352 | NO₂ | CH₃ | CH₂CH(CH₃)₂ | O |
| Ia1.2353 | NO₂ | CH₂CH₃ | CH₂CH(CH₃)₂ | O |
| Ia1.2354 | NO₂ | (CH₂)₂CH₃ | CH₂CH(CH₃)₂ | O |
| Ia1.2355 | NO₂ | (CH₂)₃CH₃ | CH₂CH(CH₃)₂ | O |
| Ia1.2356 | NO₂ | H | CH(CH₃)CH₂CH₃ | O |
| Ia1.2357 | NO₂ | CH₃ | CH(CH₃)CH₂CH₃ | O |
| Ia1.2358 | NO₂ | CH₂CH₃ | CH(CH₃)CH₂CH₃ | O |
| Ia1.2359 | NO₂ | (CH₂)₂CH₃ | CH(CH₃)CH₂CH₃ | O |
| Ia1.2360 | NO₂ | (CH₂)₃CH₃ | CH(CH₃)CH₂CH₃ | O |
| Ia1.2361 | NO₂ | H | (CH₂)₂-C₆H₅ | O |
| Ia1.2362 | NO₂ | CH₃ | (CH₂)₂-C₆H₅ | O |
| Ia1.2363 | NO₂ | CH₂CH₃ | (CH₂)₂-C₆H₅ | O |
| Ia1.2364 | NO₂ | (CH₂)₂CH₃ | (CH₂)₂-C₆H₅ | O |
| Ia1.2365 | NO₂ | (CH₂)₃CH₃ | (CH₂)₂-C₆H₅ | O |
| Ia1.2366 | NO₂ | H | CH₂CH(CH₃)-C₆H₅ | O |
| Ia1.2367 | NO₂ | CH₃ | CH₂CH(CH₃)-C₆H₅ | O |
| Ia1.2368 | NO₂ | CH₂CH₃ | CH₂CH(CH₃-C₆H₅ | O |
| Ia1.2369 | NO₂ | (CH₂)₂CH₃ | CH₂CH(CH₃)-C₆H₅ | O |
| Ia1.2370 | NO₂ | (CH₂)₃CH₃ | CH₂CH(CH₃)-C₆H₅ | O |
| Ia1.2371 | NO₂ | H | CH(CH₃)CH₂-C₆H₅ | O |
| Ia1.2372 | NO₂ | CH₃ | CH(CH₃)CH₂-C₆H₅ | O |
| Ia1.2373 | NO₂ | CH₂CH₃ | CH(CH₃)CH₂-C₆H₅ | O |
| Ia1.2374 | NO₂ | (CH₂)₂CH₃ | CH(CH₃)CH₂-C₆H₅ | O |
| Ia1.2375 | NO₂ | (CH₂)₃CH₃ | CH(CH₃)CH₂-C₆H₅ | O |
| Ia1.2376 | NO₂ | H | CH(CH₃)CH(CH₃)-C₆H₅ | O |
| Ia1.2377 | NO₂ | CH₃ | CH(CH₃)CH(CH₃)-C₆H₅ | O |
| Ia1.2378 | NO₂ | CH₂CH₃ | CH(CH₃)CH(CH₃)-C₆H₅ | O |
| Ia1.2379 | NO₂ | (CH₂)₂CH₃ | CH(CH₃)CH(CH₃)-C₆H₅ | O |
| Ia1.2380 | NO₂ | (CH₂)₃CH₃ | CH(CH₃)CH(CH₃)-C₆H₅ | O |
| Ia1.2381 | NO₂ | H | (CH₂)₂-(4-Cl-C₆H₄) | O |
| Ia1.2382 | NO₂ | CH₃ | (CH₂)₂-(4-Cl-C₆H₄) | O |
| Ia1.2383 | NO₂ | CH₂CH₃ | (CH₂)₂-(4-Cl-C₆H₄) | O |
| Ia1.2384 | NO₂ | (CH₂)₂CH₃ | (CH₂)₂-(4-Cl-C₆H₄) | O |
| Ia1.2385 | NO₂ | (CH₂)₃CH₃ | (CH₂)₂-(4-Cl-C₆H₄) | O |
| Ia1.2386 | NO₂ | H | CH₂CH(CH₃)-(4-Cl-C₆H₄) | O |
| Ia1.2387 | NO₂ | CH₃ | CH₂CH(CH₃)-(4-Cl-C₆H₄) | O |
| Ia1.2388 | NO₂ | CH₂CH₃ | CH₂CH(CH₃)-(4-Cl-C₆H₄) | O |
| Ia1.2389 | NO₂ | (CH₂)₂CH₃ | CH₂CH(CH₃)-(4-Cl-C₆H₄) | O |
| Ia1.2390 | NO₂ | (CH₂)₃CH₃ | CH₂CH(CH₃)-(4-Cl-C₆H₄) | O |
| Ia1.2391 | NO₂ | H | CH(CH₃)CH₂- (4-Cl-C₆H₄) | O |
| Ia1.2392 | NO₂ | CH₃ | CH(CH₃)CH₂-(4-Cl C₆H₄) | O |
| Ia1.2393 | NO₂ | CH₂CH₃ | CH(CH₃)CH₂-(4-Cl-C₆H₄) | O |
| Ia1.2394 | NO₂ | (CH₂)₂CH₃ | CH(CH₃)CH₂-C4-Cl-C₆H₄) | O |
| Ia1.2395 | NO₂ | (CH₂)₃CH₃ | CH(CH₃)CH₂-(4-Cl-C₆H₄) | O |
| Ia1.2396 | NO₂ | H | CH(CH₃)CH(CH₃)-(4-Cl-C₆H₄) | O |
| Ia1.2397 | NO₂ | CH₃ | CH(CH₃)CH(CH₃)-(4-Cl-C₆H₄) | O |
| Ia1.2398 | NO₂ | CH₂CH₃ | CH(CH₃)CH(CH₃)-(4-Cl-C₆H₄) | O |
| Ia1.2399 | NO₂ | (CH₂)₂CH₃ | CH(CH₃)CH(CH₃)-(4-Cl-C₆H₄) | O |
| Ia1.2400 | NO₂ | (CH₂)₃CH₃ | CH(CH₃)CH(CH₃)-(4-Cl-C₆H₄) | O |
| Ia1.2401 | NO₂ | H | (CH₂)₂-(2,4-Cl₂-C₆H₃) | O |
| Ia1.2402 | NO₂ | CH₃ | (CH₂)₂-(2,4-Cl₂-C₆H₃) | O |
| Ia1.2403 | NO₂ | CH₂CH₃ | (CH₂)₂-(2,4-Cl₂-C₆H₃) | O |
| Ia1.2404 | NO₂ | (CH₂)₂CH₃ | (CH₂)₂-(2,4-Cl₂-C₆H₃) | O |
| Ia1.2405 | NO₂ | (CH₂)₃CH₃ | (CH₂)₂-(2,4-Cl₂-C₆H₃) | O |
| Ia1.2406 | NO₂ | H | CH₂CH(CH₃)-(2,4-Cl₂-C₆H₃) | O |
| Ia1.2407 | NO₂ | CH₃ | CH₂CH(CH₃)-(2,4-Cl₂-C₆H₃) | O |
| Ia1.2408 | NO₂ | CH₂CH₃ | CH₂CH(CH₃)-(2,4-Cl₂-C₆H₃) | O |
| Ia1.2409 | NO₂ | (CH₂)₂CH₃ | CH₂CH(CH₃)-(2,4-Cl₂-C₆H₃) | O |
| Ia1.2410 | NO₂ | (CH₂)₃CH₃ | CH₂CH(CH₃)-(2,4-Cl₂-C₆H₃) | O |
| Ia1.2411 | NO₂ | H | CH(CH₃)CH₂-(2,4-Cl₂-C₆H₃) | O |
| Ia1.2412 | NO₂ | CH₃ | CH(CH₃)CH₂-(2,4-Cl₂-C₆H₃) | O |
| Ia1.2413 | NO₂ | CH₂CH₃ | CH(CH₃)CH₂-(2,4-Cl₂-C₆H₃) | O |
| Ia1.2414 | NO₂ | (CH₂)₂CH₃ | CH(CH₃)CH₂-(2,4-Cl₂-C₆H₃) | O |
| Ia1.2415 | NO₂ | (CH₂)₃CH₃ | CH(CH₃)CH₂-(2,4-Cl₂-C₆H₃) | O |
| Ia1.2416 | NO₂ | H | CH(CH₃)CH(CH₃)-(2,4-Cl₂-C₆H₃) | O |
| Ia1.2417 | NO₂ | CH₃ | CH(CH₃)CH(CH₃)-(2,4-Cl₂-C₆H₃) | O |
| Ia1.2418 | N_{O}2 | CH₂CH₃ | CH(CH₃)CH(CH₃)-(2,4-Cl₂-C₆H₃) | O |
| Ia1.2419 | NO₂ | (CH₂)₂CH₃ | CH(CH₃)CH(CH₃)-(2,4-Cl₂-C₆H₃) | O |
| Ia1.2420 | NO₂ | (CH₂)₃CH₃ | CH(CH₃)CH(CH₃)-(2,4-Cl₂-C₆H₃) | O |
| Ia1.2421 | NO₂ | H | (CH₂)₂-O-C₆H₅ | O |
| Ia1.2422 | NO₂ | CH₃ | (CH₂)₂-O-C₆H₅ | O |
| Ia1.2423 | NO₂ | CH₂CH₃ | (CH₂)₂-O-C₆H₅ | O |
| Ia1.2424 | NO₂ | (CH₂)₂CH₃ | (CH₂)₂-O-C₆H₅ | O |
| Ia1.2425 | NO₂ | (CH₂)₃CH₃ | (CH₂)₂-O-C₆H₅ | O |
| Ia1.2426 | NO₂ | H | CH₂CH(CH₃)-O-C₆H₅ | O |
| Ia1.2427 | NO₂ | CH₃ | CH₂CH(CH₃)-O-C₆H₅ | O |
| Ia1.2428 | NO₂ | CH₂CH₃ | CH₂CH(CH₃)-O-C₆H₅ | O |
| Ia1.2429 | NO₂ | (CH₂)₂CH₃ | CH₂CH(CH₃)-O-C₆H₅ | O |
| Ia1.2430 | NO₂ | (CH₂)₃CH₃ | CH₂CH(CH₃)-O-C₆H₅ | O |
| Ia1.2431 | NO₂ | H | CH(CH₃)CH₂-O-C₆H₅ | O |
| Ia1.2432 | NO₂ | CH₃ | CH(CH₃)CH₂-O-C₆H₅ | O |
| Ia1.2433 | NO₂ | CH₂CH₃ | CH(CH₃)CH₂-O-C₆H₅ | O |
| Ia1.2434 | NO₂ | (CH₂)₂CH₃ | CH(CH₃)CH₂-O-C₆H₅ | O |
| Ia1.2435 | NO₂ | (CH₂)₃CH₃ | CH(CH₃)CH₂-O-C₆H₅ | O |
| Ia1.2436 | NO₂ | H | CH(CH₃)CH(CH₃)-O-C₆H₅ | O |
| Ia1.2437 | NO₂ | CH₃ | CH(CH₃)CH(CH₃)-O-C₆H₅ | O |
| Ia1.2438 | NO₂ | CH₂CH₃ | CH(CH₃)CH(CH₃)-O-C₆H₅ | O |
| Ia1.2439 | NO₂ | (CH₂)₂CH₃ | CH(CH₃)CH(CH₃)-O-C₆H₅ | O |
| Ia1.2440 | NO₂ | (CH₂)₃CH₃ | CH(CH₃)CH(CH₃)-O-C₆H₅ | O |
| Ia1.2441 | NO₂ | H | (CH₂)₂-O-(4-Cl-C₆H₄) | O |
| Ia1.2442 | NO₂ | CH₃ | (CH₂)₂-O-(4-Cl-C₆H₄) | O |
| Ia1.2443 | NO₂ | CH₂CH₃ | (CH₂)₂-O-(4-Cl-C₆H₄) | O |
| Ia1.2444 | NO₂ | (CH₂)₂CH₃ | (CH₂)₂-O-(4-Cl-C₆H₄) | O |
| Ia1.2445 | NO₂ | (CH₂)₃CH₃ | (CH₂)₂-O-(4-Cl-C₆H₄) | O |
| Ia1.2446 | NO₂ | H | CH₂CH(CH₃)-O-(4-Cl-C₆H₄) | O |
| Ia1.2447 | NO₂ | CH₃ | CH₂CH(CH₃)-O-(4-Cl-C₆H₄) | O |
| Ia1.2448 | NO₂ | CH₂CH₃ | CH₂CH(CH₃)-O-(4-Cl-C₆H₄) | O |
| Ia1.2449 | NO₂ | (CH₂)₂CH₃ | CH₂CH(CH₃)-O-(4 Cl-C₆H₄) | O |
| Ia1.2450 | NO₂ | (CH₂)₃CH₃ | CH₂CH(CH₃)-O-(4-Cl-C₆H₄) | O |
| Ia1.2451 | NO₂ | H | CH(CH₃)CH₂-O-(4-Cl-C₆H₄) | O |
| Ia1.2452 | NO₂ | CH₃ | CH(CH₃)CH₂-O-(4-Cl-C₆H₄) | O |
| Ia1.2453 | NO₂ | CH₂CH₃ | CH(CH₃)CH₂-O-(4-Cl-C₆H₄) | O |
| Ia1.2454 | NO₂ | (CH₂)₂CH₃ | CH(CH₃)CH₂-O-(4-Cl-C₆H₄) | O |
| Ia1.2455 | NO₂ | (CH₂)₃CH₃ | CH(CH₃)CH₂-O-(4-Cl-C₆H₄) | O |
| Ia1.2456 | NO₂ | H | CH(CH₃)CH(CH₃)-O-(4-Cl-C₆H₄) | O |
| Ia1.2457 | NO₂ | CH₃ | CH(CH₃)CH(CH₃)-O-(4-Cl-C₆H₄) | O |
| Ia1.2458 | NO₂ | CH₂CH₃ | CH(CH₃)CH(CH₃)-O-(4-Cl-C₆H₄) | O |
| Ia1.2459 | NO₂ | (CH₂)₂CH₃ | CH(CH₃)CH(CH₃)-O-(4-Cl-C₆H₄ ) | O |
| Ia1.2460 | NO₂ | (CH₂)₃CH₃ | CH(CH₃)CH(CH₃)-O-(4-Cl-C₆H₄) | O |
| Ia1.2461 | NO₂ | H | (CH₂)₂-O-(2,4-Cl₂-C₆H₃) | O |
| Ia1.2462 | NO₂ | CH₃ | (CH₂)₂-O-(2,4-Cl₂-C₆H₃) | O |
| Ia1.2463 | NO₂ | CH₂CH₃ | (CH₂)₂-O-(2,4-Cl₂-C₆H₃) | O |
| Ia1.2464 | NO₂ | (CH₂)₂CH₃ | (CH₂)₂-O-(2,4-Cl₂-C₆H₃) | O |
| Ia1.2465 | NO₂ | (CH₂)₃CH₃ | (CH₂)₂-O-(2,4-Cl₂-C₆H₃) | O |
| Ia1.2466 | NO₂ | H | CH₂CH(CH₃)-O-(2,4-Cl₂-C₆H₃) | O |
| Ia1.2467 | NO₂ | CH₃ | CH₂CH(CH₃)-O-(2,4-Cl₂-C₆H₃) | O |
| Ia1.2468 | NO₂ | CH₂CH₃ | CH₂CH(CH₃)-O-(2,4-Cl₂-C₆H₃) | O |
| Ia1.2469 | NO₂ | (CH₂)₂CH₃ | CH₂CH(CH₃)-O-(2,4-Cl₂-C₆H₃) | O |
| Ia1.2470 | NO₂ | (CH₂)₃CH₃ | CH₂CH(CH₃)-O-(2,4-Cl₂-C₆H₃) | O |
| Ia1.2471 | NO₂ | H | CH(CH₃)CH₂-O-(2,4-Cl₂-C₆H₃) | O |
| Ia1.2472 | NO₂ | CH₃ | CH(CH₃)CH₂-O-(2,4-Cl₂-C₆H₃) | O |
| Ia1.2473 | NO₂ | CH₂CH₃ | CH(CH₃)CH₂-O-(2,4-Cl₂-C₆H₃) | O |
| Ia1.2474 | NO₂ | (CH₂)₂CH₃ | CH(CH₃)CH₂-O-(2,4-Cl₂-C₆H₃) | O |
| Ia1.2475 | NO₂ | (CH₂)₃CH₃ | CH(CH₃)CH₂-O-(2,4-Cl₂-C₆H₃) | O |
| Ia1.2476 | NO₂ | H | CH(CH₃)CH(CH₃)-O-(2,4-Cl₂-C₆H₃) | O |
| Ia1.2477 | NO₂ | CH₃ | CH(CH₃)CH(CH₃)-O-(2,4-Cl₂-C₆H₃) | O |
| Ia1.2478 | NO₂ | CH₂CH₃ | CH(CH₃)CH(CH₃)-O-(2,4-Cl₂-C₆H₃) | O |
| Ia1.2479 | NO₂ | (CH₂)₂CH₃ | CH(CH₃)CH(CH₃)-O-(2,4-Cl₂-C₆H₃) | O |
| Ia1.2480 | NO₂ | (CH₂) ₃CH₃ | CH(CH₃)CH(CH₃)-O-(2,4-Cl₂-C₆H₃) | O |
| Ia1.2481 | NO₂ | H | CH₃ | NH |
| Ia1.2482 | NO₂ | CH₃ | CH₃ | NH |
| Ia1.2483 | NO₂ | CH₂CH₃ | CH₃ | NH |
| Ia1.2484 | NO_{z} | (CH₂)₂CH₃ | CH₃ | NH |
| Ia1.2485 | NO₂ | (CH₂)₃CH₃ | CH₃ | NH |
| Ia1.2486 | NO₂ | H | C₂H₅ | NH |
| Ia1.2487 | NO₂ | CH₃ | C₂H₅ | NH |
| Ia1.2488 | NO₂ | CH₂CH₃ | C₂H₅ | NH |
| Ia1.2489 | NO₂ | (CH₂)₂CH₃ | C₂H₅ | NH |
| Ia1.2490 | NO₂ | (CH₂)₃CH₃ | C₂H₅ | NH |
| Ia1.2491 | NO₂ | H | (CH₂)₂CH₃ | NH |
| Ia1.2492 | NO₂ | CH₃ | (CH₂)₂CH₃ | NH |
| Ia1.2493 | NO₂ | CH₂CH₃ | (CH₂)₂CH₃ | NH |
| Ia1.2494 | NO₂ | (CH₂)₂CH₃ | (CH₂)₂CH₃ | NH |
| Ia1.2495 | NO₂ | (CH₂)₃CH₃ | (CH₂)₂CH₃ | NH |
| Ia1.2496 | NO₂ | H | CH(CH₃)₂ | NH |
| Ia1.2497 | NO₂ | CH₃ | CH(CH₃)₂ | NH |
| Ia1.2498 | NO₂ | CH₂CH₃ | CH(CH₃)₂ | NH |
| Ia1.2499 | NO₂ | (CH₂)₂CH₃ | CH(CH₃)₂ | NH |
| Ia1.2500 | NO₂ | (CH₂)₃CH₃ | CH(CH₃)₂ | NH |
| Ia1.2501 | NO₂ | H | (CH₂)₃CH₃ | NH |
| Ia1.2502 | NO₂ | CH₃ | (CH₂)₃CH₃ | NH |
| Ia1.2503 | NO₂ | CH₂CH₃ | (CH₂)₃CH₃ | NH |
| Ia1.2504 | NO₂ | (CH₂)₂CH₃ | (CH₂)₃CH₃ | NH |
| Ia1.2505 | NO₂ | (CH₂)₃CH₃ | (CH₂)₃CH₃ | NH |
| Ia1.2506 | NO₂ | H | CH₂CH(CH₃)₂ | NH |
| Ia1.2507 | NO₂ | CH₃ | CH₂CH(CH₃)₂ | NH |
| Ia1.2508 | NO₂ | CH₂CH₃ | CH₂CH(CH₃)₂ | NH |
| Ia1.2509 | NO₂ | (CH₂)₂CH₃ | CH₂CH(CH₃)₂ | NH |
| Ia1.2510 | NO₂ | (CH₂)₃CH₃ | CH₂CH(CH₃)₂ | NH |
| Ia1.2511 | NO₂ | H | CH(CH₃)CH₂CH₃ | NH |
| Ia1.2512 | NO₂ | CH₃ | CH(CH₃)CH₂CH₃ | NH |
| Ia1.2513 | NO₂ | CH₂CH₃ | CH(CH₃)CH₂CH₃ | NH |
| Ia1.2514 | NO₂ | (CH₂)₂CH₃ | CH(CH₃)CH₂CH₃ | NH |
| Ia1.2515 | NO₂ | (CH₂)₃CH₃ | CH(CH₃)CH₂CH₃ | NH |
| Ia1.2516 | NO₂ | H | (CH₂)₂-C₆H₅ | NH |
| Ia1.2517 | NO₂ | CH₃ | (CH₂)₂-C₆H₅ | NH |
| Ia1.2518 | NO₂ | CH₂CH₃ | (CH₂)₂-C₆H₅ | NH |
| Ia1.2519 | NO₂ | (CH₂)₂CH₃ | (CH₂)₂-C₆H₅ | NH |
| Ia1.2520 | NO₂ | (CH₂)₃CH₃ | (CH₂)₂-C₆H₅ | NH |
| Ia1.2521 | NO₂ | H | CH₂CH(CH₃)-C₆H₅ | NH |
| Ia1.2522 | NO₂ | CH₃ | CH₂CH(CH₃)-C₆R₅ | NH |
| Ia1.2523 | NO₂ | CH₂CH₃ | CH₂CH(CH₃)-C₆H₅ | NH |
| Ia1.2524 | NO₂ | (CH₂)₂CH₃ | CH₂CH(CH₃)-C₆H₅ | NH |
| Ia1.2525 | NO₂ | (CH₂)₃CH₃ | CH₂CH(CH₃)-C₆H₅ | NH |
| Ia1.2526 | NO₂ | H | CH(CH₃)CH₂-C₆H₅ | NH |
| Ia1.2527 | NO₂ | CH₃ | CH(CH₃)CH₂-C₆H₅ | NH |
| Ia1.2528 | NO₂ | CH₂CH₃ | CH(CH₃)CH₂-C₆H₅ | NH |
| Ia1.2529 | NO₂ | (CH₂)₂CH₃ | CH(CH₃)CH₂-C₆H₅ | NH |
| Ia1.2530 | NO₂ | (CH₂)₃CH₃ | CH(CH₃)CH₂-C₆H₅ | NH |
| Ia1.2531 | NO₂ | H | CH(CH₃)CH(CH₃)-C₆H₅ | NH |
| Ia1.2532 | NO₂ | CH₃ | CH(CH₃)CH(CH₃)-C₆H₅ | NH |
| Ia1.2533 | NO₂ | CH₂CH₃ | CH(CH₃)CH(CH₃)-C₆H₅ | NH |
| Ia1.2534 | NO₂ | (CH₂)₂CH₃ | CH(CH₃)CH(CH₃)-C₆H₅ | NH |
| Ia1.2535 | NO₂ | (CH₂)₃CH₃ | CH(CH₃)CH(CH₃)-C₆H₅ | NH |
| Ia1.2536 | NO₂ | H | (CH₂)₂-(4-Cl-C₆H₄) | NH |
| Ia1.2537 | NO₂ | CH₃ | (CH₂)₂-(4-Cl-C₆H₄) | NH |
| Ia1.2538 | NO₂ | CH₂CH₃ | (CH₂)₂-(4-Cl-C₆H₄ ) | NH |
| Ia1.2539 | NO₂ | (CH₂)₂CH₃ | (CH₂)₂-(4-Cl-C₆H₄) | NH |
| Ia1.2540 | NO₂ | (CH₂)₃CH₃ | (CH₂)₂-(4-Cl-C₆H₄) | NH |
| Ia1.2541 | NO₂ | H | CH₂CH(CH₃)-(4-Cl-C₆H₄) | NH |
| Ia1.2542 | NO₂ | CH₃ | CH₂CH(CH₃)-(4-Cl-C₆H₄) | NH |
| Ia1.2543 | NO₂ | CH₂CH₃ | CH₂CH(CH₃)-(4-Cl-C₆H₄) | NH |
| Ia1.2544 | NO₂ | (CH₂)₂CH₃ | CH₂CH(CH₃)-(4-Cl-C₆H₄) | NH |
| Ia1.2545 | NO₂ | (CH₂)₃CH₃ | CH₂CH(CH₃)-(4-Cl-C₆H₄) | NK |
| Ia1.2546 | NO₂ | H | CH(CH₃)CH₂-(4-Cl-C₆H₄) | NH |
| Ia1.2547 | NO₂ | CH₃ | CH(CH₃)CH₂-(4-Cl-C₆H₄) | NH |
| Ia1.2548 | NO₂ | CH₂CH₃ | CH(CH₃)CH₂-(4-Cl-C₆H₄) | NH |
| Ia1.2549 | NO₂ | (CH₂)₂CH₃ | CH(CH₃)CH₂-(4-Cl-C₆H₄) | NH |
| Ia1.2550 | NO₂ | (CH₂)₃CH₃ | CH(CH₃)CH₂-(4-Cl-C₆H₄) | NH |
| Ia1.2551 | NO₂ | H | CH(CH₃)CH(CH₃)-(4-Cl-C₆H₄) | NH |
| Ia1.2552 | NO₂ | CH₃ | CH(CH₃)CH(CH₃)-(4-Cl-C₆H₄) | NH |
| Ia1.2553 | NO₂ | CH₂CH₃ | CH(CH₃)CH(CH₃)-(4-Cl-C₆H₄) | NH |
| Ia1.2554 | NO₂ | (CH₂)₂CH₃ | CH(CH₃)CH(CH₃)-(4-Cl-C₆N₄) | NH |
| Ia1.2555 | NO₂ | (CH₂)₃CH₃ | CH(CH₃)CH(CH₃)-(4-Cl-C₆H₄) | NH |
| Ia1.2556 | NO₂ | H | (CH₂)₂-(2,4-Cl₂-C₆H₃) | NH |
| Ia1.2557 | NO₂ | CH₃ | (CH₂)₂-(2,4-Cl₂-C₆H₃) | NH |
| Ia1.2558 | NO₂ | CH₂CH₃ | (CH₂)₂-(2,4-Cl₂-C₆H₃) | NH |
| Ia1.2559 | NO₂ | (CH₂)₂CH₃ | (CH₂)₂-(2,4-Cl₂-C₆H₃) | NH |
| Ia1.2560 | NO₂ | (CH₂)₃CH₃ | (CH₂)₂-(2,4 -Cl₂-C₆H₃) | NH |
| Ia1.2561 | NO₂ | H | CH₂CH(CH₃)-(2,4-Cl₂-C₆H₃) | NH |
| Ia1.2562 | NO₂ | CH₃ | CH₂CH(CH₃)-(2,4-Cl₂-C₆H₃) | NH |
| Ia1.2563 | NO₂ | CH₂CH₃ | CH₂CH(CH₃)-(2,4-Cl₂-C₆H₃) | NH |
| Ia1.2564 | NO₂ | (CH₂)₂CH₃ | CH₂CH(CH₃)-(2,4-Cl₂-C₆H₃) | NH |
| Ia1.2565 | NO₂ | (CH₂)₃CH₃ | CH₂CH(CH₃)-(2,4-Cl₂-C₆H₃) | NH |
| Ia1.2566 | NO₂ | H | CH(CH₃)CH₂-(2,4-Cl₂-C₆H₃) | NH |
| Ia1.2567 | NO₂ | CH₃ | CH(CH₃)CH₂-(2,4-Cl₂-C₆H₃) | NH |
| Ia1.2568 | NO₂ | CH₂CH₃ | CH(CH₃)CH₂-(2,4-Cl₂-C₆H₃) | NH |
| Ia1.2569 | NO₂ | (CH₂)₂CH₃ | CH(CH₃)CH₂-(2,4-Cl₂-C₆H₃) | NH |
| Ia1.2570 | NO₂ | (CH₂)₃CH₃ | CH(CH₃)CH₂-(2,4-Cl₂-C₆H₃) | NH |
| Ia1.2571 | NO₂ | H | CH(CH₃)CH(CH₃)-(2,4-Cl₂-C₆H₃) | NH |
| Ia1.2572 | NO₂ | CH₃ | CH(CH₃)CH(CH₃)-(2,4-Cl₂-C₆H₃) | NH |
| Ia1.2573 | NO₂ | CH₂CH₃ | CH(CH₃)CH(CH₃)-(2,4-Cl₂-C₆H₃) | NH |
| Ia1.2574 | NO₂ | (CH₂)₂CH₃ | CH(CH₃)CH(CH₃)-(2,4-Cl₂-C₆H₃) | NH |
| Ia1.2575 | NO₂ | (CH₂)₃CH₃ | CH(CH₃)CH(CH₃)-(2,4-Cl₂-C₆H₃) | NH |
| Ia1.2576 | NO₂ | H | (CH₂)₂-O-C₆H₅ | NH |
| Ia1.2577 | NO₂ | CH₃ | (CH₂)₂-O-C₆H₅ | NH |
| Ia1.2578 | NO₂ | CH₂CH₃ | (CH₂)₂-O-C₆H₅ | NH |
| Ia1.2579 | NO₂ | (CH₂)₂CH₃ | (CH₂)₂-O-C₆H₅ | NH |
| Ia1.2580 | NO₂ | (CH₂)₃CH₃ | (CH₂)₂-O-C₆H₅ | NH |
| Ia1.2581 | NO₂ | H | CH₂CH(CH₃)-O-C₆H₅ | NH |
| Ia1.2582 | NO₂ | CH₃ | CH₂CH(CH₃)-O-C₆H₅ | NH |
| Ia1.2583 | NO₂ | CH₂CH₃ | CH₂CH(CH₃)-O-C₆H₅ | NH |
| Ia1.2584 | NO₂ | (CH₂)₂CH₃ | CH₂CH(CH₃)-O-C₆H₅ | NH |
| Ia1.2585 | NO₂ | (CH₂)₃CH₃ | CH₂CH(CH₃)-O-C₆H₅ | NH |
| Ia1.2586 | NO₂ | H | CH(CH₃)CH₂-O-C₆H₅ | NH |
| Ia1.2587 | NO₂ | CH₃ | CH(CH₃)CH₂-O-C₆H₅ | NH |
| Ia1.2588 | NO₂ | CH₂CH₃ | CH(CH₃)CH₂-O-C₆H₅ | NH |
| Ia1.2589 | NO₂ | (CH₂)₂CH₃ | CH(CH₃)CH₂-O-C₆H₅ | NH |
| Ia1.2590 | NO₂ | (CH₂)₃CH₃ | CH(CH₃)CH₂-O-C₆H₅ | NH |
| Ia1.2591 | NO₂ | H | CH(CH₃)CH(CH₃)-O-C₆H₅ | NH |
| Ia1.2592 | NO₂ | CH₃ | CH(CH₃)CH(CH₃)-O-C₆H₅ | NH |
| Ia1.2593 | NO₂ | CH₂CH₃ | CH(CH₃)CH(CH₃)-O-C₆H₅ | NH |
| Ia1.2594 | NO₂ | (CH₂)₂CH₃ | CH(CH₃)CH(CH₃)-O-C₆H₅ | NH |
| Ia1.2595 | NO₂ | (CH₂)₃CH₃ | CH(CH₃)CH(CH₃)-O-C₆H₅ | NH |
| Ia1.2596 | NO₂ | H | (CH₂)₂-O-(4-Cl-C₆H₄) | NH |
| Ia1.2597 | NO₂ | CH₃ | (CH₂)₂-O-(4-Cl-C₆H₄) | NH |
| Ia1.2598 | NO₂ | CH₂CH₃ | (CH₂)₂-O-(4-Cl-C₆H₄) | NH |
| Ia1.2599 | NO₂ | (CH₂)₂CH₃ | (CH₂)₂-O-(4-Cl-C₆H₄) | NH |
| Ia1.2600 | NO₂ | (CH₂)₃CH₃ | (CH₂)₂-O-(4-Cl-C₆H₄) | NH |
| Ia1.2601 | NO₂ | H | CH₂CH(CH₃)-O-(4-Cl-C₆H₄) | NH |
| Ia1.2602 | NO₂ | CH₃ | CH₂CH(CH₃)-O-(4-Cl-C₆H₄) | NH |
| Ia1.2603 | NO₂ | CH₂CH₃ | CH₂CH(CH₃)-O-(4-Cl-C₆H₄) | NH |
| Ia1.2604 | NO₂ | (CH₂)₂CH₃ | CH₂CH(CH₃)-O-(4-Cl-C₆H₄) | NH |
| Ia1.2605 | NO₂ | (CH₂)₃CH₃ | CH₂CH(CH₃)-O-(4-Cl-C₆H₄) | NH |
| Ia1.2606 | NO₂ | H | CH(CH₃)CH₂-O-(4-Cl-C₆H₄) | NH |
| Ia1.2607 | NO₂ | CH₃ | CH(CH₃)CH₂-O-(4-Cl-C₆H₄) | NH |
| Ia1.2608 | NO₂ | CH₂CH₃ | CH(CH₃)CH₂-O-(4-Cl-C₆H₄) | NH |
| Ia1.2609 | NO₂ | (CH₂)₂CH₃ | CH(CH₃)CH₂-O-(4-Cl-C₆H₄) | NH |
| Ia1.2610 | NO₂ | (CH₂)₃CH₃ | CH(CH₃)CH₂-O-(4-Cl-C₆H₄) | NH |
| Ia1.2611 | NO₂ | H | CH(CH₃)CH(CH₃)-O-(4-Cl-C₆H₄) | NH |
| Ia1.2612 | NO₂ | CH₃ | CH(CH₃)CH(CH₃)-O-(4-Cl-C₆H₄) | NH |
| Ia1.2613 | NO₂ | CH₂CH₃ | CH(CH₃)CH(CH₃)-O-(4-Cl-C₆H₄) | NH |
| Ia1.2614 | NO₂ | (CH₂)₂CH₃ | CH(CH₃)CH(CH₃)-O-(4-Cl-C₆H₄) | NH |
| Ia1.2615 | NO₂ | (CH₂)₃CH₃ | CH(CH₃)CH(CH₃)-O-(4-Cl-C₆H₄) | NH |
| Ia1.2616 | NO₂ | H | (CH₂)₂-O-(2,4-Cl₂-C₆H₃) | NH |
| Ia1.2617 | NO₂ | CH₃ | (CH₂)₂-O-(2,4-Cl₂-C₆H₃) | NH |
| Ia1.2618 | NO₂ | CH₂CH₃ | (CH₂)₂-O-(2,4-Cl₂-C₆H₃) | NH |
| Ia1.2619 | NO₂ | (CH₂)₂CH₃ | (CH₂)₂-O-(2,4-Cl₂-C₆H₃) | NH |
| Ia1.2620 | NO₂ | (CH₂)₃CH₃ | (CH₂)₂-O-(2,4-Cl₂-C₆H₃) | NH |
| Ia1.2621 | NO₂ | H | CH₂CH(CH₃)-O-(2,4-Cl₂-C₆H₃) | NH |
| Ia1.2622 | NO₂ | CH₃ | CH₂CH(CH₃)-O-(2,4-Cl₂-C₆H₃) | NH |
| Ia1.2623 | NO₂ | CH₂CH₃ | CH₂CH(CH₃)-O-(2,4-Cl₂-C₆H₃) | NH |
| Ia1.2624 | NO₂ | (CH₂)₂CH₃ | CH₂CH(CH₃)-O-(2,4-Cl₂-C₆H₃) | NH |
| Ia1.2625 | NO₂ | (CH₂)₃CH₃ | CH₂CH(CH₃)-O-(2,4-Cl₂-C₆H₃) | NH |
| Ia1.2626 | NO₂ | H | CH(CH₃)CH₂-O-(2,4-Cl₂-C₆H₃) | NH |
| Ia1.2627 | NO₂ | CH₃ | CH(CH₃)CH₂-O-(2,4-Cl₂-C₆H₃) | NH |
| Ia1.2628 | NO₂ | CH₂CH₃ | CH(CH₃)CH₂-O-(2,4-Cl₂-C₆H₃) | NH |
| Ia1.2629 | NO₂ | (CH₂)₂CH₃ | CH(CH₃)CH₂-O-(2,4-Cl₂-C₆H₃) | NH |
| Ia1.2630 | NO₂ | (CH₂)₃CH₃ | CH(CH₃)CH₂-O-(2,4-Cl₂-C₆H₃) | NH |
| Ia1.2631 | NO₂ | H | CH(CH₃)CH(CH₃)-O-(2,4-Cl₂-C₆H₃) | NH |
| Ia1.2632 | NO₂ | CH₃ | CH(CH₃)CH(CH₃)-O-(2,4-Cl₂-C₆H₃) | NH |
| Ia1.2633 | NO₂ | CH₂CH₃ | CH(CH₃)CH(CH₃)-O-(2,4-Cl₂-C₆H₃) | NH |
| Ia1.2634 | NO₂ | (CH₂)₂CH₃ | CH(CH₃)CH(CH₃)-O-(2,4-Cl₂-C₆H₃) | NH |
| Ia1.2635 | NO₂ | (CH₂)₃CH₃ | CH(CH₃)CH(CH₃)-O-(2,4-Cl₂-C₆H₃) | NH |
| Ia1.2636 | NO₂ | H | CH₃ | NCH₃ |
| Ia1.2637 | NO₂ | CH₃ | CH₃ | NCH₃ |
| Ia1.2638 | NO₂ | CH₂CH₃ | CH₃ | NCH₃ |
| Ia1.2639 | NO₂ | (CH₂)₂CH₃ | CH₃ | NCH₃ |
| Ia1.2640 | NO₂ | (CH₂)₃CH₃ | CH₃ | NCH₃ |
| Ia1.2641 | NO₂ | H | C₂H₅ | NCH₃ |
| Ia1.2642 | NO₂ | CH₃ | C₂H₅ | NCH₃ |
| Ia1.2643 | NO₂ | CH₂CH₃ | C₂H₅ | NCH₃ |
| Ia1.2644 | NO₂ | (CH₂)₂CH₃ | C₂H₅ | NCH₃ |
| Ia1.2645 | NO₂ | (CH₂)₃CH₃ | C₂H₅ | NCH₃ |
| Ia1.2646 | NO₂ | H | (CH₂)₂CH₃ | NCH₃ |
| Ia1.2647 | NO₂ | CH₃ | (CH₂)₂CH₃ | NCH₃ |
| Ia1.2648 | NO₂ | CH₂CH₃ | (CH₂)₂CH₃ | NCH₃ |
| Ia1.2649 | NO₂ | (CH₂)₂CH₃ | (CH₂)₂CH₃ | NCH₃ |
| Ia1.2650 | NO₂ | (CH₂)₃CH₃ | (CH₂)₂CH₃ | NCH₃ |
| Ia1.2651 | NO₂ | H | CH(CH₃)₂ | NCH₃ |
| Ia1.2652 | NO₂ | CH₃ | CH(CH₃)₂ | NCH₃ |
| Ia1.2653 | NO₂ | CH₂CH₃ | CH(CH₃)₂ | NCH₃ |
| Ia1.2654 | NO₂ | (CH₂)₂CH₃ | CH(CH₃)₂ | NCH₃ |
| Ia1.2655 | NO₂ | (CH₂)₃CH₃ | CH(CH₃)₂ | NCH₃ |
| Ia1.2656 | NO₂ | H | (CH₂)₃CH₃ | NCH₃ |
| Ia1.2657 | NO₂ | CH₃ | (CH₂)₃CH₃ | NCH₃ |
| Ia1.2658 | NO₂ | CH₂CH₃ | (CH₂)₃CH₃ | NCH₃ |
| Ia1.2659 | NO₂ | (CH₂)₂CH₃ | (CH₂)₃CH₃ | NCH₃ |
| Ia1.2660 | NO₂ | (CH₂)₃CH₃ | (CH₂)₃CH₃ | NCH₃ |
| Ia1.2661 | NO₂ | H | CH₂CH(CH₃)₂ | NCH₃ |
| Ia1.2662 | NO₂ | CH₃ | CH₂CH(CH₃)₂ | NCH₃ |
| Ia1.2663 | NO₂ | CH₂CH₃ | CH₂CH(CH₃)₂ | NCH₃ |
| Ia1.2664 | NO₂ | (CH₂)₂CH₃ | CH₂CH(CH₃)₂ | NCH₃ |
| Ia1.2665 | NO₂ | (CH₂)₃CH₃ | CH₂CH(CH₃)₂ | NCH₃ |
| Ia1.2666 | NO₂ | H | CH(CH₃)CH₂CH₃ | NCH₃ |
| Ia1.2667 | NO₂ | CH₃ | CH(CH₃)CH₂CH₃ | NCH₃ |
| Ia1.2668 | NO₂ | CH₂CH₃ | CH(CH₃)CH₂CH₃ | NCH₃ |
| Ia1.2669 | NO₂ | (CH₂)₂CH₃ | CH(CH₃)CH₂CH₃ | NCH₃ |
| Ia1.2670 | NO₂ | (CH₂)₃CH₃ | CH(CH₃)CH₂CH₃ | NCH₃ |
| Ia1.2671 | NO₂ | H | (CH₂)₂-C₆H₅ | NCH₃ |
| Ia1.2672 | NO₂ | CH₃ | (CH₂)₂-C₆H₅ | NCH₃ |
| Ia1.2673 | NO₂ | CH₂CH₃ | (CH₂)₂-C₆H₅ | NCH₃ |
| Ia1.2674 | NO₂ | (CH₂)₂CH₃ | (CH₂)₂-C₆H₅ | NCH₃ |
| Ia1.2675 | NO₂ | (CH₂)₃CH₃ | (CH₂)₂-C₆H₅ | NCH₃ |
| Ia1.2676 | NO₂ | H | CH₂CH(CH₃)-C₆H₅ | NCH₃ |
| Ia1.2677 | NO₂ | CH₃ | CH₂CH(CH₃)-C₆H₅ | NCH₃ |
| Ia1.2678 | NO₂ | CH₂CH₃ | CH₂CH(CH₃)-C₆H₅ | NCH₃ |
| Ia1.2679 | NO₂ | (CH₂)₂CH₃ | CH₂CH(CH₃)-C₆H₅ | NCH₃ |
| Ia1.2680 | NO₂ | (CH₂)₃CH₃ | CH₂CH(CH₃)-C₆H₅ | NCH₃ |
| Ia1.2681 | NO₂ | H | CH(CH₃)CH₂-C₆H₅ | NCH₃ |
| Ia1.2682 | NO₂ | CH₃ | CH(CH₃)CH₂-C₆H₅ | NCH₃ |
| Ia1.2683 | NO₂ | CH₂CH₃ | CH(CH₃)CH₂-C₆H₅ | NCH₃ |
| Ia1.2684 | NO₂ | (CH₂)₂CH₃ | CH(CH₃)CH₂-C₆H₅ | NCH₃ |
| Ia1.2685 | NO₂ | (CH₂)₃CH₃ | CH(CH₃)CH₂-C₆H₅ | NCH₃ |
| Ia1.2686 | NO₂ | H | CH(CH₃)CH(CH₃)-C₆H₅ | NCH₃ |
| la1.2687 | NO₂ | CH₃ | CH(CH₃)CH(CH₃)-C₆H₅ | NCH₃ |
| Ia1.2688 | NO₂ | CH₂CH₃ | CH(CH₃)CH(CH₃)-C₆H₅ | NCH₃ |
| Ia1.2689 | NO₂ | (CH₂)₂CH₃ | CH(CH₃)CH(CH₃)-C₆H₅ | NCH₃ |
| Ia1.2690 | NO₂ | (CH₂)₃CH₃ | CH(CH₃)CH(CH₃)-C₆H₅ | NCH₃ |
| Ia1.2691 | NO₂ | H | (CH₂)₂-(4-Cl-C₆H₄) | NCH₃ |
| Ia1.2692 | NO₂ | CH₃ | (CH₂)₂-(4-Cl-C₆H₄) | NCH₃ |
| Ia1.2693 | NO₂ | CH₂CH₃ | (CH₂)₂-(4-Cl-CₑH₄) | NCH₃ |
| Ia1.2694 | NO₂ | (CH₂)₂CH₃ | (CH₂)₂-(4-Cl-C₆H₄) | NCH₃ |
| Ia1.2695 | NO₂ | (CH₂)₃CH₃ | (CH₂)₂-(4-Cl-C₆H₄) | NCH₃ |
| Ia1.2696 | NO₂ | H | CH₂CH(CH₃)-(4-Cl-C₆H₄) | NCH₃ |
| Ia1.2697 | NO₂ | CH₃ | CH₂CH(CH₃)-(4-Cl-C₆H₄) | NCH₃ |
| Ia1.2698 | NO₂ | CH₂CH₃ | CH₂CH(CH₃)-(4-Cl-C₆H₄) | NCH₃ |
| Ia1.2699 | NO₂ | (CH₂)₂CH₃ | CH₂CH(CH₃)-(4-Cl-C₆H₄) | NCH₃ |
| Ia1.2700 | NO₂ | (CH₂)₃CH₃ | CH₂CH(CH₃)-(4-Cl-C₆H₄) | NCH₃ |
| Ia1.2701 | NO₂ | H | CH(CH₃)CH₂-(4-Cl-C₆H₄) | NCH₃ |
| Ia1.2702 | NO₂ | CH₃ | CH(CH₃)CH₂-(4-Cl-C₆H₄) | NCH₃ |
| Ia1.2703 | NO₂ | CH₂CH₃ | CH(CH₃)CH₂-(4-Cl-C₆H₄) | NCH₃ |
| Ia1.2704 | NO₂ | (CH₂)₂CH₃ | CH(CH₃)CH₂-(4-Cl-C₆H₄) | NCH₃ |
| Ia1.2705 | NO₂ | (CH₂)₃CH₃ | CH(CH₃)CH₂-(4-Cl-C₆H₄) | NCH₃ |
| Ia1.2706 | NO₂ | H | CH(CH₃)CH(CH₃)-(4-Cl-C₆H₄) | NCH₃ |
| Ia1.2707 | NO₂ | CH₃ | CH(CH₃)CH(CH₃)-(4-Cl-C₆H₄) | NCH₃ |
| Ia1.2708 | NO₂ | CH₂CH₃ | CH(CH₃)CH(CH₃)-(4-Cl-C₆H₄) | NCH₃ |
| Ia1.2709 | NO₂ | (CH₂)₂CH₃ | CH(CH₃)CH(CH₃)-(4-Cl-C₆H₄) | NCH₃ |
| Ia1.2710 | NO₂ | (CH₂)₃CH₃ | CH(CH₃)CH(CH₃)-(4-Cl-C₆H₄) | NCH₃ |
| Ia1.2711 | NO₂ | H | (CH₂)₂-(2,4-Cl₂-C₆H₃) | NCH₃ |
| Ia1.2712 | NO₂ | CH₃ | (CH₂)₂-(2,4-Cl₂-C₆H₃) | NCH₃ |
| Ia1.2713 | NO₂ | CH₂CH₃ | (CH₂)₂-(2,4-Cl₂-C₆H₃) | NCH₃ |
| Ia1.2714 | NO₂ | (CH₂)₂CH₃ | (CH₂)₂-(2,4-Cl₂-C₆H₃) | NCH₃ |
| Ia1.2715 | NO₂ | (CH₂)₃CH₃ | (CH₂)₂-(2,4-Cl₂-C₆H₃) | NCH₃ |
| Ia1.2716 | NO₂ | H | CH₂CH(CH₃)-(2,4-Cl₂-C₆H₃) | NCH₃ |
| Ia1.2717 | NO₂ | CH₃ | CH₂CH(CH₃)-(2,4-Cl₂-C₆H₃) | NCH₃ |
| Ia1.2718 | NO₂ | CH₂CH₃ | CH₂CH(CH₃)-(2,4-Cl₂-C₆H₃) | NCH₃ |
| Ia1.2719 | NO₂ | (CH₂)₂CH₃ | CH₂CH(CH₃)-(2,4-Cl₂-C₆H₃) | NCH₃ |
| Ia1.2720 | NO₂ | (CH₂)₃CH₃ | CH₂CH(CH₃)-(2,4-Cl₂-C₆H₃) | NCH₃ |
| Ia1.2721 | NO₂ | H | CH(CH₃)CH₂-(2,4-Cl₂-C₆H₃) | NCH₃ |
| Ia1.2722 | NO₂ | CH₃ | CH(CH₃)CH₂-(2,4-Cl₂-C₆R₃) | NCH₃ |
| Ia1.2723 | NO₂ | CH₂CH₃ | CH(CH₃)CH₂-(2,4-Cl₂-C₆H₃) | NCR₃ |
| Ia1.2724 | NO₂ | (CH₂)₂CH₃ | CH(CH₃)CH₂-(2,4-Cl₂-C₆H₃) | NCH₃ |
| Ia1.2725 | NO₂ | (CH₂)₃CH₃ | CH(CH₃)CH₂-(2,4-Cl₂-C₆H₃) | NCH₃ |
| Ia1.2726 | NO₂ | H | CH(CH₃)CH(CH₃)-(2,4-Cl₂-C₆H₃) | NCH₃ |
| Ia1.2727 | NO₂ | CH₃ | CH(CH₃)CH(CH₃)-(2,4-Cl₂-C₆H₃) | NCH₃ |
| Ia1.2728 | NO₂ | CH₂CH₃ | CH(CH₃)CH(CH₃)-(2,4-Cl₂-C₆H₃) | NCH₃ |
| Ia1.2729 | NO₂ | (CH₂)₂CH₃ | CH(CH₃)CH(CH₃)-(2,4-Cl₂-C₆H₃) | NCH₃ |
| Ia1.2730 | NO₂ | (CH₂)₃CH₃ | CH(CH₃)CH(CH₃)-(2,4-Cl₂-C₆H₃) | NCH₃ |
| Ia1.2731 | NO₂ | H | (CH₂)₂-O-C₆H₅ | NCH₃ |
| Ia1.2732 | NO₂ | CH₃ | (CH₂)₂-O-C₆H₅ | NCH₃ |
| Ia1.2733 | NO₂ | CH₂CH₃ | (CH₂)₂-O-C₆H₅ | NCH₃ |
| Ia1.2734 | NO₂ | (CH₂)₂CH₃ | (CH₂)₂-O-C₆H₅ | NCH₃ |
| Ia1.2735 | NO₂ | (CH₂) ₃CH₃ | (CH₂)₂-O-C₆H₅ | NCH₃ |
| Ia1.2736 | NO₂ | H | CH₂CH(CH₃)-O-C₆H₅ | NCH₃ |
| Ia1.2737 | NO₂ | CH₃ | CH₂CH(CH₃)-O-C₆H₅ | NCH₃ |
| Ia1.2738 | NO₂ | CH₂CH₃ | CH₂CH(CH₃)-O-C₆H₅ | NCH₃ |
| Ia1.2739 | NO₂ | (CH₂)₂CH₃ | CH₂CH(CH₃)-O-C₆H₅ | NCH₃ |
| Ia1.2740 | NO₂ | (CH₂)₃CH₃ | CH₂CH(CH₃) -O-C₆H₅ | NCH₃ |
| Ia1.2741 | NO₂ | H | CH(CH₃)CH₂-O-C₆H₅ | NCH₃ |
| Ia1.2742 | NO₂ | CH₃ | CH(CH₃)CH₂-O-C₆H₅ | NCH₃ |
| Ia1.2743 | NO₂ | CH₂CH₃ | CH(CH₃)CH₂-O-C₆H₅ | NCH₃ |
| Ia1.2744 | NO₂ | (CH₂)₂CH₃ | CH(CH₃)CH₂-O-C₆H₅ | NCH₃ |
| Ia1.2745 | NO₂ | (CH₂)₃CH₃ | CH(CH₃)CH₂-O-C₆H₅ | NCH₃ |
| Ia1.2746 | NO₂ | H | CH(CH₃)CH(CH₃)-O-C₆H₅ | NCH₃ |
| Ia1.2747 | NO₂ | CH₃ | CH(CH₃)CH(CH₃)-O-C₆H₅ | NCH₃ |
| Ia1.2748 | NO₂ | CH₂CH₃ | CH(CH₃)CH(CH₃)-O-C₆H₅ | NCH₃ |
| Ia1.2749 | NO₂ | (CH₂)₂CH₃ | CH(CH₃)CH(CH₃)-O-C₆H₅ | NCH₃ |
| Ia1.2750 | NO₂ | (CH₂)₃CH₃ | CH(CH₃)CH(CH₃)-O-C₆H₅ | NCH₃ |
| Ia1.2751 | NO₂ | H | (CH₂)₂-O-(4-Cl-C₆H₄) | NCH₃ |
| Ia1.2752 | NO₂ | CH₃ | (CH₂)₂-O-(4-Cl-C₆H₄) | NCH₃ |
| Ia1.2753 | NO₂ | CH₂CH₃ | (CH₂)₂-O-(4-Cl-C₆H₄) | NCH₃ |
| Ia1.2754 | NO₂ | (CH₂)₂CH₃ | (CH₂)₂-O-(4-Cl-C₆H₄) | NCH₃ |
| Ia1.2755 | NO₂ | (CH₂)₃CH₃ | (CH₂)₂-O-(4-Cl-C₆H₄) | NCH₃ |
| Ia1.2756 | NO₂ | H | CH₂CH(CH₃)-O-(4-Cl-C₆H₄) | NCH₃ |
| Ia1.2757 | NO₂ | CH₃ | CH₂CH(CH₃)-O-(4-Cl-C₆H₄) | NCH₃ |
| Ia1.2758 | NO₂ | CH₂CH₃ | CH₂CH(CH₃)-O-(4-Cl-C₆H₄) | NCH₃ |
| Ia1.2759 | NO₂ | (CH₂)₂CH₃ | CH₂CH(CH₃)-O- (4-Cl-C₆H₄) | NCH₃ |
| Ia1.2760 | NO₂ | (CH₂)₃CH₃ | CH₂CH(CH₃)-O-(4-Cl-C₆H₄) | NCH₃ |
| Ia1.2761 | NO₂ | H | CH(CH₃)CH₂-O-(4-Cl-C₆H₄) | NCH₃ |
| Ia1.2762 | NO₂ | CH₃ | CH(CH₃)CH₂-O-(4-Cl-C₆H₄) | NCH₃ |
| Ia1.2763 | NO₂ | CH₂CH₃ | CH(CH₃)CH₂-O-(4-Cl-C₆H₄) | NCH₃ |
| Ia1.2764 | NO₂ | (CH₂)₂CH₃ | CH(CH₃)CH₂-O-(4-Cl-C₆H₄) | NCH₃ |
| Ia1.2765 | NO₂ | (CH₂)₃CH₃ | CH(CH₃)CH₂-O-(4-Cl-C₆H₄) | NCH₃ |
| Ia1.2766 | NO₂ | H | CH(CH₃)CH(CH₃)-O-(4-Cl-C₆H₄) | NCH₃ |
| Ia1.2767 | NO₂ | CH₃ | CH(CH₃)CH(CH₃)-O-(4-Cl-C₆H₄) | NCH₃ |
| Ia1.2768 | NO₂ | CH₂CH₃ | CH(CH₃)CH(CH₃)-O-(4-Cl-C₆H₄) | NCH₃ |
| Ia1.2769 | NO₂ | (CH₂)₂CH₃ | CH(CH₃)CH(CH₃)-O-(4-Cl-C₆H₄) | NCH₃ |
| Ia1.2770 | NO₂ | (CH₂)₃CH₃ | CH(CH₃)CH(CH₃)-O-(4-Cl-C₆H₄) | NCH₃ |
| Ia1.2771 | NO₂ | H | (CH₂)₂-O-(2,4-Cl₂-C₆H₃) | NCH₃ |
| Ia1.2772 | NO₂ | CH₃ | (CH₂)₂-O-(2,4-Cl₂-C₆H₃) | NCH₃ |
| Ia1.2773 | NO₂ | CH₂CH₃ | (CH₂)₂-O-(2,4-Cl₂-C₆H₃) | NCH₃ |
| Ia1.2774 | NO₂ | (CH₂)₂CH₃ | (CH₂)₂-O-(2,4-Cl₂-C₆H₃) | NCH₃ |
| Ia1.2775 | NO₂ | (CH₂)₃CH₃ | (CH₂)₂-O-(2,4-Cl₂-C₆H₃) | NCH₃ |
| Ia1.2776 | NO₂ | H | CH₂CH(CH₃)-O-(2,4-Cl₂-C₆H₃) | NCH₃ |
| Ia1.2777 | NO₂ | CH₃ | CH₂CH(CH₃)-O-(2,4-Cl₂-C₆H₃) | NCH₃ |
| Ia1.2778 | NO₂ | CH₂CH₃ | CH₂CH(CH₃)-O-(2,4-Cl₂-C₆H₃) | NCH₃ |
| Ia1.2779 | NO₂ | (CH₂)₂CH₃ | CH₂CH(CH₃)-O-(2,4-Cl₂-C₆H₃) | NCH₃ |
| Ia1.2780 | NO₂ | (CH₂)₃CH₃ | CH₂CH(CH₃)-O-(2,4-Cl₂-C₆H₃) | NCH₃ |
| Ia1.2781 | NO₂ | H | CH(CH₃)CH₂-O-(2,4-Cl₂-C₆H₃) | NCH₃ |
| Ia1.2782 | NO₂ | CH₃ | CH(CH₃)CH₂-O-(2,4-Cl₂-C₆H₃) | NCH₃ |
| Ia1.2783 | NO₂ | CH₂CH₃ | CH(CH₃)CH₂-O-(2,4-Cl₂-C₆H₃) | NCH₃ |
| Ia1.2784 | NO₂ | (CH₂)₂CH₃ | CH(CH₃)CH₂-O-(2,4-Cl₂-C₆H₃) | NCH₃ |
| Ia1.2785 | NO₂ | (CH₂)₃CH₃ | CH(CH₃)CH₂-O-(2,4-Cl₂-C₆H₃) | NCH₃ |
| Ia1.2786 | NO₂ | H | CH(CH₃)CH(CH₃)-O-(2,4-Cl₂-C₆H₃) | NCH₃ |
| Ia1.2787 | NO₂ | CH₃ | CH(CH₃)CH(CH₃)-O-(2,4-Cl₂-C₆H₃) | NCH₃ |
| Ia1.2788 | NO₂ | CH₂CH₃ | CH(CH₃)CH(CH₃)-O-(2,4-Cl₂-C₆H₃) | NCH₃ |
| Ia1.2789 | NO₂ | (CH₂)₂CH₃ | CH(CH₃)CH(CH₃)-O-(2,4-Cl₂-C₆H₃) | NCH₃ |
| Ia1.2790 | NO₂ | (CH₂)₃CH₃ | CH(CH₃)CH(CH₃)-O-(2,4-Cl₂-C₆H₃) | NCH₃ |

Desweiteren sind die folgenden 2-Ben2oyl-cyclohexan-1,3-dione der Formel I insbesonderst außerordentlichst bevorzugt:
- die Verbindungen Ia2, insbesondere die Verbindungen Ia2.1-Ia2.2790, die sich von den entsprechenden Verbindungen la1.1-Ia1.2790 dadurch unterscheiden, daß R¹³ für Methyl steht: 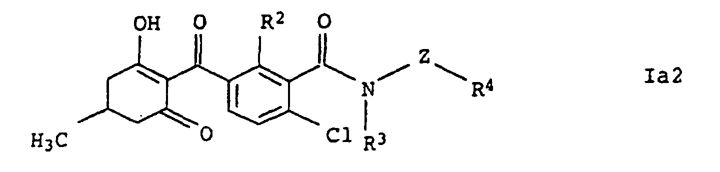
- die Verbindungen Ia3, insbesondere die Verbindungen Ia3.1-Ia3.2790, die sich von den entsprechenden Verbindungen Ia1.1-Ia1.2790 dadurch unterscheiden, daß R¹³ und R¹⁴ jeweils für Methyl stehen: 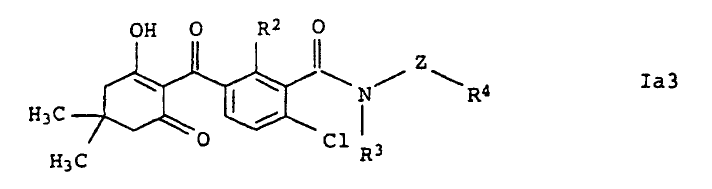
- die Verbindungen Ia4, insbesondere die Verbindungen Ia4.1-Ia4.2790, die sich von den entsprechenden Verbindungen Ia1.1-Ia1.2790 dadurch unterscheiden, daß R¹⁵ und R¹⁶ jeweils für Methyl stehen: 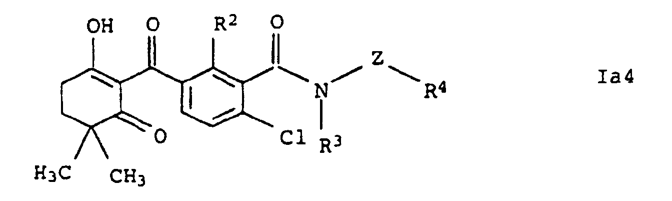
- die Verbindungen Ia5, insbesondere die Verbindungen Ia5.1-Ia5.2790, die sich von den entsprechenden Verbindungen Ia1.1-Ia1.2790 dadurch unterscheiden, daß die CR¹³R¹⁴-Einheit durch C=O ersetzt ist: 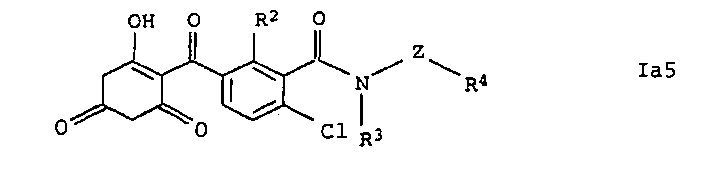
- die Verbindungen Ia6, insbesondere die Verbindungen Ia6.1-Ia6.2790, die sich von den entsprechenden Verbindungen Ia1.1-Ia1.2790 dadurch unterscheiden, daß R¹¹, R¹⁵ und R¹⁶ jeweils für Methyl stehen und die CR¹³R¹⁴-Einheit durch C=O ersetzt ist: 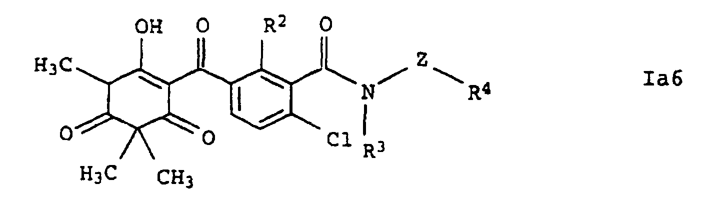
- die Verbindungen Ia7, insbesondere die Verbindungen Ia7.1-Ia7.2790, die sich von den entsprechenden Verbindungen Ia1.1-Ia1.2790 dadurch unterscheiden, daß R¹¹, R¹², R¹⁵ und R¹⁶ jeweils für Methyl stehen und die CR¹³R¹⁴-Einheit durch C=O ersetzt ist: 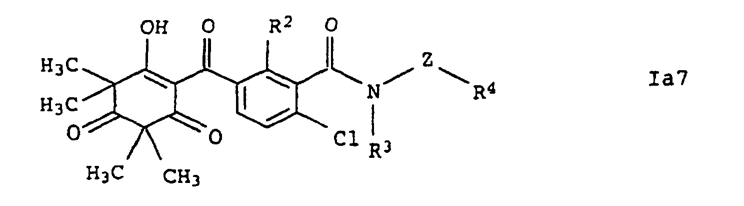
- die Verbindungen Ia8, insbesondere die Verbindungen Ia8.1-Ia8.2790, die sich von den entsprechenden Verbindungen Ia1.1-Ia1.2790 dadurch unterscheiden, daß R¹ für Nitro steht: 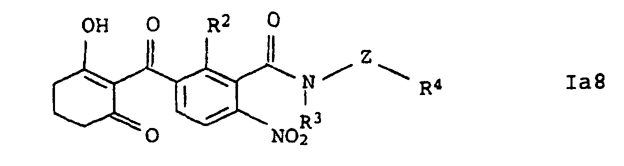
- die Verbindungen Ia9, insbesondere die Verbindungen Ia9.1-Ia9.2790, die sich von den entsprechenden Verbindungen Ia1.1-Ia1.2790 dadurch unterscheiden, daß R¹ für Nitro und R¹³ für Methyl stehen: 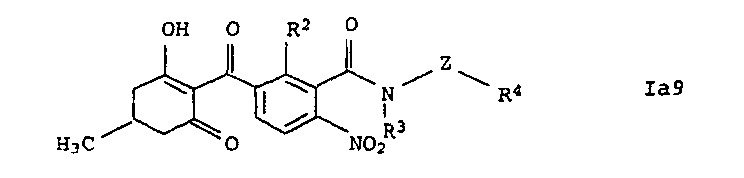
- die Verbindungen Ia10, insbesondere die Verbindungen Ia10.1-Ia10.2790, die sich von den entsprechenden Verbindungen Ia1.1-Ia1.2790 dadurch unterscheiden, daß R¹ für Nitro und R¹³ und R¹⁴ jeweils für Methyl stehen: 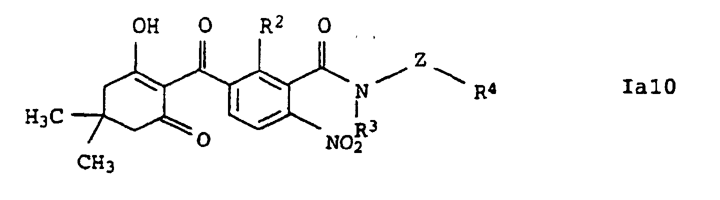
- die Verbindungen Ia11, insbesondere die Verbindungen Ia11.1-Ia11.2790, die sich von den entsprechenden Verbindungen Ia1.1-Ia1.2790 dadurch unterscheiden, daß R¹ für Nitro und R¹⁵ und R¹⁶ jeweils für Methyl stehen: 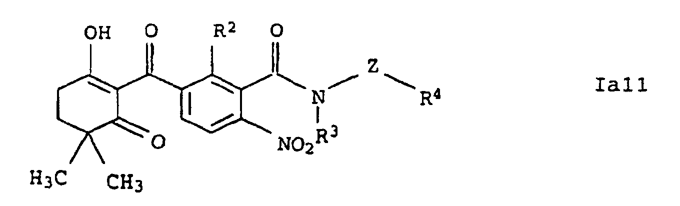
- die Verbindungen Ia12, insbesondere die Verbindungen Ia12.1-Ia12.2790, die sich von den entsprechenden Verbindungen Ia1.1-Ia1.2790 dadurch unterscheiden, daß R¹ für Nitro steht und die CR¹³R¹⁴-Einheit durch C=O ersetzt ist: 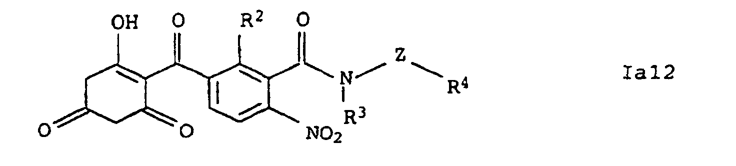
- die Verbindungen Ia13, insbesondere die Verbindungen Ia13.1-Ia13.2790, die sich von den entsprechenden Verbindungen Ia1.1-Ia1.2790 dadurch unterscheiden, daß R¹ für Nitro, R¹¹, R¹⁵ und R¹⁶ jeweils für Methyl stehen und die CR¹³R¹⁴-Einheit durch C=O ersetzt ist: 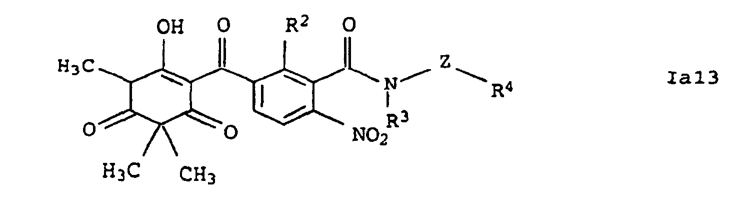
- die Verbindungen Ia14, insbesondere die Verbindungen Ia14.1-Ia14.2790, die sich von den entsprechenden Verbindungen Ia1.1-Ia1.2790 dadurch unterscheiden, daß R¹ für Nitro, R¹¹, R¹², R¹⁵ und R¹⁶ jeweils für Methyl stehen und die CR¹³R¹⁴-Einheit durch C=O ersetzt ist: 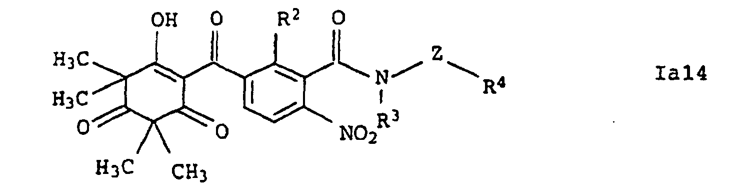
- die Verbindungen Ia15, insbesondere die Verbindungen Ia15.1-Ia15.2790, die sich von den entsprechenden Verbindungen Ia1.1-Ia1.2790 dadurch unterscheiden, daß R¹ für Methylsulfonyl steht: 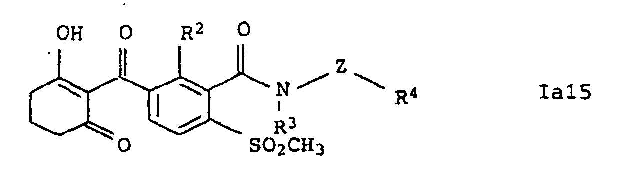
- die Verbindungen Ia16, insbesondere die Verbindungen Ia16.1-Ia16.2790, die sich von den entsprechenden Verbindungen Ia1.1-Ia1.2790 dadurch unterscheiden, daß R¹ für Methylsulfonyl und R¹³ für Methyl stehen: 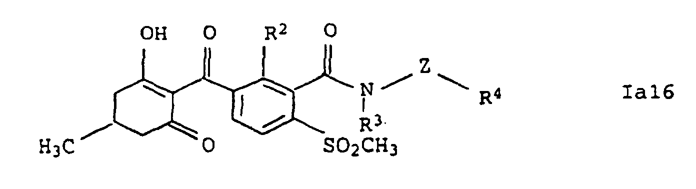
- die Verbindungen Ia17, insbesondere die Verbindungen Ia17.1-Ia17.2790, die sich von den entsprechenden Verbindungen Ia1.1-Ia1.2790 dadurch unterscheiden, daß R¹ für Methylsulfonyl und R¹³ und R¹⁴ jeweils für Methyl stehen: 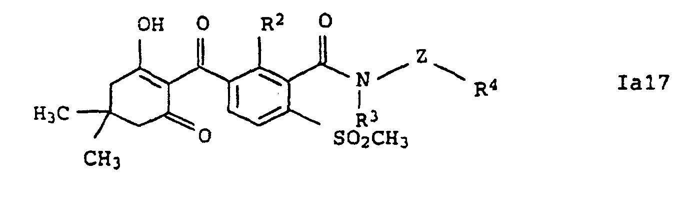
- die Verbindungen Ia18, insbesondere die Verbindungen Ia18.1-Ia18.2790, die sich von den entsprechenden Verbindungen Ia1.1-Ia1.2790 dadurch unterscheiden, daß R¹ für Methylsulfonyl und R¹⁵ und R¹⁶ jeweils für Methyl stehen: 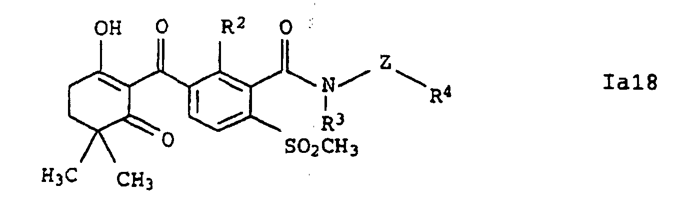
- die Verbindungen Ia19, insbesondere die Verbindungen Ia19.1-Ia19.2790, die sich von den entsprechenden Verbindungen Ia1.1-Ia1.2790 dadurch unterscheiden, daß R¹ für Methylsulfonyl steht und die CR¹³R¹⁴-Einheit durch C=O ersetzt ist: 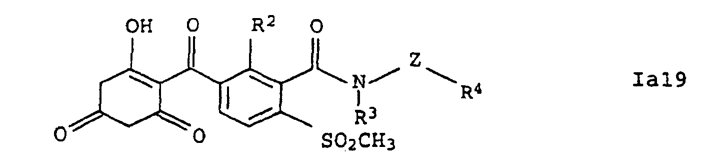
- die Verbindungen Ia20, insbesondere die Verbindungen Ia20.1-Ia20.2790, die sich von den entsprechenden Verbindungen Ia1.1-Ia1.2790 dadurch unterscheiden, daß R¹ für Methylsulfonyl. R¹¹, R¹⁵ und R¹⁶ jeweils für Methyl stehen und die CR¹³R¹⁴ -Einheit durch C=O ersetzt ist: 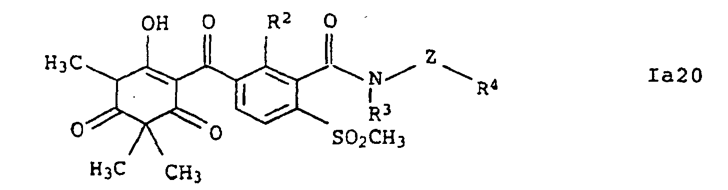
- die Verbindungen Ia.21, insbesondere die Verbindungen Ia21.1-Ia21.2790, die sich von den entsprechenden Verbindungen Ia1.1-Ia1.2790 dadurch unterscheiden, daß R¹ für Methylsulfonyl, R¹¹, R¹², R¹⁵ und R¹⁶ jeweils für Methyl stehen und die CR¹³R¹⁴-Einheit durch C=O ersetzt ist: 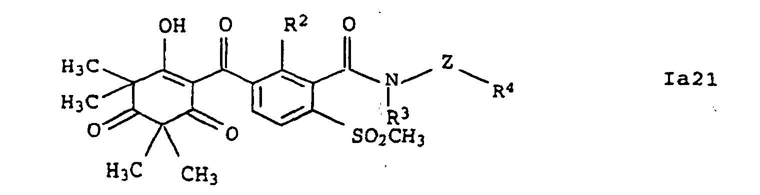
- die Verbindungen Ia22, insbesondere die Verbindungen Ia22.1-Ia22.2790, die sich von den entsprechenden Verbindungen Ia1.1-Ia1.2790 dadurch unterscheiden, daß R¹ für Trifluormethyl steht: 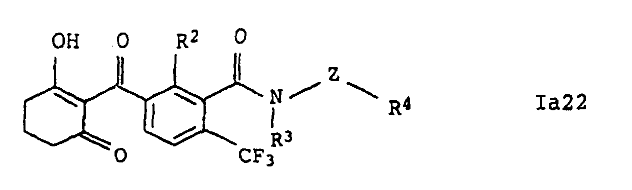
- die Verbindungen Ia23, insbesondere die Verbindungen Ia23.1-Ia23.2790, die sich von den entsprechenden Verbindungen Ia1.1-Ia1.2790 dadurch unterscheiden, daß R¹ für Trifluormethyl und R¹³ für Methyl stehen: 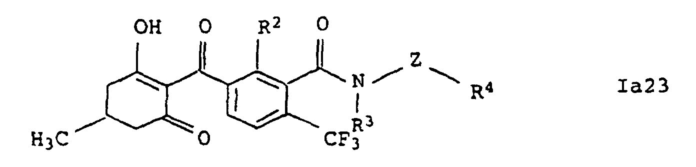
- die Verbindungen Ia24, insbesondere die Verbindungen Ia24.1-Ia24.2790, die sich von den entsprechenden Verbindungen Ia1.1-Ial.2790 dadurch unterscheiden, daß R¹ für Trifluormethyl und R¹³ und R¹⁴ jeweils für Methyl stehen: 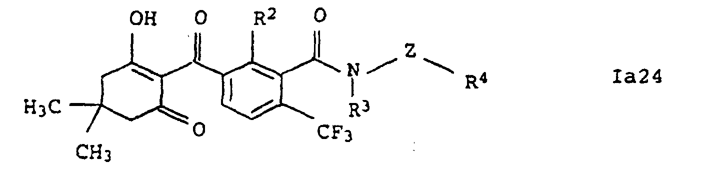
- die Verbindungen Ia25, insbesondere die Verbindungen Ia25.1-Ia25.2790, die sich von den entsprechenden Verbindungen Ia1.1-Ia1.2790 dadurch unterscheiden, daß R¹ für Trifluormethyl und R¹⁵ und R¹⁶ jeweils für Methyl stehen: 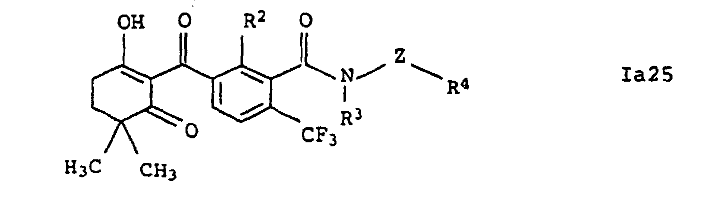
- die Verbindungen Ia26, insbesondere die Verbindungen Ia26.1-Ia26.2790, die sich von den entsprechenden Verbindungen Ia1.1-Ia1.2790 dadurch unterscheiden, daß R¹ für Trifluormethyl steht und die CR¹³R¹⁴-Einheit durch C=O ersetzt ist: 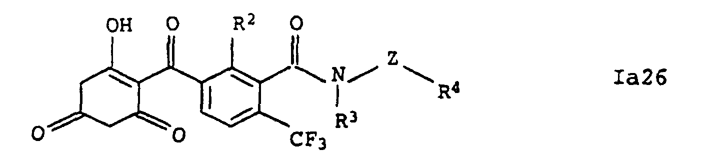
- die Verbindungen Ia27, insbesondere die Verbindungen Ia27.1-Ia27.2790, die sich von den entsprechenden Verbindungen Ia1.1-Ia1.2790 dadurch unterscheiden, daß R¹ für Trifluormethyl, R¹¹, R¹⁵ und R¹⁶ jeweils für Methyl stehen und die CR¹³R¹⁴-Einheit durch C=O ersetzt ist: 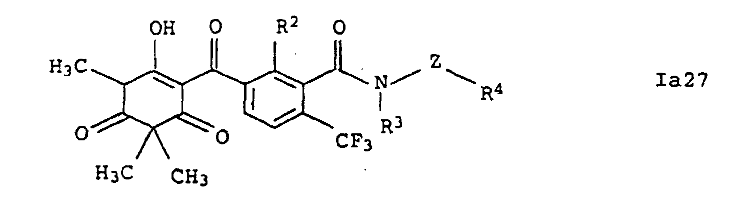
- die Verbindungen Ia28, insbesondere die Verbindungen Ia28.1-Ia28.2790, die sich von den entsprechenden Verbindungen Ia.1-1a1.2790 dadurch unterscheiden, daß R¹ für Trifluormethyl, R¹¹ R¹², R¹⁵ und R¹⁶ jeweils für Methyl stehen und die CR¹³R¹⁴-Einheit durch C=O ersetzt ist:

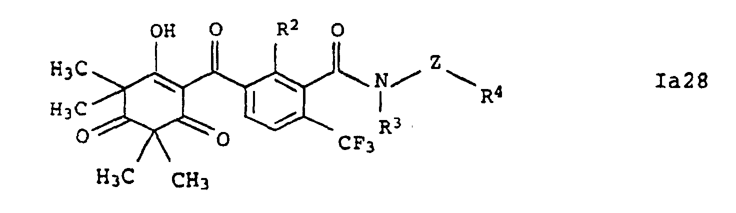

Ganz insbesonderst außerordentlich bevorzugt sind die Verbindungen der Formel Ia' (≙ I, wobei R¹ in Position 4 des Phenylrings und R² in Position 2 des Phenylrings gebunden sind), 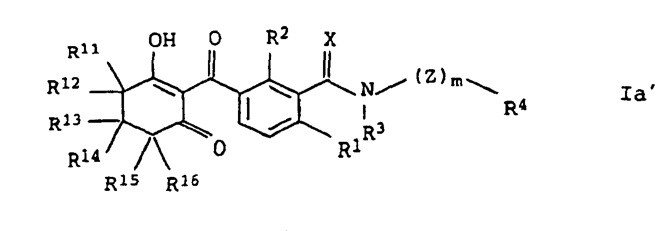 wobei die Variablen folgende Bedeutung haben:
- R¹: Halogen oder C₁-C₄-Alkylsulfonyl;
- R²: Halogen oder C₁-C₄-Alkyl;
- R³: Wasserstoff oder C₁-C₄-Alkyl;
- R⁴: Wasserstoff, C₁-C₆-Alkyl, C₃-C₆-Alkenyl, wobei die zwei letztgenannten Substituenten partiell oder vollständig halogeniert sein können und/oder eine bis drei der folgenden Gruppen tragen können: Phenyl, Phenyl-C₁-C₄-alkyl, Hetaryl oder Phenyloxy, wobei die 4 letztgenannten Reste ihrerseits partiell oder vollständig halogeniert sein können;
- X: Sauerstoff;
- Z: Sauerstoff oder NH;
- m: 0 oder 1;
- R¹¹, R¹², R¹⁴, R¹⁶,: Wasserstoff oder C₁-C₄-Alkyl;
- R¹³: Wasserstoff, C₁-C₄-Alkyl, Tetrahydropyran-3-yl, Tetrahydrothiopyran-3-yl oder 1,4-Dioxan-2-yl;
Gewünschtenfalls kann die CR¹³R¹⁴-Einheit durch C=O ersetzt werden.

Die 2-Benzoyl-cyclohexan-1,3-dione der Formel I sind auf verschiedene Art und Weise erhältlich, beispielsweise nach folgendem Verfahren:

Umsetzung von Cyclohexandionen der Formel II mit einer aktivierten Carbonsäure IIIα oder einer Carbonsäure IIIβ, die vorzugsweise in situ aktiviert wird, zu dem Acylierungsprodukt IV und anschließende Umlagerung.

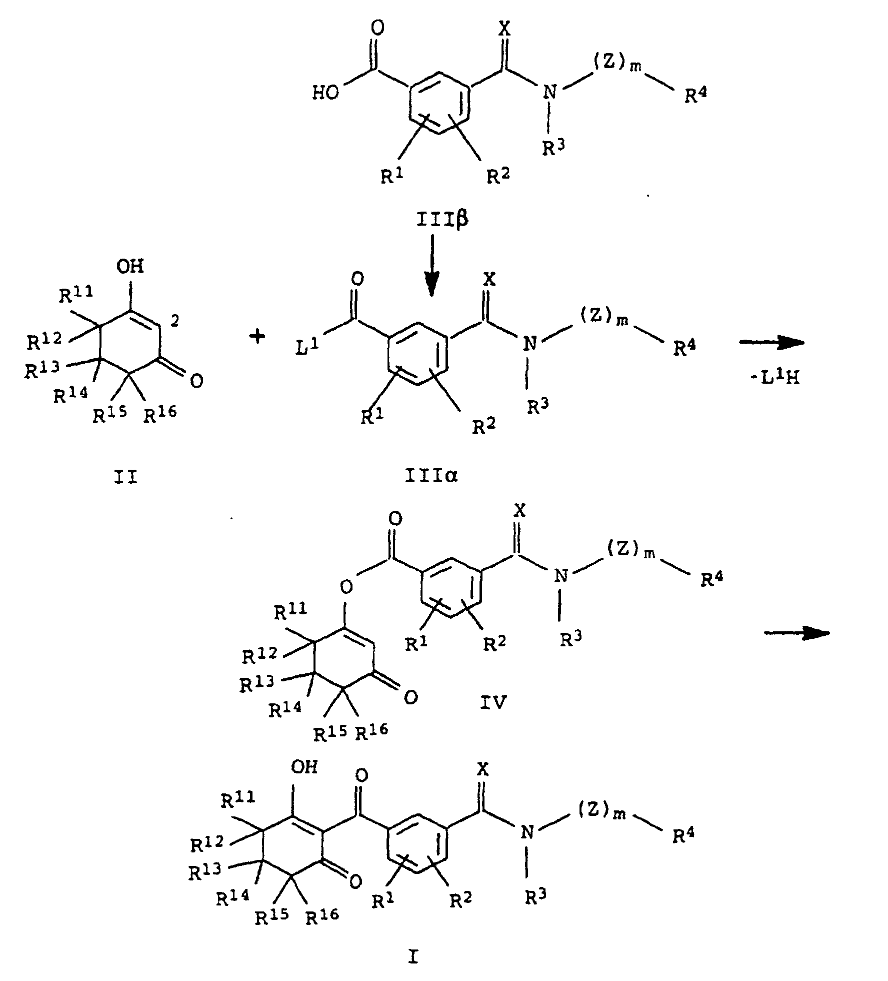

L¹ steht für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1, 2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40°C in einem Lösungsmittel und in Gegenwart einer Hilfsbase sowie gegebenenfalls mit Hilfe einer Cyanoverbindung als Katalysator.

Als Lösungsmittel können z.B. Acetonitril, Methylenchlorid, 1,2-Dichlorethan, Essigsäureethylester, Toluol oder Gemische hiervon verwendet werden. Bevorzugtes Lösungsmittel ist Acetonitril.

Geeignete Hilfsbasen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Enolester, eingesetzt werden. Bevorzugt wird Triethylamin verwendet, vorzugsweise in doppelt äouimolaren Verhältnis in Bezug auf den Enolester.

Als "Umlagerungskatalysator" kommen anorganische Cyanide, wie Natriumcyanid, Kaliumcyanid und organische Cyanoverbindungen, wie Acetoncyanhydrin, Trimethylsilylcyanid in Betracht. Sie werden üblicherweise in einer Menge von 1 bis 50 Molprozent, bezogen au den Enolester, eingesetzt. Vorzugsweise werden Acetoncyanhydrin oder Trimethylsilylcyanid, z.B. in einer Menge von 5 bis 15, vor zugsweise 10 Molprozent, bezogen auf den Enolester, eingesetzt.

Die Aufarbeitung kann in an sich bekannter Weise erfolgen. Das Reaktionsgemisch wird z.B. mit verdünnter Mineralsäure, wie z.B. 5%ige Salzsäure oder Schwefelsäure, angesäuert, mit einem organi schen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlori oder Essigsäureethylester extrahiert, getrocknet und eingeengt. (Beispiele für die Darstellung von Enolestern von Cyclohexan-1,3-dionen und für die cyanidkatalysierte Umlagerung der Enolester sind z.B. in EP-A 186 118. US 4 780 127 genannt).

Die als Ausgangsmaterialien verwendeten Cyclohexan-1,3-dione der Formel II sind bekannt oder können nach an sich bekannten Verfahren hergestellt werden (z.B. EP-A 71 707, EP-A 142 741, EP-A 243 313, US 4 249 937; WO 92/13821).

Die Benzoesäurederivate der Formel lila sind neu, 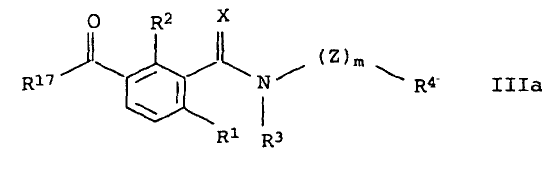 wobei die Variablen folgende Bedeutung haben:
- R¹, R²: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵, oder -S(O)ₙR⁷
- R³: Wasserstoff, C₁-C₆-Alkyl, C₁-C₆-Halogenalkyl, C₃-C₆-Alkenyl oder C₃-C₆-Alkinyl;
- R⁴: Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰. -SR¹⁰, - NR⁸R¹⁰ =NOR¹⁰, -OCOR¹⁰, -SCOR¹⁰, -NR⁸COR¹⁰, -CO₂R¹⁰, - COSR^{10,} -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Phenyl-C₁-C₄-alkyl, Hetaryl, Phenoxy, Phenyl-C₁-C₄-alkoxy und Hetaryloxy, wobei die acht letztgenannten Reste ihrerseits substituiert sein können;
- X: Sauerstoff oder Schwefel;
- Z: Sauerstoff oder NR⁸;
- m: 1;
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁷: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁸: Wasserstoff oder C₁-C₆-Alkyl;
- R⁹: C₁-C₆-Alkyl, C₃-C₆-Alkenyl, C₃-C₆-Alkinyl, Phenyl oder Benzyl;
- R¹⁰: C₁-C₆-Alkyl, C₁-C₆-Halogenalkyl, C₃-C₆-Alkenyl oder C₃-C₆-Alkinyl;
- R¹⁷: Hydroxy oder ein abhydrolysierbarer Rest.
wobei R⁴ nicht für Methyl steht, wenn Z für Sauerstoff, R¹ für Methylsulfonyl, R² für Chlor, Methyl oder Methoxy und R³ für Methyl stehen.

Beispiele für abhydrolysierbare Reste sind Alkoxy-, Phenoxy-, Alkylthio-, Phenylthio-Reste, die gegebenenfalls substituiert sind, Halogenide, Hetarylreste, die über Stickstoff gebunden sind, Amino-, Iminoreste, die gegebenenfalls substituiert sind.

Bevorzugt sind Benzoesäurehalogenide IIIaα, mit L¹ = Halogen (≙ IIIa mit R¹⁷ = Halogen), 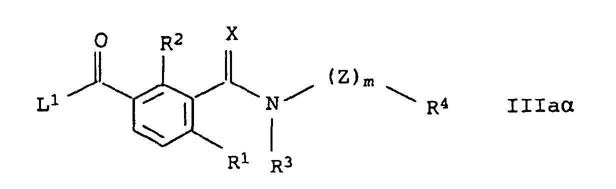 wobei die Variablen R¹ bis R⁴, X, Z und m die unter Formel IIIa genannte Bedeutung haben und
- L¹: Halogen, insbesondere Chlor oder Brom, bedeutet.

Ebenso bevorzugt sind die Benzoesäuren der Formel IIIaβ (≙ IIIa mit R¹⁷ = Hydroxy), 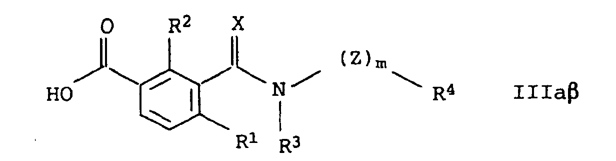 wobei die Variablen R¹ bis R⁴, X, Z und m die unter Formel IIIa genannte Bedeutung haben:

Ebenso bevorzugt sind die Benzoesäureester der Formel IIIaγ (≙ IIIa mit M = C₁-C₆-Alkoxy), 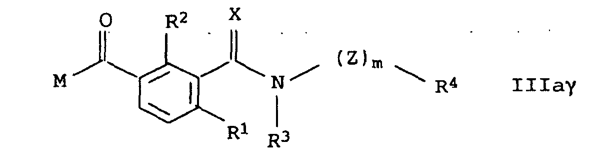 wobei die Variablen R¹ bis R⁴, X, Z und m die unter Formel IIIa genannte Bedeutung haben und
- M: C₁-C₆-Alkoxy;
bedeutet.

Die besonderen Ausführungsformen der Benzoesäureester der Formel IIIa in Bezug auf die Variablen R¹ bis R⁴, X, Z und m entsprechen denen der 2-Benzoyl-cyclohexan-1,3-dione der Formel I.

Die Verbindungen der Formel IIIα (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIβ mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIβ können in Analogie zu an sich literaturbekannten Methoden u.a. durch Verseifung der Benzoesäureester der Formel IIIγ (mit M = C₁-C₆-Alkoxy) erhalten werden.

Die Benzoesäureester der Formel IIIγ (mit X = Sauerstoff) sind auf verschiedene Art und Weise erhältlich, beispielsweise nach folgenden Verfahren:

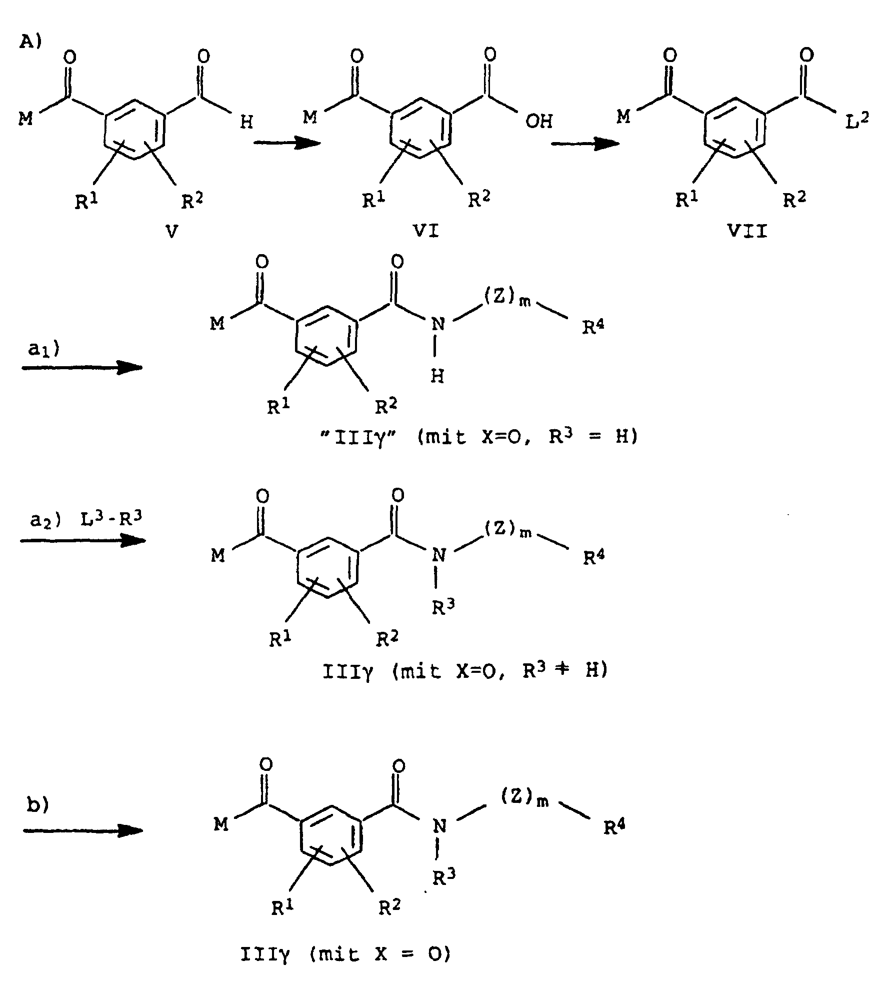

Durch Oxidation von Aldehyden der Formel V können auf an sich bekannte Art und Weise Isophthalsäurederivate der Formel VI erhalten werden (J. March, "Advanced Organic Chemistry", 3. Auflage, S. 629 ff, Wiley-Interscience Publication, 1985).

In Analogie zu literaturbekannten Verfahren können die Verbindungen der Formel VI zunächst in die entsprechenden aktivierten Carbonsäuren VII, wobei L² für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat etc. steht, überführt werden. Anschließend wird im Fall a₁) mit einem Amino-, Hydroxylamin- oder Hydrazinderivat umgesetzt, wobei R³ Wasserstoff bedeutet. Anschließende Alkylierung (a₂) führt zu entsprechenden Amid-, Hydroxamsäure- bzw. Carbonsäurehydrazidderivaten der Formel IIIγ (mit X = O und R³ ≠ H), (wobei L³ die unter L² genannte Bedeutung hat) (J. 0rg. Chem. (1971), 31, 284-294; J. Chem. Soc. Perk. II (1977), 1080-1084; Australian J. Chem. (1969), 22, 161-173; ibid (1974), 27, 1341-1349). Im Fall b) wird direkt mit R³NH-(Z)ₘ-R⁴ zum Endprodukt umgesetzt (vgl. Australian J. Chem. (1974), 27, 1341-1349).

Die Aldehyde der Formel V können in Analogie zu literaturbekannten Verfahren aus entsprechenden Toluolen der Formel VIII dargestellt werden, indem man sie in das ω-Halogentoluol IX überführt und anschließend oxidiert (vgl. Synth. Commun. 22, 1967-1971 (1992)).

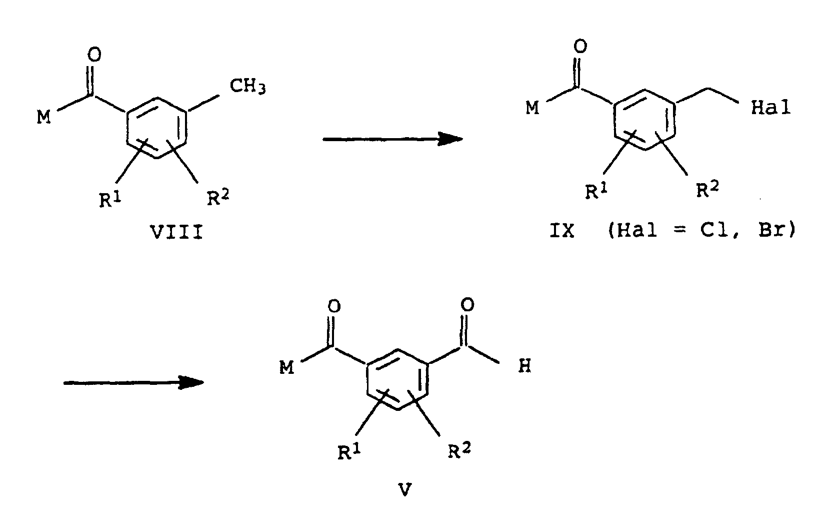

Die Carbonsäuren der Formel VI sind nach literaturbekannten Verfahren durch Hydrolyse von Nitrilen der Formel X zugänglich (J. March, "Advanced Organic Chemistry", 3. Auflage, S. 788, Wiley-Interscience Publication, 1985; Ann. Chem. (1970), S. 23-37).

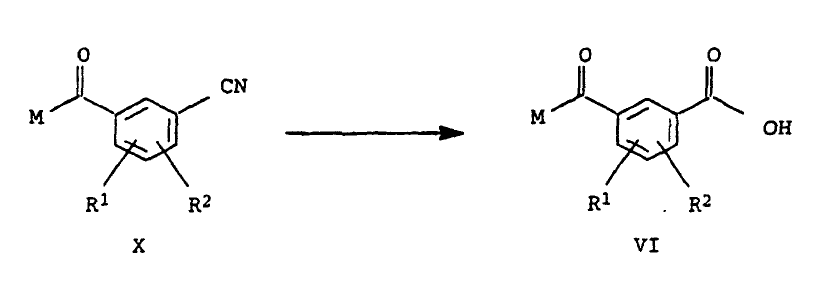

Die Nitrile der Formel X können in Analogie zu literaturbekannten Verfahren aus den entsprechenden Aldehyden V dargestellt werden (J. March, "Advanced Organic Chemistry", 3. Auflage, S. 806-807, Wiley-Interscience Publication, 1985). Ebenso ist es möglich, Nitrile der Formel X aus Anilinen der Formel XI mittels Sandmeyer-Reaktion zu erhalten oder durch Rosemund/von Braun-Reaktion aus Arylhalogeniden der Formel XII mit Metallcyaniden, insbesondere CuCN (J. March, "Advanced Organic Chemistry", 3. Auflage, S. 594, S. 648, Wiley-Interscience Publication, 1985).

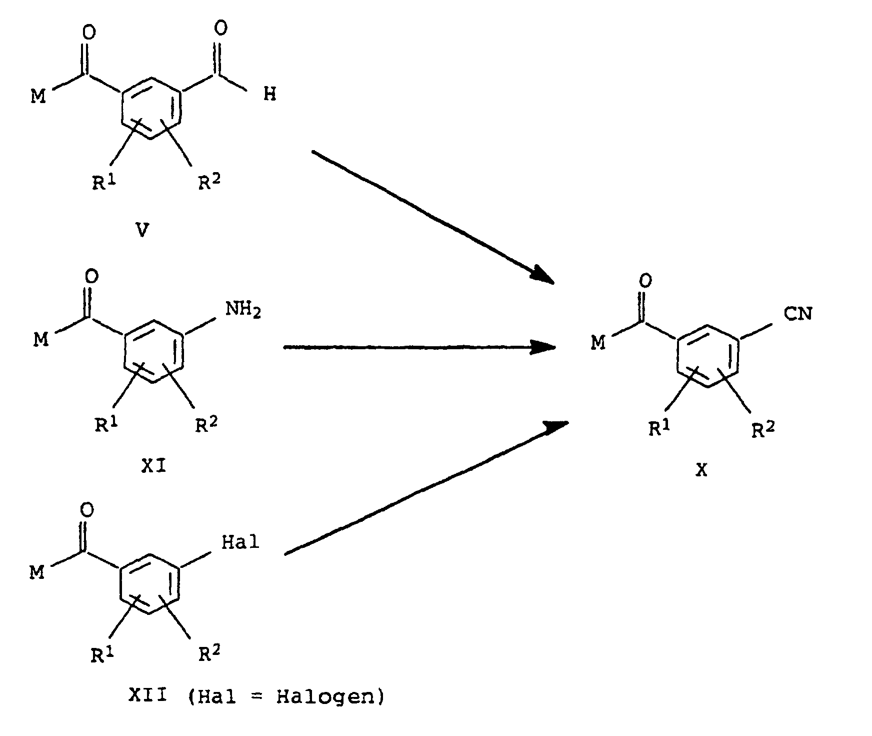

### Herstellungsbeispiele

### 2-[2,4-Dichlor-3-(N-ethyl-N-propoxy-aminocarbonyl)benzoyl]-1,3-cyclohexandion (Verbindung 2.03)

Zu einer Lösung von 0,71 g (0,0070 mol) Triethylamin und 0,79 g (0,0070 mol) 1,3-Cyclohexandion in 50 ml Methylenchlorid wurden 2,20 g (0,0065 mol) 2,4-Dichlor-3-(N-ethyl-N-propoxy-aminocarbonyl)benzoylchlorid gegeben. Nachdem die Reaktionslösung 2 Stunden bei Raumtemperatur gerührt wurde, entfernte man das Lösungsmittel am Vakuum. Der Rückstand wurde mittels Chromatographie an Kieselgel (Eluent: Toluol/Essigsäureethylester = 8/2) gereinigt. Der so erhaltene Enolester wurde in 50 ml Acetonitril aufgenommen und mit 0,50 g (0,0049 mol) Triethylamin und 0,10 g (0,0010 mol) Trimethylsilylcyanid versetzt. Nach 3 Stunden Rühren bei Raumtemperatur wurde das Lösungsmittel entfernt und der Rückstand in Methylenchlorid aufgenommen. Die organische Phase wurde mit verdünnter Phosphorsäure gewaschen, getrocknet und eingeengt. Man erhielt 1,20 g 2-[2,4-Dichlor-3-(N-ethyl-N-propoxy-aminocarbonyl)benzoyl]-1,3-cyclohexandion, das aus Diethylether ausgerührt wurde.
(Fp.: 180-183°C)

In der folgenden Tabelle 2 sind neben dem voranstehend beschriebenen 2-Benzoyl-cyclohexan-1.3-dion der Formel I noch weitere aufgeführt, die in analoger Weise hergestellt wurden oder herstellbar sind:

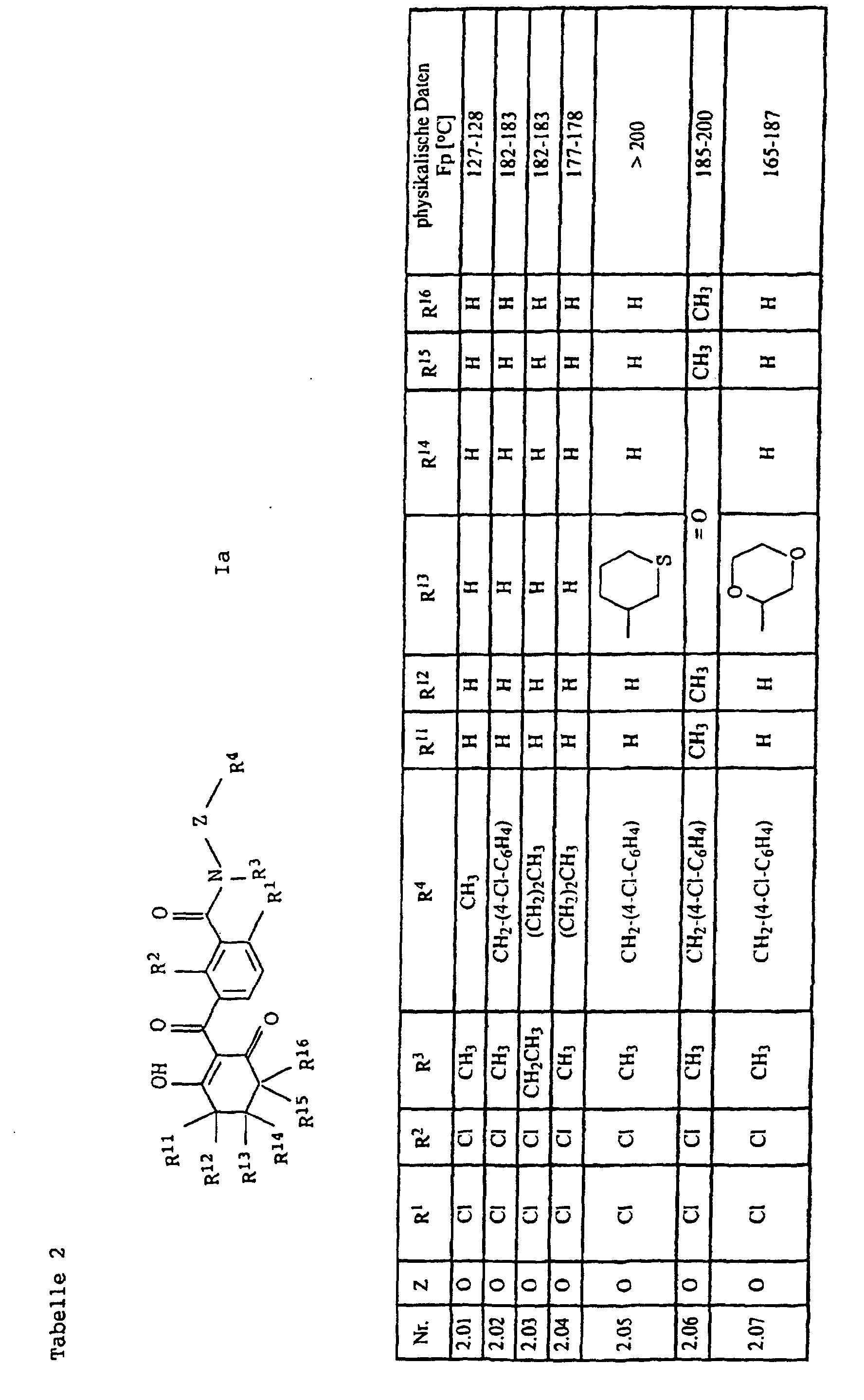

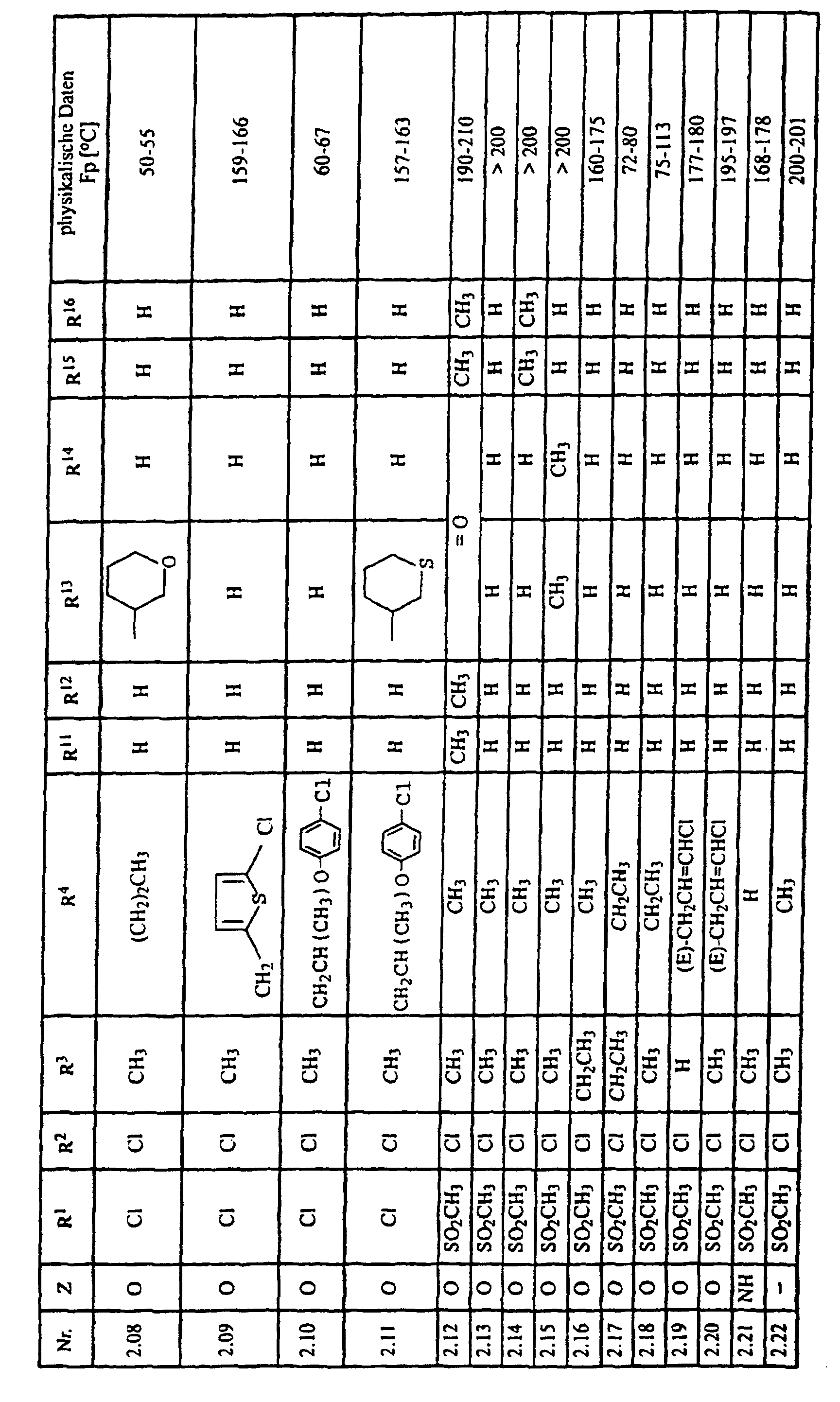

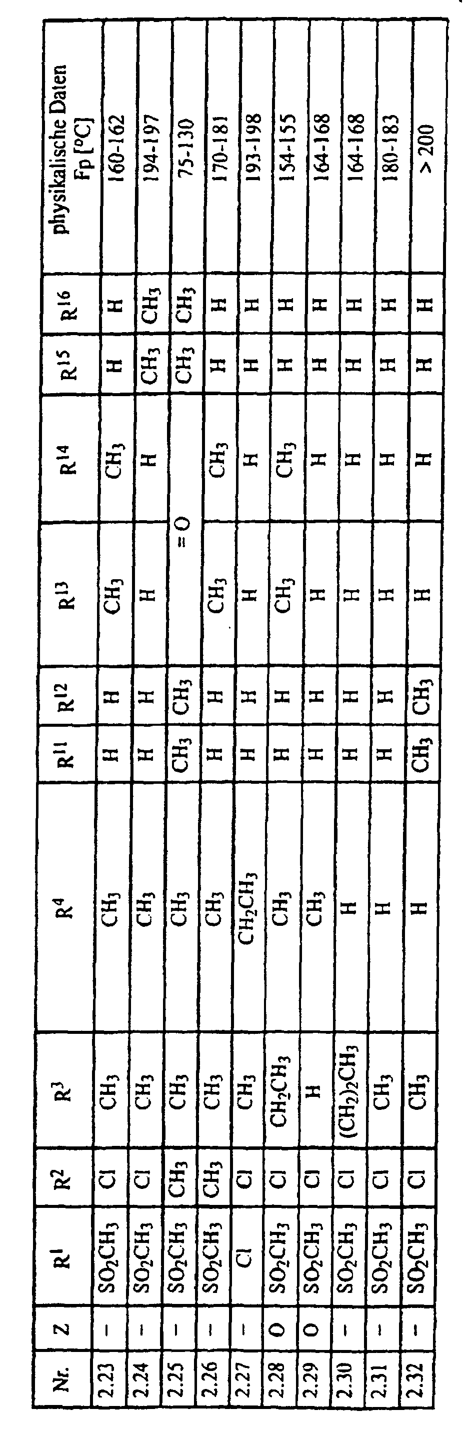

Nachfolgend sind die Synthesen einiger Ausgangsstoffe aufgeführt:

### 2-Chlor-3-(N-ethoxy-N-methyl-aminocarbonyl)-4-methylsulfonyl-benzoesäuremethylester (Verbindung 3.29)

Stufe a) 2-Chlor-3-methyl-4-methylthio-acetophenon
   Zu einer Suspension von 286 g (2,14 mol) Aluminiumtrichlorid in 420 ml 1,2-Dichlorethan wurde bei 15-20°C eine Lösung von 157 g (2 mol) Acetylchlorid in 420 mol 1,2-Dichlorethan getropft. Anschließend wurde eine Lösung von 346 g (2 mol) 2-Chlor-6-methylthio-toluol in 1 l 1,2-Dichlorethan zugetropft. Nach 12 Stunden Rühren wurde das Reaktionsgemisch in eine Mischung aus 3 1 Eis und 1 l konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert. Man erhielt 256 g (60 % d.Th.) 2-Chlor-3-methyl-4-methyl-thio-acetophenon.
   (Fp.: 46°C)
Stufe b) 2-Chlor-3-methyl-4-methylsulfonyl-acetophenon
   163,0 g (0,76 mol) 2-Chlor-3-methyl-4-methylthio-acetophenon wurden in 1,5 l Eisessig gelöst, mit 18,6 g Natriumwolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser gewaschen und getrocknet. Man erhielt 164,0 g (88 % . d.Th.) 2-Chlor-3-methyl-4-methylsulfonyl-acetophenon.
   (Fp.: 110-111°C)
Stufe c) 2-Chlor-3-methyl-4-methylsulfonyl-benzoesäure
   82 g (0,33 mol) 2-Chlor-3-methyl-4-methylsulfonyl-acetophenon wurden in 700 ml Dioxan gelöst und bei Raumtemperatur mit 1 l einer 12,5 %igen Natriumhypochloritlösung versetzt. Anschließend wurde 1 Stunde bei 80°C nachgerührt. Nach dem Abkühlen bildeten sich zwei Phasen, von denen die untere mit Wasser verdünnt und schwach angesäuert wurde. Der ausgefallene Feststoff wurde abgesaugt, mit Wasser nachgewaschen und getrocknet. Man erhielt 60 g (73 % d.Th) 2-Chlor-3-methyl-4-methylsulfonylbenzoesäure.
   (Fp.: 230-231°C)
Stufe d) 2-Chlor-3-methyl-4-methylsulfonyl-benzoesäuremethylester
   100 g (0,4 mol) 2-Chlor-3-methyl-4-methylsulfonyl-benzoesäure wurden in 1 l Methanol gelöst und bei Rückflußtemperatur 5 Stunden mit Chlorwasserstoff begast. Anschließend wurde eingeengt. Man erhielt 88,5 g (84 % d.Th.) 2-Chlor-3-methyl-4-methylsulfonyl-benzoesäuremethylester.
   (Fp.: 107-108°C)
Stufe e) 3-Brommethyl-2-chlor-4-methylsulfonyl-benzoesäuremethylester
   82 g (0,31 mol) 2-Chlor-3-methyl-4-methylsulfonyl-benzoesäuremethylester wurden in 2 l Tetrachlormethan gelöst und unter Belichtung portionsweise mit 56 g (0,31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wurde mit Petrolether versetzt, der ausgefallene Feststoff abgesaugt und getrocknet. Man erhielt 74,5 g (70 % d.Th.) 3-Brommethyl-2-chlor-4-methylsulfonyl-benzoesäuremethylester.
   (Fp.: 74-75°C)
Stufe f) 2-Chlor-3-formyl-4-methylsulfonyl-benzoesäuremethylester
   Eine Lösung von 41,0 g (0,12 mol) 3-Brommethyl-2-chlor-4-methylsulfonyl-benzoesäuremethylester in 250 ml Acetonitril wurde mit 42,1 g (0,36 mol) N-Methylmorpholin-N-oxid versetzt. Der Ansatz wurde 12 Stunden bei Raumtemperatur gerührt, anschließend eingeengt und der Rückstand in Essigsäureethylester aufgenommen. Die Lösung wurde mit Wasser extrahiert, mit Natriumsulfat getrocknet und eingeengt. Man erhielt 31,2 g (94 % d.Th.)
   2-Chlor-3-formyl-4-methylsulfonyl-benzoesäuremethylester
   (Fp.: 98-105°C)
Stufe g) 2-Chlor-3-hydroxycarbonyl-4-methylsulfonyl-benzoesäuremethylester
   Zu einer Lösung von 115,3 g (0,42 mol) 2-Chlor-3-formyl-4-methylsulfonyl-benzoesäuremethylester und 2000 ml Acetonitril wurden bei 5°C nacheinander 13,8 g (0,11 mol) Natriumhydrogenphosphatmonohydrat in 170 ml Wasser, 49,3 g (0,43 mol) 30 %ige Wasserstoffperoxidlösung und 66,2 g (0.59 mol) 80 %ige wäßrige Natriumchloritlösung gegeben. Die Reaktionslösung wurde anschließend 1 Stunde bei 5°C und 12 Stunden bei Raumtemperatur gerührt. Dann wurde mit 10 %iger Salzsäure auf pH = 1 eingestellt und 1500 ml wäßrige 40 %ige Natriumhydrogensulfit-Lösung zugegeben. Nach 1 Stunde Rühren bei Raumtemperatur wurde die wäßrige Phase dreimal mit Essigsäureethylester extrahiert. Die vereinigten organischen Phasen wurden mit Natriumhydrogensulfit-Lösung gewaschen und getrocknet. Nach Abdestillation des Lösungsmittels erhielt man 102,0 g 2-Chlor-3-hydroxycarbonyl-4-methylsulfonyl-benzoesäuremethylester.
   (¹H-NMR (d⁶-DMSO, δ in ppm) : 3,34 (s, 3H); 3,93 (s, 3H) ; 8,08 (s, 2H); 14,50 (s, br., 1H).)
Stufe h) 2-Chlor-3-chlorcarbonyl-4-methylsulfonyl-benzoesäuremethylester
   Zu einer Lösung von 6,0 g (0,021 mol) 2-Chlor-3-hydroxycarbonyl-4-methylsulfonyl-benzoesäuremethylester und 50 ml trockenem Toluol wurden 2 Tropfen Dimethylformamid und 11,9 g (0,1 mol) Thionylchlorid gegeben. Die Lösung wurde 4 Stunden unter Rückfluß erhitzt. Nach Entfernen des Lösungsmittels am Vakuum erhielt man 6,2 g 2-Chlor-3-chlorcarbonyl-4-methylsulfonyl-benzoesäuremethylester.
   (¹H-NMR (CDCl₃; δ in ppm): 3,21 (s, 3H); 4,02 (s, 3H); 8,02 (d, 1H); 8,07 (d, 1H).)
Stufe i) 2-Chlor-3-N-ethoxy-aminocarbonyl-4-methylsulfonyl-benzoesäuremethylester (Verbindung 3.28)
   Zu einer Lösung von 26,40 g (0,085 mol) 2-Chlor-3-chlorcarbonyl-4-methylsulfonyl-benzoesäuremethylester und 300 ml Dichlormethan wurden bei Raumtemperatur 11,70 g (0,120 mol) O-Ethylhydroxylamin-Hydrochlorid und 12,10 g (0,120 mol) Triethylamin zugegeben. Nach 4 Stunden Rühren bei Raumtemperatur wurde die Reaktionslösung mit verdünnter Salzsäure gewaschen, getrocknet und eingeengt. Der erhaltene Rückstand wurde aus Diethylether ausgerührt. Man erhielt 25,00 g 2-Chlor-3-N-ethoxy-aminocarbonyl-4-methylsulfonyl-benzoesäuremethylester.
   (Fp.: 90-110°C)
Stufe j) 2-Chlor-3-(N-ethoxy-N-methyl-aminocarbonyl)-4-methylsulfonylbenzoesäuremethylester (Verbindung 3.29)
   Ein Gemisch von 20,00 g (0,060 mol) 2-Chlor-3-N-ethoxyaminocarbonyl-4-methylsulfonyl-benzoesäuremethylester und 16,60 g (0,120 mol) Kaliumcarbonat in 200 ml Dimethylformamid wurde 30 Minuten bei Raumtemperatur gerührt. Anschließend wurden 25,60 g (0,180 mol) Methyliodid zugetropft und 5 Stunden bei 50°C gerührt. Nach Abkühlen des Reaktionsgemisches wurde in 1 l Eiswasser eingerührt, die wäßrige Phase mit Essigsäureethylester extrahiert und die vereinigten organischen Phasen getrocknet und eingeengt. Der Rückstand wurde an Kieselgel (Eluent: Toluol/Essigsäureethylester = 8/2) chromatographiert. Man erhielt 3,80 g 2-Chlor-3-(N-ethoxy-N-methyl-aminocarbonyl)-4-methylsulfonyl-benzoesäuremethylester.

### 2,4-Dichlor-3-(N-ethyl-N-propoxy)-aminocarbonyl-benzoylchlorid (Verbindung 3.21)

Stufe a) 2,4-Dichlor-3-methyl-acetophenon
   Zu einer Lösung von 502,0 g (3,12 mol) 2,6-Dichlortoluol und 408,0 g (3,06 mol) Aluminiumtrichlorid wurden bei 100°C unter Rühren 235,0 g (3,0 mol) Acetylchlorid über einen Zeitraum von 2 Stunden zugetropft. Nach 2 Stunden Rühren bei 100-105°C kühlte man ab und goß das Reaktionsgemisch auf 3 l Eis und 1 l Wasser. Der dabei ausgefallene Feststoff wurde abgesaugt und mit 800 ml Wasser neutral gewaschen. Nach dem Trocknen bei 40°C erhielt man 500,0 g 2,4-Dichlor-3-methyl-acetophenon als Rohprodukt, das anschließend im Hochvakuum destilliert wurde.
   (Sdp.: 121-128°C (4 mbar))
Stufe b) 2,4-Dichlor-3-methyl-benzoesäure
   In eine Lösung von 520,0 g (13 mol) Natriumhydroxid in 2600 ml Wasser wurden bei 0-10°C zunächst 655,2 g (4,1 mol) Brom und anschließend 203,0 g (1.0 mol) 2,4-Dichlor-3-methyl-acetophenon in 1300 ml 1,4-Dioxan zugetropft. Nach 12 Stunden Rühren trennte man die organische Phase ab, versetzte die wäßrige Phase mit einer 30 %igen Lösung, dargestellt aus Natriumpyrosulfit und Wasser, und stellte mit Salzsäure einen pH-Wert von 1 ein. Der ausgefallene Niederschlag wurde abgesaugt, mit Wasser gewaschen und am Vakuum bei 60°C getrocknet. Man erhielt 197,0 g 2,4-Dichlor-3-methyl-benzoesäure.
   (Fp.: 173-175°C)
Stufe c) 2.4-Dichlor-3-methyl-benzoesäuremethylester
   Zu einer Lösung von 424,0 g (2 mol) 2,4-Dichlor-3-methylbenzoesäure und 1500 ml Methanol wurden 60 ml konz. Schwefelsäure getropft. Nach 5 Stunden Erhitzen unter Rückfluß wurde das Reaktionsgemisch abgekühlt, am Vakuum eingeengt und anschließend in 1000 ml Methylenchlorid aufgenommen. Die organische Phase wurde mit Wasser, anschließend mit 5 %iger Natriumhydrogencarbonat-Lösung und dann wiederum mit Wasser gewaschen, getrocknet und am Vakuum eingeengt. Mari erhielt 401,0 g 2,4-Dichlor-3-methyl-benzoesäuremethylester.
   (Sdp: 103-107°C (1-1,5 mbar))
Stufe d) 3-Brommethyl-2,4-dichlor-benzoesäuremethylester
   Zu einer Lösung von 84,0 g (0,38 mol) 2,4-Dichlor-3-methyl-benzoesäuremethylester und 67,6 g (0,38 mol) N-Bromsuccinimid in 380 ml Tetrachlorkohlenstoff wurde 1,0 g Azobisisobutyronitril gegeben. Nach 3,5 Stunden Erhitzen unter Rückfluß wurde das Reaktionsgemisch abgekühlt und der gebildete Niederschlag abgesaugt. Das Filtrat wurde am Vakuum eingeengt und der resultierende Rückstand aus Methyl-tert.butylether ausgerührt. Man erhielt 108,0 g 3-Brommethyl-2,4-dichlor-benzoesäuremethylester.
   (Fp.: 51-54°C)
Stufe e) 2,4-Dichlor-3-formyl-benzoesäuremethylester
   Zu einer Lösung von 312,0 g (0,99 mol) 3-Brommethyl-2,4-dichlor-benzoesäuremethylester in 2 l Acetonitril wurden unter Rückfluß 696,2 g (2,97 mol) wäßrige 50 %ige N-Methylmorpholin-N-oxid-Lösung getropft. Nach 48 Stunden Rühren bei Raumtemperatur wurde die Reaktionslösung in 6 l Wasser eingerührt. Der ausgefallene Niederschlag wurde abgesaugt, mit Wasser gewaschen und am Vakuum getrocknet. Man erhielt 141,3 g 2,4-Dichlor-3-formylbenzoesäuremethylester.
   (¹H-NMR (CDCl₃, δ in ppm): 3,98 (s, 3H); 7,47 (d, 1H); 7,84 (d. 1H); 10,48 (s, 1H) .)
Stufe f) 2,4-Dichlor-3-hydroxycarbonyl-benzoesäuremethylester
   Zu einer Lösung von 40,0 g (0,172 mol) 2,4-Dichlor-3-formyl-benzoesäuremethylester und 500 ml Acetonitril wurden bei 5°C nacheinander 5,9 g (0,043 mol) Natriumdihydrogenphosphatmonohydrat in 70 ml Wasser, 20,5 g (0,181 mol) 30 %ige Wasserstoffperoxid-Lösung und 27,3 g (0,241 mol) 80 %ige Natriumchloritlösung gegeben. Die Reaktionslösung wurde 1 Stunde bei 5°C und 12 Stunden bei Raumtemperatur gerührt. Anschließend wurde mit 10 %iger Salzsäure ein pH-Wert von 1 eingestellt und 500 ml 40 %ige Natriumhydrogensulfit-Lösung zugegeben. Nach 1 Stunde Rühren bei Raumtemperatur wurde die wäßrige Phase dreimal mit Essigsäureethylester extrahiert, die vereinigten organischen Phasen mit 1,0 l 10 %iger Natriumhydrogensulfit-Lösung gewaschen und anschließend getrocknet. Nach Abdestillieren des Lösungsmittels erhielt man 40,0 g 2,4-Dichlor-3-hydroxycarbonyl-benzoesäuremethylester.
   (¹H-NMR (d⁶-DMSO, δ in ppm): 3,90 (s, 3H); 7,69 (d, 1H); 7,89 (d, 1H).)
Stufe g) 3-Chlorcarbonyl-2,4-dichlor-benzoesäuremethylester
   Zu einer Lösung von 5,00 g (0,02 mol) 2,4-Dichlor-3-hydroxycarbonyl-benzoesäuremethylester und 50 ml trockenen Toluol wurden 2 Tropfen Dimethylformamid und 11,90 g (0,1 mol) Thionylchlorid gegeben. Die Lösung wurde 4 Stunden unter Rückfluß erhitzt. Nach Abdestillieren des Lösungsmittels erhielt man 5,35 g 3-Chlorcarbonyl-2,4-dichlor-benzoesäuremethylester.
Stufe h) 2,4-Dichlor-3-(N-propoxy)-aminocarbonyl-benzoesäuremethylester (Verbindung 3.17)
   Zu einer Lösung von 10,70 g (0.040 mol) 3-Chlor-carbonyl-2,4-dichlor-benzoesäuremethylester und 200 ml Dichlormethan wurden 4,05 g (0,040 mol) Triethylamin und 4,50 g (0,040 mol) Propoxyamin-Hydrochlorid gegeben. Nach 2 Stunden Rühren bei Raumtemperatur wurde die Reaktionslösung mit verdünnter Phosphorsäure gewaschen, getrocknet und eingeengt. Der erhaltene Rückstand wurde an Kieselgel (Eluent: Toluol/Essigsäureethylester = 9/1) chramatographiert. Man erhielt 2,4-Dichlor-3-(N-propoxy)-aminocarbonyl-benzoesäuremethylester.
Stufe i) 2,4-Dichlor-3-(N-ethyl-N-propoxy)-aminocarbonyl-benzoesäuremethylester (Verbindung 3.14)
   Ein Gemisch von 12,50 g (0,041 mol) 2,4-Dichlor-3-(N-propoxy)-aminocarbonyl-benzoesäuremethylester und 11,30 g (0,082 mol) Kaliumcarbonat in 100 ml Dimethylformamid wurde 30 Minuten bei Raumtemperatur gerührt. Anschließend wurden 19,20 g (0,123 mol) Ethyliodid zugetropft. Nach 5 Stunden erhitzen auf 50°C wurde das Reaktionsgemisch abgekühlt und in 1 1 Eiswasser eingerührt. Nun wurde die wäßrige Phase mit Essigsäureethylester extrahiert, die vereinigten organischen Phasen getrocknet und das Lösungsmittel am Vakuum abdestilliert. Nach Chramatographie des Rückstandes an Kieselgel (Eluent: Toluol/Essigsäureethylester = 9/1) erhielt man 7,00 g
   2,4-Dichlor-3-(N-ethyl-N-propoxy)-aminocarbonyl-benzoesäuremethylester.
   (Fp.: 48-50°C).
Stufe j) 2,4-Dichlor-3-(N-ethyl-N-propoxy)-aminocarbonyl-benzoesäure (Verbindung 3.18)
   Eine Lösung von 7,00 g (0,021 mol) 2,4-Dichlor-3-(N-ethyl-N-propoxy)-aminocarbonyl-benzoesäuremethylester und 40 ml 10 %ige wäßrige Natriumhydroxidlösung wurde 2 Stunden bei 80°C gerührt. Nach Abkühlen wurde das Reaktionsgemisch in 200 ml Eiswasser eingerührt und mit konzentrierter Salzsäure auf pH = 1 gestellt. Die wäßrige Phase wurde mit Essigsäureethylester extrahiert, die vereinigten organischen Phasen getrocknet und am Vakuum eingeengt. Man erhielt 5,50 g 2,4-Dichlor-3-(N-ethyl-N-propoxy)-aminocarbonyl-benzoesäure.
Stufe k) 2,4-Dichlor-3-(N-ethyl-N-propoxy)-aminocarbonyl-benzoylchlorid (Verbindung 3.21)
   Eine Lösung von 4,00 g (0,0125 mol) 2,4-Dichlor-3-(N-ethyl-N-propoxy)-aminocarbonyl-benzoesäure und 14,90 g Thionylchlorid in 100 ml trockenem Toluol wurde 3 Stunden bei 100°C gerührt. Nach dem Entfernen des Lösungsmittels am Vakuum erhielt man 4,40 g 2,4-Dichlor-3-(N-ethyl-N-propoxy)-aminocarbonyl-benzoylchlorid.

### 2,4-Dichlor-3-(N-methoxy)-aminocarbonyl-benzoesäuremethylester (Verbindung 3.01)

Zu einer Lösung von 5,35 g (0,02 mol) 3-Chlorcarbonyl-2,4-dichlor-benzoesäuremethylester und 100 ml Dichlormethan wurden 4,60 g (0,045 mol) Triethylamin und 3,75 g (0,045 mol) Methoxyaminhydrochlorid gegeben. Nach 12 Stunden Rühren bei Raumtemperatur wurde die Reaktionslösung mit verdünnter Phosphorsäure gewaschen, getrocknet und eingeengt. Der erhaltene Rückstand wurde in Diethylether ausgerührt. Man erhielt 4,80 g
2,4-Dichlor-3-(N-methoxy)-aminocarbonyl-benzoesäuremethylester. (Fp.: 162-164°C)

### 2,4-Dichlor-3-(N-propoxy)-aminocarbonyl-benzoesäuremethylester (Verbindung 3.02)

Zu einer Lösung von 4,50 g (0,04 mol) Propoxyaminhydrochlorid und 4.05 g (0,04 mol) Triethylamin in 200 ml Methylenchlorid wurden langsam bei 30°C 10,7 g (0,04 mol) 3-Chlorcarbonyl-2,4-dichlorbenzoesäuremethylester in 100 ml Methylenchlorid getropft. Nach 2 Stunden Rühren bei Raumtemperatur wurde das Reaktionsgemisch mit verdünnter Phosphorsäure gewaschen, getrocknet und eingeengt. Der erhaltene Rückstand wurde an Kieselgel chromatographiert (Eluent: Toluol/Essigsäureethylester = 9/1). Man erhielt 11,50 g
2,4-Dichlor-3-(N-propoxyamino)-carbonyl-benzoesäuremethylester. (Fp.: 80-81°C)

### 3-(N-4-Chlorbenzyloxy)-aminocarbonyl-2,4-dichlor-benzoesäuremethylester (Verbindung 3.03)

Zu einer Lösung von 7,76 g (0,04 mol) 4-Chlorbenzyloxyaminhydrochlorid und 4,05 g (0,04 mol) Triethylamin in 200 ml Methylenchlorid wurden langsam bei ca. 30°C 10,70 g (0,04 mol) 3-Chlorcarbonyl-2,4-dichlor-benzoesäuremethylester in 50 ml Methylenchlorid getropft. Nach 12 Stunden Rühren bei Raumtemperatur wurde das Reaktionsgemisch mit verdünnter Phosphorsäure gewaschen, getrocknet und eingeengt. Nach Ausrühren des Rückstandes mit Diethylether erhielt man 19,00 g 3-(4-Chlorbenzyloxy)-aminocarbonyl-2,4-dichlor-benzoesäuremethylester.
(Fp.: 120-121°C)

In nachfolgender Tabelle 3 sind neben den voranstehend beschriebenen Verbindungen weitere Benzoesäurederivate der Formel IIIa aufgeführt, die in analoger Weise hergestellt wurden oder herstellbar sind.

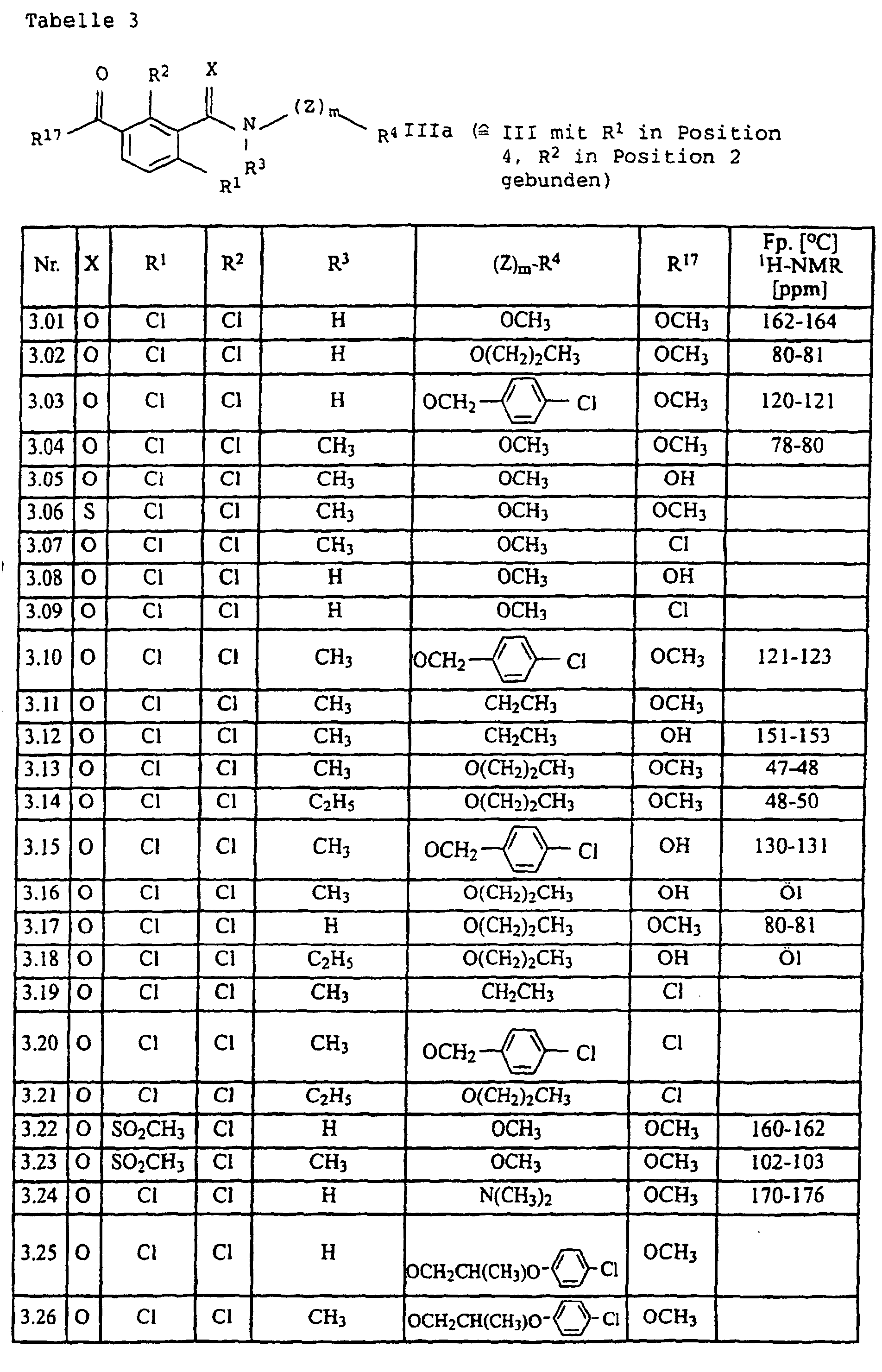

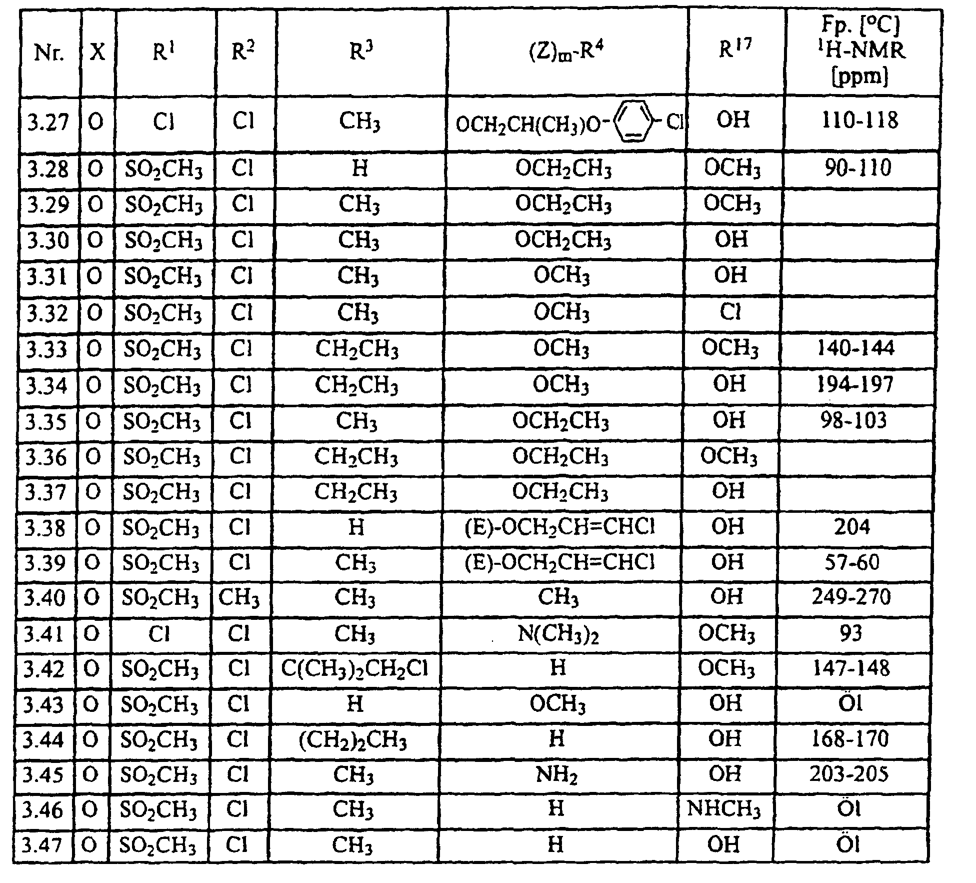

Die 2-Benzoyl-cyclohexan-1,3-dione der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes syl estre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Õl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat. Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II. 20 Gewichtsteile der Verbindung Nr. 2.03 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen wasser erhält man eine wäßrige Dispersion, die 0,02 Gew.-% des Wirkstoffs enthält.
III. 20 Gewichtsteile des Wirkstoffs Nr. 2.05 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.06 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.09 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.11 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung 2.12 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung 2.07 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadrazole, 1,3,4-Thiadiazole, Amide, Aminophosphorsäure und deren Derivate, Aminotriazole, Anilide, (Het)-Aryloxyalkansäuren und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximether, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 2-Benzoyl-cyclohexan-1,3-dione der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauf laufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,125 bzw. 0,0625 kg/ha a. S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Englischer Name | Deutscher Name |
|---|---|---|
| Chenopodium album | lambsguarters (goosefoot) | Weißer Gänsefuß |
| Ipomoea spp. | morning glory | Prunkwindenarten |
| Polygenum persicaria | ladysthumb | Plöhknöterich |
| Solanum nigrum | black nightshade | Schwarzer Nachtschatten |
| Zea mays | Indian corn | Mais |

Bei Aufwendungen von 0,125 bzw. 0,0625 kg/ha (a. S.) zeigte die Verbindung 2.01 (Tabelle 2) im Nachauflauf eine sehr gute Wirkung gegen oben genannte mono- und dikotyle Schadpflanzen und gute Verträglichkeit in Mais.

## Patentansprüche

1. 2-Benzoyl-cyclohexan-1,3-dione der Formel I 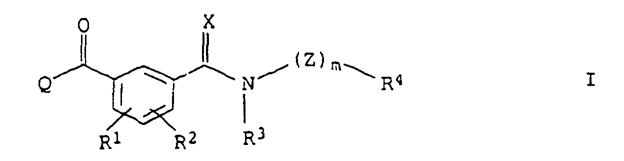 in der die Variablen folgende Bedeutung haben:
R¹, R² Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵, -OCOR⁶, -OSO₂R⁶, -SH, -S(O)ₙR⁷, -SO₂OR⁵, -SO₂NR⁵R⁸, -NR⁸SO₂R⁶ oder -NR⁸COR⁶;
R³ Wasserstoff, C₁-C₆-Alkyl, C₁-C₆-Halogenalkyl, C₃-C₆-Alkenyl oder C₃-C₆-Alkinyl;
R⁴ Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, NR⁸R¹⁰, =NOR¹⁰, -OCOR¹⁰, -SCOR¹⁰_{,} -NR⁸COR¹⁰_{,} -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Phenyl-C₁-C₄-alkyl, Hetaryl, Phenoxy, Phenyl-C₁-C₄-alkoxy und Hetaryloxy, wobei die acht letztgenannten Reste ihrerseits substituiert sein können;
X Sauerstoff oder Schwefel;
Z Sauerstoff oder NR⁸;
m 0 oder 1;
n 0, 1 oder 2;
R⁵ Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁶ C₁-C₆-Alkyl oder C₁-C₆-Halogenalkyl ;
R⁷ C₁-C₆-Alkyl, C₁-C₆-Halogenalkyl, C₁-C₆-Alkoxy-C₂-C₅-alkyl, C₃-C₆-Alkenyl oder C₃-C₆-Alkinyl;
R⁸ Wasserstoff oder C₁-C₆-Alkyl;
R⁹ C₁-C₆-Alky- C₃-C₆-Alkenyl, C₃-C₆-Alkinyl, Phenyl oder Benzyl;
R¹⁰ C₁-C₆-Alkyl, C₁-C₆-Halogenalkyl, C₃-C₆-Alkenyl oder C₃-C₆-Alkinyl;
Q ein gegebenenfalls substituierter in 2-Stellung verknüpfter Cyclohexan-1,3-dionring;
wobei m 1 bedeutet, wenn R³ für Wasserstoff steht;
sowie deren landwirtschaftlich brauchbaren Salze.

2. 2-Benzoyl-cyclohexan-1,3-dione der Formel I gemäß Anspruch 1, in der Q ein in 2-Stellung verknüpfter Cyclohexan-1,3-dion-ring der Formel II 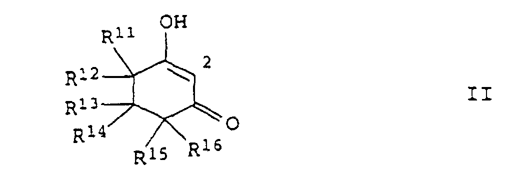 ist, wobei
R¹¹, R¹², R¹⁴ und R¹⁶ für Wasserstoff oder C₁-C₄-Alkyl stehen;
R¹³ für Wasserstoff, C₁-C₄-Alkyl oder C₃-C₄-Cycloalkyl steht, wobei die beiden letztgenannten Gruppen einen bis drei der folgenden Substituenten tragen können; Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,4-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
R¹⁵ für Wasserstoff, C₁-C₄-Alkyl oder C₁-C₆-Alkoxycarbonyl steht;
oder
R¹³ und R¹⁶ gemeinsam eine π-Bindung oder einen drei- bis sechsgliedrigen carbocyclischen Ring bilden;
oder
die CR¹³R¹⁴-Einheit durch C=O ersetzt sein kann.

3. 2-Benzoyl-cyclohexan-1,3-dione der Formel I gemäß Anspruch 1 oder 2 in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷ bedeutet;
R² für Wasserstoff oder einen wie voranstehend unter R¹ genannten Rest steht.

4. 2-Benzoyl-cyclohexan-1,3-dione der Formel I gemäß den Ansprüchen 1 bis 3, in der m für 1 steht.

5. 2-Benzoyl-clohexan-1,3-dione der Formel Ia 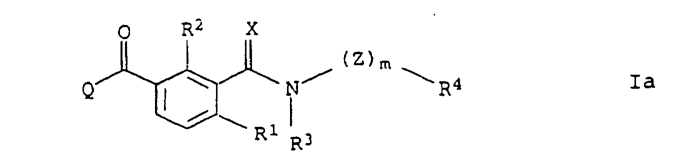 in der die Variablen R¹ bis R⁴, Q, X, Z und m die unter den Ansprüchen 1 bis 4 genannte Bedeutung haben.

6. Verfahren zur Herstellung von 2-Benzoyl-cyclohexan-1,3-dionen der Formel I gemäß den Ansprüchen 1 bis 5, **dadurch gekennzeichnet, daß** man ein gegebenenfalls substituiertes Cyclohexan-1,3-dion Q mit einer aktivierten Carbonsäure IIIα oder mit einer Carbonsäure IIIβ, 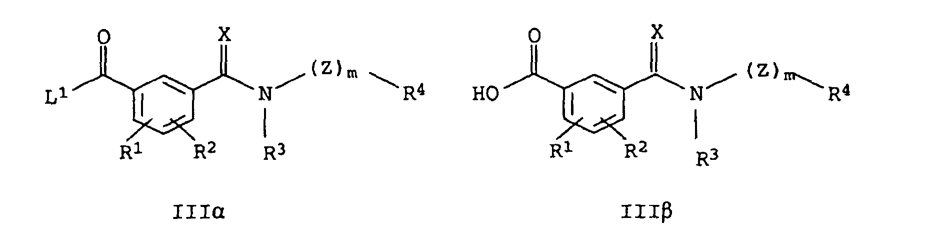 wobei die Variablen R¹ bis R⁴, X, Z und m die unter Anspruch 1 genannte Bedeutung haben und L¹ für eine nucleophil verdrängbare Abgangsgruppe steht, acyliert und das Acylierungsprodukt gegebenenfalls in Gegenwart eines Katalysators zu den Verbindungen I umlagert.

7. Benzoesäurederivate der Formel IIIa 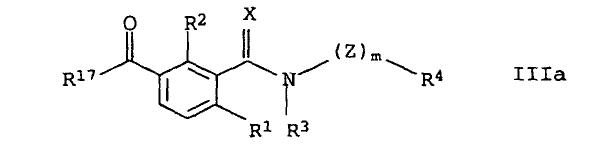 wobei
R¹, R² Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
m 1;
R¹⁷ Hydroxy oder einen abhydrolysierbaren Rest bedeutet
und
die Variablen R³ bis R⁵ und R⁷ bis R¹⁰, X und Z sowie n die in Anspruch 1 genannte Bedeutung haben;
wobei R⁴ nicht für Methyl steht, wenn Z für Sauerstoff, R¹ für Methylsulfonyl, R² für Chlor, Methyl oder Methoxy und R³ für Methyl stehen.

8. Benzoesäurederivate der Formel IIIa nach Anspruch 7, wobei R¹⁷ Halogen, Hydroxy oder C₁-C₆-Alkoxy bedeutet.

9. Mittel, enthaltend eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 5 und für die Formulierung von Pflanzenschutzmitteln übliche Hilfsmittel.

10. Verfahren zur Herstellung von herbizid wirksamen Mitteln gemäß Anspruch 9, **dadurch gekennzeichnet, daß** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 5 und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 5 auf Pflanzen, deren Lebensraum und/oder auf Samen einwirken läßt.

12. Verwendung der 2-Benzoyl-cyclohexan-1,3-dione der Formel I und deren landwirtschaftlich brauchbaren Salze gemäß den Ansprüchen 1 bis 5 als Herbizide.

## Claims

1. A 2-benzoylcyclohexane-1,3-dione of the formula I 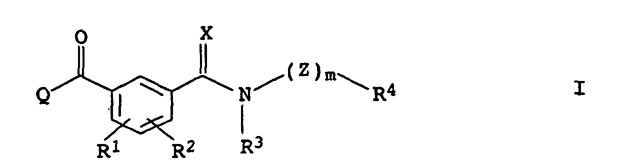 where the variables have the following meanings:
R¹, R² are hydrogen, nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵, -OCOR⁶, -OSO₂R⁶, -SH, -S(O)ₙR⁷, -SO₂OR⁵, -SO₂NR⁵R⁸, -NR⁸SO₂R⁶ or -NR⁸COR⁶;
R³ is hydrogen, C₁-C₆-alkyl, C₁-C₆-haloalkyl, C₃-C₆-alkenyl or C₃-C₆-alkynyl;
R⁴ is hydrogen, C₁-C₆-alkyl, C₃-C₆-cycloalkyl, C₃-C₆-alkenyl, C₄-C₆-cycloalkenyl, C₃-C₆-alkynyl, -COR⁹, -CO₂R⁹, -COSR⁹ or -CONR⁸R⁹, it being possible for the abovementioned alkyl, cycloalkyl, alkenyl, cycloalkenyl and alkynyl radicals and for R⁹ of the radicals -COR⁹, -CO₂R⁹, -COSR⁹ and -CONR⁸R⁹ to be partially or fully halogenated and/or to have attached to them one to three of the following groups:
hydroxyl, mercapto, amino, cyano, R¹⁰, -OR¹⁰, - SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, phenyl-C₁-C₄-alkyl, hetaryl, phenoxy, phenyl-C₁-C₄-alkoxy and hetaryloxy, it being possible for the eight last-mentioned radicals, in turn, to be substituted;
X is oxygen or sulfur;
Z is oxygen or NR⁸;
m is 0 or 1;
n is 0, 1 or 2;
R⁵ is hydrogen, C₁-C₆-alkyl, C₁-C₆-haloalkyl, C₁-C₆-alkoxy-C₂-C₆-alkyl, C₃-C₆-alkenyl or C₃-C₆-alkynyl;
R⁶ is C₁-C₆-alkyl or C₁-C₆-haloalkyl;
R⁷ is C₁-C₆-alkyl, C₁-C₆-haloalkyl, C₁-C₆-alkoxy-C₂-C₆-alkyl, C₃-C₆-alkenyl or C₃-C₆-alkynyl;
R⁸ is hydrogen or C₁-C₆-alkyl;
R⁹ is C₁-C₆-alkyl, C₃-C₆-alkenyl, C₃-C₆-alkynyl, phenyl or benzyl;
R¹⁰ is C₁-C₆-alkyl, C₁-C₆-haloalkyl, C₃-C₆-alkenyl or C₃-C₆-alkynyl;
Q is an unsubstituted or substituted cyclohexane-1,3-dione ring which is linked in the 2-position;
where m is 1 if R³ is hydrogen;
or an agriculturally useful salt thereof.

2. A 2-benzoylcyclohexane-1,3-dione of the formula I as claimed in claim 1 where Q is a cyclohexane-1,3-dione ring of the formula II 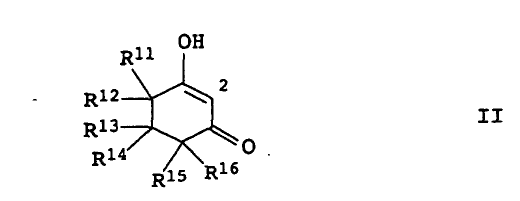 which is linked in the 2-position, where
R¹¹, R¹², R¹⁴ and R¹⁶ are hydrogen or C₁-C₄-alkyl;
R¹³ is hydrogen, C₁-C₄-alkyl or C₃-C₄-cycloalkyl, it being possible for the two last-mentioned groups to have attached to them one to three of the following substituents:
halogen, C₁-C₄-alkylthio or C₁-C₄-alkoxy;
or
is tetrahydropyran-2-yl, tetrahydropyran-3-yl, tetrahydropyran-4-yl, tetrahydrothiopyran-2-yl, tetrahydrothiopyran-3-yl, tetrahydrothiopyran-4-yl, 1,3-dioxolan-2-yl, 1,3-dioxan-2-yl, 1,4-dioxan-2-yl, 1,3-oxathiolan-2-yl, 1,3-oxathian-2-yl, 1,3-dithiolan-2-yl or 1,3-dithian-2-yl, it being possible for the 6 last-mentioned radicals to be substituted by one to three C₁-C₄-alkyl radicals;
R¹⁵ is hydrogen, C₁-C₄-alkyl or C₁-C₆-alkoxycarbonyl;
or
R¹³ and R¹⁶ together form a π bond or a three to six-membered carbocyclic ring;
or
the CR¹³R¹⁴ unit can be replaced by C=O.

3. A 2-benzoylcyclohexane-1,3-dione of the formula I as claimed in claim 1 or 2 where
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵ or -S(O)ₙR⁷;
R² is hydrogen or a radical as mentioned above for R¹.

4. A 2-benzoylcyclohexane-1,3-dione of the formula I as claimed in any of claims 1 to 3, where m is 1.

5. A 2-benzoylcyclohexane-1,3-dione of the formula Ia 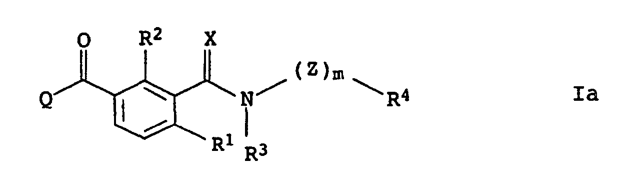 where the variables R¹ to R⁴, Q, X, Z and m have the meanings given in claims 1 to 4.

6. A process for the preparation of 2-benzoylcyclohexane-1,3-diones of the formula I as claimed in any of claims 1 to 5, which comprises acylating an unsubstituted or substituted cyclohexane-1,3-dione Q with an activated carboxylic acid IIIα or with a carboxylic acid IIIβ, 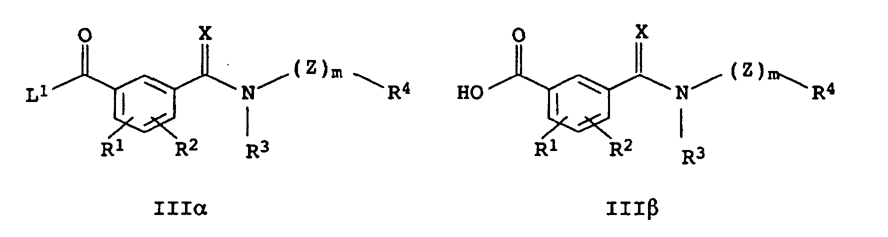 where the variables R¹ to R⁴, X, Z and m have the meanings given in claim 1 and L¹ is a nucleophilically displaceable leaving group, and, if appropriate, subjecting the acylation product to a rearrangement reaction in the presence of a catalyst to give the compounds I.

7. A benzoic acid derivative of the formula IIIa 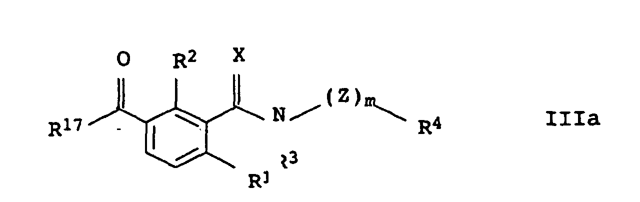 where
R¹, R² are nitro, halogen, cyano, thiocyanato, C₁-C₆-alkyI, C₁-C₂-haloalkyl, C₂-C₆-alkoxy-C₁-C₆-alkyl, C₂-C₆-alkenyl, C₂-C₆-alkynyl, -OR⁵ or -S(O)ₙR⁷;
m is 1;
R¹⁷ is hydroxyl or a radical which can be removed by hydrolysis and
the variables R³ to R⁵ and R⁷ to R¹⁰, X, Z and n have the meanings given in claim 1;
where R⁴ is not methyl if Z is oxygen, R¹ is methylsulfonyl, R² is chlorine, methyl or methoxy and R³ is methyl.

8. A benzoic acid derivative of the formula IIIa as claimed in claim 7, where R¹⁷ is halogen, hydroxyl or C₁-C₆-alkoxy.

9. A composition comprising a herbicidally active amount of at least one 2-benzoylcyclohexane-1,3-dione of the formula I or of an agriculturally useful salt of I as claimed in any of claims 1 to 5 and auxiliaries conventionally used for the formulation of crop protection products.

10. A process for the preparation of herbicidally active composition as claimed in claim 9, which comprises mixing a herbicidally active amount of at least one 2-benzoyl-cyclohexane-1,3-dione of the formula I or of an agriculturally useful salt of I as claimed in any of claims 1 to 5 and auxiliaries conventionally used for the formulation of crop protection products.

11. A method of controlling undesirable vegetation, which comprises allowing a herbicidally active amount of at least one 2-benzoylcyclohexane-1,3-dione of the formula I or of an agriculturally useful salt of I as claimed in any of claims 1 to 5 to act on plants, their environment and/or on seeds.

12. The use of a 2-benzoylcyclohexane-1,3-dione of the formula I or of an agriculturally useful salt thereof as claimed in any of claims 1 to 5 as a herbicide.

## Revendications

1. 2-benzoyl-cyclohexane-1 ,3-diones de formule 1 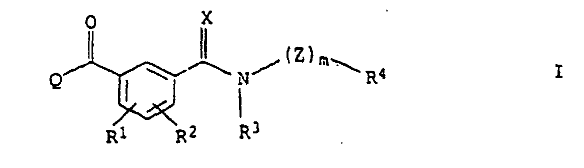 dans laquelle les variables prennent la signification suivante :
R¹_{,} R² représentent un atome d'hydrogène, un groupe nitro, un atome d'halogène, un groupe cyano, rhodano, alkyle en C₁ à C₆, halogénoalkyle en C₁ à C₆, (alcoxy en C₁ à C₆)-(alkyle en C₁ à C₆), alcényle en C₂ à C₆, alcynyle en C₂ à C₆, -OR⁵, -OCOR⁶, -OSO₂R⁶, -SH, -S(O)ₙR⁷_{,} -SO₂OR⁵, -SO₂NR⁵R⁸ , -NR⁸SO₂R⁶ ou -NR⁸COR⁶;
R³ représente un atome d'hydrogène, un groupe alkyle en C₁ à C₆, halogénoalkyle en C₁ à C₆, alcényle en C₃ à C₆, ou alcynyle en C₃ à C₆;
R⁴ représente un atome d'hydrogène, un groupe alkyle en C₁ à C₆, cycloalkyle en C₃ à C₆, alcényle en C₃ à C₆, cycloalcényle en C₄ à C₆, alcynyle en C₃ à C₆, -COR⁹, -CO₂R⁹_{,} -COSR⁹_{,} ou -CONR⁸R⁹_{,} les résidus alkyle, cycloalkyle, alcényle, cycloalcényle, et alcynyle, cités ainsi que R⁹ des résidus -COR⁹, -CO₂R⁹, -COSR⁹ et -CONR⁸R⁹ pouvant être partiellement ou complètement halogénés, et/ou pouvant porter d'un à trois des groupes suivants :
hydroxy, mercapto, amino, cyano, R¹⁰_{,} -OR¹⁰, -SR¹⁰, -NR⁸ R¹⁰, =NOR¹⁰, -OCOR¹⁰, -SCOR¹⁰, -NR⁸COR¹⁰, -CO₂R¹⁰, -COSR¹⁰, -CONR⁸R¹⁰, alkylimino-oxy en C₁ à C₄, alcoxyamino en C₁ à C₄, (alkyle en C₁ à C₄)-carbonyle, (alcoxy en C₁ à C₄)-(alcoxy en C₂ à C₆)-carbonyle, alkylsulfonyle en C₁ à C₄, hétérocyclyle, hétérocyclyloxy, phényle, phényl-(alkyle en C₁ à C₄), hétéroaryle, phénoxy, phényl-(alcoxy en C₁ à C₄) et hétéroaryloxy, les huit résidus cités en dernier pouvant être de leur côté substitués;
X représente un atome d'oxygène ou de soufre:
Z représente un atome d'oxygène ou un groupe NR⁸;
m vaut 0 ou 1 ;
n vaut 0, 1 ou 2;
R⁵ représente un atome d'hydrogène, un groupe alkyle en C₁ à C₆, halogénoalkyle en C₁ à C₆, (alcoxy en C₁ à C₆)-(alkyle en C₂ à C₆), alcényle en C₃ à C₆ ou alcynyle en C₃ à C₆;
R⁶ représente un groupe alkyle en C₁ à C₆ ou halogénoalkyle en C₁ à C₆;
R⁷ représente un groupe alkyle en C₁ à C₆, halogénoalkyle en C₁ à C₆, (alcoxy en C₁ à C₆)-(alkyle en C₂ à C₆), alcényle en C₃ à C₆ ou alcynyle en C₃ à C₆;
R⁸ représente un atome d'hydrogène ou un groupe alkyle en C₁ à C₆;
R⁹ représente un groupe alkyle en C₁ à C₆, alcycényle en C₃ à C₆, alcynyle en C₃ à C₆, phényle ou benzyle;
R¹⁰ représente un groupe alkyle en C₁ à C₆, halogénoalkyle en C₁ à C₆, alcényle en C₃ à C₆ ou alcynyle en C₃ à C₆;
Q représente un cycle cyclohexane-1,3-dione lié, en position 2, éventuellement substitué;
dans laquelle m vaut 1 lorsque R³ représente un atome d'hydrogène;
ainsi que leurs sels utilisables en agriculture.

2. 2-benzoyl-cyclohexane-1,3-diones de formule I selon la revendication 1, dans lequel Q est un cycle cyclohexane-1,3-diones lié en position 2 de formule II 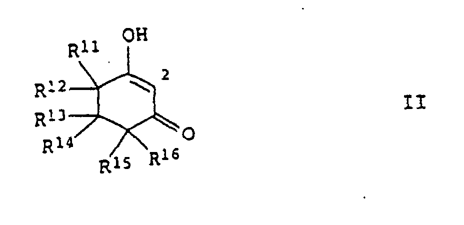 dans laquelle
R¹¹, R¹², R¹⁴ et R¹⁶ représentent un atome d'hydrogène ou un groupe alkyle en C₁ à C₄;
R¹³ représente un atome d'hydrogène, un groupe alkyle en C₁ à C₄ ou cycloalkyle en C₃ à C₄, les deux groupes cités en dernier pouvant porter de un jusqu'à trois des substituants suivants :
un atome d'halogène, un groupe alkylthio en C₁ à C₄ ou alcoxy en C₁ à C₄;
ou bien
représente un groupe tétrahydropyran-2-yle, tétrahydropyran-3-yle, tétrahydropyran-4-yle, tétrahydrothiopyran-2-yle, tétrahydrothiopyran-3-yte, tétrahydrothiopyran-4-yle, 1,3-dioxolan-2-yle, 1,3-dioxan-2-yle, 1,4-dioxan-2-yle, 1,3-oxathiolan-2-yle, 1,3-oxathian-2-yle, 1,3-dithiolan-2-yle, ou 1,3-dithian-2-yle, les six résidus cités en dernier pouvant être substitués par un jusqu'à trois résidus alkyle en C₁ à C₄;
R₁₅ représente un atome d'hydrogène, un groupe alkyle en C₁ à C₄, ou (alcoxy en C₁ à C₆)-carbonyle;
ou bien
R¹³ et R¹⁶ forment ensemble une liaison π ou un cycle carbocyclique de trois à six maillons;
ou bien
le motif CR¹³R¹⁴ peut être remplacé par C=O.

3. 2-benzoyl-cyclohexane-1,3-diones de formule | selon la revendication 1 ou 2 dans lesquelles
R¹ représente un groupe nitro, un atome d'halogène, un groupe cyano, rhodano, alkyte, en C₁ à C₆, halogénoalkyle en C₁ à C₆, (alcoxy en C₁ à C₆)-(alkyle en C₁ à C₆); alcényle en C₂ à C₆, alcynyle en C₂ à C₆, -OR⁵ ou - S(O)ₙ R⁷;
R² représente un atome d'hydrogène ou un résidu tel que cité précédemment sous R¹.

4. 2-benzoyl-cyclohexane-1,3-diones de formule I selon les revendications 1 à 3, dans lesquelles m vaut 1.

5. 2-benzoyl-cyclohexane-1,3-diones de formule la 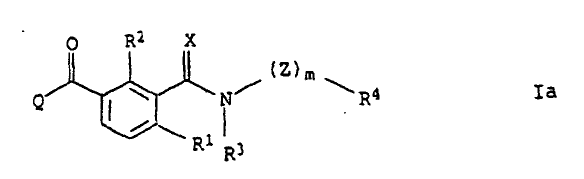 dans laquelle les variables R¹ à R⁴, Q, X, Z et m prennent la signification citée sous les revendications 1 à 4.

6. Procédé de préparation de 2-benzoyl-cyclohexane-1,3-diones de formule I selon les revendications 1 à 5, **caractérisé en ce que** l'on acyle une cyclohexane-1,3-dione Q éventuellement substituée, avec un acide carboxylique activé IIIα ou avec un acide carboxylique IIIβ, 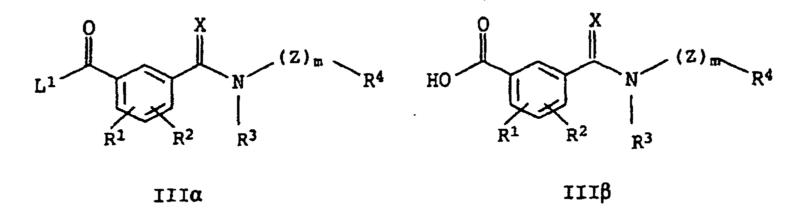 dans lesquelles les variables R¹ à R⁴, X, Z et m prennent la signification citée sous la revendication 1 et L¹ représente un groupe sortant déblocable de façon nucléophile, et l'on transpose le produit d'acylation en les composés l éventuellement en présence d'un catalyseur.

7. Dérivés de l'acide benzoïque de formule IIIa 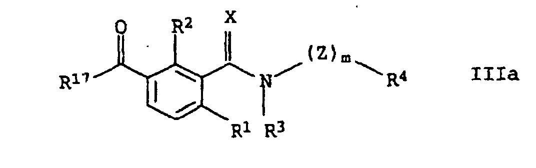 dans laquelle
R¹_{,} R² représentent un groupe nitro, un atome d'halogène, un groupe cyano, rhodano, alkyle en C₁ à C₆, halogénoalkyle en C₁ à C₆, (alcoxy en C₁ à C₆)-(alkyle en C₁ à C₆), alcényle en C₂ à C₆, alcynyle en C₂ à C₆, -OR⁵ ou -S(O)ₙR⁷;
m vaut 1;
R¹⁷ représente un groupe hydroxy ou un résidu pouvant être éliminé par hydrolyse et
les variables R³ à R⁵ et R⁷ à R¹⁰, X et Z ainsi que n prennent la signification citée dans la revendication 1;
dans laquelle R⁴ ne représente pas un groupe méthyle, lorsque Z représente un atome d'oxygène, R¹ représente un groupe méthylsulfonyle, R² représente un atome de chlore, un groupe méthyle ou méthoxy, et R³ représente un groupe méthyle.

8. Dérivés de l'acide benzoïque de formule IIIa selon la revendication 7, dans laquelle R¹⁷ représente un atome d'halogène, un groupe hydroxy ou alcoxy en C₁ à C₆.

9. Agent, contenant une quantité efficace en tant qu'herbicide, d'au moins une 2-benzoyl-cyclohexane-1,3-dione de formule I, ou d'un sel de utilisable en agriculture selon les revendications 1 à 5 et des adjuvants courants pour la formulation d'agents phytosanitaires.

10. Procédé de préparation d'agents efficaces en tant qu'herbicides selon la revendication 9, **caractérisé en ce que** l'on mélange une quantité efficace en tant qu'herbicide, d'au moins une 2-benzoyl-cyclohexane-1,3-dione de formule l ou d'un sel de | utilisable en agriculture, selon les revendications 1 à 5, et des adjuvants courants pour la formulation d'agents phytosanitaires.

11. Procédé pour lutter contre une croissance végétale non souhaitée, **caractérisé en ce que** l'on laisse agir une quantité efficace en tant qu'herbicide", d'au moins une 2-benzoyl-cyclohexane-1,3-diones de formule| ou d'un sel de | utilisable en agriculture, selon les revendications 1 à 5, sur des plantes, leur biotope et/ou leur semences.

12. Utilisation des 2-benzoyl-cyclohexane-1,3-diones de formule I et leurs sels utilisables en agriculture selon les revendications 1 à 5 en tant qu'herbicides.
